# EUROPEAN PATENT SPECIFICATION

(11) **EP 4 232 151 B1**
(45) Date of publication and mention of the grant of the patent: **22.10.2025**
(21) Application number: 21807468.0
(22) Date of filing: 25.10.2021
(51) Int. Cl.: A61K 39/395

(54) **USE OF ANTI-PRO/LATENT MYOSTATIN ANTIBODY FOR TREATING SPINAL MUSCULAR ATROPHY**
VERWENDUNG VON ANTI-PRO/LATENTEM MYOSTATIN-ANTIKÖRPER ZUR BEHANDLUNG VON SPINALER MUSKELATROPHIE
UTILISATION D'UN ANTICORPS ANTI-PRO-MYOSTATINE/MYOSTATINE LATENTE POUR LE TRAITEMENT DE L'AMYOTROPHIE SPINALE

(30) Priority: 26.10.2020 US 202063105850 P; 27.10.2020 US 202063106172 P; 05.04.2021 US 202163200955 P; 15.04.2021 US 202163201157 P; 06.06.2021 US 202163202317 P; 08.06.2021 US 202163202372 P; 29.06.2021 US 202163202900 P; 30.08.2021 US 202163260725 P; 20.09.2021 US 202163261398 P
(43) Date of publication of application: 30.08.2023
(73) Proprietor: Scholar Rock, Inc., Cambridge, MA 02142 (US)
(72) Inventor: NOMIKOS, George, Cambridge, Massachusetts 02142 (US); SONG, Guochen, Cambridge, Massachusetts 02142 (US); IARROBINO, Ryan, Cambridge, Massachusetts 02142 (US); CHYUNG, Yung, Cambridge, Massachusetts 02142 (US)
(74) Representative: Carpmaels & Ransford LLP
(86) International application number: PCT/US2021/056517
(87) International publication number: WO 2022/093724

(56) References cited:
- WO-A1-2017/218592
- ANONYMOUS: "History of Changes for Study: NCT03921528", 13 January 2020 (2020-01-13), XP055874560, Retrieved from the Internet <URL:https://clinicaltrials.gov/ct2/history/NCT03921528?V_13=View#StudyPageTop> [retrieved on 20211220]
- PLACE A ET AL: "SMA - THERAPY P.253 Clinical Development of SRK-015, a Fully Human Anti-proMyostatin Monoclonal Antibody, for the Treatment of Later-Onset Spinal Muscular Atrophy", NEUROMUSCULAR DISORDERS, ELSEVIER LTD, GB, vol. 30, 1 October 2020 (2020-10-01), XP086258190, ISSN: 0960-8966, DOI: 10.1016/J.NMD.2020.08.252
- PLACE AMY ET AL: "SRK-015: A fully human antibody that blocks cleavage of the Myostatin prodomain A Phase 2 Study to Evaluate the Efficacy and Safety of SRK-015 in Patients with Later-Onset Spinal Muscular Atrophy (TOPAZ): An Introduction Study Design (Continued) Demographics* Disease History* Functional Motor Skills", 26 May 2020 (2020-05-26), XP055874801, Retrieved from the Internet <URL:https://scholarrock.com/wp-content/uploads/2020/09/MDA-2020-TOPAZ-SMA-Introduction.pdf> [retrieved on 20211220]
- CAMBRIDGE MASS ET ET AL: "Scholar Rock Announces Positive Interim Results from Phase 1 Trial of SRK-015 in Healthy Volunteers and Updates on Future Development Plans", GLOBE NEWSWIRE) --SCHOLAR ROCK HOLDING CORPORATION, 19 February 2019 (2019-02-19), XP055874556, Retrieved from the Internet <URL:https://www.sma-europe.eu/wp-content/uploads/2019/02/Scholar-Rock-Announces-Positive-Interim-Results-from-Phase-1-Trial-of-SRK-015-in-Healthy-Volunteers-and-Updates-on-Future-Development-Plans.pdf> [retrieved on 20211220]
- DATABASE EMBASE [online] ELSEVIER SCIENCE PUBLISHERS, AMSTERDAM, NL; 2021, PLACE A ET AL: "TOPAZ: Phase 2 study evaluating efficacy and safety of apitegromab in later-onset spinal muscular atrophy", XP002805206, Database accession no. EMB-635747941
- PLACE A ET AL: "TOPAZ: Phase 2 study evaluating efficacy and safety of apitegromab in later-onset spinal muscular atrophy", JOURNAL OF NEUROMUSCULAR DISEASES 2021 IOS PRESS NLD, vol. 8, no. SUPPL 1, 2021, pages S9 CONF 20210528 to 20210529 Virtual - 16th Inte, ISSN: 2214-3602
- ANONYMOUS: "Scholar Rock Announces Positive Proof-of-Concept Data from TOPAZ Phase 2 Trial Interim Analysis of SRK-015 in Patients with Type 2 and Type 3 Spinal Muscular Atrophy | Business Wire", 27 October 2020 (2020-10-27), XP055874803, Retrieved from the Internet <URL:https://www.businesswire.com/news/home/20201027005388/en/Scholar-Rock-Announces-Positive-Proof-of-Concept-Data-from-TOPAZ-Phase-2-Trial-Interim-Analysis-of-SRK-015-in-Patients-with-Type-2-and-Type-3-Spinal-Muscular-Atrophy> [retrieved on 20211220]
- BARRETT DOREEN ET AL: "A Randomized Phase 1 Safety, Pharmacokinetic and Pharmacodynamic Study of the Novel Myostatin Inhibitor Apitegromab (SRK-015): A Potential Treatment for Spinal Muscular Atrophy", ADVANCES IN THERAPY, HEALTH COMMUNICATIONS, METUCHEN, NJ, US, vol. 38, no. 6, 8 May 2021 (2021-05-08), pages 3203 - 3222, XP037476583, ISSN: 0741-238X, [retrieved on 20210508], DOI: 10.1007/S12325-021-01757-Z
- CRAWFORD THOMAS ET AL: "Relationship of pharmacokinetics and pharmacodynamics to apitegromab efficacy in patients with later-onset spinal muscular atrophy (Types 2 and 3 SMA): Results from the TOPAZ study", JOURNAL OF NEUROLOGICAL SCIENCES, ELSEVIER SCIENTIFIC PUBLISHING CO, AMSTERDAM, NL, vol. 429, 1 October 2021 (2021-10-01), XP086816744, ISSN: 0022-510X, [retrieved on 20211008], DOI: 10.1016/J.JNS.2021.118388

## Description

### FIELD

The present disclosure relates to therapeutic methods, uses, and compositions comprising an anti-pro/latent myostatin antibody to treat spinal muscular atrophy (SMA) in human patients.

### BACKGROUND

Myostatin, also known as growth differentiation factor 8, abbreviated GDF-8 or GDF8, is a regulator of muscle homeostasis. Mutations that cause loss of myostatin, as well as pharmacological inhibition of myostatin activities, have shown to increase muscle growth in a number of species, including human. Over the past 15 years, at least 15 different myostatin inhibitor drug candidates, including small molecules and biologics, have been evaluated in human patients aimed to treat various muscle disorders but none to date have succeeded in the clinic (Hanna et al. (2019) Lancet Neurol. 18(9):834-844; Rooks et al. (2020) JAMA Netw Open. 3(10):e2020836). While many were able to show efficacy (e.g., increased muscle mass) in preclinical models, none to date have successfully translated this to achieve clinical benefit for treating muscle disorders in human patients. Furthermore, most of these inhibitors lacked selectivity and therefore antagonized other related growth factors such as Activin A, raising toxicity concerns. Most of these programs are now discontinued. Thus, in many cases, satisfactory preclinical results have not successfully translated into a safe and effective drug.

As the sole target for the treatment of muscle diseases, and for SMA in particular, myostatin inhibition has been met with a degree of skepticism. For example, it has been suggested that myostatin expression is reduced in SMA patients, which suggested that myostatin might not be an effective therapeutic target (Burch et al. (2017) J Neurol. 264(3):541-553; Mariot et al. (2017) Nat Commun. 8(1):1859. Latres et al. (April 2017) suggested that Activin A, rather than myostatin, was the more prominent regulator of muscle mass in primates, indicating that Activin A would be the better therapeutic target. In fact, the same group, despite the availability of a myostatin-selective inhibitor in possession, elected to enter the clinic with a combination of the myostatin-selective inhibitor *and* an Activin A inhibitor in IBM. The program was later discontinued.

SMA is a rare, autosomal recessive neuromuscular disease characterized by degeneration of the motor neurons in the anterior horn of the spinal cord, resulting in atrophy of the voluntary muscles of the limbs and trunk. SMA directly results from reduced levels of survival motor neuron (SMN) protein, caused by homozygous deletions and, infrequently, mutations within the survival motor neuron 1 (SMN1) gene on chromosome 5q13.2. The lack of SMN protein causes the motor neurons to become dysfunctional and, eventually, to die.

In some countries, SPINRAZA^{®} (nusinersen) is approved in the treatment of pediatric and adult SMA patients and ZOLGENSMA^{®} (onasemnogene abeparvovec-xioi) is approved for the treatment of pediatric patients less than 2 years of age with SMA with bi-allelic mutations in the SMN1 gene. EVRYSDI^{™} (risdiplam), a small molecule SMN therapy, is also approved in the United States and in Europe. Nusinersen is an SMN2-directed antisense oligonucleotide (ASO) designed to treat SMA caused by mutations that lead to SMN protein deficiency. Risdiplam works in a similar way and is a pyridazine derivative that modifies the splicing of SMN2 messenger RNA. Onasemnogene abeparvovec-xioi is a recombinant adeno-associated virus vector 9-based gene therapy designed to deliver a copy of the gene encoding the human SMN protein.

SMN therapies (or SMN-directed therapies), such as nusinersen, risdiplam, onasemnogene abeparvovec-xioi, and other products in development act primarily upon motor neurons to prevent further loss. Consistent with this notion, clinical data reported from an extended SMN-directed therapy indicate that after improvement in the first 15 months of treatment with nusinersen, as measured by mean change in HFMSE scores from baseline, the effects appear to level off to a near steady state. A limited further enhancement is observed in motor function over the next three or more years (Darras et al. (2019) Neurology 92(21):e2492-e2506). Similarly, long term evaluation of risdiplam showed only stabilization or minor/variable improvement after 12 months in the primary and secondary endpoints (Oskoui et al. "SUNFISH Part 2: 24-month efficacy and safety of risdiplam in patients with type 2 or non-ambulant type 3 spinal muscular atrophy (SMA)." Presented at MDA Clinical and Scientific Conference 2021; March 15-18. Poster 80). SMN-directed therapies may therefore help maintain motor function over time but may provide limited long-term enhancement in motor function. Thus, while approved treatments have shown improvement in motor function in younger patients with SMA, particularly during the early phase of treatment, they do not act directly on the muscle to primarily address already-existing atrophy and motor functional impairment in patients with symptomatic SMA.

Applicant of the present disclosure previously identified at least three criteria in selecting indications in which myostatin inhibition is likely to be particularly advantageous: i) the target muscle is anabolically active (e.g., still in the growth phase); ii) the motor unit (motor neuron and innervating target muscle) is at least partially functionally intact; and, iii) the target muscle preferentially relies on fast-twitch (type II) fibers (see: WO 2017/218592). Based on this recognition, Applicant demonstrated therapeutic effects of the selective inhibition of myostatin in preclinical models of SMA, in which the motor unit function is enhanced by concurrent SMN-enhancing therapy (e.g., SMN upregulator/corrector therapy) which addresses criterion (ii) above, supporting the notion that a muscle-enhancing agent, such as myostatin inhibitors, can be used to complement the effects of a motor neuron-enhancing agent, such as SMN upregulators/correctors. The Applicant also initiated trials to test the safety and efficacy of a selective inhibitor of myostatin, SRK-015/apitegromab, in human subjects. For instance, NCT03921528 describes a Phase 2 active treatment study to evaluate the efficacy and safety of SRK-015 in patients with later-onset SMA (TOPAZ). Place et al. (October 2020), describe the clinical development of SRK-015 for the treatment of later onset SMA (Place et al., Neuromuscular Disorders, Volume 30, Supplement 1, S119-S120, October 2020). Place et al. (March 2020) provide an introduction to the TOPAZ study (Poster titled "A Phase 2 Study to Evaluate the Efficacy and Safety of SRK-015 in Patients with Later-Onset Spinal Muscular Atrophy (TOPAZ)", March 2020, MDA Clinical and Scientific Conference).

There are currently no approved muscle-directed therapies (e.g., muscle-enhancing therapies) for the treatment of SMA. Consequently, there remains an unmet need for an effective muscle-directed therapy that can address muscle atrophy and motor functional impairment in patients with SMA.

### SUMMARY

The invention is defined in the claims.

The invention provides a composition comprising apitegromab for use in the treatment of spinal muscular atrophy (SMA) in a human subject, wherein the treatment comprises intravenous administration of apitegromab in an amount of 10 mg/kg at an interval of once every four weeks or once a month for at least 24 weeks or six months, wherein the treatment increases motor function as measured by a Hammersmith Functional Motor Scale Expanded score (HFMSE) at 6 months after the start of treatment relative to HFMSE at baseline.

Exemplary embodiments of apitegromab for use in the treatment of SMA are described in the dependent claims.

Herein, the terminologies "embodiment" and "embodiments" are to be construed as embodiment(s) of the invention only in as far as they fall within the scope of the appended claims. The references to methods of treatment of the human or animal body by surgery or therapy herein are to be interpreted as references to a composition comprising apitegromab for use in those methods.

The present disclosure includes, inter alia, therapeutic methods, uses, and compositions for treating SMA patients using a muscle-enhancing agent, such as apitegromab, also known as SRK-015. In various embodiments, apitegromab or a composition comprising apitegromab is used in the treatment of later-onset SMA in a human subject, either as monotherapy or as an adjunct to a motor neuron-directed therapy, such as an SMN upregulator/corrector therapy (i.e., SMN therapy). The data provided herein represent the first clinical demonstration of motor function improvement in human patients by selectively inhibiting myostatin. Thus, the present disclosure encompasses identification and selection of a SMA patient or patient population, likely to respond to or otherwise benefit from apitegromab therapy as described herein. Data from patients with type 2 and type 3 SMA who received the apitegromab therapy for 12 months point to a general trend of more robust response in younger patient populations, irrespective of the duration (or dose numbers received) of background therapies, e.g., SMN upregulator/corrector therapies. This is consistent with our earlier hypothesis that the anabolic capacity of the target muscle may be an important factor that determines responsiveness to myostatin inhibition (US 10,946,036).

Apitegromab therapy for treating subjects with SMA is provided herein. According to the present disclosure, a composition comprising apitegromab is used to treat later-onset SMA in human patients. Apitegromab therapy comprises intravenous administration of the composition to the patients at a therapeutic dose. Therapeutic dose refers to an amount of apitegromab that produces clinical benefit in patients. The clinical benefit (e.g., efficacy) may be measured or assessed by suitable clinical endpoints such as those described herein. According to the data presented herein, the therapeutic dose is greater than 2 mg/kg and up to 20 mg/kg of apitegromab, when dosed every 4 weeks (i.e., Q4W) or monthly. Therapeutic doses of less than 20 mg/kg may be considered, such as 5 mg/kg, 7.5 mg/kg, 10 mg/kg, 12 mg/kg and 15 mg/kg. According to the invention, the therapeutic dose is 10 mg/kg. According to the invention, the therapeutic dose is 10 mg/kg administered once every 4 weeks (i.e., Q4W) or once monthly. In various embodiments, apitegromab therapy may improve motor function in patients having later-onset SMA.

Pharmacokinetics (PK) analyses provide the relationship between dosage (e.g., therapeutic dose) and bioavailability (serum exposure) of therapeutics. In some embodiments, the therapeutic dose is a dose that achieves or produces between about 25 and 250 µg/mL of serum exposure to apitegromab, when the serum concentration of apitegromab is measured at trough (C_{trough}) at steady state. According to the invention, the dose achieving this result is administered intravenously.

In some embodiments, the therapeutic dose is a dose that achieves or produces up to about 1100 µg/mL of serum exposure, for example, between about 25 and 1100 µg/mL, when the serum concentration of apitegromab is measured at peak (Cₘₐₓ) within about two hours of administration (dosing). According to the invention, the dose achieving this result is administered intravenously.

Pharmacodynamics (PD) analyses allow the assessment of target engagement, as measured by serum concentrations of latent myostatin (LM). In some embodiments, the therapeutic dose is a dose that achieves or produces at least about 100 or preferably at least about 250 ng/mL of serum concentrations of latent myostatin, preferably measured at steady state, e.g., 14 days after administration of apitegromab or later. According to the invention, the dose achieving this result is administered intravenously. For example, the serum concentration of latent myostatin may be about 250 ng/mL or above, 400 ng/mL or above 550 ng/ml or above 700 ng/ml or above 950 ng/ml or above 1100 ng/ml, etc.

Apitegromab or another selective myostatin inhibitor may be used to treat SMA either alone (e.g., monotherapy) or in conjunction with another therapy, such as an SMN-directed therapy (e.g., add-on/adjunct therapy or combination therapy). In some embodiments, the subject is treated with an SMN upregulator therapy. In some embodiments, the SMN upregulator therapy is nusinersen (SPINRAZA^{®}), risdiplam (Evrysdi^{®}), and/or onasemnogene abeparvovec-xioi (ZOLGENSMA^{®}). In some embodiments, the SMN regulator therapy is nusinersen. In some embodiments, the SMN regulator therapy is risdiplam. In some embodiments, the SMN regulator therapy is onasemnogene abeparvovec-xioi. In some embodiments, the subject initiated the SMN upregulator therapy at or after the age of 5.

In some embodiments, an SMN-directed therapy and apitegromab therapy (e.g., muscle-directed therapy) are used as a combination therapy. Thus, an SMN corrector and apitegromab may be used in the treatment of later-onset SMA in a patient, wherein the treatment comprises administration of the SMN corrector and the apitegromab in amounts sufficient to treat SMA, wherein the apitegromab therapy is intravenously administered to the patient at a dose of 10 mg/kg every 4 weeks or monthly. In some embodiments, the SMN corrector is an SMN1-directed gene therapy, wherein optionally the SMN1-directed therapy is a gene therapy. In some embodiments, the SMN corrector is an SMN2-directed therapy, wherein optionally the SMN2-directed therapy is a splice modifier. In some embodiments, an SMN corrector may be administered orally, intrathecally or intravenously. In some embodiments, the patient has later-onset SMA. In some embodiments, the patient has type 2 SMA. In some embodiments, the patient has non-ambulatory type 3 SMA. In some embodiments, the patient has ambulatory type 3 SMA. In some embodiments, the patient has 2 copies of the SMN2 gene. In some embodiments, the patient has 3 copies of the SMN2 gene. In some embodiments, the patient has 4 copies of the SMN2 gene. In some embodiments, the patient has 5 copies of the SMN2 gene. In some embodiments, the patient has 6 copies of the SMN2 gene. In some embodiments, the patient commences the combination therapy before the age of 5. In some embodiments, the patient commences the combination therapy before the age of 2. In some embodiments, the patient commences the combination therapy before the age of six weeks. In some embodiments, the patient commences the combination therapy at age 5 or older. In some embodiments, the patient is diagnosed with SMA (e.g., identified as a carrier of an SMN1 mutation) by genetic screening, wherein optionally the genetic screening is a newborn screening, in utero screening, e.g., for SMN1 mutation(s). In some embodiments, the patient is presymptomatic.

In some embodiments, apitegromab therapy is used as an add-on or adjunct therapy to treat SMA. Thus, a composition comprising apitegromab may be used in the treatment of later-onset SMA in a patient, wherein the treatment comprises intravenous administration of the composition comprising a therapeutic dose of apitegromab, wherein the therapeutic dose is 10 mg/kg every 4 weeks or monthly, and wherein the patient is treated with an SMN corrector therapy. In some embodiments, the SMN corrector is an SMN1-directed therapy, wherein optionally the SMN1-directed therapy is a gene therapy. In some embodiments, the SMN corrector is an SMN2-directed therapy, wherein optionally the SMN2-directed therapy is a splice modifier. In some embodiments, any of the SMN corrector may be administered orally, intrathecally or intravenously. In some embodiments, the patient has type 2 SMA. In some embodiments, the patient has non-ambulatory type 3 SMA. In some embodiments, the patient has ambulatory type 3 SMA. In some embodiments, the patient has 2 copies of the SMN2 gene. In some embodiments, the patient has 3 copies of the SMN2 gene. In some embodiments, the patient has 4 copies of the SMN2 gene. In some embodiments, the patient has 5 copies of the SMN2 gene. In some embodiments, the patient has 6 copies of the SMN2 gene. In some embodiments, the patient commences the SMN corrector therapy before the age of 5. In some embodiments, the patient commences the SMN corrector therapy at age 5 or older. In some embodiments, the patient is diagnosed with SMA (e.g., identified as a carrier of an SMN1 mutation) by genetic screening, wherein optionally the genetic screening is a newborn screening. In some embodiments, the patient is presymptomatic. In some embodiments, the patient is treated with the SMN corrector prior to apitegromab therapy. In some embodiments, the patient is treated with apitegromab prior to receiving the SMN corrector therapy.

SMA patients who may benefit from an apitegromab therapy include those who meet one or more of the following criteria: has a documented diagnosis of 5q SMA and later-onset (and/or type 2 or 3) SMA prior to receiving a therapy for SMA; non-ambulatory subjects who are able to sit independently per WHO motor milestones definition; ambulatory subjects who are able to independently ambulate without aids over 10 meters in 30 seconds or less; subjects having a Revised Hammersmith Scale score of less than or equal to 63 and/or a Hammersmith Functional Motor Scale Expanded score of 10 or greater; subjects who do not use tracheostomy with positive pressure or chronic daytime non-invasive ventilatory support for greater than 16 hours daily within two weeks prior to treatment; subjects who do not have any acute or comorbid condition interfering with the well-being of the subject within two weeks prior to treatment; subjects who do not have severe scoliosis or contractures; and/or subjects who do not use systemic corticosteroids, valproic acid, or therapies with potential muscular or neuromuscular effects within 60 days except approved SMN-directed therapy, e.g., SMN upregulator (also known as SMN corrector) therapy. Therapies with potential muscular or neuromuscular effects include androgens, insulin-like growth factor, growth hormone, systemic beta-agonist, botulinum toxin, muscle relaxants, muscle enhancing supplements or acetylcholinesterase inhibitors. In some embodiments, the patient has a documented diagnosis of 5q SMA and later-onset (and/or type 2 or 3) SMA prior to receiving a therapy for SMA and meets one or more of the additional criteria listed above. In various embodiments, the methods disclosed herein include the selection of such a patient or patient population(s) for treatment with apitegromab, e.g., according to a dosage or regimen disclosed herein.

SMA patients who may benefit from an apitegromab therapy include those who meet one or more of the following criteria: has 1, 2, 3 or 4 copies of the smn2 genes and achieves one or more of the gross motor milestones according to the World Health Organization (WHO) motor development scale: 1) sitting up without support (e.g., head erect for at least 10 seconds; no use of arms or hands to balance); 2) crawling on hands and knees (e.g., at least 3 movements in a row and stomach does not touch supporting surface); 3) standing with assistance (e.g., upright on both feet for at least 10 seconds without leaning on any object); 4) walking with assistance (e.g., takes at least 5 steps holding a stable object); 5) standing without support (e.g., at least 10 seconds with no contact with person or object); and, 6) walking without support (e.g., takes at least 5 steps independently).

SMA patients who may benefit from an apitegromab therapy include those who meet one or more of the following criteria: has 1, 2, 3 or 4 copies of the smn2 genes and achieves one or more of the gross motor milestones according Hammersmith Functional Motor Scale Expanded (HFMSE): 1) neck holding (e.g., while laying on their stomach able to raise or hold head); 2) rolls over; 3) sits in tripod position (e.g., uses hands to support self while sitting); 4) sits without support; 5) stands with support; 6) creeps/crawls; 7) pulls to standing position (e.g., pulls to stand and cruises along furniture); 8) stands without support; 9) takes several independent steps but falls; 10) walks alone (e.g., walks independently, walks without support); 11) squats to pick up an object (e.g., a toy); 12) walks/creeps up and down the stairs; 13) jumps; 14) alternates feet going upstairs; 15) hops on one foot; 16) alternates feet going downstairs.

In some embodiments, a patient treated with apitegromab as disclosed herein has received or is treated with an SMN therapy (SMN-directed therapy), e.g., an SMN upregulator (corrector) therapy, such as nusinersen. In some embodiments, a patient initiates the SMN upregulator (corrector) therapy before the age of 5. In some embodiments, the patient initiates the SMN upregulator (corrector) therapy at or after the age of 5. In some embodiments, an SMN corrector therapy is an SMN2 upregulator therapy. In some embodiments, an SMN corrector therapy is an SMN1 gene therapy.

In some instances, a patient receives apitegromab therapy for at least 6 months (e.g., 6 months, 12 months, or longer) at a therapeutic dose that is sufficient to achieve a clinical benefit characterized by motor function improvement, disease stabilization, or delay in disease progression.

According to the invention, a patient receives apitegromab therapy and attains improvement in motor function, corresponding to an increase in an HFMSE score. For example, the patient may achieve an increase of at least 1 point, 2 points, 3 points, 4 points, 5 points or more in the HFMSE score over a baseline after 6 months or 12 months of treatment with apitegromab (i.e., apitegromab therapy). In some embodiments, 12 months of apitegromab therapy may produce 3 points or greater increase (e.g., at least 3 points, at least 5 points, at least 10 points, up to about 20 points) in the HFMSE score over baseline in patients who had initiated a background SMN therapy at an early age. In some embodiments, improvements in the HFMSE score are additive and may be synergistic with background therapy, e.g., background SMN upregulator/corrector therapy.

A patient who receives apitegromab therapy may have disease stabilization. Disease stabilization may correspond to a net zero (e.g., at least no change or an increase) or near-zero change in an HFMSE score or RHS score over a baseline. This is a clinically meaningful outcome over an untreated patient population (e.g., natural history), or one treated with prior art methods (e.g., background therapy), in which a gradual decline in motor function is expected.

A patient who receives apitegromab therapy may have a delay in disease progression. Delay in disease progression may include, for example, a slower rate of decrease in an HFMSE score over time, as compared to a suitable control (e.g., an untreated patient exhibiting the natural history of the particular patient population). Delay may include a later transition from ambulatory to non-ambulatory SMA.

In some embodiments, apitegromab is able to increase a treatment response rate in a patient population, relative to control that does not receive apitegromab.

In any of the embodiments, a therapeutically effective amount of apitegromab does not cause serious adverse events in patients following twelve months of treatment.

The present disclosure is based, at least in part, on the finding that an anti-pro/latent myostatin antibody capable of selectively inhibiting the activation of latent myostatin can improve muscle function in human patients with SMA, including SMA patients who may or may not be on a background SMN upregulator therapy (e.g., nusinersen), and do so in some cases to a surprising degree that is typically not expected or observed in the particular patient population, while avoiding adverse events (compare, for example, to Mercuri et al. (2018) New England Journal of Medicine. 378(7):625-635). Accordingly, the present disclosure provides various embodiments of methods, uses, and compositions comprising an anti-pro/latent myostatin antibody for treating SMA in a human subject. Moreover, based on the data presented herein demonstrating clinical benefit of selective myostatin inhibition for treating a neuromuscular disease, the present disclosure also encompasses the notion that other selective myostatin inhibitors may also be used in a similar fashion. These may include, for example, neutralizing antibodies capable of selectively inhibiting myostatin, but that spare other related growth factors such as Activin A, and ligand traps engineered to preferentially bind myostatin.

A therapeutically effective amount of the apitegromab of greater than 2 and up to 20 mg/kg may achieve one or more of the following in the subject: retaining motor function as compared to a decline in a control, delaying disease progression, delaying or preventing an ambulatory type 3 SMA patient from becoming non-ambulatory, delaying or preventing need for respiratory aid or intervention, reducing the rate of a decline in one or more motor function scores as compared to a control, and/or maintaining at least net zero change in one or more motor function scores as compared to a baseline. The amount may be a dose of greater than 2 and up to 20 mg/kg apitegromab intravenously administered every 4 weeks or monthly. A mean decline from baseline may be observed in this patient population but the majority of patients show disease stabilization (no change or increase in RHS). In some embodiments, a subset (e.g., 10% or greater, e.g., 15% or more, 20% or more) of patients in the patient population achieve a 3 point or more increase in RHS following 12 months of treatment with apitegromab as monotherapy.

In some embodiments, the present disclosure provides a composition comprising apitegromab for use in treating SMA in a human subject receiving an SMN upregulator, wherein the subject is administered the apitegromab by intravenous infusion every 4 weeks or monthly for at least 6 months in an amount of 10 mg/kg sufficient to produce an at least 1 point mean increase in a HFMSE score relative to a baseline score prior to treatment, e.g., in a cohort of at least 14 subjects.

In some embodiments, the SMA is later-onset SMA. In some embodiments, the SMA is type 2 SMA. In some embodiments, the SMA is non-ambulatory type 3 SMA. In some embodiments, the subject is 5 to 21 years old. In some embodiments, the SMN upregulator therapy is nusinersen, risdiplam, and/or onasemnogene abeparvovec. In some embodiments, the SMN upregulator therapy is nusinersen. In some embodiments, the therapeutic dose of apitegromab is sufficient to increase 1 or more points in HFMSE in a patient, e.g., in a majority of a patient population (e.g., over 50%, such as over 60%), and/or, increase 3 or more points in HFMSE in a patient, e.g., in at least 20% of a patient population (e.g., at least 25%).

In some embodiments, the SMA is later-onset SMA, wherein the later-onset SMA is type 2 SMA, in which the patient initiated background SMN corrector therapy at an early age (before the age of 5). Apitegromab therapy may achieve significant motor function improvements (e.g., a 5 point or greater increase in HFMSE scores) in this patient population. In some embodiments, the patient attains a 3+ point increase, 5+ point increase, or 10+ point increase after 12 months of apitegromab therapy in HFMSE over baseline, wherein the baseline is measured prior to or at the time of the first administration of apitegromab.

In some embodiments, the subject is a patient who has not gained the ability to sit without help at age 4-9 months, based on the WHO Motor Developmental Milestones classification. In some embodiments, the subject is a patient who has the ability to sit without help at age 4-9 months. In some embodiments, the subject is a patient who has not gained the ability to stand with help at age 5-11 months. In some embodiments, the subject is a patient who has the ability to stand with help at age 5-11 months. In some embodiments, the subject is a patient who has not gained the ability to crawl on hands and knees at age 5-13 months. In some embodiments, the subject is a patient who has the ability to crawl on hands and knees at age 5-13 months. In some embodiments, the subject is a patient who has not gained the ability to walk with help at age 6-14 months. In some embodiments, the subject is a patient who has the ability to walk with help at age 6-14 months. In some embodiments, the subject is a patient who has not gained the ability to stand alone at age 7-14 months. In some embodiments, the subject is a patient who has the ability to stand alone at age 7-14 months. In some embodiments, the subject is a patient who has not gained the ability to walk alone at age 8-18 months. In some embodiments, any of the above mentioned patients who are unable to walk can also be characterized as having type 2 or type 3 SMA. In some embodiments, any of the above mentioned patients who are able to walk (with or without assistance) can also be characterized as having type 3 SMA. In some embodiments, the subject initiated SMN upregulator/corrector therapy before the age of 5 years old. In some embodiments, the subject initiated SMN upregulator/corrector therapy after the age of 5 years old. In some embodiments, any of the above mentioned patients may, after 6-12 months treatment with a myostatin inhibitor (e.g., apitegromab), gain one or more new milestone(s) according to the WHO Motor Developmental Milestones. In some embodiments, any of the above mentioned patients may, after 6-12 months treatment with a myostatin inhibitor (e.g., apitegromab), gain one, two, or three new milestones according to the WHO Motor Developmental Milestones, e.g., one or more of ability to walk independently, ability to stand independently, standing with assistance, hands and knees crawling, and/or walking with assistance.

In some embodiments, a target patient population to be treated with apitegromab includes those who are 12 years old or younger at the time of starting a myostatin inhibitor (e.g., apitegromab) therapy. In some embodiments, a target patient population includes those who are 2 years or older. In some embodiments, a target patient population includes those who are between 2-12 years of age. In some embodiments, a target patient population includes those who are between 2-5 years of age.

In some embodiments, apitegromab is administered to a SMA patient as monotherapy, who cannot receive intrathecal injection (for example due to spinal fusion) required for an SMN upregulator therapy or who has opted out of the treatment.

The present disclosure provides use of apitegromab in the manufacture of a pharmaceutical composition (e.g., medicament) for the treatment of later-onset SMA in a human subject. The medicament is intended for administration to the subject at a dose of more than 2 mg/kg and up to 20 mg/kg of apitegromab every 4 weeks or monthly, wherein optionally the subject initiated a motor neuron-directed therapy for SMA before the age of 5, and wherein the motor neuron-directed therapy increases SMN1 or SMN2 expression. The manufacture process may include providing a cell line comprising a vector or vectors with nucleic acid sequences of the heavy chain and the light chain of apitegromab immunoglobulin polypeptides, capable of producing a recombinant antibody corresponding to apitegromab, or antigen-binding fragment thereof. Such cell line can be used to produce apitegromab in a cell culture, such as mammalian cell culture, e.g., CHO cells. In some embodiments, a large-scale (e.g., 250L, 1000L, 2000L 3000, 4000L, etc.) bioreactor may be employed to produce apitegromab. The recombinant antibody molecules may then be purified from the cell culture, and the purified antibody formulated into a pharmaceutical composition comprising apitegromab and one or more excipients. The process typically includes a sterile filtration step. In some embodiments, the pharmaceutical composition is a liquid formulation containing about 50 mg/mL apitegromab, suitable for intravenous administration.

In some embodiments, apitegromab is contained in a multidose vial, such as a glass vial. In some embodiments, a container containing apitegromab therein (such as glass vial) is part of a kit.

### BRIEF DESCRIPTION OF THE FIGURES

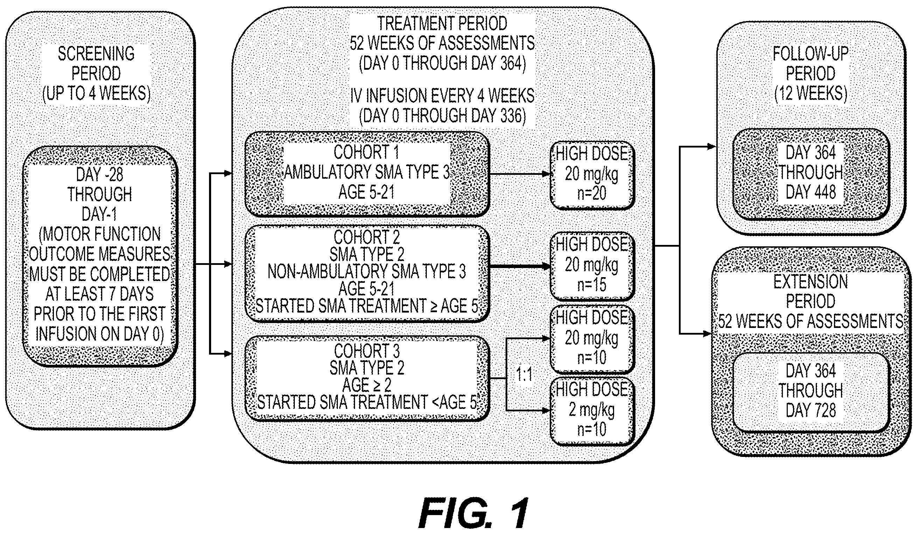

**FIG. 1** shows a schematic design for the treatment period of an active treatment study to evaluate the efficacy and safety of apitegromab in patients with later-onset SMA. During the 52-week treatment period, patients enrolled in Cohorts 1 and 2, as set forth in Example 1, Section 1.1, receive high dose (20 mg/kg) apitegromab and patients enrolled in Cohort 3 are randomized (1:1) in a blinded manner to receive either low dose (2 mg/kg) or high dose (20 mg/kg) apitegromab.

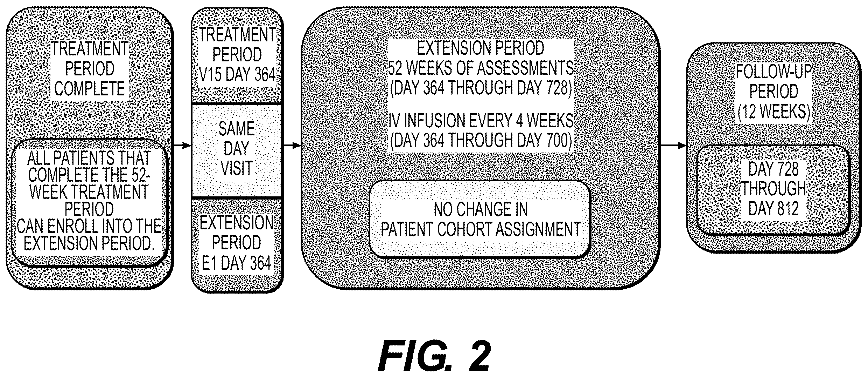

**FIG. 2** shows a schematic design for the extension period of an active treatment study to evaluate the efficacy and safety of apitegromab in patients with later-onset SMA, as set forth in Example 1. Patients who complete the 52-week treatment period **(****FIG. 1****)** have the option to enroll into the extension period for an additional 52 weeks.

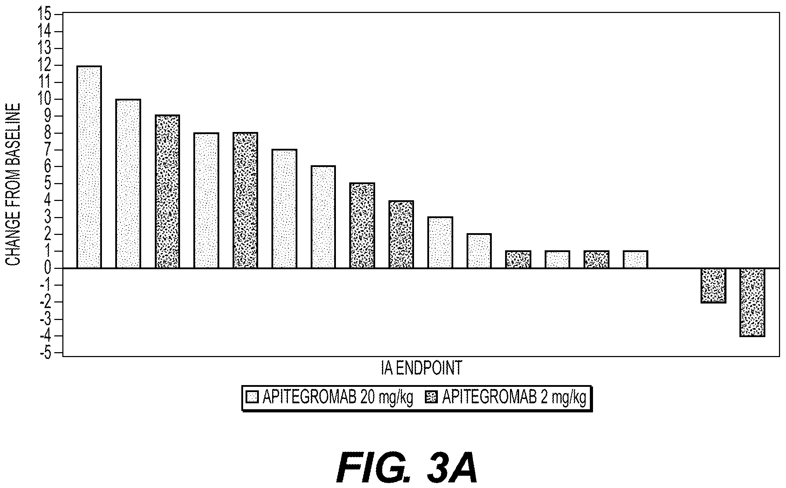

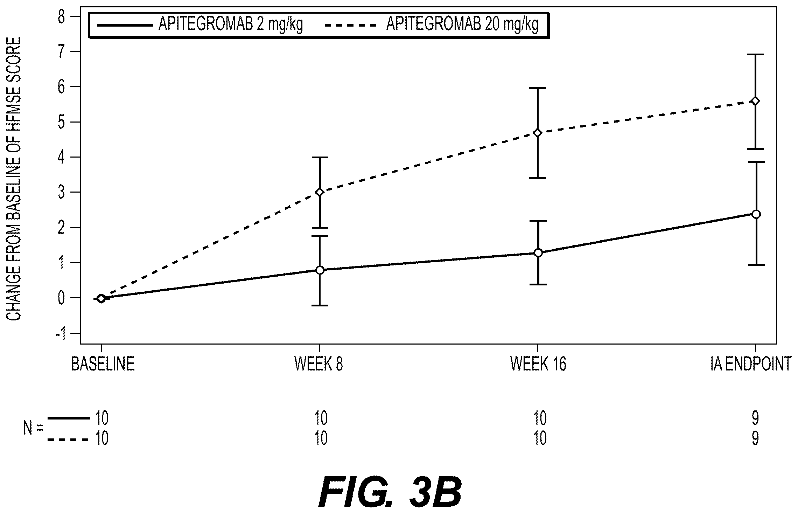

**FIG. 3A-B**show six-month interim results from Cohort 3, as set forth in Example 1, Section 1.1, a randomized, double-blind, portion of the active treatment study of apitegromab, which enrolled patients with type 2 SMA who had initiated treatment with an approved SMN upregulator (nusinersen) before 5 years of age. Patients were randomized in a 1:1 ratio to receive the low dose (2 mg /kg apitegromab once every 4 weeks or monthly (Q4W)) or high dose (20 mg/kg apitegromab Q4W); both treatment arms are in conjunction with an approved SMN upregulator therapy (nusinersen). **FIG. 3A** shows individual HFMSE responses. **FIG. 3B** shows mean (±SEM) change from baseline in HFMSE scores.

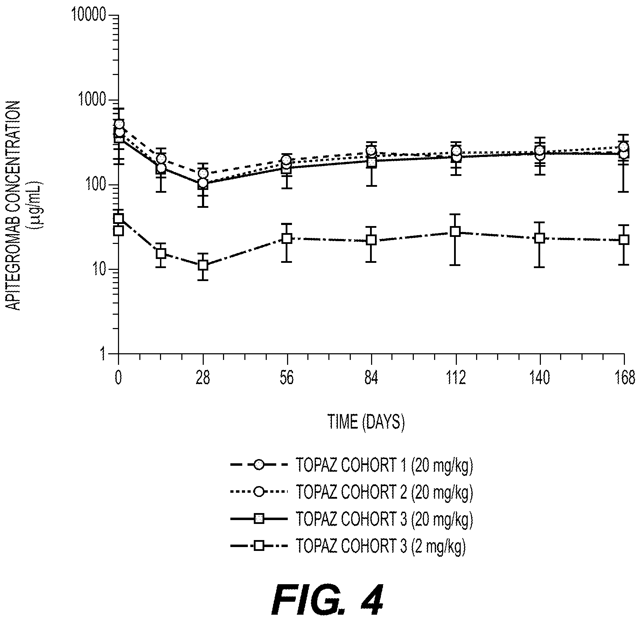

**FIG. 4** shows a pharmacokinetics (PK) analysis of six-month interim results from Cohorts 1, 2, and 3, as set forth in Example 1, Section 1.1. Starting at day 28, measures are pre-dose trough levels.

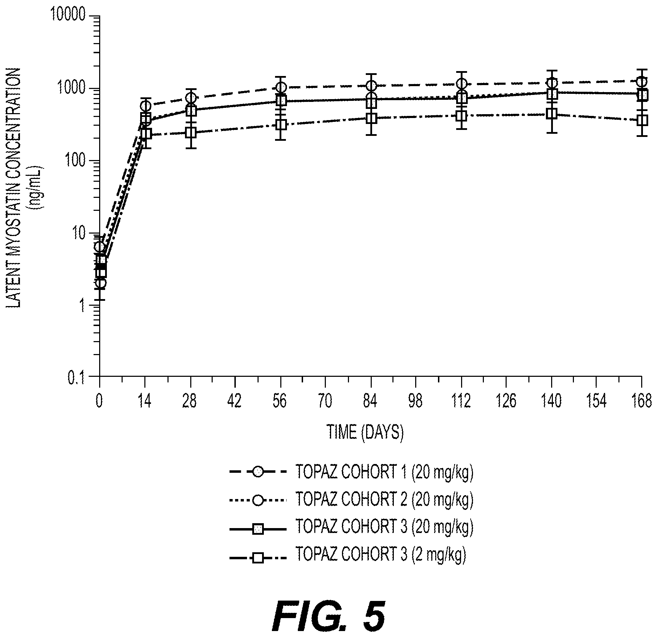

**FIG. 5** shows a pharmacodynamics (PD) analysis of six-month interim results from Cohorts 1, 2, and 3, as set forth in Example 1, Section 1.1.

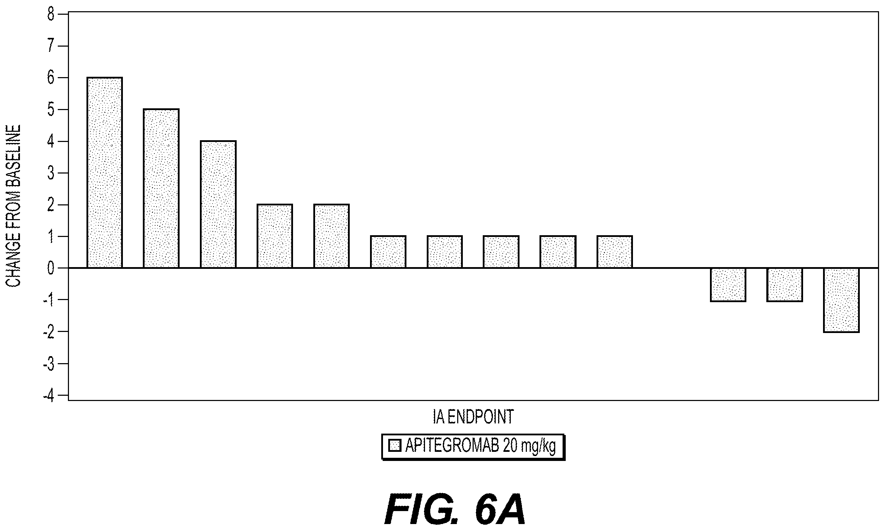

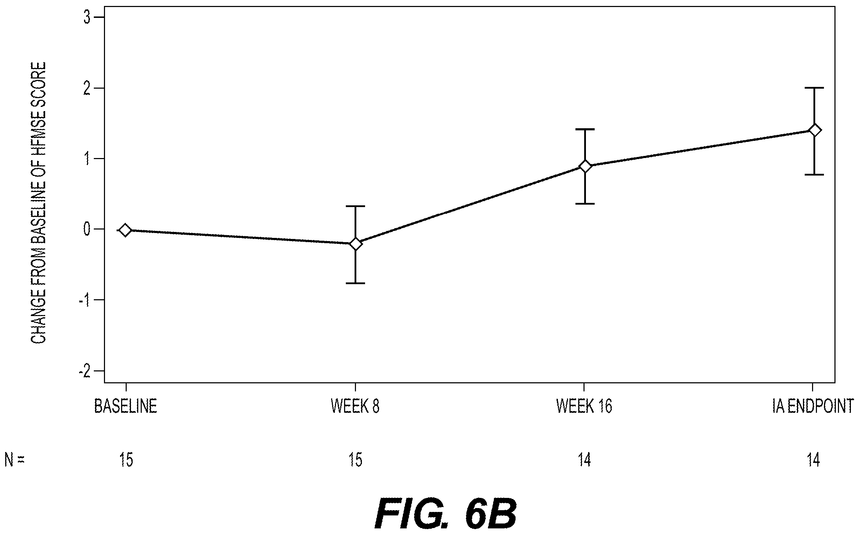

**FIG. 6A-B** show six-month interim results from Cohort 2, as set forth in Example 1, Section 1.1, an open-label, single-arm cohort which enrolled 15 patients with a mean age of 11.7 years old (range 8-19 years) with type 2 or non-ambulatory type 3 SMA and who are already receiving treatment with an approved SMN upregulator. Patients are treated with 20 mg/kg of apitegromab Q4W in conjunction with an approved SMN upregulator therapy (nusinersen). **FIG. 6A** shows individual HFMSE responses. **FIG. 6B** shows mean (±SEM) change from baseline in HFMSE scores.

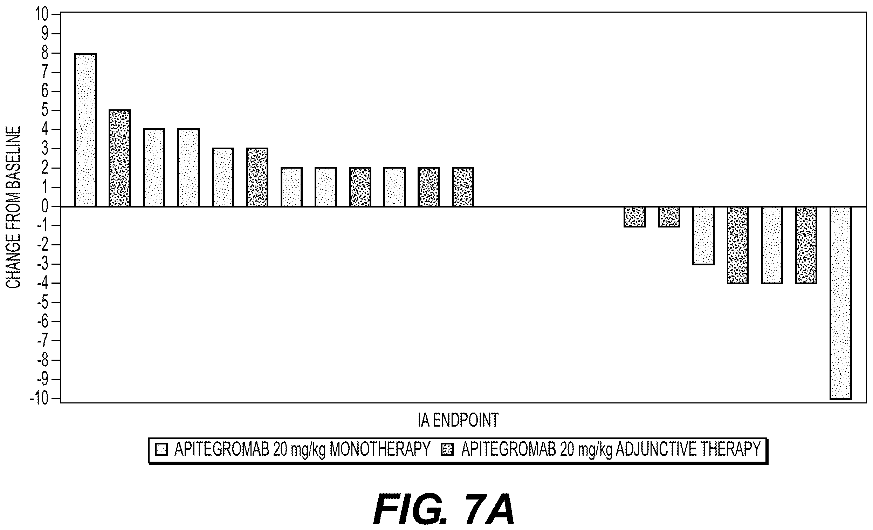

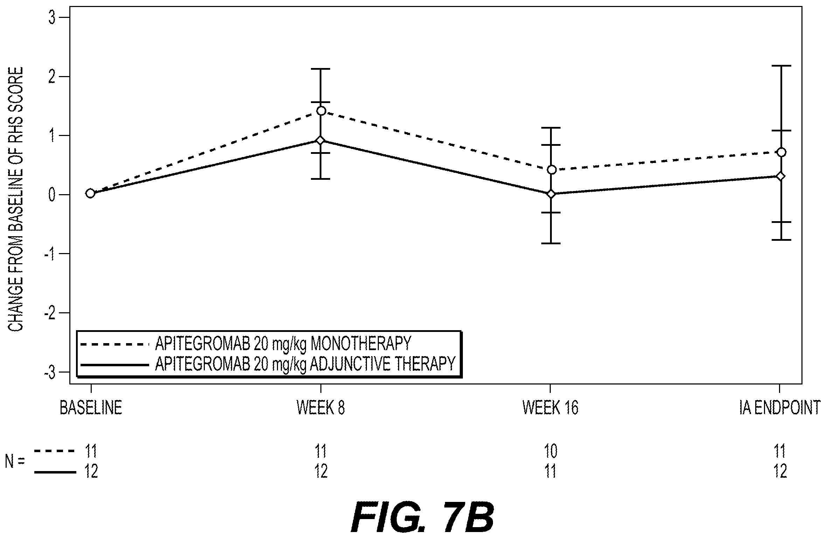

**FIG. 7A-B**show six-month interim results from Cohort 1, as set forth in Example 1, Section 1.1, an open-label, single-arm cohort which enrolled 23 patients with ambulatory type 3 SMA. Patients are treated with 20 mg/kg of apitegromab Q4W as monotherapy or in conjunction with an approved SMN upregulator therapy (nusinersen). **FIG. 7A** shows individual RHS responses. **FIG. 7B** shows mean (±SEM) change from baseline in RHS scores.

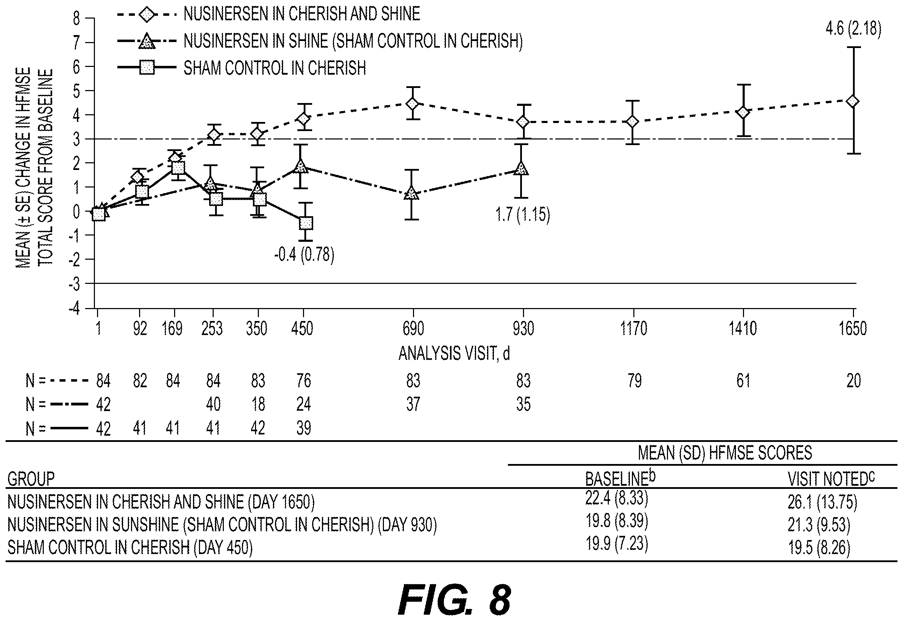

**FIG. 8** shows results from the nusinersen SHINE trial in later-onset SMA and is reproduced from Williams et al. Minimal clinically important differences of the Expanded Hammersmith Functional Motor Scale in later-onset spinal muscular atrophy: results from the phase 3 CHERISH trial. Presented at: 31st Annual Meeting of the American Academy of Managed Care Pharmacy; 25-28 March 2019; San Diego, CA, USA.

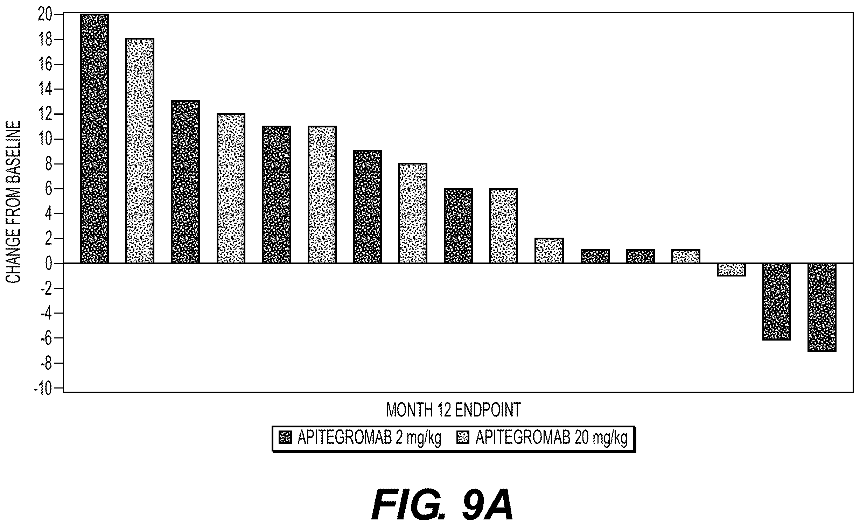

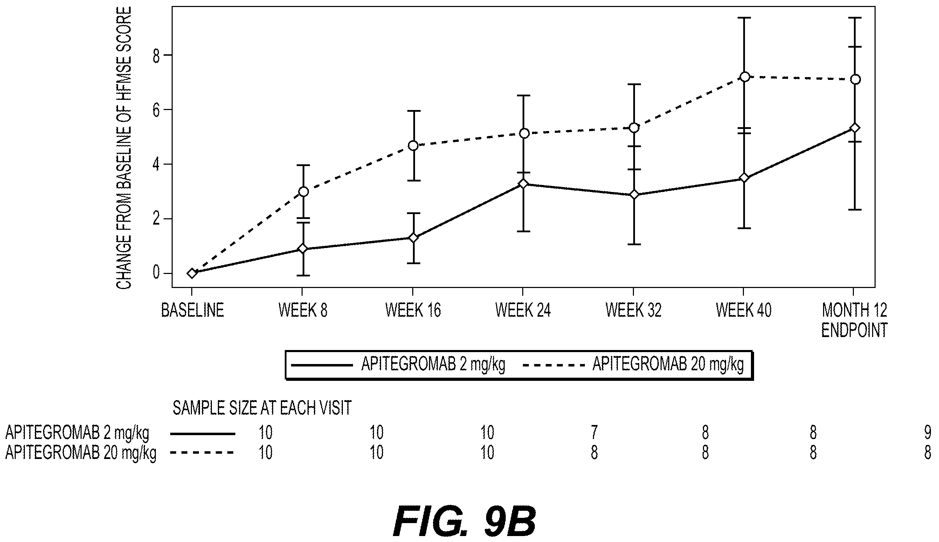

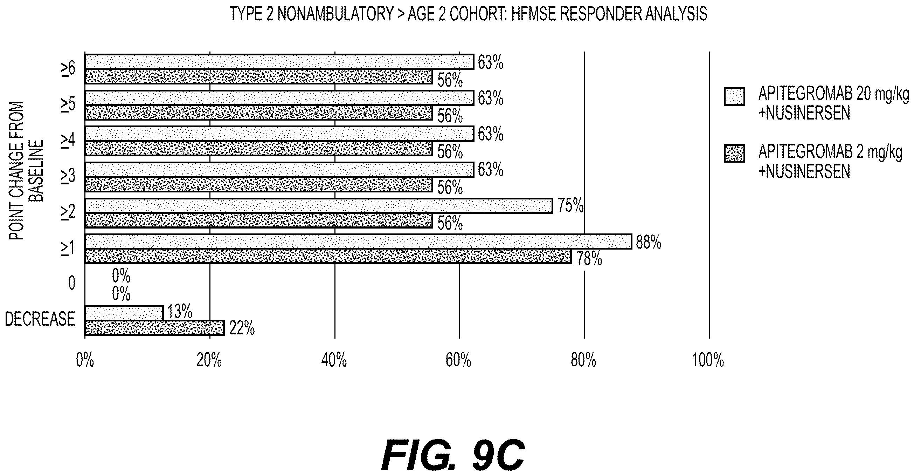

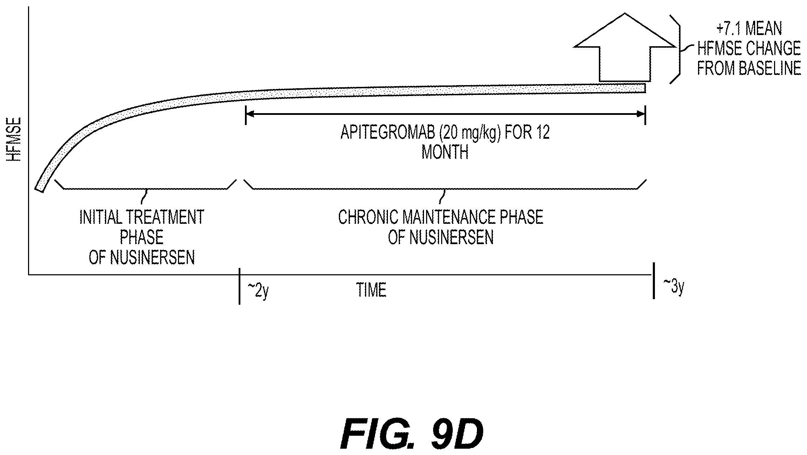

**FIG. 9A-D**show 12-month top-line results from Cohort 3, as set forth in Example 1, Section 1.1. **FIG. 9A** shows individual HFMSE responses. **FIG. 9B** shows mean (±SEM) change from baseline in HFMSE scores. **FIG. 9C** shows HFMSE responder analysis in patients from the Type 2 NonAmbulatory > age 2 cohort. **FIG. 9D** shows a schematic of overall treatment timeline and change in HFMSE scores as compared to baseline in patients from the 20 mg/kg arm of the Type 2 NonAmbulatory > age 2 cohort.

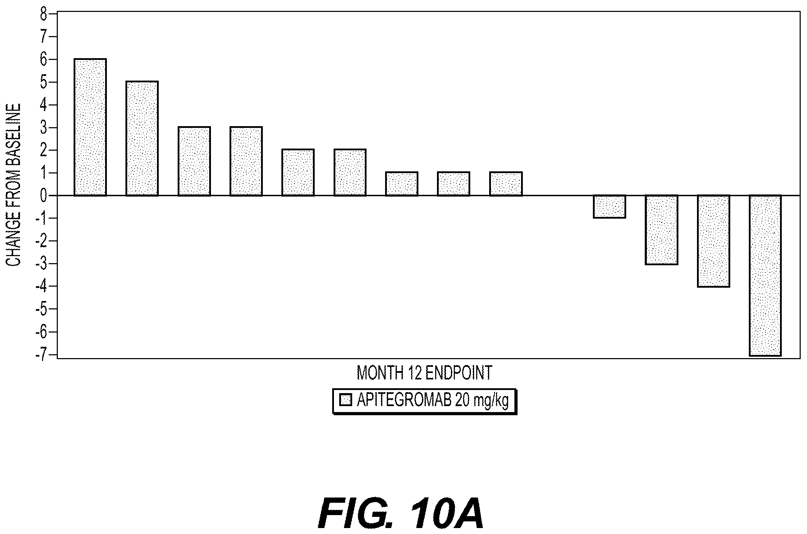

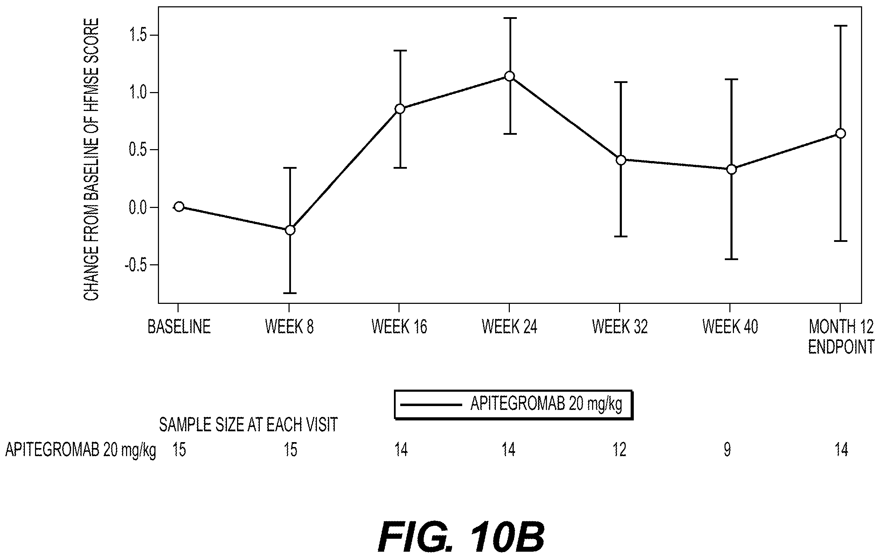

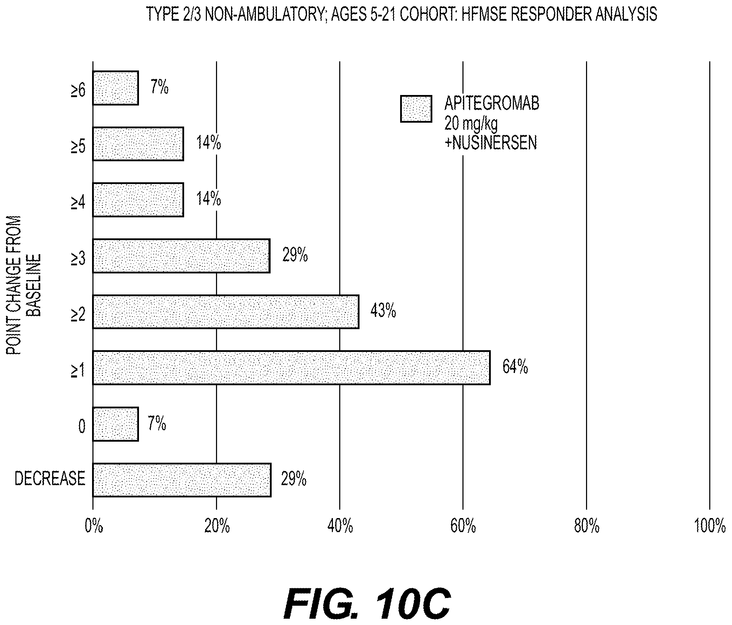

**FIG. 10A-C** show 12-month top-line results from Cohort 2, as set forth in Example 1, Section 1.1. **FIG. 10A** shows individual HFMSE responses. The per protocol analysis excludes one patient who showed a -7 point change from baseline, due to treatment with an acetylcholinesterase inhibitor. **FIG. 10B** shows mean (±SEM) change from baseline in HFMSE scores. **FIG. 10C** shows HFMSE responder analysis in patients from the Type 2/3 Non-Ambulatory Ages 5-21 Cohort.

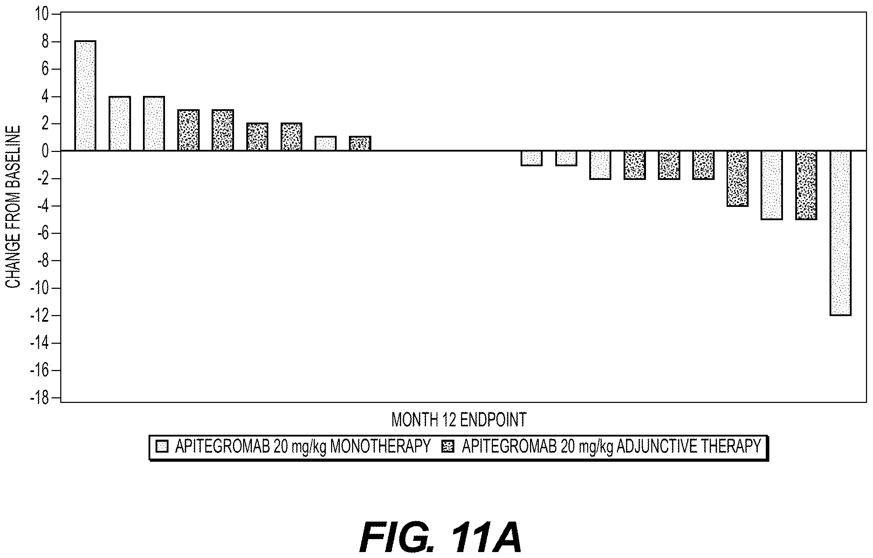

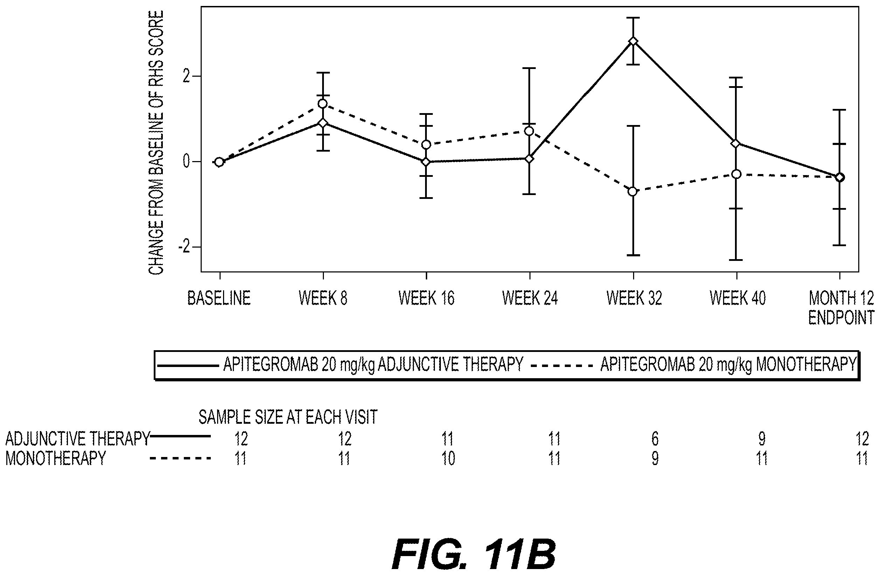

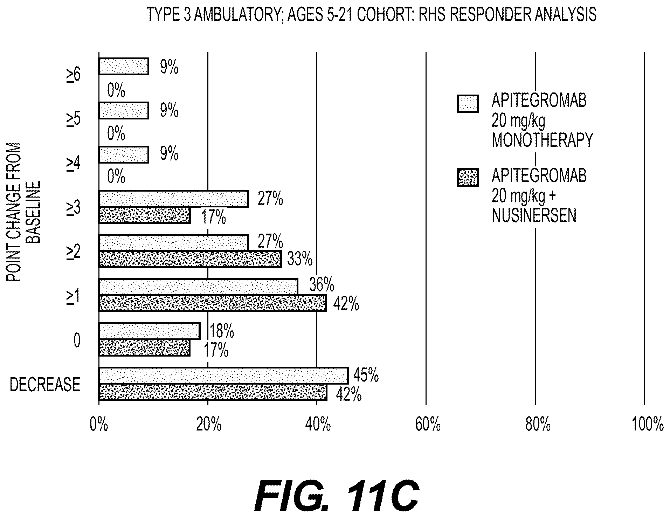

**FIG. 11A-C** show 12-month top-line results from Cohort 1, as set forth in Example 1, Section 1.1. **FIG. 11A** shows individual RHS responses. **FIG. 11B** shows mean (±SEM) change from baseline in RHS scores. **FIG. 11C** shows RHS responder analysis of patients from the Type 3 Ambulatory Ages 5-21 Cohort.

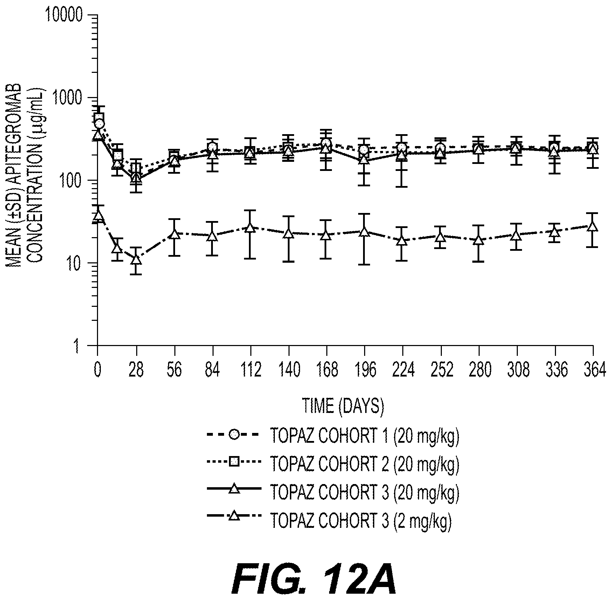

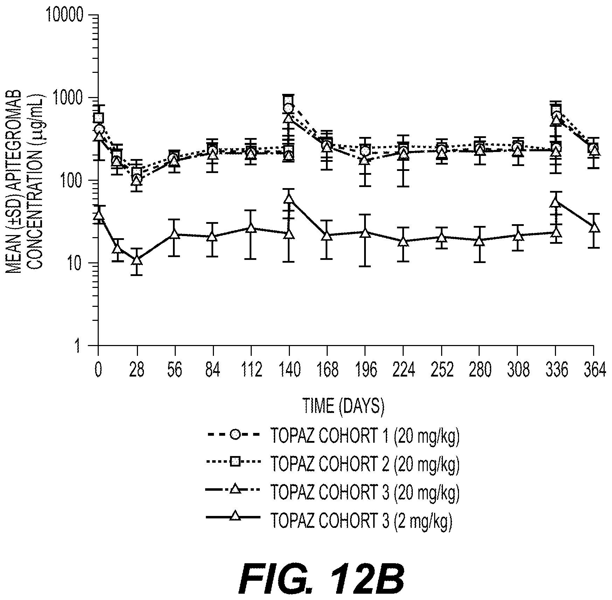

**FIG. 12A-B** show a pharmacokinetics (PK) analysis of 12-month top-line results from Cohorts 1, 2, and 3. In **FIG. 12A****,** starting at day 28, measures are pre-dose trough levels. In **FIG. 12B****,** starting at day 28, measures are pre-dose trough levels except for days 140 and 336. At days 140 and 336, measures are Cₘₐₓ levels.

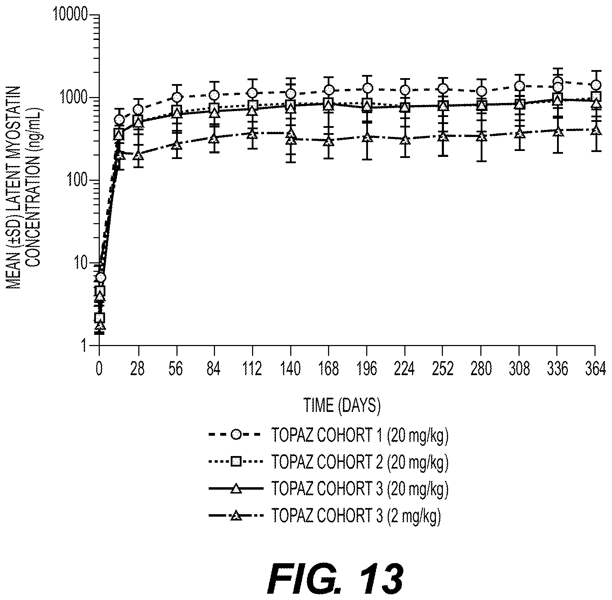

**FIG. 13** shows a pharmacodynamics (PD) analysis of 12-month top-line results from Cohorts 1, 2, and 3, as set forth in Example 1, Section 1.1.

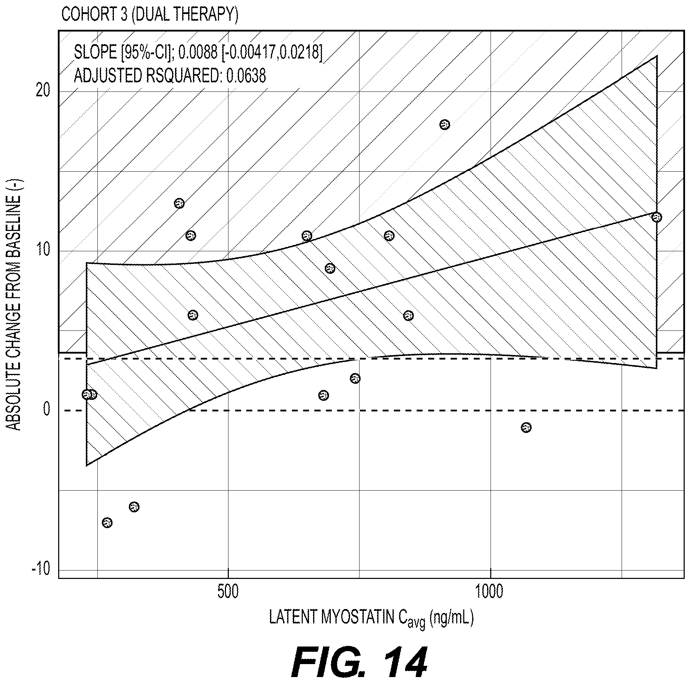

**FIG. 14** shows a graph plotting target engagement against change in HFMSE score in Cohort 3, as set forth in Example 1, Section 1.1 for patients receiving 2 mg/kg (darker circles) or 20 mg/kg (lighter circles) apitegromab in conjunction with nusinersen.

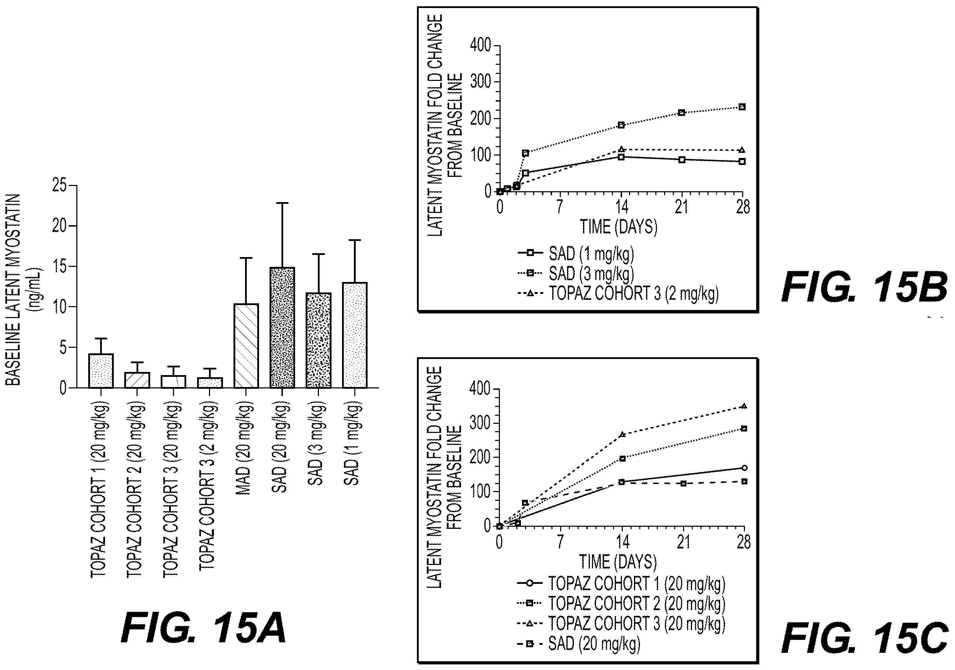

**FIG. 15A** shows a comparison between baseline latent myostatin concentrations in healthy volunteers (SAD and MAD) vs. Cohorts 1, 2, and 3, as set forth in Example 1, Section 1.1. **FIGs** 15B-C show fold change in latent myostatin concentrations in heathy volunteers (SAD) and Cohorts 1, 2, 3.

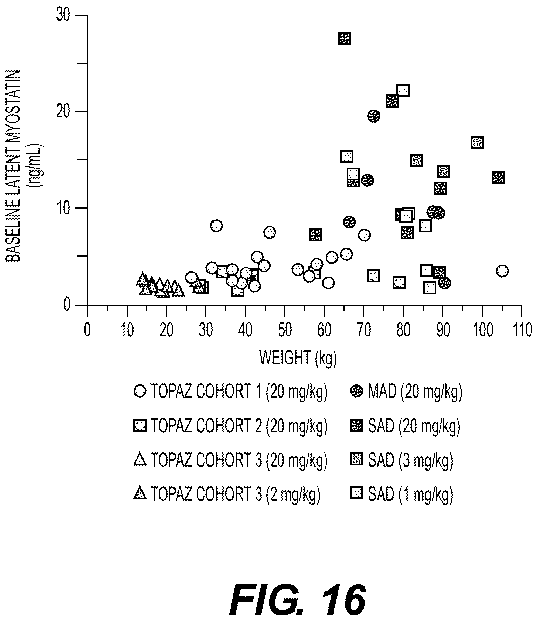

**FIG. 16** shows a correlation between baseline latent myostatin concentration and weight in healthy volunteers (SAD and MAD) vs. Cohorts 1, 2, and 3, as set forth in Example 1, Section 1.1.

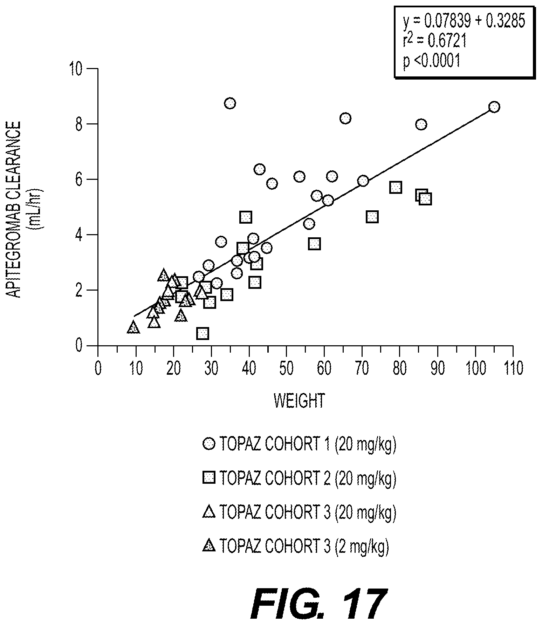

**FIG. 17** shows a linear regression for the correlation between clearance and weight in Cohorts, 1, 2, and 3, as set forth in Example 1, Section 1.1.

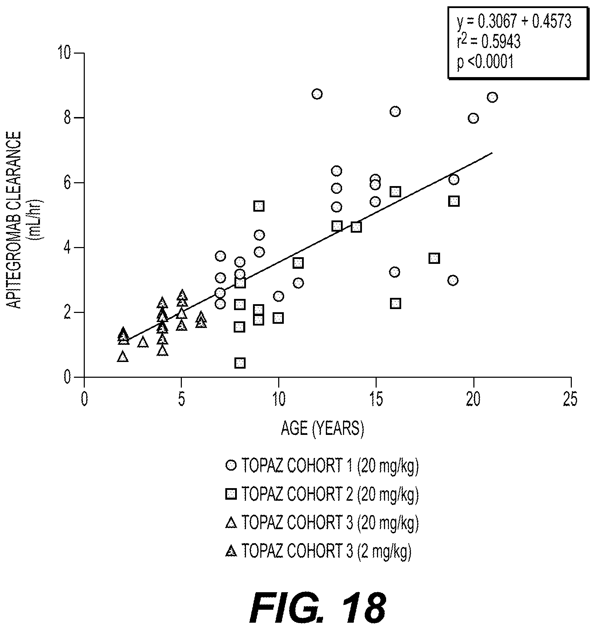

**FIG. 18** shows a linear regression for the correlation between clearance and age in Cohorts, 1, 2, and 3, as set forth in Example 1, Section 1.1.

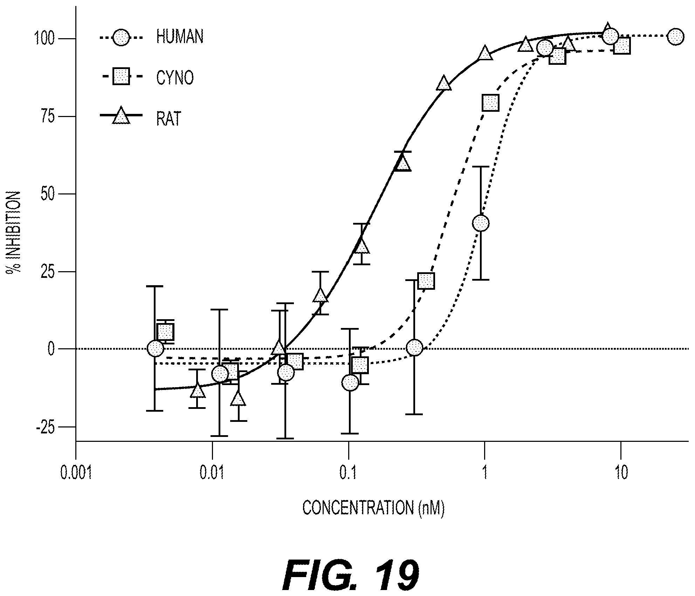

**FIG. 19** shows results from a SMAD-responsive luciferase reporter assay. Recombinant promyostatin was incubated with apitegromab, mTLL2 and furin proteases, followed by incubation on CAGA cells, transfected with a SMAD-responsive luciferase reporter vector. The ability of apitegromab to inhibit the proteolytic processing and release of the active growth factor was measured and plotted as % inhibition.

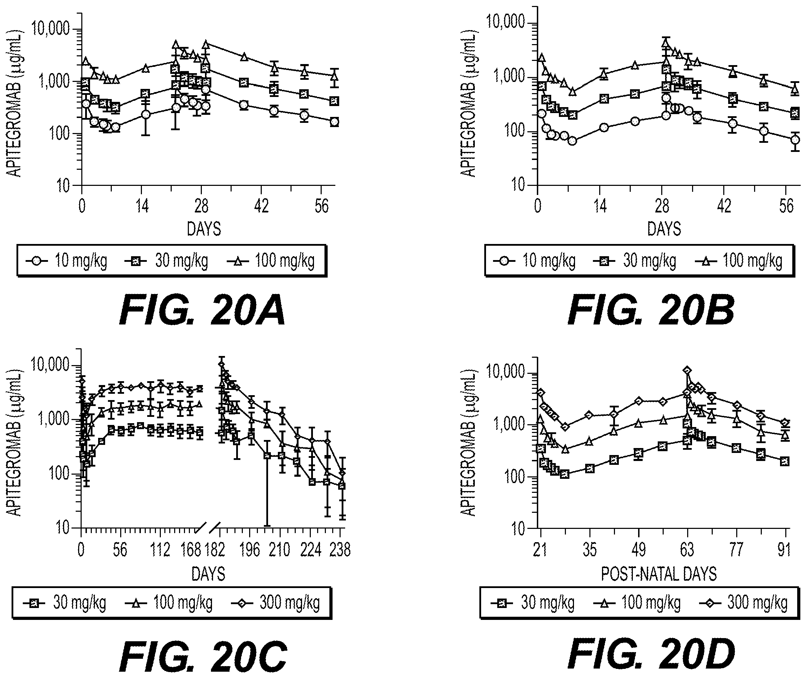

**FIG. 20A-D** show serum concentration time profiles of apitegromab following multiple doses, as set forth in Example 1. Apitegromab was measured in the serum of male and female animals following once weekly repeat IV doses for: four weeks to cynomolgus monkeys at 10, 30, and 100 mg/kg, followed by a four-week recovery phase **(****FIG. 20A****),** four weeks to adult rats at 10, 30, and 100 mg/kg, followed by a four-week recovery phase **(****FIG. 20B****),** 26 weeks to adult rats at 30, 100, and 300 mg/kg, followed by an eight-week recovery period **(****FIG. 20C****),** and seven weeks to juvenile rats at 30, 100, and 300 mg/kg, followed by a four-week recovery phase **(****FIG. 20D****).** Data shown are mean ± standard deviation. Dose symbols: 10 (circles), 30 (squares), 100 (triangles), and 300 (diamonds) mg/kg.

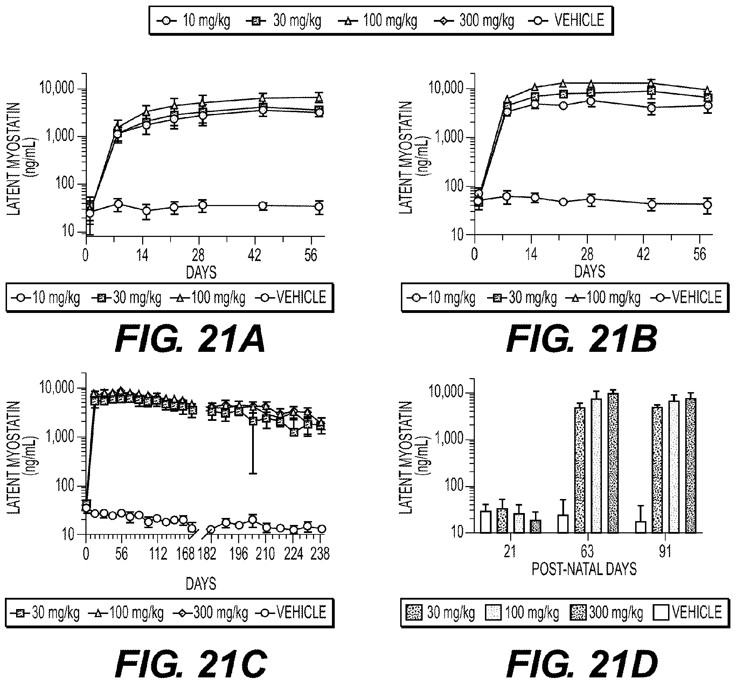

**FIG. 21A-D** show serum latent myostatin concentration time profiles of apitegromab following multiple doses, as set forth in Example 1. Serum latent myostatin was measured in animals administered weekly doses of apitegromab for: four weeks to cynomolgus monkeys at 10, 30, and 100 mg/kg, followed by a four-week recovery phase **(****FIG. 21A****),** four weeks to adult rats at 10, 30, and 100 mg/kg, followed by a four-week recovery phase **(****FIG. 21B**), 26 weeks to adult rats at 30, 100, and 300 mg/kg, followed by an eight-week recovery period **(****FIG. 21C****),** and seven weeks to juvenile rats at 30, 100, and 300 mg/kg, followed by a four-week recovery phase **(****FIG. 21D****).** Data shown are mean ± standard deviation. Dose symbols: 10 (filled circles), 30 (squares), 100 (triangles), and 300 (diamonds) mg/kg.

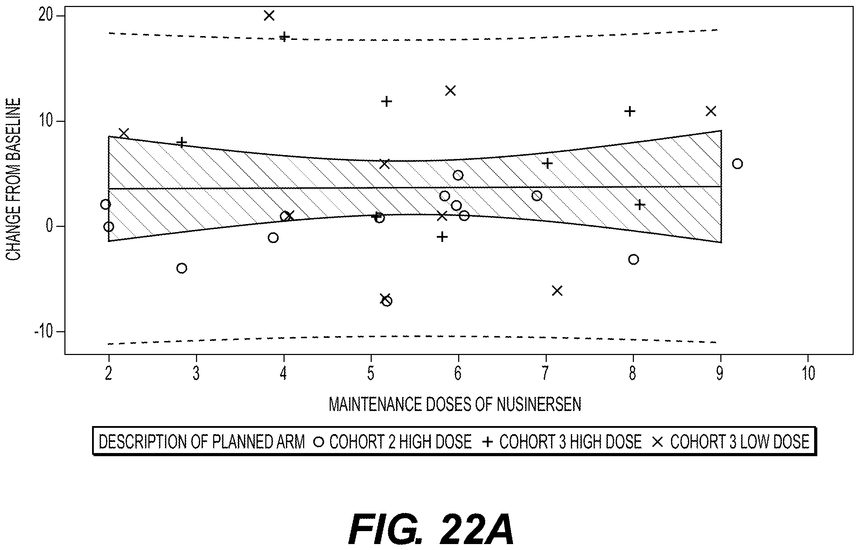

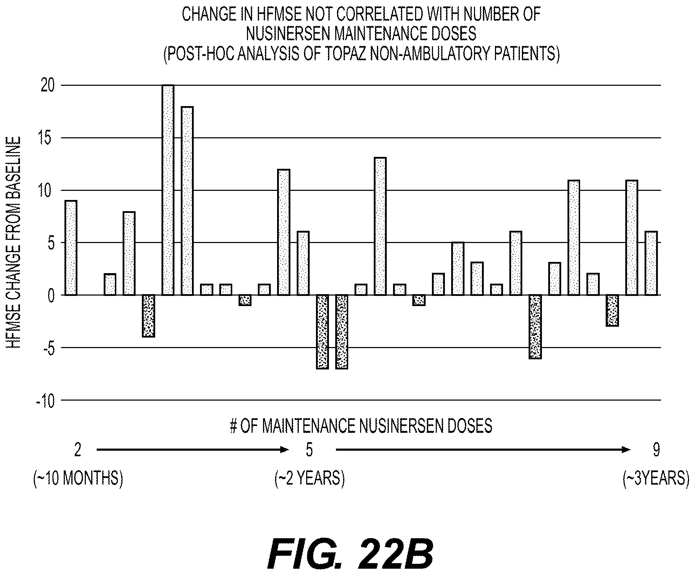

**FIG. 22A** shows 12-month HFMSE changes from baseline as a function of duration of prior nusinersen treatment in cohorts 2 and 3, as set forth in Example 1, Section 1.1. **FIG. 22B** shows 12-month HFMSE changes from baseline as a function of number of nusinersen maintenance doses received in non-ambulatory patients.

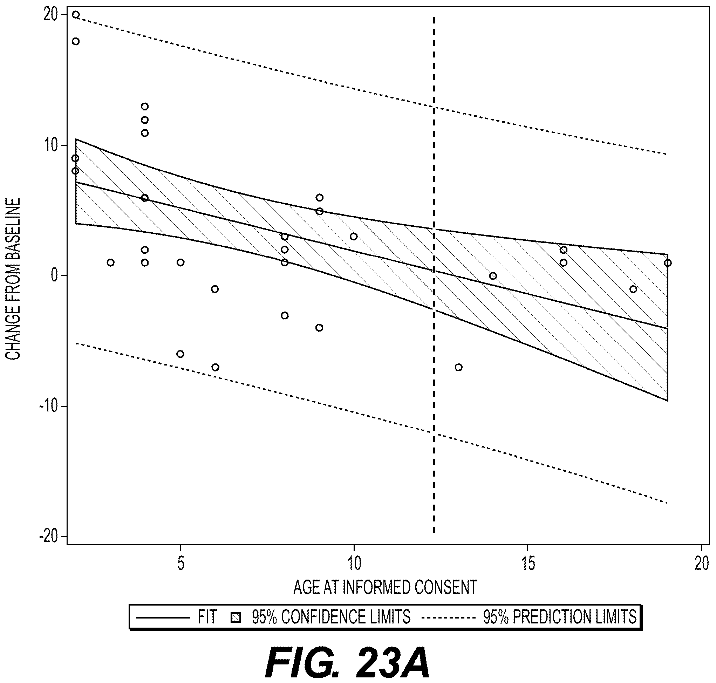

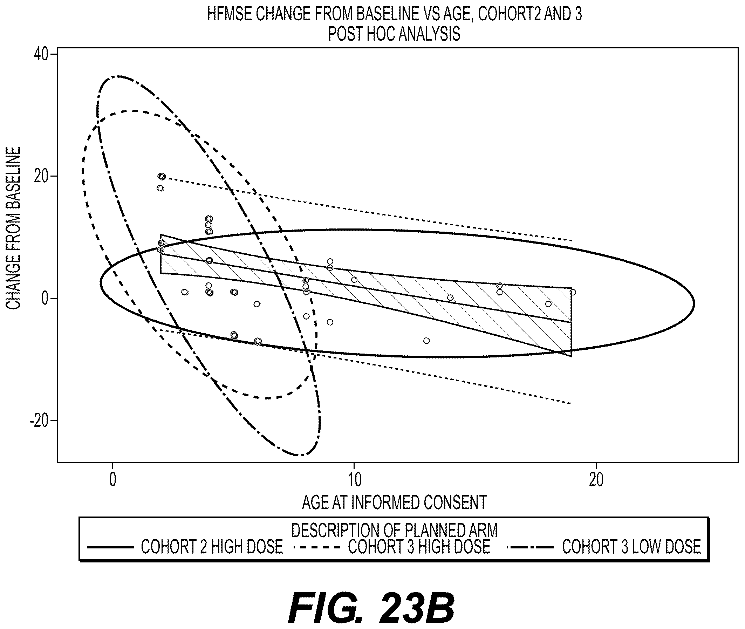

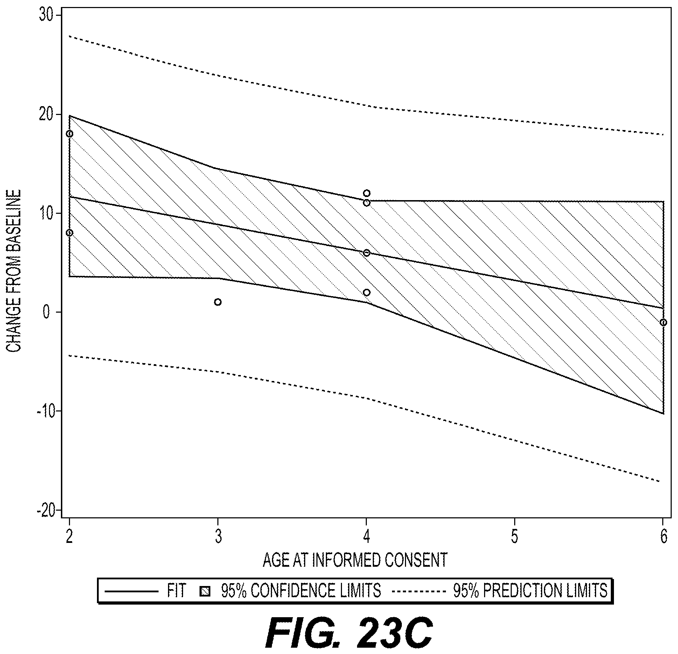

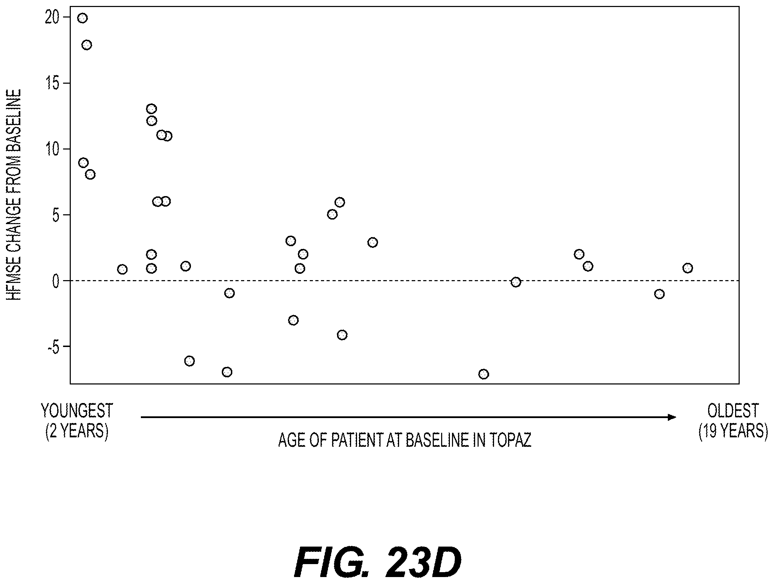

**FIG. 23A-C** show post hoc analyses of 12-month HFMSE change as a function of age for cohorts 2 and 3. as set forth in Example 1, Section 1.1. **FIG 23D** shows HFMSE change from baseline in pooled cohorts of non-ambulatory patients treated with apitegromab 20 mg/kg or 2 mg/kg.

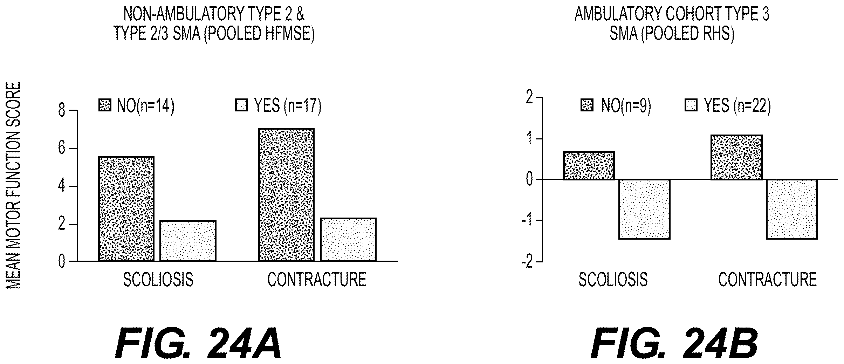

**FIG. 24** shows a comparison of 12-month motor function scores in patients with scoliosis or joint contracture relative to patients without scoliosis or joint contracture, as set forth I Example 1. **FIG. 24A** shows pooled HFMSE scores from non-ambulatory type 2 and type2/3 SMA patients. **FIG. 24B** shows pooled RHS scores from ambulatory type 3 SMA patients.

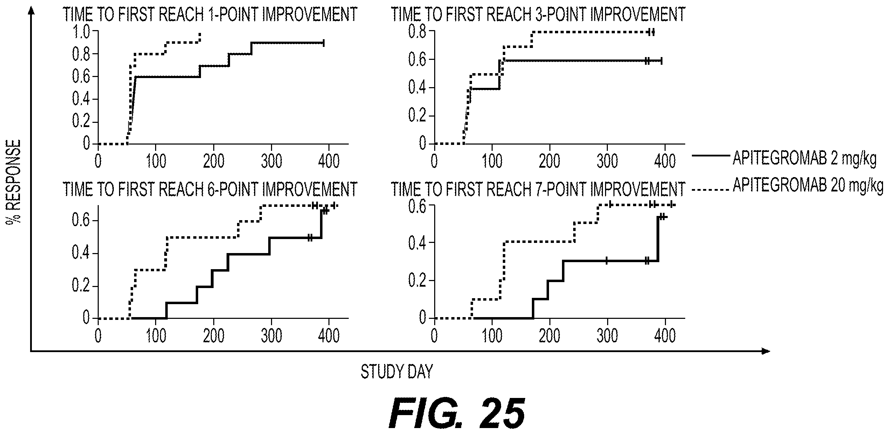

**FIG. 25** shows dose response in patients from type 2 non-ambulatory >age 2 cohort as measured by motor function scores, as set forth in Example 1.

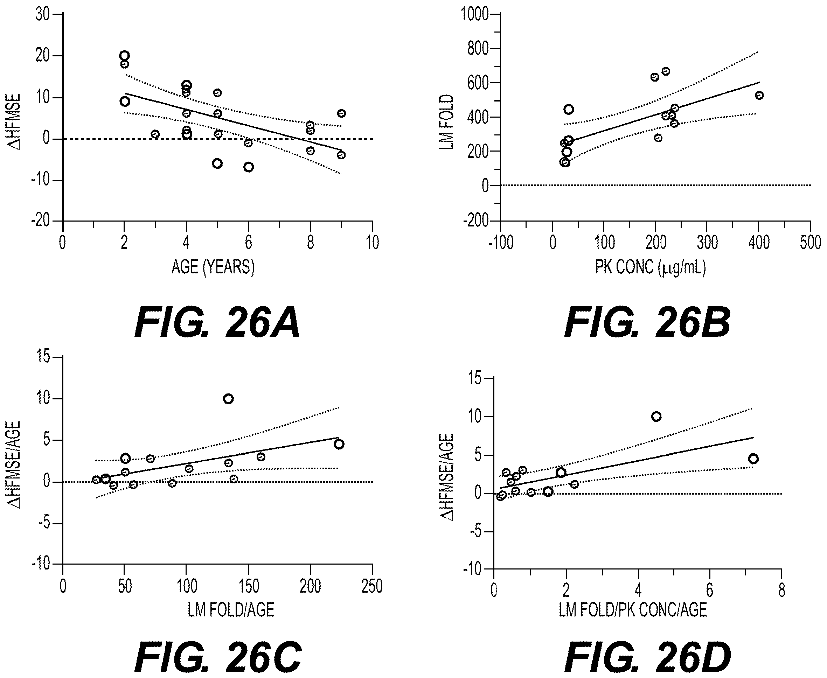

**FIG. 26** shows analysis of the relationship between improvement in motor function as measured in change of HFMSE scores with respect to age, fold-change in latent myostatin levels, and pharmacokinetic concentration of apitegromab in patients with type 2 SMA, as set forth in Example 1. **FIG. 26A** shows the relationship between change in HFMSE score and age. **FIG. 26B** shows the relationship between fold change of latent myostatin levels (LM fold) and the pharmacokinetic concentration of apitegromab. **FIG. 26C** shows the relationship between age-normalized change in motor function and age-normalized fold change in latent myostatin levels. **FIG. 26D** shows the relationship between change in age-normalized motor function and age-normalized LM Fold/PK concentration.

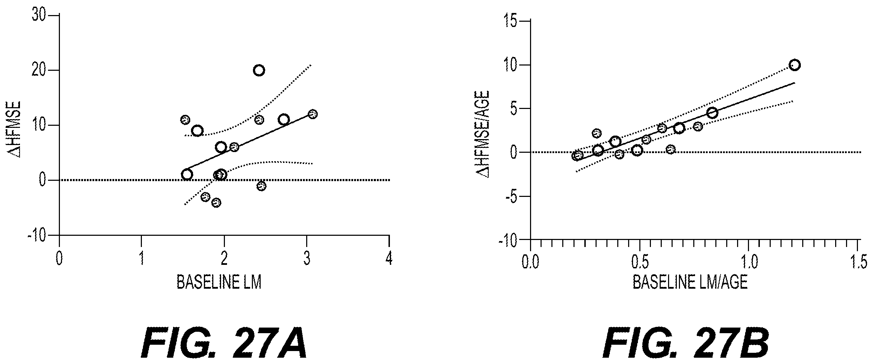

**FIG. 27A** shows the relationship between change in HFME score and baseline latent myostatin. **FIG. 27B** shows the relationship after age normalization, as set forth in Example 1.

### DETAILED DESCRIPTION OF CERTAIN EMBODIMENTS

### Definitions

In order that the disclosure may be more readily understood, certain terms are first defined. These definitions should be read in light of the remainder of the disclosure and as understood by a person of ordinary skill in the art. Unless defined otherwise, all technical and scientific terms used herein have the same meaning as commonly understood by a person of ordinary skill in the art. Additional definitions are set forth throughout the detailed description.

*Apitegromab*: Apitegromab (also referred to as "SRK-015") is an investigational, fully human, monoclonal antibody that specifically binds to the proforms of myostatin-namely, promyostatin and latent myostatin-with high affinity, thereby inhibiting myostatin activation. *See, e.g.,* CAS Reg No. 2278276-46-1; International Nonproprietary Names for Pharmaceutical Substances (INN) (2020) WHO Drug Information, Vol. 34, No. 2 at pages 272-273.; *see also* GtoPdb Ligand ID: 11180; GtoPdb PubChem SID: 434122240; IMGT/mAb-DB Database ID: 829; NIH ChemlDplus Database: RN: 2278276-46-1; UNII: UZ54216N0Y. Apitegromab is of the human immunoglobulin G4 (IgG4)/lambda isotype. Apitegromab is a selective myostatin inhibitor (a myostatin-selective activation inhibitor). *See*, *e.g.*, International Pub. No. WO 2017/049011. The term "selective myostatin inhibitor" (or "myostatin-selective inhibitor") refers to an agent capable of blocking, inhibiting, or otherwise antagonizing the activation or activities of myostatin/GDF-8 without significantly affecting structurally related growth factors, such as Activin A. Apitegromab comprises a heavy chain amino acid sequence of SEQ ID NO: 15 and a light chain amino acid sequence of SEQ ID NO: 16, as shown below in Table 3B.

*Baseline:* As used herein, the term "baseline" or "baseline score" in the context of a SMA-related parameter (e.g., a score on a motor functional scale, e.g., a Revised Hammersmith Score (RHS) score or a Hammersmith Functional Motor Scale Expanded (HFMSE) score), refers to the numerical value of the SMA-related parameter of a patient at or prior to treatment with an SMA therapy of the present disclosure (e.g., prior to or at the time of initial administration of apitegromab, alone or as an adjunct to a background therapy comprising an SMN upregulator therapy).

In some embodiments, a baseline RHS score in a patient to be treated with apitegromab, e.g., apitegromab monotherapy (e.g., a patient who has ambulatory SMA) is measured at or prior to the start of treatment with the apitegromab. In some embodiments, a baseline RHS score in a patient to be treated with apitegromab, e.g., apitegromab as an adjunct to an SMN upregulator therapy (e.g., a patient who has ambulatory SMA) is measured at or prior to the start of treatment with the apitegromab. In some embodiments, the patient is on the SMN upregulator therapy at the time of baseline measurement. In some embodiments, the patient has been on the SMN upregulator therapy for at least six months prior to baseline measurement. For example, in some embodiments, a patient to be treated with apitegromab (e.g., a patient who has ambulatory SMA) has a baseline RHS score of 26 or greater. In some embodiments, the patient has a baseline RHS score of 63 or below. In some embodiments, the patient has a baseline RHS score which ranges between 26-63.

In other embodiments, a baseline HFMSE score is measured in a patient to be treated with apitegromab, e.g., apitegromab as an adjunct to a background therapy of an SMN upregulator therapy (e.g., in a patient with later-onset SMA, e.g., a patient who has a non-ambulatory form of SMA, such as type 2 or non-ambulatory type 3 SMA). In some embodiments, the baseline score is measured at or prior to the start of treatment with the apitegromab (i.e., apitegromab therapy). In some embodiments, the patient is on the SMN upregulator therapy at the time of baseline measurement. In some embodiments, the patient has been on the SMN upregulator therapy for at least six months prior to baseline measurement. In some embodiments, the patient has been on the SMN upregulator therapy for at least 2 years at the time of initiating apitegromab therapy, at which time the baseline measurement is made. In some embodiments, the baseline scores used to assess therapeutic effects of the apitegromab therapy therefore are on top of therapeutic effects, if any, as a result of the background therapy. In some embodiments, the patient had responded to the background therapy by an increase in the motor function scores of, for example, 1-7 points measured against pre-treatment. In some embodiments, a patient to be treated with apitegromab (e.g., a patient who has a non-ambulatory form of SMA, such as type 2 and non-ambulatory type 3 SMA) has a baseline HFMSE score of 12 or greater. In some embodiments, the patient has a baseline HFMSE score of 44 or below. In some embodiments, the patient has a baseline HFMSE score which ranges between 12-44.

*Cohort:* As used herein, the term the "cohort" or "cohort population" refers to a group or population of human subjects with shared factors or influences, such as age, SMA disease severity (e.g., type 2 and/or type 3 SMA), concurrent treatments (e.g., an SMN upregulator therapy), etc. In some embodiments, as used herein, a "cohort" refers to a group of human subjects with shared age, SMA disease severity, and/or concurrent treatments. For example, in some embodiments, a cohort comprises ambulatory type 3 SMA patients, age 5 through 21 years old, including those not receiving an SMN upregulator therapy, as well as SMA patients already receiving an SMN upregulator therapy. In other embodiments, a cohort comprises type 2 and non-ambulatory type 3 patients, age 5 through 21 years old, already receiving an SMN upregulator therapy that had been started after the patient turned 5 years old. In other embodiments, a cohort comprises type 2 SMA patients, age 2 or older, already receiving an SMN upregulator therapy that had been started before the patient turns or turned 5 years old.

*Control:* The term "control" or "control sample," as used herein, refers to any clinically or scientifically relevant comparative sample, population, or counterpart, including, for example, a sample from a healthy subject, a sample from a subject having a deficiency that can cause or make the subject susceptible to a certain disease or condition, a subject with a disease or condition of interest, a sample from a subject treated with a placebo, a sample from a subject prior to treatment, a sham or buffer treated subject or sample, an untreated subject or sample, and the like. In some embodiments, a patient or patient population receiving or in need of treatment with apitegromab may be compared to a control patient or patient population. In some embodiments, the control patient or patient population is a patient or patient population that does not receive apitegromab.

*Inhibit or inhibition of:* The term "inhibit" or "inhibition of," as used herein, means to reduce by a measurable amount, and can include but does not require complete prevention or inhibition.

*Effective amount:* The terms "effective" and "therapeutically effective" refer to the ability or an amount to fulfill its intended purpose(s), i.e., a desired biological or medicinal response in a subject, and/or to achieve a statistically significant clinical benefit (e.g., efficacy) in a patient population. For example, in certain embodiments of the present disclosure, the intended purpose may be to inhibit activation of myostatin *in vivo,* to achieve clinically meaningful outcome associated with the myostatin inhibition. An "effective amount" (or therapeutically effective amount, or therapeutic dose) may be a dosage or dosing regimen that is sufficient to produce a detectable change in a parameter of a disease, e.g., a slowing, pausing, reversing, diminution, or amelioration in a parameter, symptom, or downstream effect of the disease. The term encompasses but does not require the use of an amount that completely cures a disease. References herein to a dose of an anti-pro/latent myostatin antibody (e.g., a dose of apitegromab) may be a therapeutically effective dose, as described herein.

Measure of the relevant intended purpose may be objective (i.e., measurable by some assay or marker) or subjective (i.e., subject gives an indication of or feels an effect). In some embodiments, a therapeutically effective amount is an amount that, when administered to a patient population that meets certain clinical criteria for SMA (for example, as determined by symptoms manifested, disease progression/stage, genetic profile, etc.), a statistically significant therapeutic response is obtained among the population.

In some embodiments, an effective amount is an amount that, when administered according to a particular regimen, produces a positive clinical outcome with a reasonably acceptable level of adverse effects (e.g., toxicity), such that the adverse effects, if present, are tolerable enough for a patient to continue with the therapeutic regimen, and the benefit of the therapy overweighs risk of toxicity. Those of ordinary skill in the art will appreciate that in some embodiments of the disclosure, a dosage may be considered to contain an effective amount if it contains an amount appropriate for administration in the context of a dosage regimen correlated with a positive outcome.

*Later-onset SMA:* As used herein, unless explicitly defined otherwise, the term later-onset SMA refers to a patient who does not exhibit onset of SMA symptoms until after 6 months of age (e.g., symptoms are not detected at a 6 month medical screening). A later-onset SMA patient may also be a patient characterized as having type 2, type 3 or type 4 SMA based on the traditional disease classifications known in the art. Patients with later-onset SMA typically have at least 2 copies of the SMN2 gene (e.g., 2 copies, 3 copies, 4 copies, or more, e.g., 2-4 copies). In some embodiments, a patient with later-onset SMA is treated with or has received a motor neuron-directed therapy (e.g., has received such therapy before the age of 5), such as an SMN-directed therapy, e.g., an SMN upregulator. As opposed to later-onset SMA, early-onset or infantile-onset SMA refers to SMA that manifests symptoms at or prior to six months of age (including patients who can also be categorized as type 1 SMA using the traditional classification known in the art). By comparison, an early- or infantile-onset SMA patient typically has 1 or 2 copies of the SMN2 gene.

In some embodiments, a patient with later-onset SMA has 2-4 copies of the SMN2 gene. In some embodiments, a patient with later-onset SMA has 1-2 copies of the SMN2 gene. In some embodiments, patients with later-onset SMA due to early treatment intervention (i.e., patients who otherwise would have had onset of symptoms before 6 months of age) may also be referred to as an "emerging" or "treatment-emergent" later-onset SMA patient or patient population. In some embodiments, treatment-emergent later-onset SMA patients may have different genotype (e.g., 1-2 copies of the SMN2 gene), phenotype (e.g., gait), and/or course of disease and response to myostatin treatment. In some embodiments, a patient having later-onset SMA would not have been able to walk and/or sit unassisted (e.g., would have had type 1 and/or non-ambulatory type 2 SMA) in the absence of treatment, e.g., SMN-directed treatment, but is able to do so because of treatment. In some embodiments, a treatment-emergent later-onset SMA patient who would have had non-ambulatory type 3 such that they would have lost the ability to walk by 18 months of age in the absence of treatment, e.g., SMN-directed treatment, retains the ability to walk at 18 months because of treatment. When considering treatment of ambulatory type 3 SMA patients, in some embodiments any ambulatory patient is treated. In some embodiments, a type 3 patient is one that would retain the ability to walk by 18 months in the absence of intervention. In some embodiments, a type 3 patient is one that would lose the ability to walk in the absence of intervention. In some embodiments, a patient is identified through newborn screening and is initiated in treatment, e.g., SMN-directed therapy, prior to disease onset. In some embodiments, SMA symptom onset is not observed because of early treatment initiation following newborn screening. In some embodiments, the treated patient has type 4 SMA (e.g., a patient with 4 or more copies of the SMN2 gene and much later onset of symptoms, e.g., at 18 years of age or later).

*Natural history*: Natural history of SMA refers to the progression of SMA in a person over time without treatment.

*Progression:* Progression of disease (e.g., SMA) is the process of worsening of symptoms or a decline of condition over a period of time. Absent pharmacological intervention (e.g., therapy), the progression is reflected on or corresponds to the natural history of the disease observed in a particular patient population. Disease progression may be assessed by the rate by which the condition worsens, and/or, the degree by which the condition becomes worse. Accordingly, efficacy may include the ability of a drug or therapy to delay or slow the progression of disease. For example, a patient may show a decline in a motor function score over time, but at a slower rate than what would be expected based on the natural history. Efficacy may include the ability of a drug or therapy to reduce the degree of decline. Efficacy may include stabilization of the disease, i.e., at least net zero change in functional parameter(s) over time.

*SMN therapy*/*SMN-directed therapy:* In the context of the present disclosure, the term "SMN therapy" or "SMN-directed therapy" (used interchangeably herein) refers to pharmacological agents (drugs, biologics) aimed to increase the amount or availability of the functional SMN protein in patients for purposes of treating SMA. Such agents include SMN correctors/upregulators. For example, an SMN1-directed therapy may include gene therapies intended to supplement or replace the deleted or mutated SMN1 gene. An SMN2-directed therapy may include splice modifiers that target certain exon(s) of the SMN2 backup gene so as to increase the availability of an at least partially functional SMN protein in the body. Non-limiting examples of SMN therapies include nusinersen, onasemnogene abeparvovec-xioi, and risdiplam.

*Steady-state:* As used herein, the term "steady-state" refers to the situation where the overall intake of a molecule (e.g., apitegromab) is fairly in dynamic equilibrium with its elimination. In some embodiments, a steady-state serum concentration of apitegromab is measured as a maximum serum concentration (i.e., Cₘₐₓ) or a minimum (trough) serum concentration (i.e., Cₘᵢₙ or C_{trough}) of the apitegromab after administration, e.g., to a human subject or to a cohort of human subjects. In some embodiments, maximum serum concentration of apitegromab is used to determine steady state of the apitegromab in a pharmacokinetics (PK) analysis. In some embodiments, minimum serum concentration of apitegromab is used to determine steady state of the apitegromab in a PK analysis. In some embodiments, the pharmacodynamic (PD) target is latent myostatin. In some embodiments, the PD profile of apitegromab is evaluated by measuring latent myostatin concentrations in serum. In some embodiments, the serum latent myostatin concentration is a steady-state concentration. In some embodiments, the serum latent myostatin concentration is measured as a pre-dose or trough concentration (i.e., Cₘᵢₙ or C_{trough}), e.g., in a human subject or in a cohort of human subjects.

*Subject:* The term "subject" in the context of therapeutic applications refers to an individual who receives or is in need of clinical care or intervention, such as treatment, diagnosis, etc. Suitable subjects include vertebrates, including but not limited to mammals (e.g., human and non-human mammals). Where the subject is a human subject, the term "patient" may be used interchangeably. In a clinical context, the term "a patient population" or "patient subpopulation" is used to refer to a group of individuals that falls within a set of criteria, such as clinical criteria, medical history, health status, gender, age group, genetic criteria, and/or lifestyle factors. In some embodiments, a patient or patient population is a SMA patient or patient population. In some embodiments, a patient or patient population is an early- or infantile-onset SMA patient or patient population. In some embodiments, a patient or patient population is a later-onset SMA patient or patient population. In some embodiments, a patient or patient population is a type 2 SMA patient or patient population. In some embodiments, a patient or patient population is a non-ambulatory type 3 SMA patient or patient population. In some embodiments, a patient or patient population is an ambulatory type 3 SMA patient or patient population. In some embodiments, a patient or patient population is an ambulatory later-onset SMA patient or patient population. In some embodiments, a patient or patient population is a non-ambulatory patient or patient population. In some embodiments, a patient or patient population is a presymptomatic or asymptomatic patient or patient population. In some embodiments, a patient or patient population is a symptomatic patient or patient population. In some embodiments, a patient or patient population has two or more copies of the SMN2 gene, e.g., 2-3 copies, 2-4 copies, 3-4 copies, etc. In some embodiments, a patient or patient population has later-onset SMA and has two or more copies of the SMN2 gene, e.g., 2-3 copies, 2-4 copies, 3-4 copies, etc.

*Target engagement:* As used herein, the term "target engagement" refers to the ability of a molecule (e.g., apitegromab) to bind to its intended target *in vivo* (e.g., latent myostatin), e.g., in skeletal muscle. In some embodiments, serum latent myostatin levels may be relatively low at baseline (i.e., prior to treatment) and increase following treatment with apitegromab. In some embodiments, an increase in serum latent myostatin levels indicates target engagement with apitegromab, e.g., in skeletal muscle. In some embodiments, low baseline serum levels of latent myostatin as compared to high levels following treatment indicates that a major portion of the drug target resides within skeletal muscle, rather than circulating systemically. In some embodiments, saturation in target engagement indicates a dosage sufficient to achieve certain therapeutic effects (e.g., efficacy), although it may be possible to obtain efficacy without saturation.

*Therapeutic dose:* The term "therapeutic dose" refers to an amount (e.g., of apitegromab) sufficient to achieve efficacy as determined by clinical endpoints that measure therapeutic effects (e.g., by one or more of the clinical endpoint measurements discussed herein) when administered to a patient in a particular way (e.g., using a particular dosing regimen). For example, a therapeutic dose of apitegromab, when administered intravenously every 4 weeks, may be greater than 2 mg/kg and up to 20 mg/kg. According to the invention, the therapeutic dose of apitegromab, when administered intravenously every 4 weeks, is 10 mg/kg.

*Treatment:* As used herein, the term "treating" or "treatment" of a disease or disorder (e.g., SMA) is meant slowing, delaying, or preventing the onset of such a disease or disorder, or reversing, alleviating, ameliorating, inhibiting, slowing down, stabilizing, or stopping the progression, aggravation or deterioration, the progression or severity of a condition associated with such a disease or disorder, e.g., as compared to a baseline in the absence of the treatment (e.g., where treatment stops disease progression observed in the absence of treatment). The term includes but does not necessarily require a complete treatment or prevention of the disease or disorder. In some embodiments, "treating" or "treatment" refers to the administration of one or more active agents (simultaneously or sequentially), or of one or more compositions including one or more active agents, to a subject who has SMA, with the purpose to cure, heal, alleviate, relieve, alter, remedy, ameliorate, improve, or affect the disorder, the symptom of the disease, or the predisposition toward the SMA.

*Treatment emergent patient population:* The traditional classification of SMA patient populations or types (e.g., type 0, type 1, type 2, non-ambulatory type 3, ambulatory type 3, type 4 etc.) is largely based on the natural history of the disease manifestation, which shows strong correlation with the number of SMN2 gene copies. Based on the traditional classification, type 0 is the most severe form which has in utero onset with reduced or absent movements and requires mechanical ventilation at birth. With the availability of approved SMN-directed therapies (e.g., SMN2 upregulators, SMN1 gene therapy, etc.) and newborn screening in recent years, additional classifications may be warranted to describe emerging patient populations, referred to herein as "emergent" or "treatment emergent" patient populations. In these populations, the manifestations of SMA expected based on natural history and/or genetic analysis may be altered where patients have initiated an SMN-directed therapy at an early age. In other words, early intervention can impact the course of the disease progression expected from the natural history, irrespective of the disease genotype. For example, an infant with confirmed SMN1 deletion and one copy of the SMN2 gene, who receives an early SMN therapy, may be able to sit independently at 12 months, who otherwise would have been expected to develop type 1 SMA. A SMA patient with two copies of the SMN2 gene may be expected to develop a severe (e.g., type 2) disease form without receiving SMN-directed therapy, but with SMN-directed therapy, especially with an early intervention, clinical presentation in such patient may resemble that of a milder disease form. A patient with non-ambulatory type 3 SMA (who lost the ability to walk) may regain the ability to walk, or a patient with type 2 SMA may walk for the first time, as a result of such therapy. Likewise, a patient expected to develop non-ambulatory type 3 SMA after 18 months of age may exhibit a retained ability to walk at a later age due to early SMN-directed therapy yet exhibit different phenotype (e.g., gait) and/or response to SMA treatments (Mercuri et al. (2020) Nature Reviews Neurology, 16:706-715). To accommodate changes associated with the medical advancement in SMA therapy, the field continues to evolve, including adjustments to certain nomenclature/terminology, aimed to better describe various disease. As such, the term "later-onset SMA" as used herein is intended, unless indicated otherwise, to describe a patient or patient population in which the symptomatic onset (e.g., clinical manifestation) occurs after 6 months of age, irrespective of the genotype, with or without SMN-directed therapy. Whereas a patient in which symptomatic onset occurs before 6 months of age is referred to herein as "early-onset" or "infantile-onset" SMA depending on the specific age symptoms are first detected.

*Type 1 SMA*: Typically, children with type 1 SMA rapidly decline in the natural history of the disease. Early symptoms include hypotonia, small or weak muscles, difficulty in breathing, trouble swallowing, weak cough or weak cry, and inability to sit. Infants with Type 1 are very weak and unable to sit. At 5-6 months of age, patients typically require respiratory and/or nutritional support. Over 90% of patients die before the second birthday. Such patients typically have 1-2 copies of the SMN2 gene.

*Type 2 SMA:* Children with type 2 SMA are non-ambulant absent therapeutic intervention and often have 3 copies of the SMN2 gene and become symptomatic between 6-18 months of age. Muscle weakness is very common and affects the ability to stand or walk without assistance and typically require wheelchairs. Generally, they start losing abilities before the age of 2. But they can sit independently, maintain head control and roll over.

*Type 3 SMA:* In type 3 SMA patients, symptoms can begin after 18 months of age, usually in early childhood, e.g., based on clinical classification and/or physical milestones such as sitting or walking. These patients often have 3 or 4 or more copies of the SMN2 gene, with the more severe form of non-ambulatory type 3 SMA typically being associated with 3 copies of the SMN2 gene (type 3a) and the less severe form of non-ambulatory type 3 SMA typically being associated with 4 copies (type 3b). Typically, type 3 SMA patients can at least initially walk and climb with assistance, eat with utensils and dress themselves, but they cannot generally run, jump or climb without help. They lose many motor functions as they grow older, including the ability to walk and climb. Among type 3 patients, those who are able to walk are referred to as ambulatory type 3 SMA, whilst those who have lost the ability to walk are referred to as non-ambulatory type 3 SMA. Type 3 patients often lose ambulation by ages 4-16, e.g., on average around age 10.

*Type 4 SMA:* Type 4 SMA is a rare, adult-onset form of SMA. These patients usually have 4 or more copies of the SMN2 gene and usually experience only mild muscle weakness, which may start around 18 years of age, but more often later (e.g., in the 20's or 30's).

Other than in the operating examples, or where otherwise indicated, all numbers expressing quantities of ingredients or reaction conditions used herein should be understood as modified in all instances by the term "about." The term "about" when used in connection with percentages can mean ±1%.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in some embodiments, to A without B (optionally including elements other than B); in other embodiments, to B without A (optionally including elements other than A); in yet other embodiments, to both A and B (optionally including other elements); etc.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in some embodiments, to at least one, optionally including more than one, A, with no B present (and optionally including elements other than B); in other embodiments, to at least one, optionally including more than one, B, with no A present (and optionally including elements other than A); in yet other embodiments, to at least one, optionally including more than one, A, and at least one, optionally including more than one, B (and optionally including other elements); etc.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

Ranges provided herein are understood to be shorthand for all of the values within the range. For example, a range of 1 to 10 is understood to include any number, combination of numbers, or sub-range from the group consisting of 1, 2, 3, 4, 5, 6, 7, 8, 9, or 10, e.g., 2-8, 1-5, etc.

Where a reference and the specification conflict, the specification will control. It is to be appreciated that certain features of the disclosed compositions and methods, which are, for clarity, described herein in the context of separate embodiments, may also be provided in combination in a single embodiment. Conversely, various features of the disclosed compositions and methods that are, for brevity, described in the context of a single embodiment, may also be provided separately or in any subcombination.

### Spinal Muscular Atrophy (SMA)

Spinal muscular atrophy (SMA) is a debilitating, frequently fatal neuromuscular disease and the most common genetic cause of infant mortality (Awano et al. (2014) Neurotherapeutics 11:786-795). SMA is an autosomal recessive genetic disorder involving a mutation or deletion in the survival motor neuron 1 (SMN1) gene. Specifically, SMA is caused by reductions in the level of SMN protein, sufficient amounts of which are necessary to promote survival of the anterior horn cells of the spinal cord. Loss of motor neurons results in profound muscle atrophy, often leading to death due to respiratory insufficiency (Monani (2005) Neuron 48:885-896). Muscle pathology in SMA is characterized by the presence of small atrophic fibers believed to represent denervated or partially denervated myofibers. The presence of atrophic fibers is a classic indication of motor neuron denervation caused by the loss or dysfunction of motor neurons. Unlike Duchenne muscular dystrophy (DMD) patients, SMA patients do not exhibit features of necrosis and inflammatory changes even in the most severe cases of the disease (Le Verche V. et al. Spinal Muscular Atrophy; Disease Mechanisms and Therapy 2017; Ch. 21:341-356).

While SMA patients lack a functional SMN1 gene, the paralogous gene, SMN2, produces low levels of functional SMN protein due to alternative splicing that truncates the transcript. The development of molecular medicine techniques have advanced the diagnosis of SMA patients based on genotyping of both SMN1 and SMN2 copy numbers. SMA patients are also diagnosed based on clinical presentation and categorized phenotypically based on the maximal motor milestone achieved and the age at symptom onset. Disease-modifying drugs have altered some of the traditional diagnosis and classification. Classification may, in some embodiments, be made based on one or more of age of initiation of therapy, age of symptom onset, and/or number of SMN2 copies, e.g., separately or to refine the definition of SMA phenotypes in the clinic beyond the traditional classifications defined by age of onset and severity alone (J 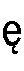 drzejowska M. et al. Degener Neurol Neuromuscul Dis. 2020;10:39-47).

As used herein, unless explicitly defined otherwise, the term "later-onset SMA" is intended to encompass type 2, type 3 (ambulatory and non-ambulatory) and type 4 SMA as defined by the traditional SMA classifications known in the art. In some embodiments, a patient with later-onset SMA is treated with or has received a motor neuron-directed therapy (e.g., has received such therapy before the age of 5), such as an SMN upregulator. In some embodiments, a patient with later-onset SMA has at least two copies of the SMN2 gene (e.g., at least three copies or at least four copies of SMN2). In some embodiments, a patient with later-onset SMA develops diagnosable symptoms of SMA after 6 months of age. A subject with SMA may be treated before exhibiting symptoms of the disease or after onset of diagnosable symptoms.

As used herein, the term "infantile-onset SMA" or "early-onset SMA" encompasses type 1 SMA as defined by the traditional SMA classifications known in the art. In some embodiments, a patient with infantile-onset SMA is treated with or has received a motor neuron-directed therapy, such as an SMN upregulator. In some embodiments, a patient with infantile-onset SMA has one copy of SMN2. In some embodiments, a patient with infantile onset SMA develops diagnosable symptoms of SMA before 6 months of age. In some embodiments, a patient with infantile onset SMA would have developed diagnosable symptoms before 6 months of age but does not because of treatment, e.g., with an SMN upregulator. In some embodiments, these subjects are diagnosed with infantile-onset SMA based on, e.g., SMN2 copy number, and are distinguished from later-onset SMA patients on the basis of other factors beyond age of disease onset, e.g., genotype (such as SMN2 copy number), phenotype (such as gait or other observed motor traits), and/or response to SMA therapy.

A summary of SMA categorization and disease characteristics is provided below.

| **Onset-based categorization** | **Classic categorization (by natural history)** | **SMN2 gene #copy (typically)** | **SMN-directed therapy** | **Apitegromab** | **TOPAZ trial** | **Examples of emerging patient populations (effects of therapy; deviation from natural history)** |
|---|---|---|---|---|---|---|
| Infantile-onset SMA (symptomatic by 6M of age) | Type 1 (never sit independently) | 1-2 | + (initiated before age 5) | + | | - Initiated SMN-directed therapy before 6M and remain largely asymptomatic at 6M (as if type 2) |
| | | | | | | - Initiated SMN-directed therapy after being symptomatic by 6M and gain ability to sit by 12M (as if type 2) |
| | | | | | | - Identified by newborn genetic screening and may remain asymptomatic after early treatment |
| | | | | | | |
| Later-onset SMA (becomes symptomatic after 6M of age) | Type 2 (never walked) | 2-3 | + (initiated before age 5) | + | Cohort 3 | - May gain ability to walk (as if ambulatory type 3) after SMN-directed treatment |
| | | | + (initiated at age 5 or later) | + | Cohort 2 | |
| | | | - | + | | |
| | Type 3 non-ambulatory (lost ability to walk) | 2-4 (3 copies = type 3a; 4 copies = type 3b) | + (initiated before age 5) | + | | - May regain ability to walk (after being non-ambulatory) after SMN-directed treatment but may lose ability at a later age |
| | | | + (initiated at age 5 or later) | + | Cohort 2 | |
| | | | - | + | | |
| | Type 3 ambulatory (retained ability to walk) | 3+ | + | + | | - May lose ability to walk at a later age |
| | | | - | + | Cohort 1 (monotherapy) | |
| | Type 4 (adult-onset) | 4+ | - | + | | |

### Genotype-based classifications of SMA

The clinical heterogeneity of SMA is due in part to the complicated genetics of the disease. Mutations in the SMN1 gene result in SMA (Lefebvre et al. (1995) Cell 80:155-165); however, in humans, a nearly identical gene, SMN2, is located in close proximity to SMN1 (Monani et al. (1999) Hum Mol Genet 8:1177-1183). The primary difference between these genes is a C to T transition which creates an exonic splice silencer, resulting in removal of exon 7 from the final mRNA transcript. The truncated SMN protein is unstable and quickly degraded. Nevertheless, approximately 10% of the mRNA produced from SMN2 is correctly spliced and produces full length SMN protein, but this amount is insufficient to fully compensate for loss of SMN1.

The copy number of SMN2 varies between individuals and strongly correlates with SMA disease severity. More copies (e.g., three or four copies) are generally associated with milder forms of SMA. SMA patients with a single SMN2 copy are rare but this copy number is highly predictive of a severe type 1 phenotype with a poor prognosis. The majority of patients with the type 1 form of SMA have one or two copies of SMN2; most patients with type 2 have three SMN2 copies; and most patients with type 3 have three or four SMN2 copies. To illustrate, according to Calucho et al. ((2018) Neuromuscular Disorders. 28:208-215 at Table 2), about 80% of type 1 SMA patients carry one or two SMN2 copies, about 94% of type 2 patients carry two or three copies, about 93% of type 3 patients carry three or four copies, and nearly 100% of type 4 patients carry four to six SMN2 copies.

Traditional SMA classifications include type 1, type 2, type 3 and sometimes type 4 SMA, in the order of severity. Type 1 SMA is typically diagnosed between birth and 6 months, and, without early intervention, patients never gain sufficient strength to sit independently. Without intervention, most type 1 patients do not survive past two years without respiratory support. People with type 2 and type 3 produce greater amounts of SMN protein and have less severe, but still life-altering forms of SMA. Type 2 patients are generally diagnosed between six and 18 months of age. While they are able to sit unassisted, they cannot walk without aid. With type 3 SMA, patients are typically diagnosed past the age of 18 months and are able to sit and walk unassisted, although they may become wheelchair-dependent later in life. Thus, type 3 SMA includes both ambulatory and non-ambulatory subpopulations. Many ambulatory type 3 SMA patients at some point transition to non-ambulatory as the disease progresses. Type 4 SMA is adult onset, mild in phenotype, and very rare.

Although SMA stratification by type is a useful clinical paradigm, the disease phenotype exists more as a continuum than as discreet classifications. For example, patients carrying an SMA genetic mutation may also be pre-symptomatic (not manifesting obvious disease phenotypes).

In some cases, a patient who carries a genetic mutation in the SMN gene (such as SMN1) may be phenotypically pre-symptomatic and identified for treatment on the basis of genetic screening. In some embodiments, the delay in disease manifestation may be at least in part due to an early intervention such as an SMN upregulator therapy (e.g., SMN2 upregulator therapy and SMN1 gene therapy). Early intervention generally means the treatment is initiated prior to age 5, e.g., prior to age 4, age 3, age 2, age 1, or soon after birth. Newborn genetic screening has become more widely accessible in recent years, which allows the identification of individuals born with genetic disorders, such as SMA. In the United States, for example, many states have included SMA in a panel of routine newborn screening among other known genetic disorders, which is typically conducted during the first few days of life. Therefore, babies carrying mutations in the SMN gene (SMN1) who are likely to develop the disease can be identified early, often when asymptomatic (pre-symptomatic). Early detection and diagnosis can lead to early treatment intervention, e.g., even before the child starts to develop symptoms. This may help prevent certain clinical manifestations and/or may delay the onset or progress of SMA.

Genetic screening may be carried out in newborn/infant subjects, as well as in utero (e.g., in a fetus). In some embodiments, a subject is or has been identified as a carrier of an SMN mutation, e.g., by genetic screening either in utero or as a newborn/infant. In some embodiments, the genetic screening is carried out as a newborn/infant (e.g., within 24 hours of birth). In other embodiments, the genetic screening is carried out in utero.

### Onset-based classifications of SMA

Severe forms of SMA typically manifest early symptoms, e.g., before six months of age.

This may be referred to as infantile-onset, as opposed to later-onset SMA. When SMA symptoms are present at birth or by the age of 6 months, the disease is also SMA type 1 (also called infantile-onset or Werdnig-Hoffmann disease). Babies typically have generalized muscle weakness, a weak cry, and breathing distress.

Irrespective of genotype-based SMA classifications such as SMN2 gene copy number, the term later-onset SMA is defined by the timing of disease onset (when symptoms appear) unless indicated otherwise. For example, later-onset SMA can be defined as disease with symptom onset after 6 months of age (most likely to be classified as SMA type 2 or type 3).

More recently, with the availability of approved SMN upregulator therapies, such as nusinersen, early intervention has been shown to be particularly effective in delaying the onset of and/or reducing the severity of more severe phenotypes of SMA based on the genotype of the particular patient. For example, some patients who were diagnosed with (or, due to a low copy number of SMN2 genes, were at risk of developing) type 1 SMA, early intervention with SMN upregulator therapy may alter the phenotype to one that is more consistent with later-onset SMA.

In some embodiments, symptom onset-based definition is useful to categorize certain patient populations that receive early intervention which changes the normal course or timeline of disease progression.

### Targeting Myostatin to Improve Muscle Function - Myostatin Inhibition

One therapeutic approach to improve patient motor function is to directly target the skeletal muscle to reduce muscle atrophy and thus improve muscle strength in subjects having muscle conditions, such as SMA. Inhibition of myostatin offers a promising approach to increase muscle mass and function in patients having muscle conditions, such as SMA patients. Myostatin is a member of the TGFβ superfamily and a negative regulator of muscle growth. Genetic loss of myostatin results in significantly increased muscle mass, resulting from both muscle cell hypertrophy and hyperplasia (McPherron et al. (1997) Nature 387:83-90). As with myostatin loss of function mutations, pharmacologic inhibition of myostatin also increases muscle mass, mediated via muscle hypertrophy but not hyperplasia (Lee et al. (2001) Proc Natl Acad Sci USA 98:9306-9311). Additionally, evidence in animal models suggests that blockade of myostatin signaling prevents muscle atrophy associated with limb immobilization, cancer cachexia, and corticosteroid treatment (Latres et al. (2015) Skelet Muscle 5:34; Smith et al. (2015) Mol Cancer Ther 14:1661-1670; Wang et al. (2017) Am J Phys Med Rehabil 96(6):430-437; Zhou et al. (2010) Cell 142:531-543). Since the initial description of the knockout mouse, mutations in myostatin and associated muscle hypertrophy have been identified in cattle, dogs, and humans. Loss of myostatin does not appear to cause any detrimental effects.

The profound effects of myostatin loss on muscle mass, as well as the lack of observed pathology with myostatin mutations, has made this growth factor an important therapeutic target for indications in which muscle wasting is a significant feature, including sarcopenia, cancer cachexia, muscular dystrophy, and disuse atrophy. Multiple companies are pursuing a variety of approaches to inhibit myostatin and therefore increase muscle mass and strength. The most common approaches to myostatin inhibition are (1) antibodies which bind to and inhibit the mature growth factor (commonly referred to as "neutralizing" antibodies), (2) antibodies against the myostatin receptor, ActRIIB, (3) soluble ligand traps such as ActRIIB-Fc, and (4) viral mediated expression of myostatin inhibitors, such as follistatin (Amato et al. (2014) Neurology 83:2239-2246; Becker et al. (2015) Lancet Diabetes Endocrinol 3:948-957; Campbell et al. (2017) Muscle Nerve 55(4):458-464; Mendell et al. (2015) Mol Ther 23:192-201; Wagner et al. (2008) Neurol 63:561-571). However, in addition to targeting myostatin, many of these therapies also inhibit related family members such as GDF-11 and Activins. The amino acid sequences of mature myostatin and GDF-11 are 90% identical, making it difficult to generate antibodies that specifically bind myostatin but not GDF-11. Myostatin, GDF-11, and Activins all signal through ActRIIB; as such, antibodies that block ActRIIB or soluble ActRIIB ligand traps will therefore inhibit activities of all three growth factors (Lee et al. (2005) Proc Natl Acad Sci USA 102:18117-18122). Several of these therapies also bind more distantly related growth factors, such as BMP9 and BMP10, albeit with reduced affinity. This lack of specificity has the potential for unwanted side effects. Together, these observations point to the importance of developing selective inhibitors of myostatin signaling, so as to minimize risk of adverse effects that may be caused by inadvertently inhibiting the signal transduction pathways of one or more of the related growth factors.

Accordingly, disclosed herein are antibodies capable of binding to pro myostatin and/or latent myostatin, thereby inhibiting myostatin activity, and uses thereof for treating diseases and disorders associated with muscle atrophy such as SMA. In some embodiments, given the prevalence of the latent complex in circulation, treatments are provided herein that specifically target more abundant and longer-lived myostatin precursors, e.g., pro myostatin and latent myostatin, rather than the mature growth factor. Without wishing to be bound by any particular theory, antibodies described herein may prevent the proteolytic activation of pro myostatin and/or latent myostatin into mature myostatin which is considered the "active" form of myostatin, capable of activating the myostatin pathway, e.g., by binding Type I (ALK4/5) and Type II (ACTRIIA/B) receptors.

As used herein, the term "antibody" encompasses any naturally-occurring, recombinant, modified or engineered immunoglobulin or immunoglobulin-like structure or antigen-binding fragment or portion thereof, or derivative thereof. Thus, the term refers to an immunoglobulin molecule that specifically binds to a target antigen, and includes, for instance, chimeric, humanized, fully human, and multispecific antibodies (including bispecific antibodies). An intact antibody will generally comprise at least two full-length heavy chains and two full-length light chains, but in some instances can include fewer chains such as antibodies naturally occurring in camelids which can comprise only heavy chains. Antibodies can be derived solely from a single source, or can be "chimeric," that is, different portions of the antibody can be derived from two different antibodies. Antibodies, or antigen binding portions thereof, can be produced in hybridomas, by recombinant DNA techniques, or by enzymatic or chemical cleavage of intact antibodies. The term antibodies, as used herein, includes monoclonal antibodies, multispecific antibodies such as bispecific antibodies, minibodies, domain antibodies, synthetic antibodies (sometimes referred to herein as "antibody mimetics"), chimeric antibodies, humanized antibodies, human antibodies, antibody fusions (sometimes referred to herein as "antibody conjugates"), respectively. In various instances of the therapeutic methods, uses, and compositions disclosed herein, an antibody is a pro/latent myostatin antibody (e.g., apitegromab). *Pro*/*latent myostatin*: As used herein, the term "pro/latent myostatin" refers to pro myostatin, latent myostatin, or both. An anti-pro/latent myostatin antibody may bind specifically to pro myostatin. An anti-pro/latent myostatin antibody may bind specifically to latent myostatin. An anti-pro/latent myostatin antibody may bind specifically to both latent myostatin and pro myostatin. According to the invention, the anti-pro/latent myostatin antibody is apitegromab and binds specifically to both latent myostatin and pro myostatin.

As used herein, the term "mature myostatin" refers to a mature, biologically active form of myostatin. In some embodiments, mature myostatin is capable of myostatin receptor binding and/or activation. Activation and release of mature myostatin *in vivo* from its pro myostatin form is accomplished by several discrete protease cleavage events. To begin with, "pro myostatin" is cleaved by a proprotein convertase, resulting in "latent myostatin," in which the mature myostatin is shielded from binding to its receptors by a portion of the prodomain. Activation and release of mature myostatin is accomplished after cleavage of latent myostatin by an additional protease from the BMP/tolloid family, such as mTLL-2. As used herein, the term "mature myostatin" can refer to both full-length mature myostatin, as well as fragments of the full-length mature myostatin which retain biological activity. So-called neutralizing antibodies that bind mature myostatin thereby interfere with its ability to bind and activate its cellular receptors.

As used herein, the term "proforms of myostatin" refers to inactive (e.g., precursor or latent) forms of the myostatin growth factor that are associated with the N-terminal latency-associated peptide (LAP) domain. Proforms of myostatin are comprised of dimers. The term encompasses both "promyostatin" and "latent myostatin." The term excludes mature growth factor (GDF-8) that is not associated with the LAP domain.

The term "pro myostatin" (or "promyostatin") refers to an inactive precursor of mature myostatin which comprises a disulfide-linked homodimer, each molecule of the homodimer comprising the amino terminal prodomain covalently bound to the carboxyl terminal mature myostatin domain. In some embodiments, "pro myostatin" has not been cleaved by either a proprotein convertase, or a protease from the BMP/tolloid family. Promyostatin and latent myostatin (see below) are proforms of myostatin/GDF-8.

As used herein, the term "latent myostatin" refers to an inactive precursor of mature myostatin which comprises a disulfide-linked homodimer, each molecule of the homodimer comprising the amino terminal prodomain non-covalently bound to the carboxyl terminal mature myostatin domain. In some embodiments, "latent myostatin" is generated from a pro myostatin that has been cleaved by a proprotein convertase, but which has not been cleaved by a protease from the BMP/tolloid family. In some embodiments, "latent myostatin" can be generated by combining the prodomain and the carboxy terminal mature myostatin domain *in vitro* and allowing them to fold properly. See, for example, Sengle et al. (2011) J. Biol. Chem., 286(7):5087-5099. Promyostatin (see above) and latent myostatin are proforms of myostatin/GDF-8.

As used herein, the term "specific binding" or "specifically binds" means that an antibody or antigen binding portion thereof exhibits a particular affinity for a particular structure (e.g., an antigenic determinant or epitope) in an antigen (e.g., a K_{D} measured by Biacore^{®}). In some embodiments, an antibody or antigen binding portion thereof specifically binds to a target, e.g., pro/latent myostatin, if the antibody has a K_{D} for the target of at least about 10⁻⁸ M, 10⁻⁹ M, 10⁻¹⁰ M, 10⁻¹¹ M, 10⁻¹² M, or less. In the context of the present disclosure, "an antibody that specifically binds an antigen with high affinity" generally refers to a K_{D} of 1.0 x 10⁻⁸ M or less. In some embodiments, an antibody or antigen binding portion thereof may also "selectively" (i.e., "preferentially") bind a target antigen if it binds that target with a comparatively greater strength than the strength of binding shown to other antigens, e.g., a 10-fold, 100-fold, 1000-fold, or greater comparative affinity for a target antigen (e.g., pro/latent myostatin) than for a non-target antigen (e.g., mature myostatin (GDF-8), GDF-11, and/or other members of the TGFβ superfamily of growth factors).

In some embodiments, an anti-pro/latent myostatin antibody for use in the therapeutic methods, uses, and compositions disclosed herein inhibits the activation step of myostatin from its precursor. In some embodiments, an anti-pro/latent myostatin antibody for use in the therapeutic methods, uses, and compositions disclosed herein inhibits the activation of latent myostatin. In some embodiments, the anti-pro/latent myostatin antibody comprises the heavy and light chain CDR sequences, the heavy and light chain variable region sequences, and/or the full heavy and light chain sequences set forth in Tables 1-4 below.

**Table 1. Amino acid sequences of Kabat CDRs for an anti-pro/latent myostatin antibody**

| **IgG chain** | **SEQ ID NO** | **Amino acid sequence** |
|---|---|---|
| HCDR1 | 1 | SSYGMH |
| HCDR2 | 2 | VISYDGSNKYYADSVKG |
| HCDR3 | 3 | DLLVRFLEWSHYYGMDV |
| LCDR1 | 4 | SGSSSNIGSNTVH |
| LCDR2 | 5 | SDNQRPS |
| LCDR3 | 6 | AAWDDSLNGV |

**Table 2. Amino acid sequences of IMGT CDRs for an anti-pro/latent myostatin antibody**

| **IgG chain** | **SEQ ID NO** | **Amino acid sequence** |
|---|---|---|
| HCDR1 | 7 | GFTFSSYGMH |
| HCDR2 | 8 | ISYDGSN |
| HCDR3 | 9 | DLLVRFLEWSHYYGMDV |
| LCDR1 | 10 | SSNIGSNT |
| LCDR2 | 11 | SDN |
| LCDR3 | 12 | AAWDDSLNGV |

**Table 3A. Amino acid sequences of variable regions for an anti-pro/latent myostatin antibody**

| **IgG chain** | **SEQ ID NO** | **Amino acid sequence** |
|---|---|---|
| Heavy chain | 13 | QVQLVESGGGVVQPGRSLRLSCAASGFTFSSYGMHWVRQAPGKGLEWVAVIS YDGSNKYYADSVKGRFTISRDNSKNTLYLQMNSLRAEDTAVYYCARDLLVRF LEWSHYYGMDVWGQGTTVTVSS |
| Light chain | 14 | QSVLTQPPSASGTPGQRVTISCSGSSSNIGSNTVHWYQQLPGTAPKLLIYSD NQRPSGVPDRFSGSKSGTSASLAISGLQSEDEADYYCAAWDDSLNGVFGGGT KLTVL |

**Table 3B. Amino acid sequences of full-length Ig chains for an anti-pro/latent myostatin antibody**

| **IgG chain** | **SEQ ID NO** | **Amino acid sequence** |
|---|---|---|
| Heavy chain | 15 | QVQLVESGGGVVQPGRSLRLSCAASGFTFSSYGMHWVRQAPGKGLEWVAVIS YDGSNKYYADSVKGRFTISRDNSKNTLYLQMNSLRAEDTAVYYCARDLLVRF LEWSHYYGMDVWGQGTTVTVSSASTKGPSVFPLAPCSRSTSESTAALGCLVK DYFPEPVTVSWNSGALTSGVHTFPAVLQSSGLYSLSSVVTVPSSSLGTKTYT CNVDHKPSNTKVDKRVESKYGPPCPPCPAPEFLGGPSVFLFPPKPKDTLMIS RTPEVTCVVVDVSQEDPEVQFNWYVDGVEVHNAKTKPREEQFNSTYRVVSVL TVLHQDWLNGKEYKCKVSNKGLPSSIEKTISKAKGQPREPQVYTLPPSQEEM |
| | | TKNQVSLTCLVKGFYPSDIAVEWESNGQPENNYKTTPPVLDSDGSFFLYSRL TVDKSRWQEGNVFSCSVMHEALHNHYTQKSLSLSLG |
| Light chain | 16 | QSVLTQPPSASGTPGQRVTISCSGSSSNIGSNTVHWYQQLPGTAPKLLIYSD NQRPSGVPDRFSGSKSGTSASLAISGLQSEDEADYYCAAWDDSLNGVFGGGT KLTVLGQPKAAPSVTLFPPSSEELQANKATLVCLISDFYPGAVTVAWKADSS PVKAGVETTTPSKQSNNKYAASSYLSLTPEQWKSHRSYSCQVTHEGSTVEKT VAPTECS |

Apitegromab (also referred to as "SRK-015") is an investigational fully human, monoclonal antibody that specifically binds to the proforms of myostatin-namely, promyostatin and latent myostatin-with high affinity. Apitegromab is of the human immunoglobulin G4 (IgG4)/lambda isotype. It comprises the sequences shown above (including the heavy chain amino acid sequence of SEQ ID NO: 15 and the light chain amino acid sequence of SEQ ID NO: 16). International Application No. PCT/US2016/052014 (International Pub. No. WO 2017/049011) and U.S. Application No. 15/760,393 (U.S. Pub. No. US 2018-0344844; U.S. Patent No. 10,751,413) disclose sequences for apitegromab. According to the invention, an anti-pro/latent myostatin antibody for use in the therapeutic methods, uses, and compositions disclosed herein is apitegromab.

In some instances, an anti-pro/latent myostatin antibody or antigen-binding fragment described herein comprises three heavy chain complementarity determining regions (HCDRs) comprising amino acid sequences of SEQ ID NO: 1 (HCDR1), SEQ ID NO: 2 (HCDR2), and SEQ ID NO: 3 (HCDR3); and three light chain complementarity determining regions (LCDRs) comprising amino acid sequences of SEQ ID NO: 4 (LCDR1), SEQ ID NO: 5 (LCDR2), and SEQ ID NO: 6 (LCDR3), as defined by the Kabat numbering system.

In some instances, an anti-pro/latent myostatin antibody or antigen-binding fragment described herein comprises three heavy chain complementarity determining regions (HCDRs) comprising amino acid sequences of SEQ ID NO: 7 (HCDR1), SEQ ID NO: 8 (HCDR2), and SEQ ID NO: 9 (HCDR3); and three light chain complementarity determining regions (LCDRs) comprising amino acid sequences of SEQ ID NO: 10 (LCDR1), SEQ ID NO: 11 (LCDR2), and SEQ ID NO: 12 (LCDR3), as defined by the IMGT numbering system.

In various instances, the anti-pro/latent myostatin antibody or antigen-binding fragment comprises a heavy chain variable region comprising an amino acid sequence of SEQ ID NO: 13, and/or a light chain variable region comprising an amino acid sequence of SEQ ID NO: 14.

In various instances, the anti-pro/latent myostatin antibody or antigen-binding fragment comprises a heavy chain amino acid sequence of SEQ ID NO: 15, and/or a light chain amino acid sequence of SEQ ID NO: 16.

Apitegromab binds the "arm" region within the prodomain of the pro/latent myostatin complex and inhibits release of mature growth factor (i.e., GDF-8) from the latent/inactive complex. Binding of apitegromab is specific to pro/latent myostatin and therefore apitegromab does not bind mature GDF-8 or GDF-11 (or any other members of the TGFβ superfamily of growth factors), thereby enabling selective targeting of myostatin signaling, without affecting other biological pathways.

Inhibition of myostatin activation by apitegromab may lead to buildup of promyostatin and latent myostatin. Since promyostatin is predominantly found within the skeletal muscle and latent myostatin is predominantly found in the serum, in some embodiments, serum latent myostatin levels may serve as a target engagement marker for apitegromab, e.g., target engagement with apitegromab in skeletal muscle. In a similar manner, serum latent myostatin levels may serve as a target engagement marker for other pro/latent myostatin antibodies such as GYM329 in an analogous manner. In some embodiments, serum latent myostatin levels may be relatively low at baseline (i.e., prior to treatment) and increase following treatment with apitegromab. In some embodiments, an increase in serum latent myostatin levels indicates target engagement with apitegromab, e.g., target engagement with apitegromab in skeletal muscle. In some embodiments, low baseline serum levels of latent myostatin as compared to high levels following treatment indicates that a major portion of the drug target resides within skeletal muscle, rather than circulating systemically. In some embodiments, saturation in target engagement indicates a dosage sufficient to achieve certain therapeutic effects (e.g., efficacy), although it may be possible to obtain efficacy without saturation.

In some embodiments, a composition comprising apitegromab is used in the treatment of SMA in a patient 2 months of age or older, wherein optionally the treatment further comprises risdiplam (EVRYSDI^{®}). In some embodiments, apitegromab and risdiplam are administered to the patient as a combination therapy. In some embodiments, apitegromab is administered as an add-on or adjunct therapy to the patient who has been treated with (i.e., who has received) risdiplam. In some embodiments, risdiplam is administered as an add-on or adjunct therapy to the patient who has been treated with (i.e., who has received) apitegromab. In some embodiments, the patient is 2 to 7 months of age, wherein optionally the patient has or is suspected to have type 1 SMA. In some embodiments, the patient is 2 to 25 years of age, wherein optionally the patient has type 2 or type 3 SMA. In some embodiments, the patient is any age (e.g., infant, child, or adult) and has type 1, type 2 or type 3 SMA.

### SMN Therapies

Multiple therapeutic approaches to restore SMN protein levels have been investigated, including SMN1 gene replacement therapy, small molecules which modulate SMN2 splicing, and the use of antisense oligonucleotides (ASO) to block an SMN2 intronic splicing silencer, thus increasing exon 7 inclusion.

SMN1 gene replacement therapy using adeno-associated viral vectors (AAV) has shown benefit in mouse models of SMA and AVXS-101, an AAV9-SMN1 vector from AveXis, has been evaluated in Phase I clinical trials (see NCT02122952) (Passini et al. (2010) J Clin Invest 120:1253-1264).

Other approaches focus on modulating SMN2 splicing, such that exon 7 is retained in a greater percentage of transcripts, leading to increased production of full-length SMN protein. Novartis and PTC Therapeutics/Roche have both developed small molecules which selectively enhance SMN2 exon 7 inclusion, resulting in increased full-length SMN protein levels and therapeutic efficacy in mouse models of SMA (Calder et al. (2016) J Med Chem 59:10067-10083; Naryshkin et al. (2014) Science 345:688-693; Palacino et al. (2015) Nat Chem Biol 11:511-517; Ratni et al. (2016) J Med Chem 59:6086-6100). These small molecules from both companies have been evaluated in Phase 2 clinical trials (see trials NCT02913482, NCT03032172, NCT02908685, NCT022688552). Oral dosing of RG7800, SMN-C2, and SMN-C3 in mild and severe preclinical models of SMA showed that the compounds increased SMN protein levels in both brain and muscle tissues in treated mice, as compared to vehicle. The molecules also efficiently crossed the blood brain barrier (BBB). In the severe SMA mouse model, both compounds normalized motor behavior and increased weight and survival compared with vehicle. However, the clinical program was put on hold due to safety concerns.

Additional small molecule-based SMN2 splice correctors are described in, for example, U.S. Patent Publication No. US 2009/0031435; and U.S. Patent No. 8,399,437. It should be appreciated, however, that other small molecule splice correctors including SMN2 splice correctors known in the art, e.g., risdiplam, and would be apparent to the skilled artisan, are within the scope of this disclosure.

A third approach is the use of antisense oligonucleotides (ASO), for example, to block an SMN2 intronic splicing silencer, thus increasing exon 7 inclusion, again rescuing disease in mouse models of SMA (Hua et al. (2010) Genes Dev 24:1634-1644; Hua et al. (2011) Nature 478:123-126; Passini et al. (2011) Sci Transl Med 3:72ra18). Biogen/lonis have developed nusinersen, an ASO splice modifier which shows clinical efficacy and was approved by the FDA and marketed as Spinraza^{™} (Chiriboga et al. (2016) Neurology 86:890-897; Finkel et al. (2016) Lancet 388:3017-3026; FDA, Nusinersen; Office of drug evaluation decisional memorandum (2016)). However, each administration of nusinersen requires intrathecal delivery under general anesthesia. Additionally, while the antisense corrector nusinersen has proven promising, clinical efficacy appears modest: 60% of patients among infantile-onset SMA (type I) are reported to be non-responders, and 43% of nusinersen-treated patients did not attain ≥ 3 point increase by Hammersmith Functional Motor Scale (Expanded) (HFMSE), while mean increase was less than 6 points in the treated patient group, as compared to placebo. Thus, only a partial improvement is achieved by nusinersen treatment.

While these molecules have all shown significant efficacy preclinically, and nusinersen clinically, none offers a complete cure for the disease. In mouse models, while both the small molecule and ASO splice modifiers significantly reduce disease severity, treated animals nevertheless have deficits in longevity, body weight, muscle mass, and muscle function compared to healthy animals (Hua et al. (2011) Nature 478:123-126; Feng et al. (2016) Hum Mol Genet 25:964-975). In a double-blinded clinical trial in infantile-onset SMA, nusinersen resulted in clinically meaningful benefits at the time of interim analysis (41% of treated versus 0% placebo had improvements in motor milestones using the Hammersmith Infant Neurological Examination). The motor function milestones reached are impressive for type I patients, with five of 81 treated patients able to sit unaided (a milestone almost never reached in these patients). Nevertheless, these patients have not achieved the full range of developmental milestones, and in a normal individual the achieved milestones would be considered disappointing. In a second placebo controlled trial in type 2 SMA, nusinersen again showed clinically meaningful improvements, with scores on the Hammersmith Functional Motor Scale - Expanded (HFMSE) increasing by 5.9 points relative to the placebo group. Note that the maximum score on the HFMSE is 66 points, with most type 2 patients scoring below 20 (Glanzman et al. (2011) J Child Neurol 26:1499-1507; Kaufmann et al. (2011) Arch Neurol 68:779-786). Nevertheless, 43% of patients failed to achieve at least a 3-point improvement in motor function in this trial. These results indicate that, while SMN2 splice modulators have potential for significant effects on SMA disease course patient quality of life, additional functional gains are needed to further improve reduce disease burden.

As used herein, the term "motor neuron-directed therapy" refers to an agent aimed to improve (e.g., enhance or restore) neuronal function. Such therapies are useful for treating conditions that involve impaired signaling between a motor neuron and its target muscle. Specifically, motor neuron-directed therapies may be particularly useful in the treatment of conditions involving partial but not complete loss of neurons that innervate muscle. In some embodiments, a motor neuron-directed therapy is a gene therapy, a small molecule, or an antisense oligonucleotide. In some embodiments, a motor neuron-directed therapy is a "SMN upregulator." Exemplary motor neuron-directed therapies (e.g., SMN upregulators) suitable for use in conjunction with apitegromab according to the present disclosure include but are not limited to: nusinersen; risdiplam; and onasemnogene abeparvovec. In some embodiments, a motor neuron-directed therapy is an agent that is capable of fully restoring motor neuron function in a cell (e.g., a cell within a subject). In some embodiments, a motor neuron-directed therapy is an agent that is capable of partially restoring motor neuron function in a cell (e.g., a cell within a subject). In some embodiments, a motor neuron-directed therapy is an agent that is capable of restoring at least 10%, 15%, 20%, 25%, 30%, 35%, 40%, 45%, 50%, 55%, 60%, 65%, 70%, 75%, 80%, 85%, 90%, 95%, 99%, or more of the motor neuron function in a cell (e.g., a cell within a subject). A skilled artisan will understand that motor neuron function typically includes membrane excitability, axonal transport, vesicle trafficking, neurotransmitter release, mitochondrial function, and/or mitochondrial availability, and such functions are measured using assays known to those of ordinary skill in the art.

As used herein, the term "SMN upregulator" or "SMN upregulator therapy" (which may also be referred to as an SMN therapy, SMN-directed therapy, an SMN corrector, an SMN enhancing therapy, or an SMN enhancer interchangeably) refers to any therapy or compound which can be used to increase or improve SMN gene expression (e.g., SMN1 gene expression and/or SMN2 gene expression), SMN protein production, and/or functional SMN activity. An SMN upregulator may be a central corrector or a systemic corrector. A central upregulator may be administered directly to the central nervous system (CNS) via the intrathecal route. In contrast, a systemic upregulator may be administered via any route, e.g., orally administered, and affects not only the CNS, but other tissues throughout the body. SMN upregulators, for example, include splice correctors/modifiers that alter the splicing of SMN2 transcripts. Systemically delivered SMN splice modifiers may also affect SMN splicing in other (i.e., non-neuronal) tissues, where SMN is expressed.

In some embodiments, a "functional SMN protein" is capable of promoting motor neuron function and/or survival. In some embodiments, a "functional SMN protein" is capable of fully restoring motor neuron function in a cell (e.g., a cell within a subject). In some embodiments, a functional SMN protein is capable of partially restoring motor neuron function in a cell (e.g., a cell within a subject). In some embodiments, a functional SMN protein is capable of restoring at least 10%, 15%, 20%, 25%, 30%, 35%, 40%, 45%, 50%, 55%, 60%, 65%, 70%, 75%, 80%, 85%, 90%, 95%, 99%, or more of the motor neuron function in a cell (e.g., a cell within a subject). In some embodiments, full-length SMN protein is the result of protein translation (e.g., in a cell) of a correctly spliced SMN mRNA. In some embodiments, a functional SMN protein is encoded from SMN2 mRNA that contains exon 7.

Examples of suitable "SMN upregulators" include splice modifiers, SMN gene replacement or gene therapy, SMN transcription enhancers, SMN protein translation enhancers, and SMN protein stabilizers. In some embodiments, such SMN upregulators may be small molecule agents, biologics, or nucleic acids. In some embodiments, the SMN upregulator is a small molecule splice modifier of SMN2. In some embodiments, the SMN upregulator is an antisense RNA splice modifier of SMN2. In some embodiments, the gene therapy comprises introduction of one or more transgenes into the patient. In some embodiments, gene transfer is achieved by the use of a suitable vector, such as viral vectors and lipid-based carriers. For viral-vector-mediated gene delivery, the gene therapy may involve the use of a particular serotype for an initial treatment, followed by a different serotype for the subsequent treatment, in order to minimize adverse immune responses in the subject. In some embodiments, the gene therapy involves targeted genome editing, such as the CRISPR/Cas9 technology or variant thereof. Non-limiting examples of SMN upregulators suitable for use in conjunction with apitegromab according to the present disclosure include but are not limited to: nusinersen; risdiplam; and onasemnogene abeparvovec. Nusinersen is an SMN2-directed antisense oligonucleotide (ASO) designed to treat SMA caused by mutations that lead to SMN protein deficiency. *See, e.g.,* Darras et.al. (2019) Neurology. 92(21) e2492-e2506; Mercuri et.al. (2018) N Engl J Med. 378:625-635. Risdiplam works in a similar way and is a pyridazine derivative that modifies the splicing of SMN2 messenger RNA. *See, e.g.,* Oskoui et al. "SUNFISH Part 2: 24-month efficacy and safety of risdiplam in patients with type 2 or non-ambulant type 3 spinal muscular atrophy (SMA)." Presented at MDA Clinical and Scientific Conference 2021; March 15-18. Poster 80. Onasemnogene abeparvovec is a recombinant adeno-associated virus vector 9-based gene therapy designed to deliver a copy of the gene encoding the human SMN protein.

In some embodiments, an SMN upregulator is a gene therapy, a small molecule, or an antisense oligonucleotide. In some embodiments, an SMN upregulator is an oligonucleotide molecule. In some embodiments, an SMN upregulator is an antisense molecule. In some embodiments, an SMN upregulator is an antisense molecule that increases expression of an SMN2 gene. In some embodiments, an SMN upregulator is an antisense molecule that increases expression of an SMN2 mRNA that contains exon 7. In some embodiments, an SMN upregulator is an antisense molecule that increases expression of functional SMN protein, for example, SMN protein encoded by SMN2 mRNA that contains exon 7. For example, antisense oligonucleotides directed to inhibit an intronic splice silencer site (ISS) in intron 7 of the SMN2 gene can modulate pre-mRNA processing, leading to a greater probability of exon 7 inclusion within the mature mRNA transcript of SMN2, resulting in the increased production of functional SMN protein.

The terms "splice corrector," "splice modulator," and "splice modifier," as used herein, are interchangeable and refer to an agent that corrects aberrant splicing of RNA transcripts, such as those encoded by the SMN2 gene and/or modulates expression of an SMN protein. In some embodiments, SMN2 splice correctors increase the inclusion of exon 7 in the SMN2 pre-mRNA. In some embodiments, increased inclusion of exon 7 in the SMN2 pre-mRNA leads to increased expression of a functional SMN protein (e.g., from an SMN2 gene) in a cell or subject, such as an SMN protein that is capable of promoting neuron function and/or survival.

In some embodiments, an SMN upregulator is a gene therapy. As used herein, the term "gene therapy" refers to any procedure that uses nucleic acids to heal, cure, or otherwise improve a condition in a subject. In gene therapy, nucleic acids are delivered into specific cells. Delivery methods include viral and non-viral means, which are known in the art. See, for example, Patil et al. (2005) AAPS J. 7(1): E61-E77; Gascon et al., Non-Viral Delivery Systems in Gene Therapy (2013); Somiari et al. (2000) Molecular Therapy, 2(3):178-187; Herweijer and Wolff (2003) Gene Therapy 10(6):453-458; Nayerossadat et al. (2012) Advanced Biomedical Research 1(2):1-11. Viral means for delivering gene therapy involve the use of viral vectors. Viral vectors are genetically modified viruses that can carry a therapeutic genetic payload and have been reprogrammed to allow for infection and subsequent transmittal of said payload into specific tissues without the side effects typically associated with wild-type viral infection. A number of viruses can be used as viral vectors, including retroviruses, adenoviruses, herpes simplex virus, lentiviruses, Poxvirus, and Epstein-Barr virus. While safer than wild-type viruses, viral vectors may induce an immune response, occasionally necessitating the use of non-viral delivery methods. In some embodiments, the viral vector is an AAV viral vector. Non-viral delivery methods include, but are not limited to, physical methods, such as injection of naked DNA, electroporation, gene gun bombardment, and ultrasound, as well as biochemical methods. Another delivery technique, magnetofection, combines physical and biochemical elements.

In some embodiments, an "effective amount" of an SMN upregulator is an amount of an agent that is capable of fully restoring motor neuron function in a cell (e.g., a cell within a subject). In some embodiments, an SMN upregulator is an agent that is capable of partially restoring motor neuron function in a cell (e.g., a cell within a subject). In some embodiments, an "effective amount" of an SMN upregulator is an amount of an agent that is capable of restoring at least 10%, 15%, 20%, 25%, 30%, 35%, 40%, 45%, 50%, 55%, 60%, 65%, 70%, 75%, 80%, 85%, 90%, 95%, 99%, or more of the motor neuron function in a cell (e.g., a cell within a subject). In some embodiments, motor neuron function includes membrane excitability, axonal transport, vesicle trafficking, neurotransmitter release, mitochondrial function, and/or mitochondrial availability.

In some embodiments, an SMN upregulator is an agent, for example, a small molecule or an oligonucleotide (e.g., an antisense oligonucleotide), that increases expression of a functional SMN protein, for example, by promoting the inclusion of exon 7 in an SMN2 mRNA transcript. In some embodiments, the cell is a cell within a subject, for example, a subject that is administered an SMN upregulator. In some embodiments, an SMN upregulator increases the relative amount of SMN2 mRNA that includes exon 7 as compared to SMN2 mRNA that does not include exon 7 in a cell, for example, a cell in a subject. In some embodiments, an "effective amount" of an SMN upregulator increases the amount of correctly spliced SMN2 mRNA in a cell (e.g., a cell within a subject), such that at least 10%, 15%, 20%, 25%, 30%, 35%, 40%, 45%, 50%, 55%, 60%, 65%, 70%, 75%, 80%, 85%, 90%, 95%, 99%, or more of the SMN2 mRNA within a cell contains exon 7. In some embodiments, an "effective amount" of an SMN upregulator increases a level of SMN2 mRNA containing exon 7 in a subject by at least 5%, 10%, 20%, 30%, 40%, 50%, 60%, 70%, 80%, 90%, 100%, 150%, 200%, 400%, 500%, 600%, 700%, 800%, 900%, 1000%, or more. In some embodiments, an "effective amount" of an SMN upregulator increases a level of functional SMN protein in a subject by at least 5%, 10%, 20%, 30%, 40%, 50%, 60%, 70%, 80%, 90%, 100%, 150%, 200%, 400%, 500%, 600%, 700%, 800%, 900%, 1000%, or more.

### Clinical Benefits of SMN Correctors

Among non-ambulatory later-onset SMA patients who are 5 years or older, based on natural history of this patient population, mean HFMSE scores are expected to decline over a 12-month period, with few patients (e.g., less than 5%) showing a 3-point or greater increase in HFMSE score (Mercuri et al. (2016) Neuromuscul Disord. 26(2):126-131). Against this background, nusinersen has been reported to provide limited clinical benefits in patients who started the therapy at age 5 or older. More specifically, in the CHERISH study, in patients who started on nusinersen at age 5 or older, there was a mean HFMSE decline of >0.5 points after 15 months of nusinersen treatment, and <15% of the patients showed a 3-point or greater increase in HFMSE score over that period (Mercuri et.al. (2018) N Engl J Med. 378:625-635, e.g., at Figure 2A). These patients were further observed beyond the 15 months in the earlier study. Long-term treatment with nusinersen appears to provide stabilization of the disease, e.g., prevention of further loss of motor function. The majority of patients in this age range do not experience HFMSE improvements and rarely achieve a 3+ point increase.

Among non-ambulatory later-onset SMA patients with early (prior to 5 years of age) initiation of nusinersen, published data suggest that nusinersen-treated patients primarily stabilize or experience modest and gradual improvements beyond the initial 15 months of therapy. Most nusinersen-treated patients in the CHERISH study were under 5 years of age at the time of therapy initiation. In the CHERISH study, the patients who received nusinersen experienced a mean HFMSE improvement of almost 4 points in the first 15 months of treatment. Beyond this initial 15 months of nusinersen treatment in CHERISH, when patients rolled over into the long-term SHINE study, these patients primarily had motor function stabilization or only a modest and gradual mean improvement during the long-term phase of nusinersen treatment. Patients showed approximately a 4.6 point increase after ~4.5 years of treatment (see FIG. 8, which is reproduced from Williams et al). Minimal clinically important differences of the Expanded Hammersmith Functional Motor Scale in later-onset spinal muscular atrophy: results from the phase 3 CHERISH trial. Presented at: 31st Annual Meeting of the American Academy of Managed Care Pharmacy; 25-28 March 2019; San Diego, CA, USA).

Natural history data from a longitudinal study in patients with ambulatory type 3 SMA offer additional background insights into this patient population. These data indicate that patients with ambulatory type 3 SMA commonly experience motor function declines, which in some cases are severe (e.g., loss of ambulation) (Coratti et al. (2020) American Neurology Association, 88:1109-1117, e.g., at Figure 1). In the study of 130 patients with ambulatory type 3 SMA (with some patients lost to follow-up over time), the mean age of 10.05 and a mean HFMSE score of 52.81 at baseline. At the 12-month assessment, the mean change in HFMSE from baseline was -0.79 points and 11 patients had lost ambulation (mean age of ambulation loss was 10.21 years (SD±6.43)). Relative stability with modest functional improvement was observed in patients until 7 years of age, followed by a steeper decline in subsequent years.

Similarly, long term evaluation of risdiplam, another SMN corrector, demonstrated therapeutic benefit but only stabilization or minor/variable improvement after 12 months in the primary and secondary endpoints (Oskoui et al. "SUNFISH Part 2: 24-month efficacy and safety of risdiplam in patients with type 2 or non-ambulant type 3 spinal muscular atrophy (SMA)." Presented at MDA Clinical and Scientific Conference 2021; March 15-18. Poster 80).

### Add-On/Adjunct Therapy, Combination Therapy

The present disclosure includes add-on therapy (also referred to as adjunct therapy) and combination therapy that comprise a first agent which is a motor neuron-directed therapy (e.g., SMN therapy) and a second agent which is a muscle-directed therapy (e.g., myostatin inhibitor, e.g., apitegromab) aimed to treat SMA in patients. The two agents are used in conjunction with each other to enhance therapeutic effects. Add-on or adjunct therapy means that therapy is administered to a patient who is on a background therapy. For example, the muscle-directed therapy such as apitegromab may be administered to SMA patients who are already receiving an SMN therapy. In comparison, a combination therapy refers to the administration of both therapies to a patient as part of a coordinated or predetermined therapeutic regimen by a physician or a team of physicians. Combinations and add-on therapy may be administered separately or in the same setting, e.g., during the same clinical visit.

In various embodiments, the present disclosure provides use of an anti-pro/latent myostatin antibody (i.e., apitegromab) for the treatment of SMA in patients who also receive a therapy to address the motor neuron defect, such as a motor neuron-directed therapy, e.g., an SMN upregulator. The present disclosure encompasses methods for treating SMA in a subject who is treated with an SMN upregulator, comprising administration of an anti-pro/latent myostatin antibody. According to the invention, the anti-pro/latent myostatin antibody is apitegromab.

In some embodiments, apitegromab used in conjunction with an SMN therapy provides additive clinical benefits. In some embodiments, apitegromab used in conjunction with an SMN therapy provides synergistic clinical benefits. "Synergistic" means that when the two agents are used in conjunction with each other, either as add-on therapy or combination therapy, they together achieve efficacy that is greater than a sum of effects obtained by monotherapy of each.

Apitegromab may be administered to SMA patients who are either responders, poor responders, or non-responders of an SMN upregulator therapy. For poor responders or non-responders, concurrent inhibition of myostatin signaling may improve neuromuscular signaling due in part to enhanced muscle function, thereby rendering the innervating motor neurons of non-responders more responsive to the SMN upregulator. Without wishing to be bound by particular theory, it is contemplated that enhancement of the muscle component may affect the neuronal component by positive feedback, and vice versa, due to the bidirectional nature of neuromuscular signaling.

Although SMN upregulator poor responders and/or non-responders may nevertheless benefit from myostatin inhibition, an exemplary patient population includes those who are responders of an SMN upregulator. It is contemplated that motor function in these individuals may be further improved by a myostatin inhibition therapy such as apitegromab used in conjunction with a motor neuron-directed therapy, such as an SMN upregulator therapy.

The phrase "in conjunction with," in the context of treatment regimens for SMA that comprise two or more therapeutic agents (e.g., apitegromab used in conjunction with an SMN upregulator therapy), means that therapeutic effects of a first therapy overlaps temporally and/or spatially with therapeutic effects of a second and/or additional therapy in the subject receiving the two or more therapies. The two or more therapies need not be administered as a single formulation, nor do they have to be administered concurrently, nor via the same route. The first, second, and/or additional compositions may be administered concurrently (e.g., simultaneously), separately, or sequentially. Thus, the two or more therapies may be formulated as a single formulation for concurrent administration, or as separate formulations, for sequential, concurrent, or simultaneous administration of the therapies. When a subject who has been treated with a first therapy to treat SMA (e.g., apitegromab) is administered with a second and additional therapies to treat the SMA (e.g., an SMN upregulator therapy), the second and additional therapies may be referred to as an "add-on" therapy or an "adjunct" or "adjunctive" therapy.

In some embodiments, the treatment regimens described herein that comprise two or more therapeutic agents (e.g., apitegromab used in conjunction with an SMN-directed therapy such as an upregulator therapy) achieve improved clinical benefits in patients as compared to a monotherapy using each agent alone. Specifically, targeting affected muscles with a myostatin inhibition therapy such as apitegromab, in conjunction with a motor neuron-directed therapy such as an SMN upregulator therapy, may produce a beneficial clinical outcome relative to the myostatin inhibition therapy or motor neuron-directed therapy alone. Such effects may be additive or synergistic as compared to the respective monotherapies. In some embodiments, the effects of combining the two or more therapies are additive, i.e., the effects of the therapies used together are equal or about equal to the sum of the effects of the therapies when used independently. In some embodiments, the effects of combining the two or more therapies are synergistic, i.e., super-additive, i.e., the effects of the therapies used together are greater than the sum of the effects of the therapies when used independently.

In some embodiments, one or more beneficial therapeutic effects of using apitegromab in conjunction with an SMN upregulator therapy (e.g., an effect on at least one symptom or the risk/rate of disease progression) is/are additive. In some embodiments, one or more beneficial therapeutic effects of using apitegromab in conjunction with an SMN upregulator therapy (e.g., an effect on at least one symptom or the risk/rate of disease progression) is/are synergistic. In some embodiments, apitegromab used in conjunction with an SMN upregulator therapy has a combined effect that is additive, synergistic, and/or provides one or more additional combination benefits. In some embodiments, apitegromab used in conjunction with an SMN upregulator therapy provides additive or synergistic effects on at least one efficacy parameter for SMA (e.g., an HFMSE score). In some embodiments, apitegromab used in conjunction with an SMN upregulator therapy provides additive or synergistic effects on an HFMSE score in a SMA patient and/or an SMA patient population. In some embodiments, apitegromab used in conjunction with an SMN upregulator therapy provides further increases in HFMSE score, as compared to treatment with an SMN upregulator therapy alone (see, e.g., Example 1; see also FIG. 8 and Darras et.al. (2019) Neurology. 92(21) e2492-e2506). In some embodiments, the further improvements in HFMSE score are additive. In some embodiments, the further improvements in HFMSE score are synergistic.

### Patient Selection

The disclosure includes identification or selection of suitable human subjects to be treated with a selective myostatin inhibitor, such as apitegromab. Suitable human subjects are patients who are likely to benefit from apitegromab therapy, either as monotherapy or in conjunction with (or in combination with) another therapy.

SMA patients who are likely to benefit from such therapy include those who meet one or more of the following criteria: has a documented diagnosis of 5q SMA and later-onset (e.g., type 2 or 3) SMA prior to receiving a therapy for SMA; a non-ambulatory subject who is able to sit independently per WHO motor milestones definition; an ambulatory subject who is able to independently ambulate without aids over 10 meters in 30 seconds or less; a subject having a Revised Hammersmith Scale (RHS) score of less than or equal to 63 and/or a Hammersmith Functional Motor Scale Expanded score of 10 or greater; a subject who does not use tracheostomy with positive pressure or chronic daytime non-invasive ventilatory support for greater than 16 hours daily within two weeks priorto treatment; a subject who does not have any acute or comorbid condition interfering with the well-being of the subject within two weeks prior to treatment; a subject who does not have severe scoliosis or contractures; and/or subject who does not use systemic corticosteroids, valproic acid, or therapies with potential muscular or neuromuscular effects within 60 days except approved SMN up-regulator (also known as SMN corrector) therapy. Therapies with potential muscular or neuromuscular effects include androgens, insulin-like growth factor, growth hormone, systemic beta-agonist, botulinum toxin, muscle relaxants, muscle enhancing supplements or acetylcholinesterase inhibitors. In some embodiments, the patient has a documented diagnosis of 5q SMA and later-onset (e.g., type 2 or 3) SMA prior to receiving a therapy for SMA and meets one or more of the additional criteria listed above. In some embodiments, there is a positive correlation between age-normalized change in motor function and age-normalized fold change in latent myostatin levels, e.g., in patients with type 2 SMA. In various embodiments, the methods disclosed herein include the selection of such a patient or patient population(s) for treatment with a myostatin inhibitor (i.e., apitegromab), e.g., according to a dosage or regimen disclosed herein.

SMA patients who may be particularly responsive to apitegromab therapy, either as monotherapy or in conjunction with (or in combination with) another therapy, include those having any type of SMA up to the age of two years, as well as those who show a phenotype of SMA type 1 or have up to three SMN2 copies. In some embodiments, apitegromab may have enhanced therapeutic efficacy in younger patients, e.g., a population who is less than 21 years of age, because the patients have greater background anabolic activity. In some embodiments, younger patients, e.g., patients less than 21 years of age, or patients who are anabolically active, receive the apitegromab therapy. In some embodiments, the patient is aged 2 years or younger, e.g., newborn to 24 months old. In some embodiments, the patient is six weeks or younger, e.g., newborn to six weeks old. In some embodiments, the patient is 2-21 years old. In some embodiments, the patient is 13-21 years old. In some embodiments, the patient is aged 12 years or younger. In some embodiments, the patient is 2-12 years old. In some embodiments, the patient is 5-12 years old. In some embodiments, apitegromab may be particularly efficacious in patients before the start of puberty (e.g., before age 12). In some embodiments, apitegromab treatment may be efficacious in preventing drastic decline in motor function associated with the start of puberty in younger patients (e.g., patients younger than 12 years old). In some embodiments, the patient is about two years old. In some embodiments, the patient is younger than two years old. In some embodiments, the patient shows a phenotype of SMA type 1. In some embodiments, the patient has up to three SMN2 copies. In various embodiments, the methods disclosed herein include the selection of such a patient or patient population(s) for treatment with a myostatin inhibitor, such as apitegromab, e.g., according to a dosage or regimen disclosed herein.

Patients who may be particularly responsive to apitegromab therapy, either as monotherapy or in conjunction with (or in combination with) another therapy, include patients having type 2 SMA with baseline (i.e., prior to the start of apitegromab therapy) serum latent myostatin (LM) concentrations of at least 1 ng/mL (e.g., greater than 1.5 ng/ml, greater than 2 ng/ml, greater than 2.5 ng/ml, greater than 3 ng/ml, or greater). In some embodiments, an apitegromab therapy, either as monotherapy or in conjunction with (or in combination with) another therapy, may be more effective in patients with higher baseline serum LM concentrations as compared to patients with lower baseline serum LM concentrations. In some embodiments, such therapy may be particularly effective in patients who are younger than 12 years of age. In some embodiments, such therapy may be particularly effective in patients who are younger than 2 years of age. In some embodiments, type 2 SMA patients who are younger than 12 years of age and have baseline serum LM concentrations of at least 1 ng/mL (e.g., greater than 1.5 ng/ml, greater than 2 ng/ml, greater than 2.5 ng/ml, greater than 3 ng/ml, or greater) may be particularly responsive to apitegromab therapy. In some embodiments, type 2 SMA patients who are younger than 2 years of age and have baseline serum LM concentrations of at least 1 ng/mL (e.g., greater than 1.5 ng/ml, greater than 2 ng/ml, greater than 2.5 ng/ml, greater than 3 ng/ml, or greater) may be particularly responsive to apitegromab therapy. According to the invention, the apitegromab therapy comprises administering 10 mg/kg apitegromab.

In some embodiments, the treatment of SMA comprises administering an apitegromab therapy, either as a monotherapy or in conjunction with (or in combination with) another therapy, to a patient having baseline serum LM concentrations of at least 1 ng/mL (e.g., greater than 1.5 ng/ml, greater than 2 ng/ml, greater than 2.5 ng/ml, greater than 3 ng/ml, or greater). In some embodiments, the treatment of SMA comprises administering an apitegromab therapy, either as a monotherapy or in conjunction with (or in combination with) another therapy, to a patient who is younger than 12 years of age and who has baseline serum LM concentrations of at least 1 ng/mL (e.g., greater than 1.5 ng/ml, greater than 2 ng/ml, greater than 2.5 ng/ml, greater than 3 ng/ml, or greater). In some embodiments, the treatment of SMA comprises administering an apitegromab therapy, either as a monotherapy or in conjunction with (or in combination with) another therapy, to a patient who is younger than 2 years of age and who has baseline serum LM concentrations of at least 1 ng/mL (e.g., greater than 1.5 ng/ml, greater than 2 ng/ml, greater than 2.5 ng/ml, greater than 3 ng/ml, or greater). According to the invention, the apitegromab therapy comprises administering 10 mg/kg apitegromab.

In some embodiments, apitegromab is used for treating SMA, either as a monotherapy or in conjunction with (or in combination with) another therapy, in a patient having baseline serum LM concentrations of at least 1 ng/mL (e.g., greater than 1.5 ng/ml, greater than 2 ng/ml, greater than 2.5 ng/ml, greater than 3 ng/ml, or greater). In some embodiments, apitegromab is used for treating SMA, either as a monotherapy or in conjunction with (or in combination with) another therapy, in a patient younger than 12 years of age having baseline serum LM concentrations of at least 1 ng/mL (e.g., greater than 1.5 ng/ml, greater than 2 ng/ml, greater than 2.5 ng/ml, greater than 3 ng/ml, or greater). In some embodiments, apitegromab is used for treating SMA, either as a monotherapy or in conjunction with (or in combination with) another therapy, in a patient younger than 2 years of age having baseline serum LM concentrations of at least 1 ng/mL (e.g., greater than 1.5 ng/ml, greater than 2 ng/ml, greater than 2.5 ng/ml, greater than 3 ng/ml, or greater). According to the invention, the apitegromab is suitable for administering at 10 mg/kg apitegromab.

In various embodiments, an SMA patient (e.g., any of the exemplary SMA patients described above) is on (is receiving) or has been treated with an SMN-directed therapy. In some embodiments, the SMN-directed therapy comprises nusinersen and/or onasemnogene abeparvovec. In some embodiments, the patient is on (is receiving) or has been treated with nusinersen. In some embodiments, the patient has been previously treated with nusinersen. In some embodiments, the patient has previously received onasemnogene abeparvovec.

In various embodiments, the present disclosure provides a composition comprising apitegromab for use in methods of treating a patient or patient population(s), alone or in conjunction with (or in combination with) a SMN-directed therapy, wherein the patient or patient population(s) meets one or more of the following criteria: has any type of SMA before or up to the age of two years, shows a phenotype of SMA type 1, and/or has up to three SMN2 copies. In some embodiments, treatment comprises apitegromab therapy as monotherapy, e.g., according to a dosage or regimen disclosed herein. In some embodiments, treatment comprises apitegromab therapy in conjunction with (or in combination with) a SMN-directed therapy, such nusinersen or onasemnogene abeparvovec, e.g., according to a dosage or regimen disclosed herein. In some embodiments, the patient is on (is receiving) or has been treated with nusinersen. In some embodiments, the patient has been previously treated with nusinersen. In some embodiments, the patient has previously received onasemnogene abeparvovec.

In some embodiments, the patient is symptomatic or pre-symptomatic.

Pre-symptomatic patients include those who have been genetically identified as carriers of one or more mutations in the SMN1 gene, alone or in combination with identification of one or more additional genetic modifications (e.g., a determination of SMN2 copy number). Genetic identification of SMA patients may be carried out as part of newborn screening. At the time of the identification (e.g., diagnosis), the patient (e.g., newborn) may not show obvious/apparent symptoms (asymptomatic), but based on the genetic characteristics and possibly other factors, the patient may be expected to develop the disease.

In severe cases, newborn babies may already show sign of the disease (i.e., symptomatic). Typically, such a patient is likely to have type 1 SMA based on the natural history of SMA and is not generally expected to gain the ability to sit independently without intervention.

In some embodiments, the patient is a newborn patient (age of zero to six months). In some embodiments, the patient is a pediatric patient, between the age of six months and 17 years. In some embodiments, the patient is younger than 5 years old. In some, the patient is 5 years old or younger, e.g., 2-5 years of age. In some embodiments, the patient is aged 2 years or younger, e.g., newborn to 24 months old. In some embodiments, the patient is aged six weeks or younger, e.g., newborn to six weeks old. In some embodiments, the patient is 2 years old or older. In some embodiments, the patient is 2-12 years of age. In some embodiments, the patient is 2-12 years of age. In some embodiments, the patient is 13-21 years of age. In some embodiments, the patient is 5 years old or older, e.g., 5-17 years of age. In some embodiments, the patient is between the ages of 5-21 years. In some embodiments, the patient is an adult.

In some embodiments, the patient has not received or is not treated with an SMN corrector therapy, such as an SMN2 upregulator and/or an SMN1 gene therapy. In some embodiments, the patient has received or is treated with an SMN corrector therapy, such as an SMN2 upregulator therapy and/or an SMN1 gene therapy. In some embodiments, the patient initiates or initiated with the SMN corrector therapy before the age of 5. In some embodiments, the patient initiates or initiated the SMN corrector therapy at or after the age of 5.

In some embodiments, the patient is showing disease progression such that motor function is declining, indicated by a 1 point or greater decrease in a motor function assessment test scores (e.g., HFMSE and RHS scores) over a period of 12 months, prior to treatment with a selective myostatin inhibitor, i.e., apitegromab.

In some embodiments, a patient treated herein is in a period of stable disease decline such that the rate of decline in motor function (e.g., as measured by HFMSE and RHS scores) has not significantly changed over a period of at least six months prior to starting treatment with a selective myostatin inhibitor (i.e., apitegromab). In some embodiments, the patient has stable disease such that the patient's motor function (e.g., as measured by HFMSE and RHS scores) has not significantly changed over a period of at least six months prior to starting treatment with a selective myostatin inhibitor (i.e., apitegromab). In some embodiments, the patient has non-ambulatory SMA. In some embodiments, the patient has type 2 or type 3 non-ambulatory SMA. In some embodiments, the patient has ambulatory SMA. In some embodiments, the patient has type 3 ambulatory SMA. In some embodiments, the patient initiated SMN upregulator/corrector therapy before the age of 5. In some embodiments, the patient initiated SMN upregulator/corrector therapy after the age of 5. In some embodiments, the patient is aged 2 years or younger, e.g., newborn to 24 months old. In some embodiments, the patient is 2-12 years old. In some embodiments, the patient is aged six weeks or younger, e.g., newborn to six weeks old. In some embodiments, the patient is 2-12 years old and initiated SMN upregulator/corrector therapy (e.g., nusinersen) before the age of 5. In some embodiments, the patient is 2-12 years old and initiated SMN upregulator/corrector therapy (e.g., nusinersen) after the age of 5. In some embodiments, the patient is 5-12 years old and initiated SMN upregulator/corrector therapy (e.g., nusinersen) after the age of 5.

In some embodiments, the patient is a patient with 5q spinal muscular atrophy (SMA) with a bi-allelic mutation in the SMN1 gene and a clinical diagnosis of SMA Type 1, or the patient is a patient with 5q SMA with a bi-allelic mutation in the SMN1 gene and up to 3 copies of the SMN2 gene. Such patient may be treated with a selective myostatin inhibitor in conjunction with a gene therapy (e.g., SMN1 gene therapy). In some embodiments, the patient weighs between 2.6 kg and 21.0 kg at the time of receiving the gene therapy. In some embodiments, the patient's motor milestones may be monitored, such as head control, rolling from back to sides, sitting without support for 30 seconds, sitting without support for at least 10 seconds, etc.

In some embodiments, the patient is a type 2 SMA patient who is able to sit but has never walked, wherein the patient is between 2 and 18 years of age. In some embodiments, an SMN1 gene therapy is used in the treatment of type 2 SMA in a patient who is between 2-18 years of age and who is able to sit but has never gained the ability to walk, and wherein the patient is treated with a myostatin-selective inhibitor (i.e., apitegromab). In some embodiments, a myostatin inhibitor is used in the treatment of type 2 SMA in a patient who is between 2-18 years of age and who is able to sit but has never gained the ability to walk, and wherein the patient is treated with an SMN1 gene therapy, wherein optionally the SMN1 gene therapy comprises onasemnogene abeparvovec-xioi. According to the invention, the myostatin inhibitor is a myostatin-selective inhibitor, apitegromab. In some embodiments, an SMN1 gene therapy and a myostatin inhibitor are used as a combination therapy in the treatment of SMA in a patient who is between 2-18 years of age and who is able to sit but has never gained the ability to walk, and wherein the patient is treated with a myostatin-selective inhibitor (i.e., apitegromab), and wherein further optionally, the SMN1 gene therapy comprises onasemnogene abeparvovec-xioi. An antibody variant may have nucleic acid and/or amino acid sequences of significant homology (e.g., >90% sequence identity) and retains one or more physical and/or functional properties as compared to a known antibody.

In some embodiments, the patient has a stable disease as assessed by motor function, such that the motor function assessment test score (e.g., HFMSE and RHS scores) has not significantly changed over a period of at least six months prior to treatment with a selective myostatin inhibitor (i.e., apitegromab). In some embodiments, the patient has received or is treated with an SMN corrector therapy.

In some embodiments, the patient has undergone spinal fusion, e.g., primary posterior spinal fusion.

According to the present disclosure, patient selection or categorization may be based on the highest motor milestone achieved, relative to the number of the smn2 gene copies. Thus, the present disclosure includes therapeutic use of a myostatin-selective inhibitor (such as apitegromab, GYM329, or trevogrumab) in the treatment of SMA in a patient, wherein the treatment comprises administration of a composition comprising the myostatin-selective inhibitor. According to the invention, the myostatin-selective inhibitor is apitegromab, which is intravenously administered at a therapeutic dose, wherein the therapeutic dose is 10 mg/kg. In some embodiments, the patient has 1, 2, 3 or 4 copies of the smn2 genes and achieves one or more of the gross motor milestones of: 1) raises/supports head (i.e., neck holding, e.g., while laying on their stomach able to raise or hold head); 2) rolls over; 3) sits in tripod position (e.g., uses hands to support self while sitting) or sit with support; 4) sits without support; 5) stands with support/assistance; 6) creeps/crawls; 7) pulls to standing position; 8) walks with assistance (e.g., cruises along furniture); 9) stands without support; 10) takes several independent steps but falls; 11) walks alone (e.g., walks independently, walks without support); 12) squats to pick up an object (e.g., a toy); 13) walks/creeps up and down the stairs; 14) runs; 15) jumps; 16) alternates feet going upstairs; 17) hops on one foot; 18) alternates feet going downstairs.

In some embodiments, the patient has 1 copy of the smn2 gene, and the highest motor milestone achieved is to raise or hold head while lying on stomach. In some embodiments, the patient has 1 copy of the smn2 gene, and the highest motor milestone achieved is to roll over. In some embodiments, the patient has 1 copy of the smn2 gene, and the highest motor milestone achieved is to sit with support. In some embodiments, the patient has 1 copy of the smn2 gene, and the highest motor milestone achieved is to sit without support. In some embodiments, the patient has 1 copy of the smn2 gene, and the highest motor milestone achieved is to stand with support. In some embodiments, the patient has 1 copy of the smn2 gene, and the highest motor milestone achieved is to crawl. In some embodiments, the patient has 1 copy of the smn2 gene, and the highest motor milestone achieved is to pull to standing position. In some embodiments, the patient has 1 copy of the smn2 gene, and the highest motor milestone achieved is to walk with assistance. In some embodiments, the patient has 1 copy of the smn2 gene, and the highest motor milestone achieved is to stand without support. In some embodiments, the patient has 1 copy of the smn2 gene, and the highest motor milestone achieved is to walk independently (without support). In some embodiments, the patient has 1 copy of the smn2 gene, and the highest motor milestone achieved is to run. In some embodiments, the patient has 1 copy of the smn2 gene, and the highest motor milestone achieved is to jump. In some embodiments, the patient has 1 copy of the smn2 gene, and the highest motor milestone achieved is to alternate feet going upstairs. In some embodiments, the patient has 1 copy of the smn2 gene, and the highest motor milestone achieved is to hop on one foot. In some embodiments, the patient has 1 copy of the smn2 gene, and the highest motor milestone achieved is to alternate feet going downstairs.

In some embodiments, the patient has 2 copies of the smn2 gene, and the highest motor milestone achieved is to raise or hold head while lying on stomach. In some embodiments, the patient has 2 copies of the smn2 gene, and the highest motor milestone achieved is to roll over. In some embodiments, the patient has 2 copies of the smn2 gene, and the highest motor milestone achieved is to sit with support. In some embodiments, the patient has 2 copies of the smn2 gene, and the highest motor milestone achieved is to sit without support. In some embodiments, the patient has 2 copies of the smn2 gene, and the highest motor milestone achieved is to stand with support. In some embodiments, the patient has 2 copies of the smn2 gene, and the highest motor milestone achieved is to crawl. In some embodiments, the patient has 2 copies of the smn2 gene, and the highest motor milestone achieved is to pull to standing position. In some embodiments, the patient has 2 copies of the smn2 gene, and the highest motor milestone achieved is to walk with assistance. In some embodiments, the patient has 2 copies of the smn2 gene, and the highest motor milestone achieved is to stand without support. In some embodiments, the patient has 2 copies of the smn2 gene, and the highest motor milestone achieved is to walk independently (without support). In some embodiments, the patient has 2 copies of the smn2 gene, and the highest motor milestone achieved is to run. In some embodiments, the patient has 2 copies of the smn2 gene, and the highest motor milestone achieved is to jump. In some embodiments, the patient has 2 copies of the smn2 gene, and the highest motor milestone achieved is to alternate feet going upstairs. In some embodiments, the patient has 2 copies of the smn2 gene, and the highest motor milestone achieved is to hop on one foot. In some embodiments, the patient has 2 copies of the smn2 gene, and the highest motor milestone achieved is to alternate feet going downstairs.

In some embodiments, the patient has 3 copies of the smn2 gene, and the highest motor milestone achieved is to raise or hold head while lying on stomach. In some embodiments, the patient has 3 copies of the smn2 gene, and the highest motor milestone achieved is to roll over. In some embodiments, the patient has 3 copies of the smn2 gene, and the highest motor milestone achieved is to sit with support. In some embodiments, the patient has 3 copies of the smn2 gene, and the highest motor milestone achieved is to sit without support. In some embodiments, the patient has 3 copies of the smn2 gene, and the highest motor milestone achieved is to stand with support. In some embodiments, the patient has 3 copies of the smn2 gene, and the highest motor milestone achieved is to crawl. In some embodiments, the patient has 3 copies of the smn2 gene, and the highest motor milestone achieved is to pull to standing position. In some embodiments, the patient has 3 copies of the smn2 gene, and the highest motor milestone achieved is to walk with assistance. In some embodiments, the patient has 3 copies of the smn2 gene, and the highest motor milestone achieved is to stand without support. In some embodiments, the patient has 3 copies of the smn2 gene, and the highest motor milestone achieved is to walk independently (without support). In some embodiments, the patient has 3 copies of the smn2 gene, and the highest motor milestone achieved is to run. In some embodiments, the patient has 3 copies of the smn2 gene, and the highest motor milestone achieved is to jump. In some embodiments, the patient has 3 copies of the smn2 gene, and the highest motor milestone achieved is to alternate feet going upstairs. In some embodiments, the patient has 3 copies of the smn2 gene, and the highest motor milestone achieved is to hop on one foot. In some embodiments, the patient has 3 copies of the smn2 gene, and the highest motor milestone achieved is to alternate feet going downstairs.

In some embodiments, the patient has 4 copies of the smn2 gene, and the highest motor milestone achieved is to sit with support. In some embodiments, the patient has 4 copies of the smn2 gene, and the highest motor milestone achieved is to sit without support. In some embodiments, the patient has 4 copies of the smn2 gene, and the highest motor milestone achieved is to stand with support. In some embodiments, the patient has 4 copies of the smn2 gene, and the highest motor milestone achieved is to crawl. In some embodiments, the patient has 4 copies of the smn2 gene, and the highest motor milestone achieved is to pull to standing position. In some embodiments, the patient has 2 copies of the smn2 gene, and the highest motor milestone achieved is to walk with assistance. In some embodiments, the patient has 4 copies of the smn2 gene, and the highest motor milestone achieved is to stand without support. In some embodiments, the patient has 4 copies of the smn2 gene, and the highest motor milestone achieved is to walk independently (without support). In some embodiments, the patient has 4 copies of the smn2 gene, and the highest motor milestone achieved is to run. In some embodiments, the patient has 4 copies of the smn2 gene, and the highest motor milestone achieved is to jump. In some embodiments, the patient has 4 copies of the smn2 gene, and the highest motor milestone achieved is to alternate feet going upstairs. In some embodiments, the patient has 4 copies of the smn2 gene, and the highest motor milestone achieved is to hop on one foot. In some embodiments, the patient has 4 copies of the smn2 gene, and the highest motor milestone achieved is to alternate feet going downstairs.

In some embodiments, the patient has more than 4 copies of the smn2 gene, and the highest motor milestone achieved is to sit with support. In some embodiments, the patient has more than 4 copies of the smn2 gene, and the highest motor milestone achieved is to sit without support. In some embodiments, the patient has more than 4 copies of the smn2 gene, and the highest motor milestone achieved is to stand with support. In some embodiments, the patient has more than 4 copies of the smn2 gene, and the highest motor milestone achieved is to crawl. In some embodiments, the patient has more than 4 copies of the smn2 gene, and the highest motor milestone achieved is to pull to standing position. In some embodiments, the patient has 2 copies of the smn2 gene, and the highest motor milestone achieved is to walk with assistance. In some embodiments, the patient has more than 4 copies of the smn2 gene, and the highest motor milestone achieved is to stand without support. In some embodiments, the patient has more than 4 copies of the smn2 gene, and the highest motor milestone achieved is to walk independently (without support). In some embodiments, the patient has more than 4 copies of the smn2 gene, and the highest motor milestone achieved is to run. In some embodiments, the patient has more than 4 copies of the smn2 gene, and the highest motor milestone achieved is to jump. In some embodiments, the patient has more than 4 copies of the smn2 gene, and the highest motor milestone achieved is to alternate feet going upstairs. In some embodiments, the patient has more than 4 copies of the smn2 gene, and the highest motor milestone achieved is to hop on one foot. In some embodiments, the patient has more than 4 copies of the smn2 gene, and the highest motor milestone achieved is to alternate feet going downstairs.

In some embodiments, the patient has 1 copy of the smn2 gene and has achieved the WHO Motor Developmental Milestone of walking independently. In some embodiments, the patient has 1 copy of the smn2 gene and has achieved the WHO Motor Developmental Milestone of standing independently. In some embodiments, the patient has 1 copy of the smn2 gene and has achieved the WHO Motor Developmental Milestone of standing with assistance. In some embodiments, the patient has 1 copy of the smn2 gene and has achieved the WHO Motor Developmental Milestone of hands and knees crawling. In some embodiments, the patient has 1 copy of the smn2 gene and has achieved the WHO Motor Developmental Milestone of walking with assistance.

In some embodiments, the patient has 2 copies of the smn2 gene and has achieved the WHO Motor Developmental Milestone of walking independently. In some embodiments, the patient has 2 copies of the smn2 gene and has achieved the WHO Motor Developmental Milestone of standing independently. In some embodiments, the patient has 2 copies of the smn2 gene and has achieved the WHO Motor Developmental Milestone of standing with assistance. In some embodiments, the patient has 2 copies of the smn2 gene and has achieved the WHO Motor Developmental Milestone of hands and knees crawling. In some embodiments, the patient has 2 copies of the smn2 gene and has achieved the WHO Motor Developmental Milestone of walking with assistance.

In some embodiments, the patient has 3 copies of the smn2 gene and has achieved the WHO Motor Developmental Milestone of walking independently. In some embodiments, the patient has 3 copies of the smn2 gene and has achieved the WHO Motor Developmental Milestone of standing independently. In some embodiments, the patient has 3 copies of the smn2 gene and has achieved the WHO Motor Developmental Milestone of standing with assistance. In some embodiments, the patient has 3 copies of the smn2 gene and has achieved the WHO Motor Developmental Milestone of hands and knees crawling. In some embodiments, the patient has 3 copies of the smn2 gene and has achieved the WHO Motor Developmental Milestone of walking with assistance.

In some embodiments, the patient has 4 copies of the smn2 gene and has achieved the WHO Motor Developmental Milestone of walking independently. In some embodiments, the patient has 4 copies of the smn2 gene and has achieved the WHO Motor Developmental Milestone of standing independently. In some embodiments, the patient has 4 copies of the smn2 gene and has achieved the WHO Motor Developmental Milestone of standing with assistance. In some embodiments, the patient has 4 copies of the smn2 gene and has achieved the WHO Motor Developmental Milestone of hands and knees crawling. In some embodiments, the patient has 4 copies of the smn2 gene and has achieved the WHO Motor Developmental Milestone of walking with assistance.

In some embodiments, the patient has more than 4 copies of the smn2 gene and has achieved the WHO Motor Developmental Milestone of walking independently. In some embodiments, the patient has more than 4 copies of the smn2 gene and has achieved the WHO Motor Developmental Milestone of standing independently. In some embodiments, the patient has more than 4 copies of the smn2 gene and has achieved the WHO Motor Developmental Milestone of standing with assistance. In some embodiments, the patient has more than 4 copies of the smn2 gene and has achieved the WHO Motor Developmental Milestone of hands and knees crawling. In some embodiments, the patient has more than 4 copies of the smn2 gene and has achieved the WHO Motor Developmental Milestone of walking with assistance.

**Table 4 Early- and late-onset SMA patient populations with or without background SMN therapy**

| **Highest motor milestone achieved (with or without treatment)** | **Smn2 gene copy number** |
|---|---|
| Raise/support head | 1-3 |
| Roll over | 1-3 |
| Sit with support or sit in tripod position | 1-4 |
| Sit without support | 1-4 |
| Stand with support | 1-4+ |
| Stand without support | 1-4+ |
| Creep or crawl | 1-4+ |
| Pull to standing position | 1-4+ |
| Walk with assistance | 1-4+ |
| Walk without assistance | 1-4+ |
| Run | 1-4+ |
| Jump | 1-4+ |
| Squat to pick up an object | 1-4+ |
| Walk or creep up and down the stairs | 1-4+ |
| Alternate feet going upstairs | 1-4+ |
| Alternate feet going downstairs | 1-4+ |
| Hop on one foot | 1-4+ |

In some embodiments, the subject is able to raise or support head before the age of 3 months, 6 months, 9 months or 12 months.

In some embodiments, the subject is able to roll over before the age of 6 months, 9 months or 12 months.

In some embodiments, the subject is able to sit with support or sit in tripod position before the age of 6 months, 9 months or 12 months.

In some embodiments, the subject is able to sit without support before the age of 6 months, 9 months or 12 months.

In some embodiments, the subject is able to stand with support before the age of 9 months or 12 months.

In some embodiments, the subject is able to stand without support before the age of 12 months.

In some embodiments, the subject is able to creep or crawl before the age of 6 months, 9 months or 12 months.

In some embodiments, the subject is able to pull to standing position before the age of 9 months or 12 months.

In some embodiments, subject is able to walk with assistance before the age of 12 months or 15 months.

In some embodiments, the subject is able to walk without assistance before the age of 12 months, or 15 months.

In some embodiments, the subject is able to run before the age of 15 months, 18 months or 24 months.

In some embodiments, the subject is able to jump before the age of 15 months, 18 months or 24 months.

In some embodiments, the subject is able to squat to pick up an object before the age of 15 months, 18 months or 24 months.

In some embodiments, the subject is able to walk or creep up and down the stairs before the age of 15 months, 18 months or 24 months.

In some embodiments, the subject is able to alternate feet going upstairs before the age of 30 months or 36 months.

In some embodiments, the subject is able to alternate feet going downstairs before the age of 36 months (3 years).

In some embodiments, the subject is able to hop on one foot before the age of 4 years or 5 years.

The present disclosure provides therapeutic use of apitegromab in the treatment of SMA in a patient, wherein the treatment comprises intravenous administration of a composition comprising apitegromab at a therapeutic dose, wherein the therapeutic dose is greater than 2 mg/kg and up to 20 mg/kg (such as 10 mg/kg), and wherein optionally the patient may be selected from any of the following category or categories, or the patient is characterized in that:
The patient is aged 2 years or younger, e.g., newborn to 24 months old.

The patient is aged six weeks or younger, e.g., newborn to six weeks old.

The patient is aged 2 years or older.

The patient is 2-21 years old.

The patient is 2-12 years old.

The patient is 2-5 years old.

The patient is 5-12 years old.

The patient is 13-21 years old.

The patient has later-onset SMA.

The patient has type 2 SMA.

The patient has type 3 SMA, wherein optionally the patient has non-ambulatory type 3 SMA.

The patient has type 3 SMA, wherein optionally the patient has ambulatory type 3 SMA.

The patient has at least 2 copies of the SMN2 gene, e.g., 2, 3, 4, 5, or 6 copies of the SMN2 gene; or, wherein optionally the patient has 2-4 copies of the SMN2 gene.

The patient has at least 2 copies of the SMN2 gene, e.g., 2, 3, 4 copies of the SMN2 gene; or, wherein optionally the patient has 2-4 copies of the SMN2 gene, wherein optionally the patient has type 2 SMA or non-ambulatory type 3 SMA.

The patient has at least 3 copies of the SMN2 gene, e.g., 3, 4, 5, or 6 copies of the SMN2 gene; or, wherein optionally the patient has 3-5 copies of the SMN2 gene, wherein optionally the patient has ambulatory SMA, wherein further optionally the ambulatory SMA is type 4 SMA.

The patient has SMA (e.g., any type of SMA) and is up to 2 years of age, wherein optionally the patient has been treated with an SMN therapy, wherein further optionally the SMN therapy comprises an SMN2 upregulator (such as nusinersen) or an SMN1 gene therapy.

The patient has type 1 SMA with up to 3 copies of the SMN2 gene, wherein optionally the patient has been treated with an SMN therapy, wherein further optionally the SMN therapy comprises an SMN2 upregulator (such as nusinersen) or an SMN1 gene therapy.

The patient is symptomatic of SMA.

The patient has presymptomatic SMA.

The patient is identified as a carrier of SMA mutation(s) but is asymptomatic (pre-symptomatic).

The patient has not been treated with SMN-directed therapy.

The patient is on (is receiving) or has been treated with an SMN-directed therapy, wherein optionally the SMN-directed therapy is a gene therapy, splice modifier, or combination thereof, wherein further optionally the splice modifier is a nucleic acid-based agent or low molecular weight compound that modifies SMN2 splicing.

The patient is on (is receiving) or has been treated with an SMN-directed therapy, wherein optionally the SMN-directed therapy is a gene therapy, splice modifier, or combination thereof, wherein further optionally the splice modifier is a nucleic acid-based agent or low molecular weight compound that modifies SMN2 splicing; and wherein the patient commenced the SMN-directed therapy before the age of 5.

The patient is on (is receiving) or has been treated with an SMN-directed therapy, wherein optionally the SMN-directed therapy is a gene therapy, splice modifier, or combination thereof, wherein further optionally the splice modifier is a nucleic acid-based agent or low molecular weight compound that modifies SMN2 splicing, and wherein the patient commenced the SMN-directed therapy at age 5 or older.

The patient is on an SMN-directed therapy for at least 12 months at the time of starting apitegromab therapy (e.g., prior to receiving a first dose of apitegromab).

The patient is on an SMN-directed therapy for at least 15 months at the time of starting apitegromab therapy (e.g., prior to receiving a first dose of apitegromab).

The patient is on an SMN-directed therapy for at least 24 months at the time of starting apitegromab therapy (e.g., prior to receiving a first dose of apitegromab).

The patient is 5 years old or younger at the time of starting apitegromab therapy.

The patient is 2 years old or younger at the time of starting apitegromab therapy.

The patient is six weeks old or younger at the time of starting apitegromab therapy.

The patient is before the age of 5 at the time of receiving a first dose of apitegromab.

The patient has a baseline HFMSE score of at least 10, e.g., at least 13, e.g., a score of 13-39, e.g., no greater than 39.

In some embodiments, the present disclosure provides therapeutic use of apitegromab in the treatment of SMA in a patient who is 2-12 years old and has received at least 6 months of an SMN upregulator/corrector therapy (e.g., nusinersen or risdiplam) before starting apitegromab treatment, wherein the apitegromab treatment comprises intravenous administration of a composition comprising apitegromab at 10 mg/kg, once every 4 weeks or once monthly. In some embodiments, the patient initiated the SMN upregulator/corrector therapy before the age of 5. In some embodiments, the patient initiated the SMN upregulator/corrector therapy after the age of 5. In some embodiments, present disclosure provides therapeutic use of apitegromab in the treatment of SMA in a patient who is 2-12 years old and has received at least 6 months of risdiplam before starting apitegromab treatment, wherein the apitegromab treatment comprises intravenous administration of a composition comprising apitegromab at 10 mg/kg once every 4 weeks or once monthly. In some embodiments, present disclosure provides therapeutic use of apitegromab in the treatment of SMA in a patient who is 2-12 years old and has received at least 10 months of nusinersen before starting apitegromab treatment, wherein the apitegromab treatment comprises intravenous administration of a composition comprising apitegromab at 10 mg/kg once every 4 weeks or once monthly.

In some embodiments, the present disclosure provides therapeutic use of apitegromab in the treatment of SMA in a patient who is 5-12 years old and has received at least 6 months of an SMN upregulator/corrector therapy (e.g., nusinersen or risdiplam) before starting apitegromab treatment, wherein the apitegromab treatment comprises intravenous administration of a composition comprising apitegromab at 10 mg/kg, once every 4 weeks or once monthly. In some embodiments, the patient initiated the SMN upregulator/corrector therapy before the age of 5. In some embodiments, the patient initiated the SMN upregulator/corrector therapy after the age of 5. In some embodiments, present disclosure provides therapeutic use of apitegromab in the treatment of SMA in a patient who is 5-12 years old and has received at least 6 months of risdiplam before starting apitegromab treatment, wherein the apitegromab treatment comprises intravenous administration of a composition comprising apitegromab at 10 mg/kg once every 4 weeks or once monthly. In some embodiments, present disclosure provides therapeutic use of apitegromab in the treatment of SMA in a patient who is 5-12 years old and has received at least 10 months of nusinersen before starting apitegromab treatment, wherein the apitegromab treatment comprises intravenous administration of a composition comprising apitegromab at 10 mg/kg once every 4 weeks or once monthly.

In some embodiments, the present disclosure provides therapeutic use of apitegromab in the treatment of SMA in a patient who is aged 5 years or younger, wherein the patient has not received an SMN upregulator/corrector therapy before starting apitegromab treatment, wherein the treatment comprises concurrent (e.g., simultaneous, separate, or sequential) administration of a composition comprising apitegromab and a composition comprising an SMN upregulator/corrector therapy, e.g., nusinersen or risdiplam). According to the invention, the composition comprising apitegromab is administered intravenously. According to the invention, the composition comprising apitegromab is administered at a frequency of once every four weeks or once monthly.

In some embodiments, the present disclosure provides therapeutic use of apitegromab in the treatment of SMA in a patient who is aged 2 years or younger, e.g., newborn to 24 months old, wherein the patient has not received an SMN upregulator/corrector therapy before starting apitegromab treatment, wherein the treatment comprises concurrent (e.g., simultaneous, separate, or sequential) administration of a composition comprising apitegromab and a composition comprising an SMN upregulator/corrector therapy(e.g., nusinersen or risdiplam). In some embodiments, the present disclosure provides therapeutic use of apitegromab in the treatment of SMA in a patient who is aged six weeks or younger, e.g., newborn to six weeks old, wherein the patient has not received an SMN upregulator/corrector therapy before starting apitegromab treatment, wherein the treatment comprises concurrent (e.g., simultaneous, separate, or sequential) administration of a composition comprising apitegromab and a composition comprising an SMN upregulator/corrector therapy, (e.g., nusinersen or risdiplam). In some embodiments, the patient has presymptomatic SMA. In some embodiments, the patient has 2 copies of the SMN2 gene. According to the invention, the composition comprising apitegromab is administered intravenously.According to the invention, the composition comprising apitegromab is administered at a frequency of once every four weeks or once monthly.

In some embodiments, the present disclosure provides therapeutic use of a myostatin-selective inhibitor, i.e., apitegromab, in the treatment of presymptomatic SMA in a human patient. In some embodiments, the patient has not previously received an SMN upregulator/corrector therapy (e.g., SMN1 gene therapy) before starting the myostatin-selective inhibitor treatment. In some embodiments, the patient has previously received or is receiving an SMN upregulator/corrector therapy (e.g., an SMN1 gene therapy). In some embodiments, the treatment comprises concurrent (e.g., simultaneous, separate, or sequential) administration of a composition comprising a myostatin-selective inhibitor, i.e., apitegromab, and a composition comprising an SMN upregulator/corrector therapy, (e.g., an SMN1 gene therapy). In some embodiments, the patient has 2 copies of the SMN2 gene. According to the invention, the composition comprising a myostatin-selective inhibitor, i.e., apitegromab, is administered intravenously. According to the invention, the composition comprising a myostatin-selective inhibitor, i.e., apitegromab, is administered at a frequency of once every four weeks or once monthly. The myostatin-selective inhibitor binds selectively to pro-latent myostatin.According to the invention, the myostatin-selective inhibitor is apitegromab.

In some embodiments, the present disclosure provides therapeutic use of a myostatin-selective inhibitor, i.e., apitegromab, in the treatment of SMA in a patient who is aged 5 years or younger. In some embodiments, the patient has not previously received an SMN upregulator/corrector therapy (e.g., an SMN1 gene therapy) before starting the myostatin-selective inhibitor treatment. In some embodiments, the patient has previously received or is receiving an SMN upregulator/corrector therapy (e.g., an SMN1 gene therapy). In some embodiments, the patient is administered the SMN upregulator/corrector therapy (e.g., SMN1 gene therapy) at age 5 years or younger. In some embodiments, the treatment of SMA comprises concurrent (e.g., simultaneous, separate, or sequential) administration of a composition comprising a myostatin-selective inhibitor, i.e., apitegromab, and a composition comprising an SMN upregulator/corrector therapy, e.g., an SMN1 gene therapy. According to the invention, the composition comprising a myostatin-selective inhibitor, i.e., apitegromab, is administered intravenously. In some embodiments, the patient has presymptomatic SMA. In some embodiments, the patient has 2 copies of the SMN2 gene. According to the invention, the composition comprising a myostatin-selective inhibitor, i.e., apitegromab, is administered at a frequency of once every four weeks or once monthly. The myostatin-selective inhibitor binds selectively to pro-latent myostatin. According to the invention, the myostatin-selective inhibitor is apitegromab.

In some embodiments, the present disclosure provides therapeutic use of a myostatin-selective inhibitor, i.e., apitegromab, in the treatment of SMA in a patient who is aged 2 years or younger, e.g., newborn to 24 months old. In some embodiments, the patient has not previously received an SMN upregulator/corrector therapy (e.g., an SMN1 gene therapy) before starting the myostatin-selective inhibitor treatment. In some embodiments, the patient has previously received or is receiving an SMN upregulator/corrector therapy (e.g., an SMN1 gene therapy). In some embodiments, the patient is administered the SMN upregulator/corrector therapy (e.g., SMN1 gene therapy) at age 2 years or younger (e.g., newborn to 24 months). In some embodiments, the treatment comprises concurrent (e.g., simultaneous, separate, or sequential) administration of a composition comprising a myostatin-selective inhibitor, i.e., apitegromab, and a composition comprising an SMN upregulator/corrector therapy (e.g., an SMN1 gene therapy). In some embodiments, the patient has presymptomatic SMA. In some embodiments, the patient has 2 copies of the SMN2 gene. According to the invention, the composition comprising a myostatin-selective inhibitor, i.e., apitegromab, is administered intravenously. According to the invention, the composition comprising a myostatin-selective inhibitor, i.e., apitegromab, is administered at a frequency of once every four weeks or once monthly. The myostatin-selective inhibitor binds selectively to pro-latent myostatin. According to the invention, the myostatin-selective inhibitor is apitegromab.

In some embodiments, the present disclosure provides therapeutic use of a myostatin-selective inhibitor, i.e., apitegromab, in the treatment of SMA in a patient who is aged six weeks or younger, e.g., newborn to six weeks old, wherein the treatment comprises concurrent (e.g., simultaneous, separate, or sequential) administration of a composition comprising the myostatin-selective inhibitor, i.e., apitegromab, and a composition comprising an SMN upregulator/corrector therapy, (e.g., an SMN1 gene therapy). In some embodiments, the patient has not previously received an SMN upregulator/corrector therapy (e.g., SMN1 gene therapy) before starting the myostatin-selective inhibitor treatment. In some embodiments, the patient has previously received or is receiving an SMN upregulator/corrector therapy (e.g., an SMN1 gene therapy). In some embodiments, the patient has presymptomatic SMA. In some embodiments, the patient has 2 copies of the SMN2 gene. According to the invention, the composition comprising a myostatin-selective inhibitor, i.e., apitegromab, is administered intravenously. According to the invention, the composition comprising a myostatin-selective inhibitor, i.e., apitegromab, is administered at a frequency of once every four weeks or once monthly. The myostatin-selective inhibitor binds selectively to pro-latent myostatin. According to the invention, the myostatin-selective inhibitor is apitegromab.

In some embodiments, the present disclosure provides a myostatin-selective inhibitor and an SMN upregulator/corrector therapy (e.g., SMN1 gene therapy) for use in the treatment of presymptomatic SMA in a patient, wherein the treatment comprises administration of the myostatin-selective inhibitor and the SMN upregulator/corrector therapy (e.g., SMN1 gene therapy) to a presymptomatic patient in amounts effective to treat SMA. In some embodiments, the patient has 2 copies of the SMN2 gene, and/or wherein the patient is six weeks or younger at the time of administration of the SMN upregulator/corrector therapy (e.g., SMN1 gene therapy). According to the invention, the myostatin-selective inhibitor is apitegromab.

In some embodiments, the present disclosure provides a myostatin-selective inhibitor and an SMN upregulator/corrector therapy (e.g., SMN1 gene therapy) for use in the treatment of presymptomatic SMA in a patient, wherein the treatment comprises administration of the myostatin-selective inhibitor and the SMN upregulator/corrector therapy (e.g., SMN1 gene therapy) to a presymptomatic patient in amounts effective to treat SMA, wherein the patient has 2 copies of the SMN2 gene, wherein the patient is six weeks or younger at the time of administration of the SMN upregulator/corrector therapy (e.g., SMN1 gene therapy), and wherein the myostatin-selective inhibitor is apitegromab.

In some embodiments, the present disclosure provides a myostatin-selective inhibitor for use in the treatment of presymptomatic SMA in a patient, wherein the treatment comprises administration of the myostatin-selective inhibitor to a presymptomatic patient in amounts effective to treat SMA. In some embodiments, the patient has 2 copies of the SMN2 gene. In some embodiments, the patient is administered with an SMN upregulator/corrector therapy (e.g., SMN1 gene therapy) at six weeks or younger. In some embodiments, the myostatin-selective inhibitor is apitegromab.

In some embodiments, the present disclosure provides a myostatin-selective inhibitor for use in the treatment of presymptomatic SMA in a patient, wherein the treatment comprises administration of the myostatin-selective inhibitor to a presymptomatic patient in amounts effective to treat SMA, wherein the patient has 2 copies of the SMN2 gene, wherein the patient is administered with an SMN upregulator/corrector therapy (e.g., SMN1 gene therapy) at six weeks or younger, and wherein the myostatin-selective inhibitor is apitegromab.

In some embodiments, the present disclosure provides therapeutic use of an SMN upregulator/corrector therapy (e.g., SMN1 gene therapy), in the treatment of presymptomatic SMA in a human patient, wherein the treatment comprises concurrent (e.g., simultaneous, separate, or sequential) administration of a composition comprising an SMN upregulator/corrector therapy, (e.g., an SMN1 gene therapy) and a composition comprising a myostatin-selective inhibitor, i.e., apitegromab. In some embodiments, the patient has not previously received a myostatin-selective inhibitor, e.g., apitegromab, GYM329, or trevogrumab before starting the SMN upregulator/corrector therapy. In some embodiments, the patient has previously received or is receiving a myostatin-selective inhibitor, e.g., apitegromab, GYM329, or trevogrumab. In some embodiments, the patient is administered the SMN upregulator/corrector therapy (e.g., SMN1 gene therapy) at age five years or younger. In some embodiments, the patient is administered the SMN upregulator/corrector therapy (e.g., SMN1 gene therapy) at two years or younger (e.g., newborn to 24 months old). In some embodiments, the patient is administered the SMN upregulator/corrector therapy (e.g., SMN1 gene therapy) at age six weeks or younger (e.g., newborn to six weeks old). In some embodiments, the patient has 2 copies of the SMN2 gene. According to the invention, the composition comprising a myostatin-selective inhibitor, i.e., apitegromab, is administered intravenously. According to the invention, the composition comprising a myostatin-selective inhibitor, i.e., apitegromab, is administered at a frequency of once every four weeks or once monthly. The myostatin-selective inhibitor binds selectively to pro-latent myostatin. According to the invention, the myostatin-selective inhibitor is apitegromab.

In some embodiments, a myostatin-selective inhibitor is used in the treatment of SMA in a patient who is 2 months or older, who is treated with an SMN2 upregulator, such as risdiplam.

In some embodiments, a myostatin-selective inhibitor is used in the treatment of presymptomic SMA in a patient with up to 3 copies (e.g., 2 copies, 3 copies) of the SMN2 gene or who has been diagnosed with type 1 SMA, wherein the patient receives a gene therapy, such as onasemnogene abeparvovec . Optionally, the patient is 6 weeks or younger. Further optionally, the patient weighs between 2.6 kg and 21.0 kg.

In some embodiments, the present disclosure provides an SMN1 upregulator/corrector therapy (e.g., SMN1 gene therapy) for use in the treatment of presymptomatic SMA in a patient, wherein the treatment comprises administration of the SMN1 upregulator/corrector therapy (e.g., SMN1 gene therapy) to a presymptomatic patient in amounts effective to treat SMA, wherein the patient is administered the SMN1 upregulator/corrector therapy (e.g., SMN1 gene therapy) at six weeks or younger, wherein the patient is further treated with a myostatin-selective inhibitor, and wherein the myostatin-selective inhibitor is apitegromab.

### Additional Indications

Data presented herein indicate that the disclosed treatment regimens, e.g., including dose selections, for apitegromab may provide clinical benefit for human patients beyond those with SMA. Apitegromab may provide clinical benefit for human patients diagnosed with other indications that share certain attributes of SMA. Such attributes include one or more of the criteria such as: disease with relatively young patient population, disease in which muscles are structurally intact or function is retained, disease that impacts fast-twitch fiber, and the availability of an established endpoint that relies on fast-twitch fibers.

Beyond SMA, additional indications that may benefit from treatment with the antibodies disclosed herein (e.g., apitegromab) include but are not limited to dystrophies such as Becker's muscular dystrophy, Duchenne's muscular dystrophy, and other muscular dystrophies, as well as late-onset Pompe disease, post-cancer muscle recovery, and glucocorticoid-induced myopathy (e.g., in a subset of patients unable to discontinue steroid therapy). Becker's muscular dystrophy may be particularly suitable for treatment with the antibodies disclosed herein (e.g., apitegromab). Becker's muscular dystrophy patients typically have higher levels of circulating myostatin as compared to Duchenne's muscular dystrophy patients (see, e.g., Burch et al. (2017) J Neurol. 264(3):541-553; Mariot et al. (2017) Nat Commun. 8: 1859). In other cases, the indication treated is post-cancer muscle recovery, e.g., post-cancer muscle recovery in pediatric patients (as some children may develop severe muscle wasting from chemotherapy). The antibodies disclosed herein (e.g., apitegromab) may be used as monotherapy or as add-on therapy to provide muscle-directed approaches to enhance other stabilizing treatments (e.g., treatments such as gene therapy in Duchenne's muscular dystrophy or enzyme replacement therapy in lysosomal storage disorders).

### Dosage Selection and Administration

According to the invention, an effective amount of an anti-pro/latent myostatin antibody (i.e., apitegromab) is administered to a human subject in need of the treatment via intravenous administration, e.g., by continuous infusion over a period of time.

The terms "administer," "administering," and "administration" include any method of delivery of a therapeutic agent, e.g., an anti-pro/latent myostatin antibody (e.g., apitegromab), into a subject's system or to a particular region in or on a subject (systemic and local administration, respectively). According to the invention, the therapeutic agent is apitegromab. According to the invention, the apitegromab is formulated for administration as a composition, e.g., a pharmaceutical composition. In some embodiments, apitegromab is formulated for intravenous administration. For example, in some embodiments, the apitegromab is administered by intravenous injection/infusion, e.g., by intravenous infusion. In some embodiments, the apitegromab is administered by intravenous infusion, e.g., over approximately 1 to 2 hours. In some embodiments, the apitegromab is administered by intravenous infusion over approximately 2 hours. In some embodiments, the infusion duration is less than two hours but no shorter than 1 hour.

According to the invention, apitegromab is administered to a patient as a weight-based dose, i.e., a dose dependent on the patient's bodyweight. In some cases, suitable dosages of apitegromab include greater than 2 and up to 20 mg/kg, optionally about 5, 10, 15, or 20 mg/kg. According to the invention, the therapeutic dose of apitegromab is 10 mg/kg. In some other cases, the therapeutic dose of apitegromab is 20 mg/kg. In some cases, a dose greater than 20 mg/kg may be used while retaining a similar safety profile, although there may be diminished need for higher dosage given the surprisingly good therapeutic and PK profile of e.g., 20 mg/kg and 2 mg/kg, respectively.

Target engagement analyses from 6-month and 12-month studies (see Examples herein) indicate that apitegromab at 20 mg/kg achieved target saturation, whilst 2 mg/kg showed partial target engagement. These observations support that therapeutic dose for intravenously administered apitegromab can be 20 mg/kg or below, but greater than 2 mg/kg.

PK analyses of apitegromab show a correlation between drug clearance and age (see FIG. 18) or weight (see FIG. 17). These data indicate that younger patients (with lighter body weight) show slower clearance of apitegromab than older patients (with heavier body weight). This finding may point to dose selection for suitable intermediate doses to be, for example, 5 mg/kg, 7.5 mg/kg, 10 mg/kg, 12 mg/kg, 15 mg/kg, etc.

In some cases, a suitable dosage of apitegromab is 20 mg/kg. According to the invention, a suitable dosage of apitegromab is 10 mg/kg. In some cases, the apitegromab is intravenously administered to a patient every 4 weeks or monthly at 5 mg/kg, 7.5 mg.kg, 10 mg/kg or 12 mg/kg.

In some cases, apitegromab is administered to a patient at a dose of 20 mg/kg. In some cases, due to its favorable safety profile when administered at a dose of 20 mg/kg, apitegromab may be administered to a patient at a dose of more than 20 mg/kg, e.g., wherein the dose is up to 30 mg/kg, e.g., 25 mg/kg. In some cases, apitegromab is administered to a patient at a dose of more than 20 mg/kg (e.g., about 25 mg/kg, about 30 mg/kg). In some cases, apitegromab is intravenously administered to a patient at a dose of 10-20 mg/kg every 4 weeks or monthly.

In some embodiments, apitegromab is administered to a patient about once every 4 weeks, once a month, etc. Such antibody may be administered via intravenous injection/infusion, e.g., by intravenous infusion, or another suitable route of administration (e.g., subcutaneously (e.g., under the skin) or intrathecally (e.g., intra spinally). Similarly, an SMN upregulator, e.g., splice modifier, may be administered orally, e.g., by mouth, or another suitable route of administration.

In some embodiments, the subject has received an SMN upregulator prior to the administration of the apitegromab. In some embodiments, the subject is concurrently receiving an SMN upregulator at the same time as the administration of the apitegromab. In some embodiments, the subject will receive an SMN upregulator after administration of the apitegromab.

In some embodiments, the subject receiving an SMN upregulator is administered apitegromab at least 24 hours (e.g., at least 36 hours, at least 48 hours, or more) prior to a dose (e.g., a maintenance dose) of the SMN upregulator (e.g., nusinersen). In some embodiments, the subject receiving an SMN upregulator is administered apitegromab at least 14 days (e.g., least 21 days, or more) after a dose (e.g., a maintenance dose) of the SMN upregulator (e.g., nusinersen).

In some embodiments, the subject has received an SMN upregulator within 6 months of administration of the apitegromab. In some embodiments, the subject has received an SMN upregulator within 3 months of administration of the apitegromab. In some embodiments, the subject has received an SMN upregulator within 6 months, 5 months, 4 months, 3 months, 2 months, or 1 month of administration of the apitegromab. In some embodiments, the subject has received an SMN upregulator within 4 weeks, 3 weeks, 2 weeks, or 1 week of administration of the apitegromab. In some embodiments, the subject has received an SMN upregulator on the same day as administration of the apitegromab.

In some embodiments, the subject is expected to receive an SMN upregulator within 6 months of administration of the apitegromab. In some embodiments, the subject is expected to receive an SMN upregulator within 3 months of administration of the apitegromab. In some embodiments, the subject is expected to receive an SMN upregulator within 6 months, 5 months, 4 months, 3 months, 2 months, or 1 month of administration of the apitegromab. In some embodiments, the subject is expected to receive an SMN upregulator within 4 weeks, 3 weeks, 2 weeks, or 1 week of administration of the apitegromab.

In some embodiments, the SMN upregulator is an antisense nucleotide and is administered to the central nervous system of the subject via intrathecal injection. In some embodiments, the antisense nucleotide is administered to the subject every few months, e.g., monthly, every two months, every three months, every four months, every five months, every six months or every 12 months. In other embodiments, an initial treatment may involve more frequent dosing, followed by a less frequent maintenance dose thereafter.

In some embodiments, the SMN upregulator is a small molecule and is orally administered to the subject. In some embodiments, the small molecule is administered to the subject daily. In other embodiments, the small molecule is administered to the subject weekly, biweekly or monthly.

In some embodiments, the SMN upregulator is a gene therapy and is administered via intravenous injection. In some embodiments, the SMN upregulator is a gene therapy and is administered via intrathecal injection. In some embodiments, an initial treatment may include more frequent dosing, followed by a less frequent maintenance dose thereafter. Less frequent maintenance doses may be preferable in order to avoid improper immune responses to the gene therapy.

According to the invention, the apitegromab is administered to the subject via intravenous administration, e.g., by intravenous infusion. According to the invention, the apitegromab is administered once every 4 weeks or monthly, to the subject. In some cases, an initial treatment may involve more frequent dosing, followed by a less frequent maintenance dose thereafter. In some embodiments, apitegromab may be initially dosed at a higher dose (e.g., a loading dose) followed by one or more subsequent lower doses (e.g., one or more maintenance doses). In some embodiments, an initial dose or doses of apitegromab may be administered at 20 mg/kg, followed by one or more lower dose (e.g., 15 mg/kg. 10 mg/kg, 5 mg/kg, 2 mg/kg, or 1 mg/kg), e.g., four weeks or one month after administration of the initial 20 mg/kg dose, e.g., wherein the one or more lower doses are administered once every four weeks or once monthly thereafter, or at longer intervals than was used for the loading dose.

An "effective amount" for treating SMA, as used herein, may be an amount to achieve clinical efficacy, including but are not limited to: retaining motor function as compared to a decline in a control, delaying disease progression, delaying or preventing an ambulatory type 3 SMA patient from becoming non-ambulatory, delaying or preventing need for respiratory aid or intervention, reducing the rate of a decline in one or more motor function scores as compared to a control, and/or maintaining at least net zero change in one or more motor function scores as compared to a baseline, etc.

Clinical benefits of SMA therapy may include 1) motor function improvement, 2) motor function maintenance (disease stabilization) or 3) delay in disease progression.

Motor function may be assessed by suitable means, such as HFMSE and/or RHS, the latter of which is often used to assess the motor function of ambulatory patients. In some cases, HFMSE is more frequently used to assess the motor function of non-ambulatory patients. An increase in the respective score over an appropriate baseline indicates motor function improvement. According to the invention, treatment with apitegromab increases motor function as measured by HFMSE at 6 months after the start of treatment relative to HFMSE at baseline. In some embodiments, the increase is at least 1 point, at least 2 points, at least 3 points, at least 4 points, or at least 5 points. In some embodiments, the increase is 1 point or greater, 2 points or greater, 3 points or greater, 4 points or greater, or 5 points or greater. In some embodiments, an SMA therapy comprising apitegromab enhances motor function, such that a HFMSE score or a RHS score (e.g., a HFMSE score) measured at 6 months or 12 months after initiation of treatment is at least 1, 3, 5, 7, or 10 points over a baseline, and wherein the baseline is obtained at or prior to initiation of the treatment. In some embodiments, an SMA therapy disclosed herein (e.g., an SMA therapy comprising apitegromab) produces at least a 5 point increase in a HFMSE score relative to a baseline score after at least 12 months of treatment. In some embodiments, an SMA therapy disclosed herein (e.g., an SMA therapy comprising apitegromab) produces a 6-20 point or greater increase in a HFMSE score relative to a baseline score after at least 12 months of treatment. In some embodiments, an SMA therapy disclosed herein (e.g., an SMA therapy comprising apitegromab) produces at least a 7 point increase in a HFMSE score relative to a baseline score after at least 12 months of treatment.

In some embodiments, an improvement in a motor function score (e.g., a HFMSE or RHS score) may be positively correlated with SMA severity and/or the length of treatment time with an SMN corrector therapy (e.g., nusinersen). In some embodiments, an improvement in a motor function score (e.g., an HFMSE or RHS score) may be inversely correlated with age and/or characteristics of advanced disease. In some embodiments, a characteristic of advanced disease includes scoliosis and/or joint contractures. In some embodiments, joint contracture can occur due to shortening of muscles, tendons, ligaments, causing deformity and symptoms include pain and loss of movement in the joint. In some embodiments, treatment of SMA comprises administering apitegromab therapy, either as monotherapy or in conjunction with (or in combination with) another therapy, to a patient lacking scoliosis and/or joint contracture. In some embodiments, apitegromab is used for treating SMA, either as monotherapy or in conjunction with (or in combination with) another therapy, in a patient lacking scoliosis and/or joint contracture.

In some cases, the SMA therapy comprises intravenous administration of greater than 2 mg/kg and up to 20 mg/kg of apitegromab every 4 weeks or monthly. In some cases, the SMA therapy comprises intravenous administration of about 20 mg/kg of apitegromab every 4 weeks or monthly. In some cases, an SMA therapy disclosed herein (e.g., an SMA therapy comprising apitegromab) may achieve disease stabilization such that a patient's motor function is maintained over time, or in other words, prevented from declining, against the natural history of the disease, which predicts progression over time. In some cases, a net zero change in motor function scores over an appropriate baseline reflects disease stabilization. In some cases, an SMA therapy disclosed herein (e.g., an SMA therapy comprising apitegromab) stabilizes disease progression, such that a HFMSE score or a RHS score measured at 6 months or 12 months after initiation of the treatment is no less than a baseline or no more than a 0.1, 0.2, 0.3, 0.4, or 0.5 point decline, and wherein the baseline is obtained at or prior to initiation of the treatment. In some cases, an SMA therapy disclosed herein (e.g., an SMA therapy comprising apitegromab) stabilizes disease progression, such that an RHS score comprises less than a 1 point, 2 point, or 3 point decline in the RHS score relative to a baseline score after at least 12 months of treatment. In some cases, the SMA therapy comprises intravenous administration of greater than 2 mg/kg and up to 20 mg/kg of apitegromab every 4 weeks or monthly. In some cases, the SMA therapy comprises intravenous administration of about 20 mg/kg of apitegromab every 4 weeks or monthly. In some embodiments, the SMA therapy comprises administering a therapeutically effective amount of apitegromab such that the administration is sufficient to increase the motor function of the subject by at least 1 milestone according to the WHO Motor Developmental Milestones. In some embodiments, the SMA therapy comprises administering a therapeutically effective amount of apitegromab such that the administration is sufficient to increase the motor function of the subject by 1, 2, or 3 milestones according to the WHO Motor Developmental Milestones. In some embodiments, the WHO Motor Developmental Milestones include one or more of ability to walk independently, ability to stand independently, standing with assistance, hands and knees crawling, and/or walking with assistance.

An SMA therapy disclosed herein (e.g., an SMA therapy comprising apitegromab) may help maintain the disease status in a patient population that receives apitegromab, as compared to control group that does not. Maintenance of the disease status refers to preventing further deterioration of affected muscle, for example as assessed by changes in motor function over time, in these patients. Treatment may slow the disease progression, e.g., as assessed by a slower rate of change in disease function as compared to a suitable baseline (e.g., an untreated patient). Therefore, even where no improvement in motor function test scores is shown, the apitegromab may provide clinical benefits by countering disease progression. Thus, such clinical benefits may present as a longer period of time observed where the patient population treated with the apitegromab maintains prior test scores, or shows a slower rate of decrease in scores over time, as compared to a control group.

An SMA therapy disclosed herein (e.g., an SMA therapy comprising apitegromab) may slow the progression of the disease, e.g., by delaying a decrease in motor function as measured by a decreased score by HFMSE or RHS, and/or by delaying a transition from ambulatory to non-ambulatory, delaying the need for respiratory aid or intervention, etc.

Based on the surprising degree of clinical benefit achieved by selective inhibition of myostatin in human patients as demonstrated herein, it is contemplated that different myostatin-selective inhibitors (other than apitegromab) may be used in the treatment of SMA or other muscle disorders. Accordingly, the present disclosure includes a myostatin-selective inhibitor for use in the treatment of a muscle disorder, such as SMA, in a human subject, wherein optionally, the subject is further treated with a motor neuron-directed therapy, wherein further optionally the motor neuron-directed therapy comprises an SMN upregulator therapy, such as SMN2 upregulator therapies and SMN1 gene therapies.

### Biological Effects of Treatment

An SMA therapy disclosed herein (e.g., an SMA therapy comprising apitegromab) may be suitable for the treatment of any form of SMA, particularly later-onset forms of SMA, in a human subject.

Patient populations who may benefit from the therapies described herein include those with non-ambulatory SMA and those with ambulatory SMA. In some embodiments, an SMA therapy disclosed herein is considered for non-ambulatory forms of SMA, such as type 2 and non-ambulatory type 3 SMA. In other embodiments, an SMA therapy disclosed herein is considered for ambulatory forms of SMA, such as ambulatory type 3 SMA.

Genotype-based diagnosis allows for the identification of patients carrying an SMA genetic mutation, including those who may be pre-symptomatic (not manifesting obvious disease phenotypes). In some embodiments, a subject suitable for treatment with the SMA therapies disclosed herein has a direct diagnosis of a mutation in SMN1 (e.g., a chromosomal direct diagnosis of 5q SMA). In some embodiments, the subject has a diagnosis of 5q SMA, in addition to a diagnosis based on motor phenotype.

In some embodiments, the subject having SMA is 2 years of age or older. In some embodiments, the subject having SMA is 5 to 21 years old. Clinical effects of an anti-pro/latent myostatin antibody (e.g., apitegromab), administered alone or in conjunction with an SMN upregulator, can be monitored and/or evaluated for effectiveness by various means. Exemplary such biologically beneficial effects are provided herein. Beneficial biological effects in a subject can be achieved by administration of an anti-pro/latent myostatin antibody (e.g., apitegromab) as a monotherapy or in conjunction with an SMN upregulator. In some embodiments, apitegromab as a monotherapy or in conjunction with an SMN upregulator is administered in an amount effective to cause one or more of the biological effects described below.

The ability to assess functional scales that can be reliably measured in SMA patients may be used in tracking patients' disease progression as well as effects of therapy over time. Whilst muscle function may be assessed by physiological measurements, such as muscle strength and force generation, motor functional scales monitor disease progression in ways that relate to patients' functionality in everyday life and carries more meaning and relevance than a measure that quantitates strength *per se.* In some embodiments, a patient in need of treatment or being treated for SMA is evaluated using a functional scale, such as any one or more of the motor functional assessment tests or functional outcome measures described herein (e.g., one or more of HFMSE, RHS, 6MWT, WHO Motor Milestones, RULM, 30-second Sit-to-Stand, Endurance Shuttle Nine Hole Peg Test [ESNHPT], and/or Endurance Shuttle Box and Block Test [ESBBT]).

In some embodiments, a patient with SMA (e.g., later-onset SMA) is evaluated using the Revised Hammersmith Scale (RHS). In some embodiments, administration of the RHS includes the Timed Rise from Floor and 10-meter Walk/Run tests. In some embodiments, the RHS is a 36-item clinical assessment for physical abilities of patients with type 2 SMA, and ambulatory and non-ambulatory patients with type 3 SMA. The RHS includes 33 items that are graded on a scale of 0, 1, 2, where 0 denotes the lowest level of ability/function and 2 denotes the highest level of ability (Ramsey et al. (2017) PLoS One. 12(2):e0172346). The remaining 3 items are scored 0, 1, where 0 denotes an inability and 1 denotes an ability to achieve. The maximum achievable score is 69.

In some embodiments, a patient to be treated with apitegromab (e.g., a patient with ambulatory SMA) has a baseline RHS score of 26 or greater prior to treatment. In some embodiments, the patient has a baseline RHS score of 63 or below. In some embodiments, the patient has a baseline RHS score which ranges between 26-63. In some embodiments, the patient has ambulatory SMA, e.g., ambulatory type 3 SMA.

In some embodiments, a patient with SMA (e.g., later-onset SMA) is evaluated using a 10-meter Walk/Run test. In some embodiments, the 10-meter Walk/Run test is an enhanced function of the RHS used for ambulatory patients with type 3 SMA. The 10-meter Walk/Run test is a measure of the time taken to walk/run 10 meters.

In some embodiments, a patient with SMA (e.g., later-onset SMA) is evaluated using a Timed Rise from Floor test. In some embodiments, the Timed Rise from Floor test is an enhanced function of the RHS used for ambulatory patients with type 3 SMA. The Timed Rise from Floor test is a measure of the time taken to rise to standing from the floor.

In some embodiments, a patient with SMA (e.g., later-onset SMA) is evaluated using a 6-minute Walk Test (6MWT). The 6MWT is an assessment of exercise capacity and fatigue that has been used in clinical studies of ambulatory later-onset SMA patients (Young et al. (2016). Muscle Nerve. 54(5):836-842). Patients are directed to walk along a 25-meter course as fast as possible over 6 minutes. The minute distances and total distance walked over 6 minutes are measured.

In some embodiments, a patient with SMA (e.g., later-onset SMA) is evaluated using a 30-second Sit-to-Stand Test. The 30-second Sit-to-Stand Test is used by researchers and clinicians as an assessment of functional lower limb strength (Jones et al. (1999) Res Q Exerc Sport. 70:113-119). The test was modified for the ambulatory SMA population based on research of the modified 30-second Sit-to-Stand being a reliable, feasible tool for use in a general geriatric population with a lower level of function (McAllister and Palombaro (2019). J Geriatr Phys Ther. 0(0):1-6) and was related to fall risk in institutionalized veterans (Applebaum et al. (2017). PLoS One. 12(5):e0176946; Le Berre et al. (2016) Percept Mot Skills. 123:138-152). The test measures the maximal number of times the patient can transition from sitting to standing in 30 seconds.

According to the invention, a patient with SMA (e.g., later-onset SMA) is evaluated using the Hammersmith Functional Motor Scale Expanded (HFMSE) (Main M, Kairon H, Mercuri E, Muntoni F. The Hammersmith functional motor scale for children with spinal muscular atrophy: a scale to test ability and monitor progress in children with limited ambulation. Eur J Paediatr Neurol. 2003;7(4):155-9). In some embodiments, the HFMSE is used to assess the physical abilities of patients with type 2 and type 3 SMA (O'Hagen et al. (2007) Neuromuscul Disord. 17(9-10):693-697; Glanzman et al. (2011) J Child Neurol. 26(12):1499-1507). In some embodiments, the HFMSE is used to evaluate non-ambulatory SMA patients. The HFMSE consists of 33 items to assess an individual's ability to perform various activities, including:
1. Plinth/chair sitting
2. Long sitting
3. One hand to head in sitting
4. Two hands to head in sitting
5. Supine to side-lying
6. Rolls prone to supine over R
7. Rolls prone to supine over L
8. Rolls supine to prone over R
9. Rolls supine to prone over L
10. Sitting to lying
11. Props on forearms
12. Lifts head from prone
13. Prop on extended arms
14. Lying to sitting
15. Four-point kneeling
16. Crawling
17. Lifts head from supine
18. Supporting standing
19. Stand unsupported
20. Stepping
21. Right hip flexion
22. Left hip flexion in supine
23. High kneeling to right half kneel
24. High kneeling to left half kneel
25. High kneeling to stand leading with left leg
26. High kneeling to stand leading with right leg
27. Stand to sit
28. Squat
29. Jump 12 inches forward
30. Ascends stairs with rail
31. Descends stairs with rail
32. Ascends stairs without rail
33. Descends stairs without rail

Quality and execution of each movement for the parameters listed above are graded on a scale of 0, 1, 2, where 0 denotes unable, 1 denotes performed with modification or adaption, and 2 denotes without modification or adaptation. The maximum achievable score is 66.

In some embodiments, a patient to be treated with apitegromab (e.g., a patient with a non-ambulatory form of SMA, such as type 2 or non-ambulatory type 3 SMA) has a baseline HFMSE score of 12 or greater prior to treatment. In some embodiments, the patient has a baseline HFMSE score of 44 or below. In some embodiments, the patient has a baseline HFMSE score which ranges between 12-44. In some embodiments, the patient has a non-ambulatory form of SMA. In some embodiments, the patient has type 2 SMA. In some embodiments, the patient has non-ambulatory type 3 SMA.

In some embodiments, a patient with SMA (e.g., later-onset SMA) is evaluated using the Revised Upper Limb Module (RULM). The RULM is a 20-item assessment of upper limb function in non-ambulatory SMA patients (young children as well as adults) (Mazzone et al. (2017) Muscle Nerve. 55(6):869-874). The 19 scored items test functions that relate to everyday life, such as placing hands from lap, pressing a button, and picking up a token. The items are scored 0, 1, 2, where 0 denotes unable, 1 denotes able with modification, and 2 denotes able with no difficulty. The maximum score achievable is 37. In some embodiments, the RULM is used to evaluate non-ambulatory SMA patients. In some embodiments, the RULM is completed by patients who are 30 months of age or older, e.g., at the time of the baseline assessment.

In some embodiments, a patient with SMA (e.g., later-onset SMA) is evaluated using World Health Organization (WHO) Motor Development Milestones. The WHO Multicentre Growth Reference Study (MGRS) generated growth curves for assessing the growth and development of infants and young children around the world (de Onis et al. (2004) Food Nutr Bull. 25 Suppl:S1-89). The MGRS had as its primary objective the construction of curves and related tools to assess growth and development in children from birth to 5 years of age. Another feature of the MGRS is that it included the collection of ages of achievement of motor milestones, including gross motor development milestones such as sitting without support, hands-and-knees crawling, standing with assistance, walking with assistance, standing alone, and walking alone (Wijnhoven et al. (2004) Food Nutr Bull. 25(1 Suppl):S37-45; WHO Multicentre Growth Reference Study Group (2006) Acta Paediatr Suppl;450:86-95). In some embodiments, WHO MGRS performance criteria for gross motor development are utilized to assess motor development milestones, e.g., in patients with type 2 and non-ambulatory type 3 SMA.

In some embodiments, a patient with SMA (e.g., later-onset SMA) is evaluated using the Endurance Shuttle Nine Hole Peg Test (ESNHPT). The ESNHPT is an endurance test for severely affected patients with SMA. Patients are instructed to repeatedly perform the original 9 Hole Peg Test at 75% of their individual maximum speed. The shuttles refer to the set speed marked by auditory cues, with the test ending when the patient missed 2 consecutive beeps. Primary outcome parameter is "time to limitation (Tlim)," the time a task can be maintained at the pre-set intensity. Maximum test duration is 20 minutes (Stam et al. (2018) BMJ Open. 8(7):e019932). In some embodiments, the ESNHPT is used to evaluate non-ambulatory SMA patients. In some embodiments, the ESNHPT is completed by patients who are 8 years of age or older.

In some embodiments, a patient with SMA (e.g., later-onset SMA) is evaluated using the Endurance Shuttle Box and Block Test (ESBBT). ESBBT is an endurance test for moderately affected patients with SMA. Patients are instructed to repeatedly perform the original Box and Block Test at 75% of their individual maximum speed. The shuttles refer to the set speed marked by auditory cues, with the test ending when the patient missed 2 consecutive beeps. Primary outcome parameter is "time to limitation (Tlim)," the time a task can be maintained at the pre-set intensity. Maximal test duration is 20 minutes (Stam et al. (2018) BMJ Open. 8(7):e019932). In some embodiments, the ESBBT is used to evaluate non-ambulatory SMA patients. In some embodiments, the ESBBT is completed by patients who are 8 years of age or older.

In some embodiments, a patient with SMA (e.g., later-onset SMA) is evaluated using the Pediatric Evaluation of Disability Inventory Computer Adaptive Test (PEDICAT). In some embodiments, a caregiver (who may or may not be a parent and/or legal guardian) completes the PEDICAT assessment. In some embodiments, the PEDICAT assessment is not administered to the patient or if a caregiver is not present. The PEDICAT is a questionnaire completed by the caregiver that assesses the patient's ability to perform daily functions (Haley et al. (2005) Arch Phys Med Rehabil. 86(5):932-939). In some embodiments, the PEDICAT is filled out by the caregiver in a location where they are not watching the patient perform any functional assessment tests. The answers are scored on a 4-point scale (unable to easy). The test is suitable to assess function in newborns to 21-year-olds. Properties of the PEDICAT have been studied in the SMA population. A Rasch analysis with results published in 2016 revealed that the distribution of abilities for the Mobility and Daily Activities domains of the PEDICAT are best represented in the type 2 and type 3 populations (Pasternak et al. (2016) Muscle Nerve. 54(6):1097-1107).

In some embodiments, a patient with SMA (e.g., later-onset SMA) is evaluated using the Patient-reported Outcomes Measurement Information System (PROMIS). The PROMIS is a person-centered measure intended to be completed by the patient or parent proxy without help from anyone (Ader (2007) Med Care. 5(5):S1-S2). The fatigue profile domain measures a range of symptoms, from mild subjective feelings of tiredness to an overwhelming, debilitating, and sustained sense of exhaustion. The self-report measures are suitable for children 8-17 years old and the parent proxy report measures are suited for children 5-17 years old. Patients age 18 through 21 may complete an adult form of PROMIS. In some embodiments, the PROMIS is completed by patients who are 5 years of age or older.

### Methods, Uses, and Compositions for Treating SMA

In various embodiments, apitegromab therapy is administered (alone or in combination with at least one additional therapy) in an amount to achieve an average serum concentration (C_{trough}) of between about 25 and 250 micrograms per milliliter. In some embodiments, apitegromab therapy is administered (alone or in combination with at least one additional therapy) in an amount to achieve an average serum concentration (Cₘₐₓ) of between about 25 and 700 micrograms per milliliter.

In some embodiments, the present disclosure provides a composition comprising apitegromab for use in treating SMA in a human subject, wherein the subject is administered apitegromab by intravenous infusion every 4 weeks or monthly for at least 6 months or 12 months in an amount sufficient to prevent or delay a reduction in a RHS score relative to a baseline score prior to treatment.

In some embodiments, the SMA is later-onset SMA. In some embodiments, the SMA is ambulatory type 3 SMA. In some embodiments, the subject is 5 to 21 years old.

In some cases, the apitegromab is administered in an amount to stabilize or produce a less than 0.4 point decrease in an RHS score, e.g., after at least 12 months of treatment. In some embodiments, the apitegromab is administered in an amount to produce an at least 0.2 point mean increase in a RHS score relative to a baseline score prior to treatment, optionally a 0.3 point, 0.5 point, at least 0.7 point, at least 1 point, at least 2 point, or at least 3 point mean increase in a RHS score relative to a baseline, e.g., a mean increase in a cohort of at least 11 subjects. In some embodiments, the apitegromab is administered in an amount to produce an at least 1 point mean increase in a RHS score relative to a baseline, e.g., a mean increase in a cohort of at least 11 subjects. In some embodiments, the apitegromab is administered in an amount to produce an at least 3 point mean increase in a RHS score relative to a baseline, e.g., a mean increase in a cohort of at least 11 subjects. In some embodiments, the apitegromab is administered in an amount to stabilize an RHS score, e.g., as evaluated after at least 12 months of treatment relative to baseline. In some cases, the apitegromab is administered in an amount to limit a reduction in an RHS score, e.g., as evaluated after at least 12 months of treatment relative to baseline, of about 0.5, 0.4, 0.3, 0.2 or 0.1.

In some embodiments, the subject is treated with an SMN upregulator therapy. In some embodiments, the SMN upregulator therapy is nusinersen, risdiplam, and/or onasemnogene abeparvovec. In some embodiments, the SMN regulator therapy is nusinersen. In some embodiments, the subject initiated the SMN upregulator therapy at or after the age of 5. In some cases, the apitegromab is administered in an amount to stabilize an RHS score, e.g., as evaluated after at least 12 months of treatment relative to baseline. In some cases, the apitegromab is administered in an amount to limit a reduction in an RHS score, e.g., as evaluated after at least 12 months of treatment relative to baseline, of about 0.5, 0.4, 0.3, 0.2 or 0.1. In some embodiments, the apitegromab is administered in an amount to produce an at least 0.3 point mean increase in a RHS score relative to a baseline, e.g., a mean increase in a cohort of at least 11 subjects. In some embodiments, the apitegromab is administered in an amount to produce an at least 0.3 point mean increase in a RHS score relative to a baseline, e.g., a mean increase in a cohort of at least 11 subjects, after 8 weeks.

In some embodiments, the subject is not treated with an SMN upregulator therapy. In some cases, the apitegromab is administered in an amount to stabilize an RHS score, e.g., as evaluated after at least 12 months of treatment relative to baseline. In some cases, the apitegromab is administered in an amount to limit a reduction in an RHS score, e.g., as evaluated after at least 12 months of treatment relative to baseline, of about 0.5, 0.4, 0.3, 0.2 or 0.1. In some embodiments, the apitegromab is administered in an amount to produce an at least 0.7 point mean increase in a RHS score relative to a baseline, e.g., a mean increase in a cohort of at least 11 subjects. In some embodiments, the apitegromab is administered in an amount to produce an at least 0.7 point mean increase in a RHS score relative to a baseline, e.g., a mean increase in a cohort of at least 11 subjects, after 8 weeks.

In some embodiments, the apitegromab is administered in an amount to achieve a serum concentration (C_{trough}) of at least about 100 micrograms per milliliter, e.g., about 100 to 500 micrograms per milliliter (e.g., about 100, 200, 300, 400, or 500 micrograms per milliliter), e.g., as determined in an average over at least a 12-month course of treatment. In some embodiments, the serum concentration (C_{trough}) is about 100-450 micrograms per milliliter.

In some embodiments, the apitegromab is administered in an amount to achieve a serum concentration (Cₘₐₓ) of at least about 300 micrograms per milliliter, e.g., as determined in an average over at least a 12-month course of treatment. In some embodiments, the serum concentration (Cₘₐₓ) is at least about 300-1100 micrograms per milliliter, e.g., 300-800. In some embodiments, the serum concentration (Cₘₐₓ) is at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days.

In some embodiments, the apitegromab is administered in an amount sufficient to achieve a target engagement as measured by a serum latent myostatin concentration of at least about 100 nanograms per milliliter, as determined at steady state over the course of at least 12 months of treatment. In some embodiments, the apitegromab is administered in an amount sufficient to achieve a target engagement as measured by a serum latent myostatin concentration of at least about 250 nanograms per milliliter, as determined at steady state over the course of at least 12 months of treatment. In some embodiments, the apitegromab is administered in an amount sufficient to achieve a target engagement as measured by a serum latent myostatin concentration of at least about 400 nanograms per milliliter. In some embodiments, the apitegromab is administered in an amount sufficient to achieve a target engagement as measured by a serum latent myostatin concentration of at least 500 nanograms per milliliter (e.g., at least 500, 550, 600, 650, 700, 750, 800, 850, 900, 950, 1000, 1050 or 1100 nanograms per milliliter). In some embodiments, the serum latent myostatin concentration is between about 550-2400 nanograms per milliliter. In some embodiments, the serum latent myostatin concentration is a steady-state concentration measured at trough.

In some embodiments, the present disclosure provides a composition comprising apitegromab for use in treating SMA in a human subject receiving an SMN upregulator, wherein the subject is administered the apitegromab by intravenous infusion every 4 weeks or monthly for at least 6 months or 12 months in an amount sufficient to produce an at least 0.5 or 1 point mean increase in a HFMSE score relative to a baseline score prior to treatment, e.g., a mean increase in a cohort of at least 13 or 14 subjects.

In some embodiments, the SMA is later-onset SMA. In some embodiments, the SMA is type 2 SMA. In some embodiments, the SMA is non-ambulatory type 3 SMA. In some embodiments, the subject is 5 to 21 years old. In some embodiments, the SMN upregulator therapy is nusinersen, risdiplam, and/or onasemnogene abeparvovec. In some embodiments, the SMN upregulator therapy is nusinersen.

In some embodiments, the apitegromab is administered in an amount to produce an at least 0.5 point or 1 point increase in a HFMSE score relative to a baseline, e.g., a mean increase in a cohort of at least 13 or 14 subjects, after 16 weeks. In some embodiments, the apitegromab is administered in an amount to produce an at least 2 point, at least 3 point, at least 4 point, or at least 5 point increase in a HFMSE score relative to a baseline, e.g., a mean increase in a cohort of at least 13 or 14 subjects. In some embodiments, the apitegromab is administered in an amount to produce an at least 3 point increase in a HFMSE score relative to a baseline, e.g., a mean increase in a cohort of at least 13 or 14 subjects. In some embodiments, the apitegromab is administered in an amount to produce an at least 5 point increase in a HFMSE score relative to a baseline, e.g., a mean increase in a cohort of at least 13 or 14 subjects.

In some embodiments, the apitegromab is administered in an amount to achieve a serum concentration (Cₘₐₓ) of at least about 300 micrograms of apitegromab per milliliter, e.g., at least about 400, 500, 600, 700, 800, 900, 1000, or 1100. In some embodiments, the apitegromab is administered in an amount to achieve a serum concentration of at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days.

In some embodiments, the apitegromab is administered in an amount to achieve a serum concentration (C_{trough}) of at least about 100 micrograms per milliliter, e.g., at least about 200, 300, or 400 micrograms per milliliter.

In some embodiments, the apitegromab is administered in an amount sufficient to achieve a target engagement as measured by a serum latent myostatin concentration of at least about 250 nanograms per milliliter, e.g., at least about 400 or 500 nanograms per milliliter (e.g., at least 500, 550, 600, or 650 nanograms per milliliter). In some embodiments, the serum latent myostatin concentration is between about 550-1650 nanograms per milliliter. In some embodiments, the serum latent myostatin concentration is a steady-state concentration measured at trough..

In some embodiments, the present disclosure provides a composition comprising apitegromab for use in treating SMA in a human subject receiving an SMN upregulator, wherein the subject is administered apitegromab by intravenous infusion every 4 weeks or monthly for at least 6 months or 12 months in an amount sufficient to produce an at least 2 point mean increase in a HFMSE score relative to a baseline score prior to treatment, e.g., a mean increase in a cohort of at least 8 or 9 subjects, and wherein the subject initiated the SMN upregulator therapy before the age of 5.

In some embodiments, the SMA is later-onset SMA. In some embodiments, the SMA is type 2 SMA. In some embodiments, the subject is 2 years of age or older. In some embodiments, the SMN upregulator therapy is nusinersen, risdiplam, and/or onasemnogene abeparvovec. In some embodiments, the SMN upregulator therapy is nusinersen.

In some embodiments, the apitegromab is administered in an amount to produce an at least 2 point mean increase in a HFMSE score relative to a baseline, e.g., a mean increase in a cohort of at least 8 or 9 subjects, after 8 weeks or after 12 months. In some embodiments, the apitegromab is administered in an amount to produce an at least 3 point, at least 4 point, at least 5 point, at least 6 point, or at least 7 point mean increase in a HFMSE score relative to a baseline, e.g., a mean increase in a cohort of at least 8 or 9 subjects, e.g., after at least 12 months of treatment. In some embodiments, the apitegromab is administered in an amount to produce an at least 3 point mean increase in a HFMSE score relative to a baseline, e.g., a mean increase in a cohort of at least 8 or 9 subjects. In some embodiments, the apitegromab is administered in an amount to produce an at least 5 point mean increase in a HFMSE score relative to a baseline, e.g., a mean increase in a cohort of at least 8 or 9 subjects. In some embodiments, the apitegromab is administered in an amount to produce an at least 6 point mean increase in a HFMSE score relative to a baseline, e.g., a mean increase in a cohort of at least 8 or 9 subjects. In some embodiments, the apitegromab is administered in an amount to produce an at least 7 point mean increase in a HFMSE score relative to a baseline, e.g., a mean increase in a cohort of at least 8 or 9 subjects.

In some embodiments, the apitegromab is administered in an amount to achieve a serum concentration (Cₘₐₓ) of at least about 25-1100 micrograms of apitegromab per milliliter. In some cases, the apitegromab is administered at 2 mg/kg to obtain a serum concentration of about 25-55 micrograms per milliliter. In some cases, the apitegromab is administered at greater than 2 mg/kg and up to 20 mg/kg to obtain a serum concentration of about 250-1100 micrograms per milliliter. In some embodiments, the serum concentration is 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days or after about five half lives. In some cases, the amount greater than 2 mg/kg and up to 20 mg/kg is a dose of 20 mg/kg apitegromab every 4 weeks or monthly. Increasing the dose of apitegromab may yield a dose response in serum concentration (Cₘₐₓ), e.g., increasing the dose ten fold from 2 mg/kg and up to 20 mg/kg yields a ten fold increase in Cₘₐₓ.

In some embodiments, the apitegromab is administered in an amount to achieve a serum concentration (C_{trough}) of at least about 25 micrograms of apitegromab per milliliter, e.g., at least about 50, 100, 200, 300, or 400 micrograms of apitegromab per milliliter. In some cases, the apitegromab is administered at 2 mg/kg to obtain a serum concentration of about 25-55 micrograms per milliliter. In some cases, the apitegromab is administered at greater than 2 mg/kg and up to 20 mg/kg to obtain a serum concentration of about 100-400 micrograms per milliliter. In some cases, the amount greater than 2 mg/kg and up to 20 mg/kg is a dose of 20 mg/kg apitegromab every 4 weeks or monthly. Increasing the dose of apitegromab may yield a dose response in serum concentration (C_{trough}), e.g., increasing the dose ten fold from 2 mg/kg and up to 20 mg/kg yields a ten fold increase in C_{trough}.

In some embodiments, the apitegromab is administered in an amount sufficient to achieve a target engagement as measured by a serum latent myostatin concentration of at least about 250 nanograms per milliliter, e.g., at least about 400 nanograms per milliliter or 500 nanograms per milliliter (e.g., at least 500, 550, 600, or 650 nanograms per milliliter). In some embodiments, the serum latent myostatin concentration is between about 550-1100 or 600-1000 nanograms per milliliter. In some embodiments, the serum latent myostatin concentration is a steady-state concentration measured at trough.

In some embodiments, the present disclosure provides a composition comprising apitegromab for use in treating SMA, wherein a patient population is administered apitegromab by intravenous infusion every 4 weeks or monthly for at least 6 months or 12 months in an amount sufficient to increase a clinical response rate (proportion of responders) in the patient population, and wherein the clinical response rate is measured by a 6-minute Walk Test and/or a 30-second Sit-to-Stand Test.

In some embodiments, the patient population is a later-onset SMA population. In some embodiments, the patient population is an ambulatory type 3 SMA population. In some embodiments, the patient population comprises human subjects 5 to 21 years old. In some embodiments, the patient population is treated with an SMN upregulator therapy. In some embodiments, the SMN upregulator therapy is nusinersen, risdiplam, and/or onasemnogene abeparvovec. In some embodiments, the SMN regulator therapy is nusinersen. In some embodiments, the patient population initiated the SMN upregulator therapy after the age of 5.

In some embodiments, the apitegromab is administered in an amount to achieve a serum concentration of at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days.

In some embodiments, the apitegromab is administered in an amount sufficient to achieve a target engagement as measured by a serum latent myostatin concentration of at least 500 nanograms per milliliter (e.g., at least 500, 550, 600, or 650 nanograms per milliliter). In some embodiments, the serum latent myostatin concentration is between about 550-1100 or 600-1000 nanograms per milliliter. In some embodiments, the serum latent myostatin concentration is a steady-state concentration measured at trough.

In some embodiments, the present disclosure provides a composition comprising apitegromab for use in treating SMA, wherein a patient population is administered apitegromab by intravenous infusion every 4 weeks or monthly for at least 6 months or 12 months in an amount sufficient to increase a clinical response rate (proportion of responders) in the patient population, and wherein the clinical response rate is measured by RULM and/or WHO milestones.

In some embodiments, the patient population is a later-onset SMA population. In some embodiments, the patient population is a type 2 SMA population. In some embodiments, the patient population is a non-ambulatory type 3 SMA population. In some embodiments, the patient population comprises human subjects ages 2 or older. In some embodiments, the patient population comprises human subjects 5 to 21 years old. In some embodiments, the patient population is treated with an SMN upregulator therapy. In some embodiments, the SMN upregulator therapy is nusinersen, risdiplam, and/or onasemnogene abeparvovec. In some embodiments, the SMN regulator therapy is nusinersen.

In some embodiments, the apitegromab is administered in an amount to achieve a serum concentration of at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days.

In some embodiments, the apitegromab is administered in an amount sufficient to achieve a target engagement as measured by a serum latent myostatin concentration of at least 500 nanograms per milliliter (e.g., at least 500, 550, 600, or 650 nanograms per milliliter). In some embodiments, the serum latent myostatin concentration is between about 550-1100 or 600-1000 nanograms per milliliter. In some embodiments, the serum latent myostatin concentration is a steady-state concentration measured at trough.

In some embodiments, the present disclosure provides apitegromab for use in treating later-onset SMA in a human subject who is 2 years of age or older, wherein a dose of 10 mg/kg of the apitegromab is administered by intravenous infusion monthly.

In some embodiments, the apitegromab is administered as an adjunct to an SMN upregulator therapy. In some embodiments, the apitegromab is administered as an adjunct to an SMN upregulator therapy comprising nusinersen.

In some embodiments, the apitegromab is administered in an amount to achieve a serum concentration of at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days.

In some embodiments, the apitegromab is administered in an amount sufficient to achieve a target engagement as measured by a serum latent myostatin concentration of at least 500 nanograms per milliliter (e.g., at least 500, 550, 600, or 650 nanograms per milliliter). In some embodiments, the serum latent myostatin concentration is between about 550-1100 or 600-1000 nanograms per milliliter. In some embodiments, the serum latent myostatin concentration is a steady-state concentration measured at trough.

In some embodiments, the present disclosure provides apitegromab for use in treating later-onset SMA in a human subject, wherein the treatment comprises administering to the subject 10 mg/kg of apitegromab every 4 weeks or monthly, wherein the subject initiated a motor neuron-directed therapy for SMA before the age of 5, and wherein the motor neuron-directed therapy increases SMN1 or SMN2 expression. R

In some embodiments, the treatment results in at least a 2 point mean increase in a HFMSE score relative to a baseline score prior to treatment, e.g., a mean increase in a cohort of at least 8 or 9 subjects, after 6 months or 12 months. In some embodiments, the motor neuron-directed therapy is nusinersen, risdiplam, and/or onasemnogene abeparvovec. In some embodiments, the motor neuron-directed therapy is nusinersen. In some embodiments, the apitegromab is administered by intravenous infusion. In some embodiments, the later-onset SMA patient is also categorized as a type 2 SMA, type 3 SMA, or type 4 SMA patient. In some embodiments, the type 3 SMA is ambulatory or non-ambulatory.

In some embodiments, the apitegromab is administered in an amount to achieve a serum concentration of at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days. In some embodiments, the apitegromab is administered in an amount sufficient to achieve a target engagement as measured by a serum latent myostatin concentration of at least 500 nanograms per milliliter (e.g., at least 500, 550, 600, or 650 nanograms per milliliter). In some embodiments, the serum latent myostatin concentration is between about 550-1100 or 600-1000 nanograms per milliliter. In some embodiments, the serum latent myostatin concentration is a trough concentration.

In some embodiments, the present disclosure provides apitegromab for use in treating SMA in a human subject who did not receive an SMN upregulator therapy before the age of 5, wherein the treatment comprises administering to the subject a dose of 10 mg/kg of the apitegromab every 4 weeks or monthly, wherein the apitegromab stabilizes disease progression, such that a HFMSE score or a RHS score measured at 6 months after initiation of the treatment is no less than a baseline, and wherein the baseline is obtained at or prior to initiation of the treatment.

In some embodiments, the apitegromab is administered by intravenous infusion.

In some embodiments, the apitegromab is administered in an amount to achieve a serum concentration of at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days. In some embodiments, the apitegromab is administered in an amount sufficient to achieve a target engagement as measured by a serum latent myostatin concentration of at least 500 nanograms per milliliter (e.g., at least 500, 550, 600, or 650 nanograms per milliliter). In some embodiments, the serum latent myostatin concentration is between about 550-1100 or 600-1000 nanograms per milliliter. In some embodiments, the serum latent myostatin concentration is a trough concentration.

In some embodiments, the present disclosure provides apitegromab for use in the treatment of later-onset SMA in a human subject, wherein the treatment comprises administering apitegromab in an amount to achieve a maximum serum concentration of at least 600 micrograms of apitegromab per milliliter (e.g., at least 600, 700, 800, 900, 1000 micrograms per milliliter or greater).

In some embodiments, the maximum serum concentration is at least 600 micrograms per milliliter. In some embodiments, the maximum serum concentration is between about 600-1000 micrograms per milliliter. In some embodiments, the serum concentration is a steady-state serum concentration. In some embodiments, the apitegromab is administered in an amount sufficient to produce an at least 1 point mean increase in a HFMSE score relative to a baseline score prior to treatment, e.g., a mean increase in a cohort of at least 8 or 9 subjects. In some embodiments, the apitegromab is administered in an amount sufficient to produce an at least 2 point mean increase in a HFMSE score relative to a baseline score prior to treatment, e.g., a mean increase in a cohort of at least 9 subjects. In some embodiments, the later-onset SMA patient is a type 2 or type 3 SMA patient, and optionally wherein the subject is on an SMN upregulator therapy, and optionally wherein the subject initiated the SMN upregulator therapy before the age of 5.

In some embodiments, the present disclosure provides apitegromab for use in the treatment of later-onset SMA in a human subject, wherein the treatment comprises administering apitegromab in an amount sufficient to achieve a target engagement as measured by a serum latent myostatin concentration of at least 500 nanograms per milliliter (e.g., at least 500, 550, 600, or 650 nanograms per milliliter).

In some embodiments, the serum latent myostatin concentration is between about 550-1100 or 600-1000 nanograms per milliliter. In some embodiments, the serum latent myostatin concentration is a steady-state concentration. In some embodiments, the apitegromab is administered in an amount sufficient to produce an at least 1 point mean increase in a HFMSE score relative to a baseline score prior to treatment, e.g., a mean increase in a cohort of at least 8 or 9 subjects. In some embodiments, the apitegromab is administered in an amount sufficient to produce an at least 2 point mean increase in a HFMSE score relative to a baseline score prior to treatment, e.g., a mean increase in a cohort of at least 9 subjects. In some embodiments, the later-onset SMA is in a patient categorized as having type 2 or type 3 SMA, and optionally wherein the subject is on an SMN upregulator therapy, and optionally wherein the subject initiated the SMN upregulator therapy before the age of 5.

In some cases, the present disclosure provides a composition comprising apitegromab for use in the treatment of SMA in a human subject, wherein the treatment comprises administration of apitegromab at a dose sufficient to achieve at least 600 micrograms per milliliter of serum exposure, which produces a clinical benefit characterized by: motor function improvement, disease stabilization, or delay in disease progression.

In some embodiments, the SMA is a later-onset SMA, wherein optionally the later-onset SMA is type 2 SMA, type 3 SMA, or type 4 SMA, wherein further optionally the type 3 SMA is ambulatory or non-ambulatory. In some embodiments, the SMA is a later-onset SMA, wherein optionally the later-onset SMA is phenotypically characterized by symptom onset of six months of age or later. In some embodiments, the SMA is a later-onset SMA, wherein optionally the later-onset SMA is genotypically characterized by having two or more copies of the SMN2 gene, wherein optionally the subject carries 2, 3, 4, 5, or 6 copies of the SMN2 gene.

In some embodiments, the human subject initiated an SMN upregulator therapy before the age of 5, wherein optionally the subject has a baseline HFMSE score of between 12-44 or 14-42 prior to or at the time of initiation of the apitegromab treatment. In some embodiments, the human subject initiated an SMN upregulator therapy at or after the age of 5, wherein optionally: a) the subject has a baseline HFMSE score of between 13-39, and/or b) the subject has a baseline RHS score of between 44-62, prior to or at the time of initiation of the apitegromab treatment. In some embodiments, the human subject is not treated with an SMN upregulator therapy, wherein optionally the subject has a baseline RHS score of between 26-63.

In some embodiments, the motor function improvement comprises an at least 1 point increase over a baseline in a motor function assessment score after six months of treatment with the apitegromab. In some embodiments, the motor function improvement comprises an at least 2 point increase over a baseline in a motor function assessment score after six months of treatment with the apitegromab. In some embodiments, the motor function improvement comprises an at least 3 point increase over a baseline in a motor function assessment score after six months of treatment with the apitegromab. In some embodiments, the motor function improvement comprises an at least 4 point increase over a baseline in a motor function assessment score after six months of treatment with the apitegromab. In some embodiments, the motor function improvement comprises an at least 5 point increase over a baseline in a motor function assessment score after six months of treatment with the apitegromab. In some embodiments, the disease stabilization comprises no net loss in a motor function assessment score after six months of treatment with the apitegromab, wherein optionally, the subject is classified in a patient population that statistically manifests disease progression characterized by a decline in a motor function assessment score over a 12 month period. In some embodiments, the motor function assessment score is a HFMSE score or a RHS score.

In some cases, the present disclosure provides a method of achieving a target saturation in a SMA patient using apitegromab, the method comprising administering to the SMA patient about 10 mg/kg or about 20 mg/kg of apitegromab by intravenous infusion every 4 weeks or monthly.

In some embodiments, the present disclosure provides a method of achieving a target saturation in a SMA patient using apitegromab, the method comprising administering to the SMA patient apitegromab at a dose sufficient to achieve at least 600 micrograms per milliliter of serum exposure, wherein optionally the apitegromab is administered by intravenous infusion every 4 weeks or monthly.

In some embodiments, the present disclosure provides a a composition comprising apitegromab for use in a method of treating ambulatory SMA in a human subject, the method comprising administering to the subject a composition comprising apitegromab by intravenous infusion every 4 weeks or monthly for at least 6 months, wherein the subject has a baseline RHS score of at least 26 prior to or at initiation of the apitegromab treatment, and wherein the apitegromab treatment that achieves a serum exposure of at least 600 micrograms per milliliter of apitegromab is sufficient to improve or stabilize the disease. In some embodiments, the subject initiated an SMN upregulator therapy at or after the age of 5. In some embodiments, the baseline RHS score is no greater than 63.

In some embodiments, the present disclosure provides a a composition comprising apitegromab for use in a method of treating type 2 or non-ambulatory type 3 SMA in a human subject, the method comprising administering to the subject a composition comprising apitegromab by intravenous infusion every 4 weeks or monthly for at least six months, wherein the subject initiated an SMN upregulator therapy at or after the age of 5, and wherein the subject has a baseline HFMSE score of at least 13, no greater than 39, or both (between 13-39) prior to or at initiation of the apitegromab treatment that achieves a serum exposure of at least 600 micrograms per milliliter. In some embodiments, the composition is sufficient to achieve at least 1 point increase in the HFMSE score over the baseline after six months.

In some embodiments, the present disclosure provides a a composition comprising apitegromab for use in a method of treating type 2 or non-ambulatory SMA in a human subject, the method comprising administering to the subject a composition comprising apitegromab by intravenous infusion every 4 weeks or monthly for at least six months, wherein the subject initiated an SMN upregulator therapy prior to the age of 5, and the subject has a baseline HFMSE score of at least 12, no greater than 44, or both (between 12-44) prior to or at initiation of the apitegromab treatment that achieves a serum exposure of at least 600 micrograms per milliliter. In some embodiments, the composition is sufficient to achieve an at least 1 point or 3 point increase in the HFMSE score over the baseline after six months.

In some cases, the present disclosure provides a selective myostatin inhibitor for use in the treatment of later-onset SMA in a human patient, wherein the human patient has a non-ambulatory form of SMA, wherein the patient has a baseline HFMSE score ranging 12-44, and wherein the patient is treated with an SMN upregulator therapy, wherein optionally, the selective myostatin inhibitor is an antibody that specifically binds a latent myostatin complex, thereby preventing release of mature myostatin. In some cases, the selective myostatin inhibitor is a neutralizing antibody or a ligand trap that binds mature myostatin.

In some cases, the present disclosure provides a selective myostatin inhibitor and an SMN upregulator for use in the treatment of later-onset SMA in a human patient, wherein the patient has a baseline HFMSE score of no more than 44, wherein optionally, the selective myostatin inhibitor is an antibody that specifically binds a latent myostatin complex, thereby preventing release of mature myostatin, wherein further optionally, the SMN upregulator is an SMN2 upregulator and/or an SMN1 gene therapy. In some cases, the selective myostatin inhibitor is a neutralizing antibody or a ligand trap that binds mature myostatin.

In some embodiments, the present disclosure provides a composition comprising apitegromab for use in the treatment of SMA in a human subject, wherein the treatment comprises intravenous administration of the composition to the subject at a dose of 10 mg/kg of apitegromab every 4 weeks or monthly, wherein the subject initiates or initiated an SMN upregulator therapy before the age of 5. In some embodiments, the SMA is later-onset SMA. In some embodiments, the subject has two or more copies of the SMN2 gene. In some embodiments, the SMA is type 2 SMA, wherein optionally, the subject has 2-4 copies of the SMN2 gene. In some embodiments, the subject is non-ambulatory. In some embodiments, the subject is 2 years of age or older. In some embodiments, the subject is genetically identified as a carrier of a mutation in the SMN1 gene, wherein optionally, the subject is asymptomatic or symptomatic of SMA. In some embodiments, the subject has a baseline HFMSE score of at least 12, prior to or at the time of receiving a first dose of apitegromab. In some embodiments, the composition is administered to the subject in an amount sufficient to achieve sustained (steady-state) target engagement of at least about 250 ng/mL of serum latent myostatin. In some embodiments, the subject has received at least 4 doses of the SMN upregulator therapy at the time of receiving a first dose of apitegromab. In some embodiments, the subject has type 2 SMA. In some embodiments, the apitegromab is administered in a dose sufficient to produce at least a 5 point increase in a HFMSE score relative to a baseline score after at least 12 months of treatment. In some embodiments, the apitegromab is administered in a dose sufficient to produce a 6-20 point or greater increase in a HFMSE score, and preferably at least a 7 point increase, relative to a baseline score after at least 12 months of treatment. In some embodiments, the composition is formulated at a concentration of about 50 mg/mL.

In some embodiments, the present disclosure provides a method of obtaining an at least 7 point increase in a HFMSE score in a human subject, relative to a baseline score, comprising administering to the subject a composition comprising 10 mg/kg of apitegromab by intravenous infusion every 4 weeks or monthly for at least 12 months, wherein the subject has later-onset SMA and initiated treatment with nusinersen before the age of 5.

In some embodiments, the present disclosure provides a method for monitoring treatment of SMA in a human subject, comprising administering to the subject a composition comprising apitegromab by intravenous infusion every 4 weeks or monthly in an amount of 10 mg/kg; and detecting a serum latent myostatin concentration, wherein a serum latent myostatin concentration of at least about 250 ng/mL indicates treatment efficacy, wherein the subject has later-onset (optionally type 2) SMA and initiated treatment with nusinersen before the age of 5. In some embodiments, the subject is non-ambulatory. In some embodiments, the subject is 2 years of age or older. In some embodiments, the method further comprises administering an additional dose of apitegromab to the subject having a serum latent myostatin concentration of at least about 250 ng/mL.

In some embodiments, the present disclosure provides a a composition comprising apitegromab for use in a method of treating SMA in a human subject, comprising administering to the subject a composition comprising apitegromab, wherein the apitegromab is administered every 4 weeks or monthly in an amount of 10 mg/kg, wherein up to three consecutive doses of the apitegromab may be skipped if the subject has a serum latent myostatin concentration of at least about 250 ng/mL, wherein the subject has late onset SMA and initiated an SMN upregulator therapy before the age of 5. In some embodiments, the apitegromab is administered by intravenous infusion. In some embodiments, the subject is non-ambulatory and/or has type 2 SMA. In some embodiments, the subject is 2 years of age or older.

In some embodiments, the present disclosure provides a a composition comprising apitegromab for use in a method of treating SMA in a human subject, comprising administering to the subject a composition comprising apitegromab, and wherein the apitegromab is administered in an amount to obtain a serum latent myostatin concentration of at least about 100 ng/mL or preferably at least about 250 ng/mL at steady state in the subject, wherein the subject has later-onset (optionally type 2) SMA and initiated an SMN upregulator therapy before the age of 5. In some embodiments, the serum concentration is a steady state concentration. In some embodiments, the subject is non-ambulatory. In some embodiments, the subject is 2 years of age or older. In some embodiments, the apitegromab is administered by intravenous infusion

In some embodiments, the present disclosure provides a composition comprising apitegromab for use in the treatment of SMA in a human subject, wherein the treatment comprises intravenous administration of the composition to the subject at a dose of 10 mg/kg of apitegromab every 4 weeks or monthly, wherein the subject is treated with an SMN upregulator therapy, wherein the subject initiates or initiated the SMN upregulator therapy at or after the age of 5. In some embodiments, the SMA is type 2 SMA or type 3 SMA, wherein optionally, the subject has 2-4 copies of the SMN2 gene. In some embodiments, the subject has non-ambulatory type 3 SMA. In some embodiments, the subject is genetically identified as a carrier of a mutation in the SMN1 gene, wherein optionally, the subject is asymptomatic or symptomatic of SMA. In some embodiments, the subject has a baseline HFMSE score of at least 13, prior to or at the time of receiving a first dose of apitegromab. In some embodiments, the composition is administered to the subject in an amount sufficient to achieve sustained (steady-state) target engagement of at least about 100 ng/mL or preferably at least about 250 ng/mL of serum latent myostatin. In some embodiments, the subject is 5-21 years old. In some embodiments, the apitegromab is administered in a dose sufficient to achieve no or minimal reduction in a HFMSE score relative to a baseline score after at least 12 months of treatment. In some embodiments, the apitegromab is administered in a dose sufficient to produce an at least 1 point increase in a HFMSE score relative to a baseline score, and preferably at least a 3 point increase, after 12 months of treatment.

In some cases, the present disclosure provides a method of stabilizing disease progression in a human subject having non-ambulatory SMA, comprising administering to the subject a composition comprising apitegromab by intravenous infusion every 4 weeks or monthly for at least 12 months, wherein the subject initiates or initiated the SMN upregulator therapy at or after the age of 5. In some cases, the apitegromab is administered in a dose sufficient to achieve no or minimal reduction in a HFMSE score relative to a baseline score after at least 12 months of treatment.

In some embodiments, the present disclosure provides a composition comprising apitegromab for use in the treatment of SMA in a human subject, wherein the treatment comprises intravenous administration of the composition to the subject at a dose of 10 mg/kg of apitegromab every 4 weeks or monthly, wherein the subject has ambulatory SMA, and wherein optionally, the subject has not received an SMN upregulator therapy. In some embodiments, the subject has ambulatory type 3 SMA, wherein optionally, the subject has 3 or more copies of the SMN2 gene. In some embodiments, the subject is genetically identified as a carrier of a mutation in the SMN1 gene, wherein optionally, the subject is asymptomatic or symptomatic of SMA. In some embodiments, the subject has a baseline RHS score of at least 26, prior to or at the time of receiving a first dose of apitegromab. In some embodiments, the composition is administered to the subject in an amount sufficient to achieve sustained (steady-state) target engagement of at least about 100 ng/mL or preferably at least about about 250 ng/mL of serum latent myostatin. In some embodiments, the apitegromab is administered in a dose sufficient to stabilize an RHS score relative to a baseline score after at least 12 months of treatment. In some embodiments, stabilizing the RHS score comprises less than a 1 point, 2 point, or 3 point decline in the RHS score relative to a baseline score. In some embodiments, the subject initiated SMN upregulator therapy at or after the age of 5.

In some embodiments, the present disclosure provides a composition comprising apitegromab for use in a method of preventing or delaying loss of ambulation in a human subject having SMA, comprising administering a composition comprising apitegromab to the subject at a dose of 10 mg/kg of apitegromab every 4 weeks or monthly for at least 12 months, wherein the subject has ambulatory SMA, and wherein optionally, the subject has not received an SMN upregulator therapy. In some embodiments, the patient has ambulatory type 3 SMA. In some embodiments, the progression is assessed by a delay in ambulatory-to-non-ambulatory transition following 12 months of apitegromab treatment, relative to the natural history of an ambulatory SMA patient population. In some cases, the progression is assessed by a slower rate of or lesser degree of decline in a motor function score over a baseline following 12 months of apitegromab treatment, relative to the natural history of an ambulatory SMA patient population. In some embodiments, the composition is formulated at a concentration of about 50 mg/mL apitegromab. In some embodiments, the SMN upregulator therapy comprises nusinersen, onasemnogene abeparvovec-xioi, and/or risdiplam. In some embodiments, the SMN upregulator therapy comprises nusinersen. In some embodiments, the SMN upregulator therapy comprises intrathecal administration.

In some embodiments, the present disclosure provides an SMN upregulator therapy and a muscle-directed therapy for use in the treatment of SMA in a human subject, wherein the treatment comprises initiation of the SMN upregulator therapy prior to the age of 5, and wherein the muscle-directed therapy comprises intravenous administration of a composition comprising apitegromab every 4 weeks or monthly at a dose of 10 mg/kg. In some embodiments, the composition comprising apitegromab is formulated at a concentration of about 50 mg/mL. In some embodiments, the SMN upregulator therapy comprises nusinersen, onasemnogene abeparvovec-xioi, and/or risdiplam.

In some embodiments, the present disclosure provides a composition comprising apitegromab for use in a method for treating later-onset SMA in a human subject, the method comprising intravenously administering to the subject a composition comprising apitegromab at a therapeutic dose of 10 mg/kg every 4 weeks or monthly, wherein the composition is formulated at a concentration of about 50 mg/mL. In some embodiments, the therapeutic dose is a dose sufficient to achieve a steady-state serum concentration of at least about 100 ng/mL or preferably at least about 250 ng/mL of latent myostatin. In some embodiments, the subject has 2-4 copies of the SMN2 gene. In some embodiments, the subject has type 2 SMA. In some embodiments, the subject has or had the ability to sit up at age 12 months and lacks or lacked ability to walk at age 18 months. In some embodiments, the subject has non-ambulatory type 3 SMA. In some embodiments, the subject lost ability to walk by 18 months of age. In some embodiments, the subject has 3-6 copies of the SMN2 gene. In some embodiments, the subject has ambulatory type 3 SMA. In some embodiments, the subject has retained ability to walk at age 18 months. In some embodiments, the subject undergoes an ambulatory-to-non-ambulatory transition after 18 months of age. In some embodiments, the subject is treated with an SMN-directed therapy. In some embodiments, the subject initiates or initiated the SMN-directed therapy prior to age 5. In some embodiments, the subject initiates or initiated the SMN-directed therapy at age 5 or older. In some embodiments, the SMN-directed therapy is an SMN1-directed therapy and/or an SMN2-directed therapy. In some embodiments, the SMN1-directed therapy is a gene therapy. In some embodiments, the gene therapy comprises a viral vector comprising an SMN1 gene replacement. In some embodiments, the SMN2-directed therapy is or comprises a splice-modifying agent. In some embodiments, the splice-modifying agent comprises a nucleic acid that binds an SMN2 exon, thereby modifying splicing of the exon. In some embodiments, the splice-modifying agent comprises a low molecular weight compound that binds an SMN2 exon, thereby modifying splicing of the exon. In some embodiments, the therapeutic dose is sufficient to cause enhanced motor function in the subject after 12 months of the apitegromab therapy, wherein optionally the motor function is assessed by HFMSE or RHS. In some embodiments, the enhanced motor function comprises an increase in a motor function score over a baseline, wherein optionally the increase is at least 1 point, 3 points, 5 points, or 10 points. In some embodiments, the baseline comprises a motor function score assessed following an SMN-directed therapy. In some embodiments, the baseline comprises a motor function score assessed in a subject who has not received an SMN-directed therapy. In some cases, the therapeutic dose is sufficient to cause a delay in disease progression, relative to the natural history of a SMA patient population to which the subject is categorized.

In some embodiments, the present disclosure provides a composition comprising apitegromab for use in the treatment of later-onset SMA in a human subject, wherein the treatment comprises intravenous administration of the composition to the subject at a therapeutic dose of 10 mg/kg every 4 weeks or monthly, wherein optionally the composition is formulated at a concentration of about 50 mg/mL, wherein further optionally, the therapeutic dose is sufficient to achieve a steady-state serum concentration of at least about 100 ng/mL or preferably at least 250 ng/mL of latent myostatin. In some embodiments, the subject has 2-4 copies of the SMN2 gene. In some embodiments, the subject has type 2 SMA. In some embodiments, the subject has or had the ability to sit up at age 12 months and lacks or lacked ability to walk at age 18 months. In some embodiments, the subject has non-ambulatory type 3 SMA. In some embodiments, the subject lost ability to walk by 18 months of age. In some embodiments, the subject has 3-6 copies of the SMN2 gene. In some embodiments, the subject has ambulatory type 3 SMA. In some embodiments, the subject has retained ability to walk at age 18 months. In some embodiments, the subject undergoes an ambulatory-to-non-ambulatory transition after 18 months of age. In some embodiments, the subject is treated with an SMN-directed therapy. In some embodiments, the subject initiates or initiated the SMN-directed therapy prior to age 5. In some embodiments, the subject initiates or initiated the SMN-directed therapy at age 5 or older. In some embodiments, the SMN-directed therapy is an SMN1-directed therapy and/or an SMN2-directed therapy. In some embodiments, the SMN1-directed therapy is a gene therapy. In some embodiments, the gene therapy comprises a viral vector comprising an SMN1 gene replacement. In some embodiments, the SMN2-directed therapy is or comprises a splice-modifying agent. In some embodiments, the splice-modifying agent comprises a nucleic acid that binds an SMN2 exon, thereby modifying splicing of the exon. In some embodiments, the splice-modifying agent comprises a low molecular weight compound that binds an SMN2 exon, thereby modifying splicing of the exon. In some embodiments, the therapeutic dose is sufficient to cause enhanced motor function in the subject after 12 months of the apitegromab therapy, wherein optionally the motor function is assessed by HFMSE or RHS. In some embodiments, the enhanced motor function comprises an increase in a motor function score over a baseline, wherein optionally the increase is at least 1 point, 3 points, 5 points, or 10 points. In some embodiments, the baseline comprises a motor function score assessed following an SMN-directed therapy. In some embodiments, the baseline comprises a motor function score assessed in a subject who has not received an SMN-directed therapy. In some cases, the therapeutic dose is sufficient to cause a delay in disease progression, relative to the natural history of a SMA patient population to which the subject is categorized.

In some embodiments, the present disclosure provides a a composition comprising apitegromab for use in a method for treating non-ambulatory or later-onset SMA in a human subject in need thereof, the method comprising intravenously administering to the subject apitegromab at a Q4W (i.e., every 4 weeks) or monthly dosage of 10 mg/kg. In some embodiments, the monthly dosage is 10 mg/kg administered via intravenous infusion.

In some embodiments, the present disclosure provides a a composition comprising apitegromab for use in a method for treating later-onset SMA in a human subject in need thereof, the method comprising intravenously administering to the subject apitegromab in an amount sufficient to achieve a steady-state serum latent myostatin concentration of at least about 10, 20, 30, 40, 60, 80, 100, 200, 300 or 400 ng/mL

In some embodiments, the present disclosure provides a a composition comprising apitegromab for use in a method for treating non-ambulatory or later-onset SMA in a human subject in need thereof, the method comprising intravenously administering to the subject apitegromab in an amount sufficient to achieve a steady-state serum latent myostatin concentration of at least about 250 ng/mL.

In some cases, the present disclosure provides a method for slowing progression of SMA in a human subject with ambulatory SMA, the method comprising intravenously administering to the subject apitegromab at a Q4W (i.e., every 4 weeks) or monthly dosage of greater than 2 mg/kg and up to 20 mg/kg. In some embodiments, the subject has ambulatory type 3 SMA. In some embodiments, the progression is assessed by a delay in ambulatory-to-non-ambulatory transition following 12 months of apitegromab treatment, relative to the natural history of an ambulatory SMA patient population. In some cases, the progression is assessed by a slower rate of or lesser degree of decline in a motor function score over a baseline following 12 months of apitegromab treatment, relative to the natural history of an ambulatory SMA patient population.

It will be readily apparent to those skilled in the art that other suitable modifications and adaptations of the methods of the invention described herein are obvious and may be made using suitable equivalents without departing from the scope of the invention or the embodiments disclosed herein. Having now described the invention in detail, the same will be more clearly understood by reference to the following examples, which are included for purposes of illustration only and are not intended to be limiting.

### EXAMPLES

### Example 1. Active Treatment Study of Apitegromab in Patients with Later-Onset SMA

In SMA, there are now three approved SMN upregulators available to help address the underlying genetic defect that causes SMA, but patients may still have significant impairments in motor function. With such SMN upregulators (also known as SMN correctors) now available to help stabilize the SMA disease course, a complementary muscle-directed therapy aimed at driving improvements in motor function is desirable. In addition to improving motor function, such a therapy would have a safety profile that enables chronic dosing, including in a pediatric population, have a low drug administration burden, and may be applicable broadly across the SMA population.

Apitegromab, a fully human monoclonal antibody that binds to human pro/latent myostatin, is a muscle-directed therapy intended to complement the disease-stabilizing benefits of SMN upregulators. By targeting the latent form of myostatin, apitegromab is able to avoid closely related growth factors that play varying roles outside of muscle biology.

To evaluate the safety and efficacy of apitegromab in later-onset SMA patients (e.g., type 2 and type 3 SMA patients) age 2 through 21 years old, patients are administered apitegromab on top of an approved SMA treatment or apitegromab as monotherapy. With the exception of the monotherapy patients, all patients receive background nusinersen. Such patients are required to be past the loading phase of treatment, and these patients had received an average of about 5 maintenance doses of nusinersen, which translates to about 2 years of treatment, before enrolling into the study. Even after 2 years on nusinersen, the baseline HFMSE scores for these patients continued to be in the low to mid 20s.

In this study (also referred to as "TOPAZ"; ClinicalTrials.gov Identifier: NCT03921528), approximately 58 male and female patients with later-onset SMA are enrolled across 3 separate parallel subpopulations subsequently described as cohorts. Patients receive apitegromab every 4 weeks or monthly during the 52-week Treatment Period, with patients in Cohorts 1 and 2 directly assigned high dose (20 mg/kg) apitegromab and patients in Cohort 3 randomized 1:1 double-blind between low dose (2 mg/kg) and high dose (20 mg/kg) apitegromab.
- Cohort 1, N=23: Ambulatory type 3 patients, age 5 through 21 years old, approximately 10 of whom are not receiving an approved SMA treatment, as well as patients already receiving an approved SMA treatment that had been started after the patient turned 5 years old.
- Cohort 2, N=15: Type 2 and non-ambulatory type 3 patients, age 5 through 21 years old, already receiving an approved SMA treatment that had been started after the patient turned 5 years old.
- Cohort 3, N=20: Type 2 patients, age ≥2 years old, already receiving an approved SMA treatment that had been started before the patient turned 5 years old.

During the Screening Period, all patient screening and eligibility determinations are conducted after informed consent (and, as required by local authorities, patient informed assent) has been provided and within 28 days prior to first dose. Screening motor function outcome measures are conducted at least 7 days prior to the first dose. All subsequent motor function outcome measures are conducted within 96 hours prior to dosing. Information obtained in the Screening Period provide a "treatment baseline" (also referred to as a "baseline").

Patients are monitored through 2 hours post-dose. Following each Treatment Period dose, patients are contacted within 7 days for a safety check-in. Patients are seen in clinic 14 days after the first Treatment Period dose. After the second dose, visits occur every 4 weeks or monthly through the end of the study. Patients that complete the 52-week Treatment Period have the option to enroll into an Extension Period, for an additional 52-week period. There is no break between the completion of the Treatment Period and the start of the Extension Period. Upon signing consent for participation in the Extension Period, patients complete the Treatment Period Day 364 (Visit 15) and immediately begin the Extension Period Day 364 (Extension 1) on the same day.

Patients who complete the 52-week Treatment Period and choose not to enroll in the Extension Period are followed for a 12-week safety Follow-up Period after Day 364 (Visit 15). Patients who complete the Extension Period are followed for a 12-week safety Follow-up Period after Day 728 (Extension 14). Patients are monitored throughout the Treatment Period, Extension Period, and Follow-up Period for safety.

### 1 STUDY DESIGN

### 1.1 Overall Study Design

In an active treatment study of apitegromab in patients age 2 through 21 years old with later-onset SMA, patients receive apitegromab as monotherapy or in addition to an SMN upregulator therapy approved for SMA (Table 5). The study enrolls approximately 55 SMA patients across 3 separate parallel subpopulations subsequently described as cohorts:
- Cohort 1, N=20: Ambulatory type 3 patients, age 5 through 21 years old, at least 10 of whom are not receiving an approved SMA treatment, as well as patients already receiving an approved SMA treatment that had been started after the patient turned 5 years old.
- Cohort 2, N=15: Type 2 or non-ambulatory type 3 patients, age 5 through 21 years old, already receiving an approved SMA treatment that had been started after the patient turned 5 years old.
- Cohort 3, N=20: Type 2 patients, age ≥2 years old, already receiving an approved SMA treatment that had been started before the patient turned 5 years old.

Patient participation in the study consists of 3 parts, Screening, Treatment, and Follow-up:
1. During the Screening Period, all patient screening and eligibility determinations are conducted after informed consent (and, as required by local authorities, patient informed assent) has been provided and within 28 days prior to first dose. Screening motor function outcome measures are conducted at least 7 days prior to the first dose. All subsequent motor function outcome measures are conducted within 96 hours prior to dosing.
2. During the 52-week Treatment Period, patients enrolled in Cohorts 1 and 2 receive high dose (20 mg/kg) apitegromab and patients enrolled in Cohort 3 are randomized (1:1) in a blinded manner to receive either low dose (2 mg/kg) or high dose (20 mg/kg) apitegromab (FIG. 1). All treatments are administered by IV infusion over approximately 2 hours once every 4 weeks or monthly. The first dose is administered on Day 0 and the final dose on Day 336, for a total of 13 doses. There is a ±7-day window around each dosing visit, with a minimum of 21 days and a maximum of 35 days between doses.
   Patients are monitored at the study site through approximately 2 hours post-dose. If there are no acute reactions following the first two doses for a patient, and if determined safe to do so, the infusion duration can be changed to less than two hours but no shorter than 1 hour.
   Following each dose, patients are contacted by the site within 7 days as a safety check-in. Patients are seen in clinic 14 days after the first dose. After the second dose, visits occur every 4 weeks or monthly through the end of the study (Table 6). All dosing and motor function outcome measures are conducted at the study site during the Treatment Period.
   The last Treatment Period visit is on Day 364 (Visit 15).
   Patients who complete the 52-week Treatment Period have the option to enroll into the Extension Period for an additional 52 weeks (FIG. 2). There is no interruption in dosing between Treatment and Extension Periods. The first dose is administered on Day 364, following completion of the Treatment Period at Visit 15, and the final dose is administered on Day 700, for a total of 13 doses.
   All treatments are administered by intravenous (IV) infusion over approximately 2 hours once every 4 weeks or monthly. There is a ±7-day window around each dosing visit, with a minimum of 21 days and a maximum of 35 days between doses.
   Patients are monitored at the study site through approximately 2 hours post-dose. The infusion duration can be no shorter than 1 hour.
3. Patients who complete the 52-week Treatment Period and choose not to enroll in the optional Extension Period are followed for a 12-week safety Follow-up Period after Day 364. Patients who complete the 52-week Extension Period are followed for a 12-week safety Follow-up Period after Day 728.

### 1.2 Treatment Assignment

Eligible patients in Cohorts 1 and 2 receive high dose (20 mg/kg) apitegromab. Patients in Cohort 3 are randomized 1:1 to low (2 mg/kg) or high dose (20 mg/kg) apitegromab without stratification. Patients enrolled in the optional Extension Period may or may not continue to receive the same dose they received during the Treatment Period as determined by the interim and/or primary analysis.

**Table 5. Study Design**

| | **Ambulatory Patients (Revised Hammersmith Scale)** | | **Non-Ambulatory Patients (Hammersmith Functional Motor Scale Expanded)** | |
|---|---|---|---|---|
| | **Cohort 1** | | **Cohort 2** | **Cohort 3** |
| **Design** | • N= 23; ages 5 to 21 | | • N= 15; ages 5 to 21 | • N= 20; ages ≥2 |
| | • Open-label, single-arm | | • Open-label, single-arm | • Double-blind, randomized (1:1) to 2 mg/kg or 20 mg/kg apitegromab IV Q4W |
| | • 20 mg/kg apitegromab IV Q4W | | • 20 mg/kg apitegromab IV Q4W | |
| | • 12-month treatment period | | • 12-month treatment period | |
| | | | | • 12-month treatment period |
| **Patients** | • Ambulatory type 3 SMA | | • Type 2 or non-ambulatory type 3 SMA | • Type 2 SMA |
| | • Two subgroups: | | | • Receiving background nusinersen (initiated before age 5) |
| | | 1) Receiving background nusinersen; | • Receiving background nusinersen | |
| | | 2) Apitegromab monotherapy | | |
| **Primary Objectives** | • Safety | | • Safety | • Safety |
| | • Mean change from baseline in RHS | | • Mean change from baseline in HFMSE | • Mean change from baseline in HFMSE |

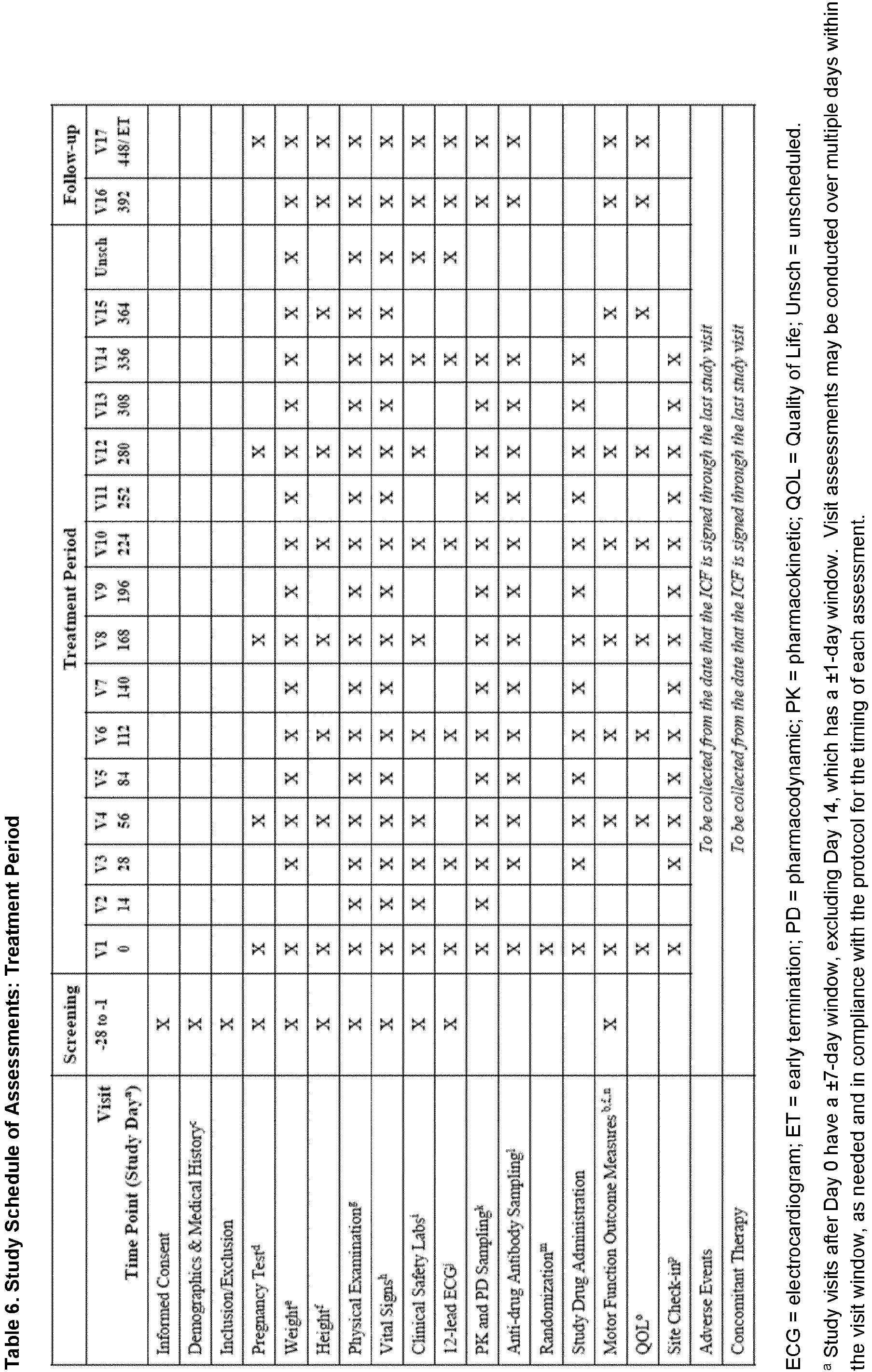

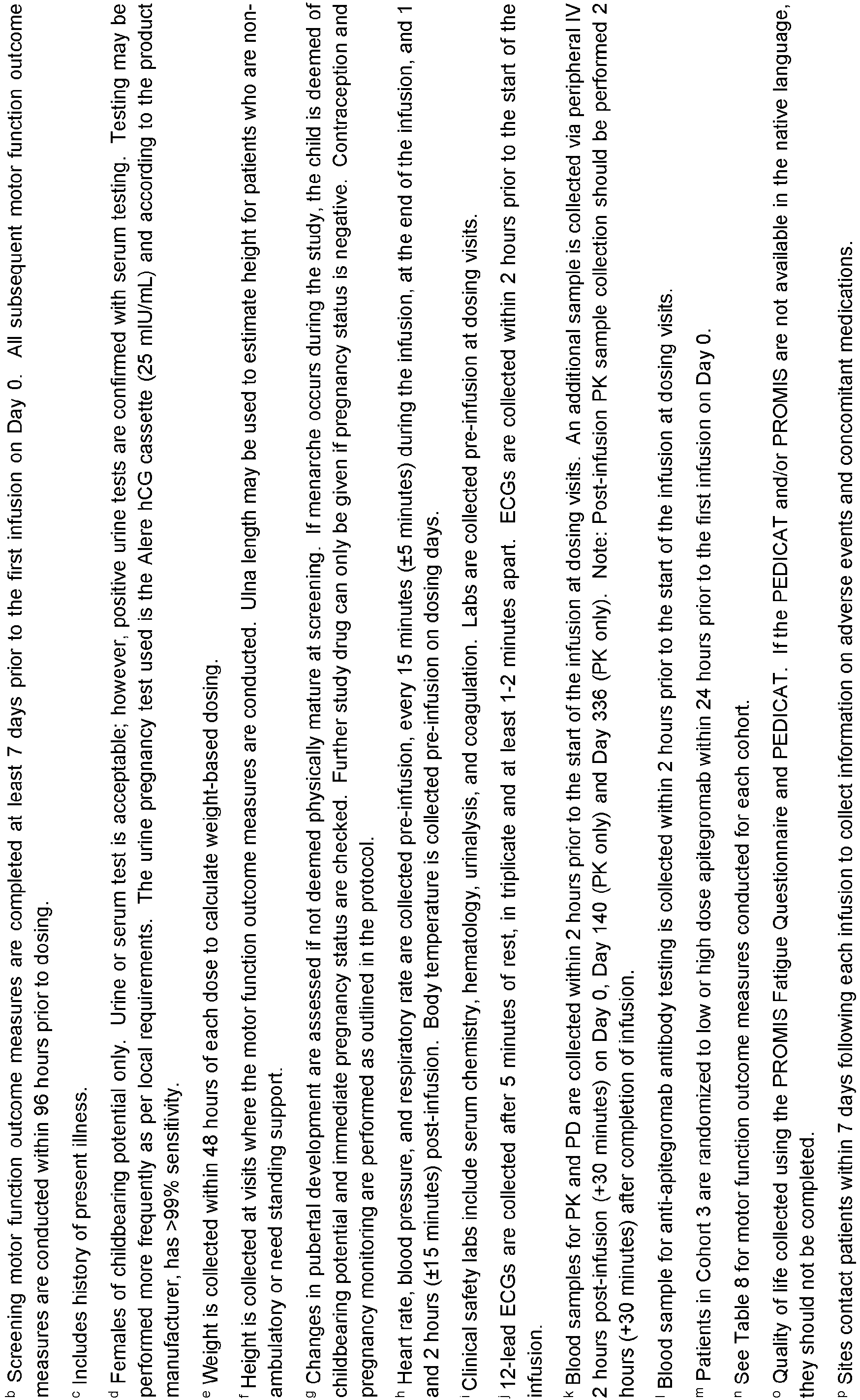

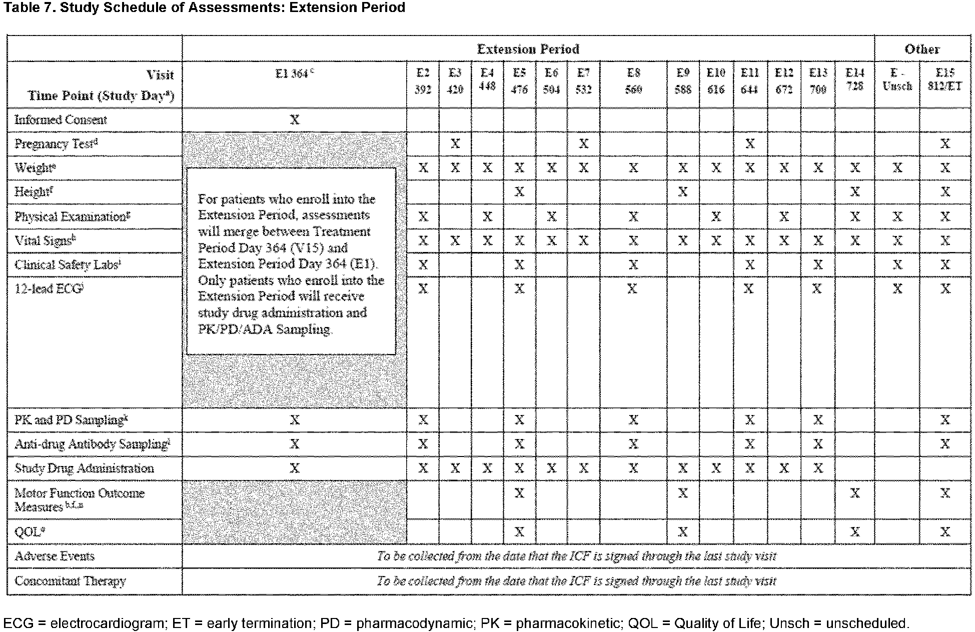

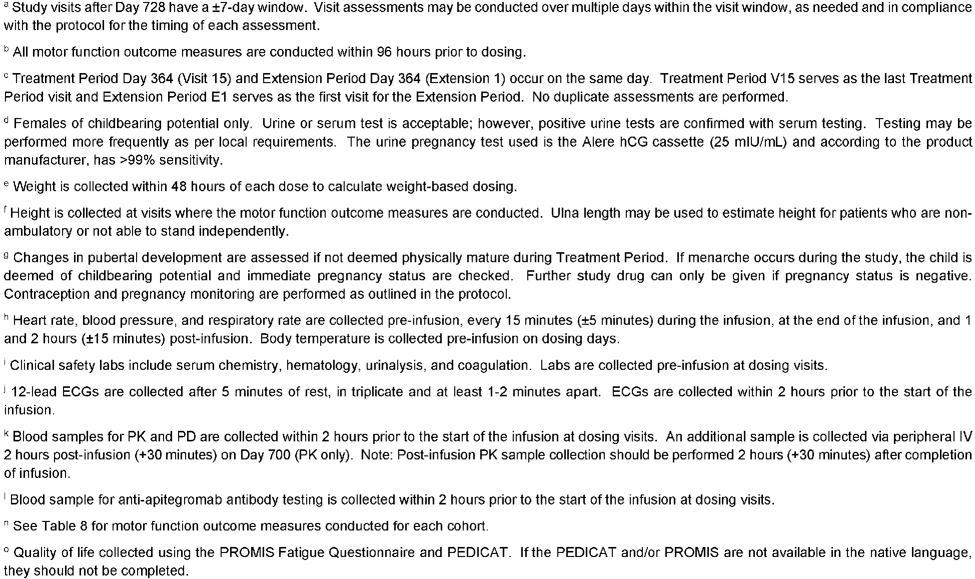

**Table 8. Motor Function Outcome Measures**

| **Cohort 1** | | **Cohorts 2 and 3** | |
|---|---|---|---|
| | • Revised Hammersmith Scale (RHS) | | • Hammersmith Functional Motor Scale Expanded (HFMSE) |
| | • 6-minute Walk Test (6MWT) | | |
| | • 30-Second Sit-to-Stand | | • World Health Organization (WHO) Motor Development Milestones |
| | • 10-meter Walk/Run (from RHS) | | |
| | • Timed Rise from Floor (from RHS) | | • Revised Upper Limb Module (RULM) |
| | | | • Endurance Shuttle Nine Hole Peg Test (ESNHPT) or Endurance Shuttle Box and Block Test (ESBBT)^{a} |

| | | | |
|---|---|---|---|
| ^{a} Patients who score ≤3 on Item A of the RULM at Screening perform the ESNHPT at Screening and all subsequent assessments, while patients who score >3 on Item A of the RULM at screening perform the ESBBT at screening and all subsequent assessments. If patient maxes out of an endurance test, the PT Advisory Board is consulted to potentially change assessment designation. ^{b} The motor function outcome measures are videotaped to ensure that they are being conducted properly and consistently by the physical therapists, upon consent. | | | |

### 1.3 Safety Oversight and Stopping Rules

### 1.3.1 Safety Surveillance

Safety data are reviewed approximately every 12 weeks, and on an ad hoc basis as needed, throughout the duration of the study (inclusive of Treatment and Extension Periods) to assess patient safety.

### 1.3.2 Pharmacokinetic Criteria for Dose Adjustment

PK and PD data are reviewed for each cohort as part of a prespecified interim analysis (refer to Section 6.4). Drug exposure and target engagement levels in SMA patients are evaluated in order to compare them to the levels seen in a prior Phase 1 healthy adult study. Data from multiple cohorts may be combined as necessary and are provided by a biostatistician. Based on these results, and taking into consideration any available safety data, cohort-specific dose levels may be adjusted. Any adjustment in dose level does not change the frequency of dosing (i.e., every 4 weeks or monthly) and the highest dose does not exceed 30 mg/kg, as tested in the Phase 1 study.

### 1.3.3 Study Stopping Rules

Dosing in the study may be suspended at any time for an emergent safety concern until the event(s) are completely evaluated and an appropriate course of action is recommended.

Recommendations may be made to restart dosing and continue the study with no changes, continue the study with changes to the protocol, terminate the study, or require more data, input, and deliberation prior to making a decision. Criteria for study termination are based on the assessment of safety concerns that may arise during the conduct of the study or from data from the apitegromab preclinical and clinical program. The study may be discontinued at any time by discretion, or if it is determined that further drug exposure would pose an undue risk to patients. If the study is terminated prematurely, patients continue to be followed for 12 weeks after their final dose.

### 1.3.4 Individual Stopping Rules

Dosing for any individual patient may be suspended or discontinued if the patient experiences a apitegromab-related serious adverse event (SAE), or a apitegromab-related, clinically significant non-serious AE that warrants suspension or discontinuation from further dosing for that patient's well-being. For patients who have had their dosing suspended, dosing may resume only after their AE has resolved and only if it would be considered safe to do so. For patients who have discontinued dosing, the patient continues to be followed for 12 weeks after their final dose or until the resolution of any ongoing clinically significant AE, whichever occurs later. (For the definition of AE resolution, refer to Section 5.2.1.)

Dosing of apitegromab for an individual patient may also be suspended or discontinued for safety concerns other than those described above if the patient's safety may be threatened. Any single event or combination of events that may jeopardize the safety of the patient or the reliability of the data is evaluated.

Patients who develop either an SAE or other toxicity meeting the individual stopping criteria are carefully monitored, which may include the following:
- Additional clinical laboratory tests and/or other clinical investigations
- Additional visits or extended duration of follow-up
- Obtaining a specialist consultation

### 2 SELECTION AND WITHDRAWAL OF PATIENTS

### 2.1 Patient Inclusion Criteria

1. Age 5 through 21 years old at the time of Screening for Cohorts 1 and 2; Age ≥2 years old at the time of screening for Cohort 3.
2. Estimated life expectancy >2 years from Screening.
3. Informed consent document signed by the patient if the patient is legally an adult. If the patient is legally a minor, informed consent document signed by the patient's parent or legal guardian and patient's oral or written assent obtained, if applicable and in accordance with the regulatory and legal requirements of the participating location.
4. Documented diagnosis of 5q SMA.
5. Diagnosed as later-onset (e.g., type 2 or type 3) SMA prior to receiving any treatment with therapy approved for SMA,
6. Non-ambulatory patients are able to sit independently (sits up straight with head erect for at least 10 seconds; does not use arms or hands to balance body or support position) per World Health Organization (WHO) motor milestones definition at Screening. Patients who never had the ability to walk independently are classified as type 2. Patients who previously had the ability to walk unaided are classified as type 3.
7. Ambulatory patients have the ability to independently ambulate without aids or orthotics over 10 meters at Screening.
8. For Cohort 1, RHS score no greater than 63 at Screening.
9. For Cohorts 2 and 3, HFMSE score no less than 10 at Screening.
10. Receiving the same background SMA therapy (e.g., on an approved SMN upregulator therapy such as nusinersen, or not on any SMA therapy) for at least 6 months prior to Screening and anticipated to remain on that therapy throughout the duration of the study.
   a. If receiving the SMN upregulator therapy nusinersen, completed the loading regimen and initiated maintenance dosing (i.e., completed at least one maintenance dose) with at least 4 weeks after the first maintenance dose having elapsed prior to Screening.
11. Nutritional status stable over the past 6 months and anticipated to be stable throughout the duration of the study.
12. Have no physical limitations that would prevent the patient from undergoing motor function outcome measures throughout the duration of the study.
13. Able to receive study drug infusions and provide blood samples through the use of a peripheral IV, or a long-term IV access device that the patient has placed for reasons independent from the study (i.e., for background medical care and not for the purpose of receiving apitegromab in the study), throughout the duration of the study.
14. Able to adhere to the protocol, including travel to the study center and completing all study procedures and study visits.
15. For patients who are expected to have reached reproductive maturity by the end of the study, adhere to study specific contraception requirements:
   a. Females of childbearing potential (see Section 5.1.7.4 for definition) have a negative pregnancy test at Screening and agree to employ highly effective contraceptive measures (failure rate of 1% or less per year when used consistently and correctly) for the duration of the study and for 18 weeks following the last dose of study drug. Effective contraception methods are restricted to combined (estrogen and progestogen containing) hormonal contraception associated with inhibition of ovulation (oral, intravaginal, transdermal), progestogen-only hormonal contraception associated with inhibition of ovulation (oral, injectable, implantable), intrauterine device (IUD), intrauterine hormone-releasing system ( IUS), bilateral tubal occlusion, vasectomized partner, or sexual abstinence. In the context of this guidance, sexual abstinence is considered a highly effective method only if defined as refraining from heterosexual intercourse during the entire period of risk associated with the study treatments. The reliability of sexual abstinence needs to be evaluated in relation to the duration of the study and the preferred and usual lifestyle of the patient.
   b. Male patients with female partners of childbearing potential are abstinent or agree to employ the use of a condom with or without spermicide throughout the duration of the study and for 18 weeks following the last dose of study drug.

### 2.2 Patient Exclusion Criteria

1. Use of tracheostomy with positive pressure
2. Use of chronic daytime non-invasive ventilatory support for >16 hours daily in the 2 weeks prior to dosing, or anticipated to regularly receive such daytime ventilator support chronically over the duration of the study.
3. Any acute or comorbid condition interfering with the well-being of the patient within 14 days of Screening, including active systemic infection, the need for acute treatment or inpatient observation due to any reason.
4. Severe scoliosis and/or contractures at Screening. Based on clinical judgement, any scoliosis or contractures present is stable over the past 6 months, anticipated to be stable for the duration of the study and not prevent the patient from being evaluated on any functional outcome measures throughout the duration of the study.
5. Pregnant or breastfeeding.
6. Major orthopedic or other interventional procedure, including spine or hip surgery, considered to have the potential to substantially limit the ability of the patient to be evaluated on any functional outcome measures, within 6 months prior to Screening, or anticipated for the duration of the study
7. Prior history of a hypersensitivity reaction to a mAb or recombinant protein bearing an Fc domain (such as a soluble receptor-Fc fusion protein), apitegromab, or excipients of apitegromab.
8. Use of systemic corticosteroids within 60 days prior to Screening. Inhaled or topical steroids are allowed.
9. Treatment with investigational drugs within 3 months or 5 half-lives, whichever is longer prior to Screening.
10. Use of therapies with potentially significant muscle effects (such as androgens, insulin-like growth factor, growth hormone, systemic beta-agonist, botulinum toxin, or muscle relaxants or muscle-enhancing supplements) or potentially significant neuromuscular effects (such as acetylcholinesterase inhibitors) other than an approved SMN upregulator therapy within 60 days prior to Screening.
11. Use of valproic acid within 60 days prior to Screening.
12. Patient has any other condition, which in the opinion of the Investigator may compromise safety or compliance, would preclude the patient from successful completion of the study, or interfere with the interpretation of the results.

### 2.3 Patient Withdrawal Criteria

Patients may withdraw consent at any time. Participation in the study may be terminated at any time without the patient's consent. A patient may be withdrawn from the study for any of the following reasons:
- Protocol violation
- Serious or intolerable AE (see Section 1.3.4)
- Clinically significant change in a laboratory parameter (see Section 1.3.4)
- Sponsor or Investigator decision
- Patient and/or parent or guardian request

### 2.4 Patient Rescreening, Replacement, and Over-enrollment

A patient who is determined to be eligible based on screening assessments and experiences an acute or comorbid condition that causes a delay in their Baseline visit and first infusion (Day 0) may remain eligible up to an additional 14 days after the end of the Screening Period without having to rescreen. The patient may proceed with entering the Treatment Period after the resolution of the event and if deemed safe to do so. Screening patients who experience an acute clinical event that results in a delay of more than 14 days outside of the Screening Period rescreen in order to determine eligibility.

Patients who withdraw from the study for non-safety related reasons before receiving 4 doses of apitegromab and undergoing 2 post-dose HFMSE or RHS assessments may be replaced.

Patients who are in the Screening Period at the time a cohort has enrolled its required number of patients may continue to be enrolled after they are determined to be eligible.

In order to preserve the robustness of the analyses, the additional patients may be enrolled into a cohort as a result of patients in that cohort who significantly change their background approved SMA treatment while on study (e.g., a patient discontinues nusinersen or begins a new approved treatment as part of standard of care for their SMA).

### 2.5 Patient Enrollment into the Optional Extension Period

Patients who choose to enroll into the Extension Period should review and sign the informed consent form prior to performing any assessments being conducted on Day 364 (Visit 15). A patient is determined to be eligible for enrollment into the Extension Period after completion of the 52-week Treatment Period.

### 3 STUDY TREATMENT

### 3.1 Study Drug Description

Apitegromab drug product is a clear, colorless to slightly yellow solution, essentially free from visible particulates. The drug product formulation does not contain excipients of animal origin.

Apitegromab is a sterile, preservative-free, liquid stored in a 6R clear, USP/EP Type I borosilicate glass vial with a 20 mm Flurotec^{®}-coated, bromobutyl rubber stopper, and 20 mm crimp seal with a royal blue flip-off cap. Apitegromab is sterile filtered and filled to provide sufficient volume for withdrawal. Each vial is intended for single use administration only. Apitegromab is stable at 2 to 8°C.

### 3.2 Study Drug Preparation and Administration

Instructions for preparation of each IV dose of study drug are provided. Preparation and dispensing of the study drug are handled by the site pharmacy. Instructions for safe handling of the study drug are provided.

Apitegromab is administered to patients at one of two dose levels: 20 mg/kg and 2 mg/kg, according to the cohort assignments (Section 1.2). Doses are diluted in normal saline and administered by IV over 2 hours (+10-minute window). If there are no acute reactions following the first two doses for a patient, and if it is determined that it would be safe to do so, the infusion duration can be changed to less than two hours but no shorter than 1 hour.

All doses are administered via peripheral IV (or via long-term IV access device such as a peripherally inserted central catheter or port, if the patient has such a device for their background medical care). The placement of a new long-term IV access device is not part of this study, not required for participation, and should not be conducted if the only reason for it is to receive apitegromab.

If an acute reaction occurs, further dosing is suspended, and the risk represented by the acute reaction is evaluated. Such an evaluation incorporates consideration of the nature of the event, relatedness to the drug, and seriousness and severity of the event.

Intervention for patients who experience an acute infusion reaction should be performed in accordance with standard procedures and may include restarting the infusion at a slower rate, terminating the infusion, administration of medications, or other medically supportive measures, as necessary.

### 3.3 Randomization and Blinding

Cohorts 1 and 2 are directly assigned to the 20 mg/kg dose of apitegromab while Cohort 3 patients are randomized (1:1) in a double-blind manner to receive either 2 mg/kg or 20 mg/kg apitegromab via an Interactive Web-based Randomization System (IWRS).

With the exception of the Pharmacist, all are blinded to Cohort 3 apitegromab dose level assignments as specified here. The site Pharmacist remains unblinded throughout the duration of the study. Individual efficacy data is blinded until prespecified interim analysis are performed (see Section 6.4). Efficacy data not included as part of the interim analyses remains blinded until the Treatment Period database lock or deemed necessary. Select personnel have access to efficacy data to ensure study oversight.

For unblinding procedures, refer to Section 5.7.1.5. Based upon interim analysis at 6 months (Day 168) and/or primary analysis at 52 weeks (Day 364), the assignment and blind designation in Cohort 3 may remain the same or change.

### 3.4 Study Drug Compliance

Study drug is administered under the supervision of qualified personnel. The study site is required to adhere to all applicable laws, regulations, and guidelines including, but not limited to, the United States (US) Code of Federal Regulations (CFR), the International Council for Harmonisation (ICH) of Technical Requirements for Registration of Pharmaceuticals for Human Use, the Health Insurance Portability and Accountability Act of 1996, as well as any applicable local and federal regulations.

### 3.5 Study Drug Packaging and Labeling

Study drug is supplied, packaged, and labeled according to applicable local and regulatory requirements for investigational studies.

### 3.6 Study Drug Storage and Accountability

All supplies of apitegromab are stored refrigerated (2°C to 8°C/36°F to 46°F) in the carton and protected from light, in a securely locked area, and accessible to authorized persons only, until needed for dose preparation.

Accurate accountability records of the investigational product are maintained throughout the clinical study. The site Pharmacist inventories the investigational product received and maintains records of disposition of the drug, including dates, quantity, and use. All dispensing and accountability records are available for review. Study drug accountability is verified during on-site monitoring visits. At the end of the study, a final accountability of all study drugs is conducted.

### 3.7 Study Drug Disposal and Return

Following accountability of study drug by a study monitor, used vials may be destroyed at the site according to local standard operating procedures (SOPs) containing well-documented destruction procedures. Unused vials should be returned (e.g., to its designated storage location) for final disposition.

### 3.8 Concomitant Treatment

Concomitant treatment or interventional procedures that are medically indicated for any AEs the patient experiences during the study or that are provided as part of standard supportive care for the patient, is permitted by discretion and supersedes any of the restrictions outlined in this protocol.

Patients may receive an approved SMA treatment in accordance with the cohort they are enrolled into (Section 1.2). Patients are expected to remain on the same background SMA therapy (e.g., on an SMN upregulator therapy such as nusinersen, or not on any SMA therapy) for at least 6 months prior to the first dose of study drug and throughout the duration of the study. Investigators who contemplate changing a patient's standard of care treatment for their SMA are encouraged to discuss this in advance.

Patients that receive the SMN upregulator treatment, nusinersen, receive their maintenance doses at least 24 hours after receiving apitegromab, or at least 14 days prior to any scheduled apitegromab study drug administration visit. Any change in the timing of the patient's nusinersen treatment that would fall within 14 days prior to the scheduled apitegromab administration is discussed in advance.

Investigational therapies are not permitted 3 months (or 5 half-lives, whichever is longer) prior to Screening and throughout the duration of the study.

The concomitant use of the following drugs or products has the potential to interfere with the assessment of treatment effect in this study. Accordingly, the use of the following is prohibited from Screening through the final visit.
- Live vaccinations within 14 days of any study visit where motor function outcome measures are conducted.
- Systemic corticosteroids. Inhaled and topical steroids are allowed.
- Any therapy with potentially significant muscle effects (such as androgens, insulin-like growth factor, growth hormone, systemic beta-agonist, botulinum toxin, muscle relaxants or muscle-enhancing supplements) or potentially significant neuromuscular effects (such as acetylcholinesterase inhibitors) other than an approved SMN upregulator therapy.
- Valproic acid.

### 4 ASSESSMENT OF EFFICACY, PHARMACOKINETICS, AND PHARMACODYNAMICS

### 4.1 Efficacy Endpoints

Primary, secondary, and tertiary efficacy endpoints are assessed at the times indicated in Table 6 and Table 7. The efficacy endpoints for each cohort are outlined in Table 9. Functional outcome measures (e.g., HFMSE/RHS, 6MWT, WHO Motor Milestones, RULM, 30-second Sit-to-Stand, Endurance Shuttle Nine Hole Peg Test [ESNHPT], and/or Endurance Shuttle Box and Block Test [ESBBT]) are conducted and assessed by the Physical Therapist. Requirements for the order of assessments, duration of assessments, and rest periods between assessments are outlined in a separate Physical Therapist training manual.

Study procedures are not conducted for patients who develop an AE (e.g., musculoskeletal injury) during the study, irrespective of relatedness to study drug, that would make it unsafe to perform a functional outcome assessment. Upon resolution of the AE, and if it is safe to do so, performance of that study procedure is resumed at the next scheduled assessment as indicated in Table 6 and Table 7.

**Table 9. Efficacy Endpoints by Cohort**

| | **Cohort 1** | | **Cohorts 2 and 3** | |
|---|---|---|---|---|
| **Primary:** | Change from Baseline in the RHS total score at Day 364 (V15) | | Change from Baseline in the HFMSE total score at Day 364 (V15) | |
| **Secondary:** | | • Change from Baseline in the RHS total score at other prespecified timepoints. | | • Change from Baseline in the HFMSE total score at other prespecified timepoints. |
| | | • Proportion of patients achieving various magnitudes of change in RHS score from Baseline | | • Proportion of patients achieving various magnitudes of change in HFMSE score from Baseline |
| | | • Change from Baseline in the 6MWT | | • Change from Baseline in the RULM total score |
| | | • Change from Baseline in the 30-second Sit-to-Stand | | • Proportion of patients achieving various magnitudes of change in RULM from Baseline |
| | | • Change from Baseline in the 10-meter Walk/Run (from the RHS) | | • Change from Baseline in the number of WHO motor development milestones attained |
| | | • Change from Baseline in the Timed Rise from Floor (from the RHS) | | |
| | | | | • Proportion of patients who attain a new WHO motor development milestone relative to Baseline |
| **Tertiary:** | | • Change from Baseline in the | | • Change from Baseline in time to limitation on the ESNHPT or ESBBT |
| | | • PEDICAT | | |
| | | • Change from Baseline in the | | • Change from Baseline in the |
| | | • PROMIS Fatigue Questionnaire | | PEDICAT |
| | | | | • Change from Baseline in the PROMIS Fatigue Questionnaire |
| | | | **Cohort 3 only:** | |
| | | | | • Time to therapeutic effect (delineated in the Statistical Analysis Plan [SAP]) as compared between low and high dose apitegromab |

| | | | | |
|---|---|---|---|---|
| 6MWT = 6-minute Walk Test; ESBBT = Endurance Shuttle Box and Block Test; ESNHPT = Endurance Shuttle Nine Hole Peg Test; HFMSE = Hammersmith Functional Motor Scale Expanded; PEDICAT = Pediatric Evaluation of Disability Inventory Computer Adaptive Test; PROMIS = Patient-Reported Outcomes Measurement Information System; RHS = Revised Hammersmith Scale; RULM = Revised Upper Limb Module; SAP = Statistical Analysis Plan; WHO = World Health Organization. | | | | |

### 4.1.1 Efficacy Parameters

### 4.1.1.1 Revised Hammersmith Scale (RHS)

The RHS is performed for the ambulatory patient subgroup (Cohort 1). Administration of the RHS includes the Timed Rise from Floor and 10-meter Walk/Run tests. The RHS is a 36-item clinical assessment for physical abilities of patients with type 2 SMA, and ambulatory and non-ambulatory patients with type 3 SMA. The RHS includes 33 items that are graded on a scale of 0, 1, 2, where 0 denotes the lowest level of ability/function and 2 denotes the highest level of ability (Ramsey et al. (2017) PLoS One. 12(2):e0172346). The remaining 3 items are scored 0, 1, where 0 denotes an inability and 1 denotes an ability to achieve. The maximum achievable score is 69.

### 4.1.1.2 10-meter Walk/Run

The 10-meter Walk/Run test is an enhanced function of the RHS used for ambulatory patients with type 3 SMA. It is a measure of the time taken to walk/run 10 meters.

### 4.1.1.3 Timed Rise from Floor

The Timed Rise from Floor test is an enhanced function of the RHS used for ambulatory patients with type 3 SMA. It is a measure of the time taken to rise to standing from the floor.

### 4.1.1.4 6-minute Walk Test (6MWT)

The 6MWT is an assessment of exercise capacity and fatigue that has been used in clinical studies of ambulatory later-onset SMA patients (Young et al. (2016). Muscle Nerve. 54(5):836-842). Ambulatory patients in Cohort 1 complete the 6MWT. Patients are directed to walk along a 25-meter course as fast as possible over 6 minutes. The minute distances and total distance walked over 6 minutes are measured.

### 4.1.1.5 30-second Sit-to-Stand

The 30-second Sit-to-Stand Test is used by researchers and clinicians as an assessment of functional lower limb strength (Jones et al. (1999) Res Q Exerc Sport. 70:113-119). The test was modified for the ambulatory SMA population based on research of the modified 30-second Sit-to-Stand being a reliable, feasible tool for use in a general geriatric population with a lower level of function (McAllister and Palombaro (2019). J Geriatr Phys Ther. 0(0):1-6) and was related to fall risk in institutionalized veterans (Applebaum et al. (2017). PLoS One. 12(5):e0176946; Le Berre et al. (2016) Percept Mot Skills. 123:138-152). The test measures the maximal number of times the patient can transition from sitting to standing in 30 seconds.

### 4.1.1.6 Hammersmith Functional Motor Scale Expanded (HFMSE)

The HFMSE assesses the physical abilities of patients with type 2 and type 3 SMA (O'Hagen et al. (2007) Neuromuscul Disord. 17(9-10):693-697; Glanzman et al. (2011) J Child Neurol. 26(12):1499-1507) and is performed for non-ambulatory patient subgroups (Cohorts 2 and 3). It consists of 33 items to assess an individual's ability to perform various activities, including:
1. Plinth/chair sitting
2. Long sitting
3. One hand to head in sitting
4. Two hands to head in sitting
5. Supine to side-lying
6. Rolls prone to supine over R
7. Rolls prone to supine over L
8. Rolls supine to prone over R
9. Rolls supine to prone over L
10. Sitting to lying
11. Props on forearms
12. Lifts head from prone
13. Prop on extended arms
14. Lying to sitting
15. Four-point kneeling
16. Crawling
17. Lifts head from supine
18. Supporting standing
19. Stand unsupported
20. Stepping
21. Right hip flexion
22. Left hip flexion in supine
23. High kneeling to right half kneel
24. High kneeling to left half kneel
25. High kneeling to stand leading with left leg
26. High kneeling to stand leading with right leg
27. Stand to sit
28. Squat
29. Jump 12 inches forward
30. Ascends stairs with rail
31. Descends stairs with rail
32. Ascends stairs without rail
33. Descends stairs without rail

Quality and execution of each movement is graded on a scale of 0, 1, 2, where 0 denotes unable, 1 denotes performed with modification or adaption, and 2 denotes without modification or adaptation. The maximum achievable score is 66.

### 4.1.1.7 Revised Upper Limb Module (RULM)

The RULM is a 20-item assessment of upper limb function in non-ambulatory SMA patients (young children as well as adults) (Mazzone et al. (2017) Muscle Nerve. 55(6):869-874). The 19 scored items test functions that relate to everyday life, such as placing hands from lap, pressing a button, and picking up a token. The items are scored 0, 1, 2, where 0 denotes unable, 1 denotes able with modification, and 2 denotes able with no difficulty. The maximum score achievable is 37. Non-ambulatory patient subgroups (Cohorts 2 and 3) perform the RULM. The RULM is completed by patients who are 30 months of age or older at the time of the baseline assessment.

### 4.1.1.8 WHO Motor Development Milestones

The World Health Organization (WHO) Multicentre Growth Reference Study performance criteria is utilized to assess motor development milestones of patients with type 2 and non-ambulatory type 3 SMA enrolled in Cohort 2 and Cohort 3.

### 4.1.1.9 Endurance Shuttle Nine Hole Peg Test (ESNHPT)

The ESNHPT is an endurance test for severely affected patients with SMA. Patients are instructed to repeatedly perform the original 9 Hole Peg Test at 75% of their individual maximum speed. The shuttles refer to the set speed marked by auditory cues, with the test ending when the patient missed 2 consecutive beeps. Primary outcome parameter is "time to limitation (Tlim)," the time a task can be maintained at the pre-set intensity. Maximum test duration is 20 minutes (Stam et al. (2018) BMJ Open. 8(7):e019932). Non-ambulatory patients who score ≤3 on Item A of the RULM at Screening perform the ESNHPT at Screening and all subsequent assessments. The ESNHPT is completed by patients who are or will turn 8 years of age or older during the study.

### 4.1.1.10 Endurance Shuttle Box and Block Test (ESBBT)

ESBBT is an endurance test for moderately affected patients with SMA. Patients are instructed to repeatedly perform the original Box and Block Test at 75% of their individual maximum speed. The shuttles refer to the set speed marked by auditory cues, with the test ending when the patient missed 2 consecutive beeps. Primary outcome parameter is "time to limitation (Tlim)," the time a task can be maintained at the pre-set intensity. Maximal test duration is 20 minutes (Stam et al. (2018) BMJ Open. 8(7):e019932). Non-ambulatory patients who score >3 on Item A of the RULM at Screening perform the ESBBT at Screening and all subsequent assessments. The ESBBT is completed by patients who are or will turn 8 years of age or older during the study.

### 4.1.1.11 Pediatric Evaluation of Disability Inventory Computer Adaptive Test (PEDICAT)

A caregiver (who may or may not be a parent and/or legal guardian) completes the PEDICAT assessment. The PEDICAT assessment should not be administered if a caregiver is not present. The PEDICAT assessment should not be administered to the patient. The PEDICAT is a questionnaire completed by the caregiver that assesses the patient's ability to perform daily functions (Haley et al. (2005) Arch Phys Med Rehabil. 86(5):932-939). The PEDICAT is filled out by the caregiver in a location where they are not watching the patient perform any functional assessment tests. The same caregiver should fill out the assessment throughout the study duration. The answers are scored on a 4-point scale (unable to easy). The test is suitable to assess function in newborns to 21-year-olds. Properties of the PEDICAT have been studied in the SMA population. A Rasch analysis with results published in 2016 revealed that the distribution of abilities for the Mobility and Daily Activities domains of the PEDICAT are best represented in the type 2 and type 3 populations (Pasternak et al. (2016) Muscle Nerve. 54(6):1097-1107).

### 4.1.1.12 Patient-reported Outcomes Measurement Information System (PROMIS)

The PROMIS is a person-centered measure intended to be completed by the patient or parent proxy without help from anyone (Ader (2007) Med Care. 5(5):S1-S2). The fatigue profile domain measures a range of symptoms, from mild subjective feelings of tiredness to an overwhelming, debilitating, and sustained sense of exhaustion. The self-report measures are suitable for children 8-17 years old and the parent proxy report measures are suited for children 5-17 years old. If a caregiver completes this form, the same caregiver completes the form throughout the study duration. Patients age 18 through 21 complete an adult form of PROMIS. The PROMIS is completed by patients who are or will turn 5 years of age or older at the time of the baseline assessment.

### 4.2 Pharmacokinetics (PK) Blood Sample Collection

Blood samples for the measurement of apitegromab concentrations are obtained prior to study drug infusion as indicated in the schedule of assessments in Table 6 and Table 7. The 2-hour (+30 minute window) post-dose (from the stop of the infusion) sample collection on Day 0, Day 140, Day 336, and Day 700 is collected via peripheral venipuncture.

Each collected sample is split into approximately equal volume sample sets to allow for retesting, if required. Aliquots from the PK blood samples may be used for additional immunogenicity or PD testing as appropriate, and Baseline samples may be used to support PK, PD, and/or anti-drug antibody (ADA) assay validation.

### 4.3 Pharmacodynamics (PD) Blood Sample Collection

Blood samples are collected for the measurement of latent myostatin levels. These blood samples are obtained prior to infusion as indicated in the schedule of assessments in Table 6 and Table 7. The 2-hour (+30 minute window) post-dose (from stop of infusion) sample collection on Day 0 is collected via peripheral venipuncture.

Each collected sample is split into approximately equal volume sample sets to allow for retesting, if required. Aliquots from the PD blood samples may be used for additional PK or immunogenicity testing as appropriate, and Baseline and post-dose samples may be used to support PK, PD, and/or ADA assay validation.

### 4.4 Immunogenicity Blood Sample Collection

Blood samples for the measurement of anti-apitegromab antibodies are obtained prior to infusion as indicated in the schedule of assessments in Table 6 and Table 7.

Each collected sample is split into approximately equal volume sample sets to allow for retesting, if required. Aliquots from the immunogenicity samples may be used for additional PK or PD testing as appropriate, and Baseline samples may be used to support PK, PD, and/or ADA assay validation.

### 5 ASSESSMENT OF SAFETY

### 5.1 Safety Parameters

### 5.1.1 Demographic/Medical History

Patient demographics and medical history are recorded on the source document and electronic case report form (eCRF). Demographic characteristics include age, sex, race, and ethnicity. Medical history captures the patient's SMA history as well as current and past relevant medical status (surgeries, allergies, and concomitant medications).

### 5.1.2 Vital Signs

Vital signs are performed according to the schedule of assessments provided in Table 6 and Table 7. Routine vital sign assessments include heart rate, blood pressure, and respiratory rate to be collected pre-infusion, every 15 minutes (±5 minutes) during the infusion from the start of the infusion, at the end of the infusion, and 1 and 2 hours (±15 minutes) post-infusion. Body temperature is collected pre-infusion.

### 5.1.3 Weight and Height

Weight and height are collected according to the schedule of assessments provided in Table 6 and Table 7. Weight is collected within 48 hours of each dose in order to calculate weight-based dosing and height is collected at visits where the motor function outcome measures are conducted. Standing height is collected for all individuals who are able to independently stand. Surrogate height may be estimated using ulna length if the patient is non-ambulatory or needs standing support.

### 5.1.4 Physical Examination

A complete physical examination is performed according to the schedule of assessments provided in Table 6 and Table 7. The findings of each examination are recorded on the source documents and eCRF. The physical examination includes an assessment of the following: general appearance, skin, lymph nodes, head-eyes-ears-nose-throat, neck, abdomen, extremities, and the respiratory, cardiovascular, musculoskeletal, and neurologic body systems.

### 5.1.5 Electrocardiogram

A 12-lead electrocardiogram (ECG) is collected at the times indicated in Table 6 and Table 7. ECGs are performed with the patient having rested for at least 5 minutes before each reading. ECGs are performed in triplicate, each approximately 1 to 2 minutes apart. On infusion days, ECG is collected within 2 hours prior to the start of the infusion. ECGs are sent to a central reading vendor for assessment.

### 5.1.6 Concomitant Medications

All concomitant medications are collected from the time the patient signs the ICF through 12 weeks after the final dose.

### 5.1.7 Laboratory Assessments

Laboratory testing (eligibility screening, serum chemistry, hematology, urinalysis, and coagulation, PK and PD sample draw, and anti-drug antibody testing) is performed using established methods at the times indicated in Table 6 and Table 7. When multiple sample collection types are performed at the same assessment time point, the samples are drawn in the following order (depending on what sample types are to be collected at that time point): laboratory safety samples (hematology, coagulation, serum chemistry, urinalysis), PK, PD, anti-drug antibodies. Aliquots from the PK, PD, and anti-drug antibody samples may be retained as back-up for retesting if necessary. The total blood draw for each patient who completes the 52-week Treatment Period, is approximately 158 mL. Patients who do not enroll into the Extension Period and complete the 12-week safety Follow-up Period have approximately 25 mL of additional blood drawn.

The total blood draw for each patient who completes the 52-week Extension Period is approximately 77 mL. Patients who then complete the 12-week safety Follow-up Period have approximately 13 mL of additional blood drawn.

*Anti-Drug Antibody (ADA) Testing.* Standard procedures are used to assess ADA in patient samples. Briefly, an initial screening step generates a positive or negative result in terms of ADA response. A bridging assay may be used to detect ADA (i.e., an electrochemiluminescent assay that uses labeled versions of apitegromab to form a "bridge" between the two arms of an anti-apitegromab antibody). A bridging assay may offer e.g., improved sensitivity, as compared to other detection methods (e.g., a traditional ELISA). Samples that screen negative are considered to lack ADA and samples that screen positive, in some instances, proceed to a confirmatory assay.

In the confirmatory assay, samples are tested with and without spiking in apitegromab. Spiked apitegromab can bind to any ADA present in a sample. When compared to an un-spiked sample, the specificity of the response to apitegromab can be confirmed. Samples confirmed as having a positive response to apitegromab may be re-assayed using serial dilutions to assess the relative strength (i.e., titer) of the antibody response. This value is typically represented as the highest dilution of a patient sample that still has a positive response in the confirmatory assay.

In other instances, the confirmatory assay is skipped and titering data is used to confirm positive ADA responses. Samples with low titers (i.e., titers under a predefined threshold, e.g., under a threshold of 1:32 dilution) are considered negative and samples with high titers (i.e., titers above a predefined threshold, e.g., above a threshold of 1:32 dilution) are considered positive. A titer threshold of 1:32 dilution may be used to assess ADA response in samples. Titering information can be collected simultaneously.

### 5.1.7.1 Hematology and Coagulation

The following hematology and coagulation parameters are assessed:
- Hemoglobin
- Red blood cell count
- Hematocrit
- White blood cell count with differential
- Absolute platelet count
- Mean corpuscular hemoglobin
- Mean corpuscular hemoglobin concentration
- Mean corpuscular volume
- Activated partial thromboplastin time (APTT)
- Prothrombin Time/International Normalized Ratio (PT/INR)

### 5.1.7.2 Blood Chemistry

The following blood chemistry parameters are assessed:
- Albumin
- Alanine aminotransferase
- Alkaline phosphatase
- Aspartate aminotransferase
- Bilirubin (total and direct)
- Blood urea nitrogen
- Calcium
- Carbon dioxide
- Chloride
- Creatinine
- Creatine phosphokinase
- Gamma-glutamyl transferase
- Glucose
- Pregnancy (females only)
- Lactate dehydrogenase
- Magnesium
- Phosphate
- Potassium
- Sodium
- Total cholesterol
- Total protein
- Triglycerides
- Uric acid

### 5.1.7.3 Urinalysis

The following urinalysis parameters are assessed:
- Bilirubin
- Blood microscopy (if urinalysis is abnormal)
- Glucose
- Ketones
- Nitrite
- pH
- Protein
- Specific gravity

### 5.1.7.4 Pregnancy Testing

A female is considered of childbearing potential, following menarche and until becoming postmenopausal unless permanently sterile. Permanent sterilization methods include hysterectomy, bilateral salpingectomy and bilateral oophorectomy. A postmenopausal state is defined as no menses for 12 months without an alternative medical cause.

Pregnancy testing is conducted for females of childbearing potential at the times indicated in Table 6 and Table 7. A urine or serum test is acceptable however, positive urine tests are confirmed with serum testing.

Patients who become pregnant during the study should not receive further study drug and should be followed according to the procedures outlined in Section 5.7.1.3.

### 5.2 Adverse and Serious Adverse Events

### 5.2.1 Definition of Adverse Events

### 5.2.1.1 Adverse Event

An adverse event (AE) is any untoward medical occurrence in a patient or clinical study patient administered a pharmaceutical product that does not necessarily have to have a causal relationship with the treatment administered. An AE can, therefore, be any unfavorable or unintended sign (including an abnormal laboratory finding), symptom, or disease temporally associated with the use of a medicinal product, whether or not related to the medicinal product.
- AEs may be new events or may be pre-existing conditions that have become aggravated or have worsened in severity, seriousness, or frequency from Baseline at any time during the study.
- AEs may be clinically significant changes from Baseline in physical examination, laboratory tests, or other diagnostic investigation.

Treatment-emergent adverse events (TEAEs) are AEs that start after the first dose of study drug or start prior to the administration of study drug and worsen in severity/grade or relationship to the investigational medication after the administration of study drug.

Any medical condition already present at Screening should be recorded as medical history and not be reported as an AE unless the medical condition or signs or symptoms present changes in severity, frequency, or seriousness at any time during the study. In this case, it should be reported as an AE.

### 5.2.1.2 Serious Adverse Event

An SAE is any adverse experience occurring at any dose that results in any of the following outcomes:
- Death
- Life-threatening experience; Note: "Life-threatening" refers to a situation in which the patient was at immediate risk of death at the time of the event; it does not refer to an event that might have caused death if it were more severe.
- Inpatient hospitalization or prolongation of existing hospitalization. The following are excluded as per this criterion:
   ∘ Emergency room visits/hospital admissions for AEs less than 24 hours in duration do not meet SAE criterion unless they meet any of the other SAE criteria in this list.
   ∘ A scheduled or elective hospitalization for medical/surgical procedure planned prior to informed consent for a pre-existing condition that has not worsened from Baseline during participation in the study. However unexpected complications and/or prolongation of hospitalization that occur during elective surgery should be recorded as AEs and assessed for seriousness. Admission to the hospital for social or situational reasons (i.e., no place to stay, too far away to come for hospital visits, respite care) is not considered inpatient hospitalization.
- Persistent or significant disability or incapacity or substantial disruption of the ability to conduct normal life functions
- Congenital anomaly or birth defect
- Is considered to be an important medical event. Important medical events are those that may not be immediately life-threatening or result in death or hospitalization but may jeopardize the patient or may require intervention to prevent one of the outcomes listed in the definition above. Examples of such medical events include allergic bronchospasm requiring intensive treatment in an emergency room or at home, blood dyscrasias or convulsions that do not result in inpatient hospitalizations, or the development of drug dependency.

### 5.2.1.3 Dosing Errors

All dosing errors (including, but not limited to, route of administration and wrong dose) are reported as protocol deviations. A brief description should be provided in the deviation report, including information about whether the patient was symptomatic or not. Dosing details should be captured in the eCRF.

An overdose is defined as a significant variation from the recommended/scheduled dosage for a product. The dosing for this study is conducted in a controlled clinical setting and an overdose is not anticipated. However, in the event of an accident, for this study, an overdose of apitegromab is considered a dose that is at least 2-fold higher than 20 mg/kg.

Overdoses are not considered AEs and should not be recorded as an AE in the eCRF unless an AE or an SAE occurs. All overdoses (regardless of whether or not they result in an AE) are recorded on an overdose form and reported within 24 hours of the site becoming aware of the overdose. If an overdose results in an SAE, both the SAE and overdose forms are completed and reported.

### 5.2.1.4 Pregnancy

Pregnancy is not an AE; however, if a female patient or female partner of a male study patient becomes pregnant during the conduct of the study, Medpace is notified according to the procedures provided in Section 5.7.1.3. A patient becoming pregnant while on study drug is immediately withdrawn from further dosing and followed according to the procedures provided in Section 5.7.1.3.

### 5.2.1.5 Treatment-emergent Adverse Event

An AE is treatment-emergent if the onset time is after administration of the first dose of study drug through the final follow-up visit or, in the event that onset time precedes study drug administration, the AE increases in severity during the safety Follow-up Period.

### 5.3 Adverse Event Monitoring

Each patient is monitored for the occurrence of AEs, including SAEs, from the signing of the Informed Consent Form (ICF) through the final follow-up visit.
- Patients are questioned and/or examined for evidence of AEs. The questioning of patients with regard to the possible occurrence of AEs is generalized such as, "How have you been feeling since your last visit?" The presence or absence of specific AEs should not be elicited from patients.
- Patients having AEs are monitored until resolution or stabilization (in the case of persistent impairment), or until the event becomes chronic in nature, or the patient dies.
- AEs, actions taken as a result of AEs, and follow-up results are recorded in the eCRF as well as in the patient's source documentation. Follow-up laboratory results should be filed with the patient's source documentation.

For any SAEs or AEs that require the patient to be discontinued from dosing, relevant clinical assessments and laboratory tests are repeated as clinically appropriate, until final resolution or stabilization of the event(s). Patients continue to be followed through 12 weeks after the final dose.

All safety laboratory analyses are performed at a central laboratory. The clinical laboratory values are reported and reviewed for clinical significance and consideration of abnormal values as potential AEs.

### 5.3.1 Relationship to Study Drug (Causality Assessment)

The relationship of any AE (including SAEs) to the use of the investigational product, as related or not related, is assessed based on clinical judgment and using all available information, and may include consideration of the following factors:
- The temporal sequence from study drug administration
- Underlying, concomitant, intercurrent diseases
- Concomitant use of other drugs
- Whether the manifestations of the AE are consistent with known actions or toxicity of the investigational product
- Exposure to physical and/or mental stresses
- The pharmacology and pharmacokinetics of the study drug
- The AE resolved or improved with decreasing the dose or stopping the use of the investigational product (dechallenge). Judgment should be used if multiple products are discontinued at the same time.

The causal relationship between the study drug and the AE is assessed using one of the following categories:
- Not Related: Factors consistent with an assessment of Not Related may include:
   ∘ Temporal relationship is lacking (e.g., the event did not occur within a reasonable time frame following administration of the study drug); or
   ∘ Other causative factors more likely explain the event (e.g., a pre-existing condition, other concomitant medications)
- Related: Factors consistent with an assessment of Related may include:
   ∘ There is a positive temporal relationship (e.g., the event occurred within a reasonable time frame following administration of study drug)
   ∘ The AE is more likely explained by the investigational product than by another cause (e.g., the AE shows a pattern consistent with previous knowledge of the investigational product or the class of the investigational product).

### 5.4 Assessment of Expectedness

As part of the regulatory reporting requirements, an assessment of expectedness (expected/unexpected from the perspective of previously observed, not on the basis of what might be anticipated from the pharmacological properties of a medicinal product) for AEs is performed. Adverse reactions are considered unexpected if the nature, seriousness, severity or outcome of the reaction(s) is not consistent with reference safety information.

### 5.5 Common Terminology Criteria for Adverse Events

Clinical and laboratory AEs are graded using the National Cancer Institute (NCI) Common Terminology Criteria for Adverse Events (CTCAE), Version 5.0.

The term "severe" is often used to describe the intensity (severity) of a specific event (as in mild, moderate, or severe myocardial infarction); the event itself, however, may be of relatively minor medical significance (such as severe nausea). This is not the same as "serious," which is based on patient/event outcome or action criteria usually associated with events that pose a threat to a patient's life or functioning.

### 5.6 Recording Adverse Events

Each patient is monitored for the occurrence of AEs, including SAEs, from the signing of the ICF through the final follow-up visit. Patients and/or their legal guardians are questioned, and the patients may be examined for evidence of AEs. Patients having AEs are monitored with relevant clinical assessments and laboratory tests.

For each AE, the requested information is evaluated and recorded in the eCRF and the patient's source documentation.

### 5.6.1 Instructions for Recording AEs in eCRF

### 5.6.1.1 Recording Diagnosis Versus Signs and Symptoms

If a patient reports signs and symptoms that represent a medical diagnosis/syndrome, the final diagnosis/syndrome should be recorded in the eCRF rather than each sign and symptom (e.g., cough, runny nose, fever = upper respiratory infection). However, if a medical diagnosis cannot be made, then each sign or symptom should be recorded as an individual SAE or AE, as appropriate.

### 5.6.1.2 Abnormal Laboratory Values or Vital Signs

Protocol defined laboratory values and vital signs should not be reported as AEs unless the abnormal laboratory value or vital sign meets at least one of the following:
- Requires an adjustment in the study drug(s) or discontinuation of treatment;
- Requires medical or surgical intervention;
- Is associated with accompanying signs/symptoms that are not considered part of a pre-existing diagnosis or syndrome (or if considered part of a pre-existing diagnosis or syndrome, is associated with disease worsening); or
- Is considered clinically significant

### 5.7 Reporting Serious Adverse Events

### 5.7.1 Serious Adverse Event Reporting

### 5.7.1.1 Initial Reports

All SAEs occurring from the time of informed consent through the final study visit are reported to Medpace Clinical Safety within 24 hours of the awareness of the occurrence (this refers to any AE that meets any of the aforementioned serious criteria). All SAEs considered related to study drug occurring after the study are reported.

To report an SAE, the SAE form is completed electronically in the electronic data capture (EDC) system for the study. When the form is completed, Medpace Safety personnel are notified electronically and retrieve the form. Any supporting documentation (e.g., discharge summary, diagnostic results) are redacted by the site and sent to Medpace Clinical Safety.

### 5.7.1.2 Follow-up Reports

SAEs are followed until resolution, stabilization (in the case of persistent impairment), or until the event becomes chronic in nature, or the patient dies. SAE resolution is defined as:
- Resolved with or without residual effects
- A return to Baseline for a pre-existing condition
- Laboratory values have returned to Baseline or stabilized
- No further improvement or worsening of the event is expected
- Fatal outcome - if an autopsy is performed, the autopsy report should be provided as soon as it is available.

Within 24 hours of receipt or awareness of follow-up information, the SAE form is updated electronically in the EDC system for the study and any supporting documentation (e.g., patient discharge summary or autopsy reports) is submitted to Medpace Clinical Safety. All supporting patient documentation is redacted for personal details as per Good Clinical Practice (GCP). If it is not possible to access the EDC system, refer to the procedures outlined above for initial reporting of SAEs.

### 5.7.1.3 Reporting Pregnancies

If a female patient or the female partner of a male patient becomes pregnant during the course of the study, the pregnancy is reported to Medpace Clinical Safety within 24 hours of awareness of the event. Medpace Clinical Safety then forward the paper Exposure in Utero (EIU) form for completion. The EIU form is completed and sent back to Medpace Clinical Safety within 24 hours. Consent to collect pregnancy information (including the status of the newborn, if applicable) is obtained.

The patient or partner should be followed until completion of the pregnancy, whenever possible. Once the outcome of the pregnancy is known, the follow-up EIU form should be completed and sent to Medpace Clinical Safety within 24 hours. At the completion of the pregnancy, the outcome of the pregnancy is documented. If the outcome of the pregnancy meets the criteria for immediate classification as an SAE (i.e., postpartum complication, spontaneous abortion, stillbirth, neonatal death, or congenital anomaly), the procedures for reporting an SAE are followed.

### 5.7.1.4 Reporting to Institutional Review Board/Independent Ethics Committee

Any expedited reports are reported to the local IRB or IEC, per local reporting regulations.

### 5.7.1.5 Unblinding a Patient's Treatment During the Study

In the event of a drug-related, serious, unexpected AE, Medpace clinical safety may provide a Cohort 3 patient's dose level for the purpose of regulatory authority agency reporting.

Because all patients in the study receive apitegromab, unblinding of a Cohort 3 patient's dose level (i.e., high versus low dose) is discouraged. In the event of a drug-related SAE, if deemed medically necessary in order to provide patient care, the patient's dose level may be obtained from the IWRS system.

### 5.7.2 Expedited Reporting to a Regulatory Health Authority

The expedited reporting of relevant safety information to concerned health authorities and to all investigators is managed in accordance with local laws and regulations.

### 6 STATISTICS

### 6.1 General Methodology

All analyses are performed under GCP standards using a prospective SAP. Analyses are conducted using Statistical Analysis System (SAS) (Version 9.4 or later, Cary, North Carolina).

The study is regarded as 3 separate cohorts; therefore, no Type I error is allocated towards separate cohort testing. No formal statistical testing is applied for the interim analysis and no alpha spending is applied. Two-sided 95% confidence intervals are reported.

There is an interim analysis conducted when prespecified enrollment (see below) and treatment duration have been reached for all three cohorts (see Section 6.4); the final analysis is conducted once each cohort completes the 52-week Treatment Period. Biostatistics Data Review Meetings are held for the final analyses in order to distinguish between major and minor protocol violations. Both Per-Protocol and Intention-to-Treat (ITT) populations are analyzed, with the ITT population being the primary analysis population.

### 6.2 Analysis Strategy

All prespecified safety and efficacy endpoints are analyzed by cohort and by dose group for Cohort 3. All patients with any post-baseline data are included in the interim and primary analyses. In addition, safety and efficacy data for the high dose are analyzed for different cohort/dose combinations taking cohort, dose level, and receiving background SMA therapy into consideration. For the change from Baseline analyses (to utilize the patient as their own control for response analyses), the last observation before the first dose is used as the Baseline. Cohort 3 low versus high dose treatment outcomes are also evaluated for the time to response as a descriptive outcome.

### 6.2.1 Safety Analyses

Safety data are evaluated for vital signs, laboratory outcomes, concomitant medications, ECGs, and AEs. Clinically significant outcomes are noted. Vital signs and laboratory outcomes are evaluated using paired t-tests within cohorts and unpaired t-tests between the two dose groups for Cohort 3; corresponding 2-sided 95% confidence intervals (Cls) are computed as well for the paired and unpaired differences. Similar analyses for different cohort/dose combinations are explored. AEs are coded in the Medical Dictionary for Regulatory Activities (MedDRA); the emphasis is on treatment-emergent adverse events (TEAEs); the TEAE type, incidence, severity, relationship, onset and resolution time is displayed descriptively; the incidence rate is assessed using an exact binomial test for single groups and using a Fisher's Exact test to compare low versus high dose treatment groups within Cohort 3. Times to first TEAE event, first related TEAE, and first severe TEAE are displayed using a Kaplan-Meier lifetable.

### 6.2.2 Efficacy Analyses

Efficacy data are evaluated for multiple measures with continuous endpoints (e.g., HFMSE score, RHS score, RULM score, etc.) at time points specified in Table 6 and Table 7. The primary endpoints and other efficacy continuous endpoints are analyzed using paired t-tests within cohorts and unpaired t-tests between the two dose groups for Cohort 3; corresponding 2-sided 95% Cls are computed as well for the paired and unpaired differences. Similar analyses for different cohort/dose combinations are explored. Efficacy data are further explored by longitudinal models addressing population and by subject variation with time, dose, and dose time interaction as covariates, with age, nusinersen treatment duration, and screening HFMSE score added as potential covariates. Any changes in the analysis as specified in the protocol are delineated in the SAP.

The HFMSE, RHS, and WHO motor development endpoints are further categorized in order to assess the specific distributions of the change from Baseline (waterfall displays of unit changes from Baseline) and the proportion of patients reaching certain milestones. The proportion and 95% confidence interval based on Wilson method are reported.

Waterfall charts are generated to display the distribution of improvement from Baseline for HFMSE, RHS, RULM, and other unit-based measures at post-Baseline milestones in support of efficacy and future study planning.

Time to outcome improvement is assessed using Kaplan-Meier lifetables and a log rank test to compare time to efficacy for Cohort 3 low versus high dose.

### 6.2.3 Pharmacokinetic and Pharmacodynamic Analysis

Apitegromab concentrations and parameters and circulating latent myostatin concentrations are determined using blood samples collected before and after dosing through the final follow-up visit. Apitegromab concentrations are listed and summarized in tabular formats using descriptive statistics and plotted against time points by cohort. The latent myostatin concentrations are listed for each patient and summarized by cohort.

### 6.2.4 Anti-Apitegromab Antibody Analysis

Anti-drug antibody data are listed and summarized in tabular format using descriptive statistics. Confirmed positive samples are further tested for the presence of neutralizing activity.

### 6.3 Sample Size Rationale

Sample sizes for each cohort were based on practical considerations. Sample sizes of 15-20 patients for each cohort provide 80% power to reject the null hypothesis involving mean changes from Baseline within each treatment group relative to prespecified alternative hypothesis using a paired t-test with two-sided 5% Type I error. Table 10 presents the effect sizes within dose groups that can be detected with 80% power and two-sided 5% Type I error as well as the effect sizes that reject the null hypothesis of no difference according to two-sided p=0.05. As illustration, assuming a 2.5 unit standard deviation (SD) for the change from Baseline for HFMSE, a mean 1.7-unit improvement for HFMSE (n=20) and a mean 2-unit improvement for HFMSE (n=15) can be detected with 80% power while a mean 1.2 unit improvement in HFMSE (n=20) and a mean 1.3-unit improvement in HFMSE reaches two-sided p=0.05 (rules out a 0 as the two-sided 95% CI for the change from Baseline).

**Table 10. Paired T-test: Reject Null Hypothesis No Mean Change from Baseline**

| | **80% Power** | | **Two-sided p=0.05** | |
|---|---|---|---|---|
| **Test significance level, α** | 0.05 | 0.05 | 0.05 | 0.05 |
| **Effect size, e = \|Δ₁-Δ₂\| / s** | 0.66 | 0.778 | 0.462 | 0.543 |
| **n (after losses)** | 20 | 15 | 20 | 15 |

In addition, the total sample size for Cohort 3 (n= 20) provides 80% power to reject the null hypothesis of no mean change from Baseline against the prespecified difference between doses for a clinically meaningful improvement at a two-sided 5% Type I error for a 1:1 randomization (high dose versus low dose).

Table 11 presents the effect size which could be detected with 80% power and a two-sided 5% Type I error as well as the effect sizes required to reject the null hypothesis of no change from Baseline for comparing Cohort 3 low versus high dose. If the SD of the change from Baseline is 2.5 units for HFMSE, then a mean 2.4-unit difference between low and high dose will reach two-sided p=0.05 (rules out 0 as the lower two-sided 95% CI as the mean difference between dose groups).

**Table 11. Unpaired T-Test: Reject Null Hypothesis: No Mean Difference from Baseline**

| | **80% Power** | **Two-sided p=0.05** |
|---|---|---|
| **Two-sided significance level, α** | 0.05 | 0.05 |
| **Effect size, e = \|Δ_{A}-Δ_{O}\| / s** | 1.325 | 0.926 |
| **n per group (after losses)** | 10 | 10 |

### 7 INTERIM EFFICACY AND SAFETY RESULTS

### 7.1 Summary

Interim efficacy and safety data demonstrate proof-of-concept in patients with type 2 or type 3 SMA, subpopulations which together represent over 85% of the patients living with SMA. Treatment with apitegromab led to improvements in Hammersmith scale scores (primary efficacy endpoint) in all three cohorts of patients (Table 12). The majority of patients achieved at least a 1-point gain, which is meaningful on an individual level. A substantial proportion of patients in each cohort also achieved at least a 3-point gain, which is highly clinically meaningful and otherwise uncommon occurrence in any given patient. No safety signals were identified following six months of treatment.

At the six-month interim analysis timepoint, mean increases from baseline in Hammersmith scale scores were observed in all three cohorts (67% of total patients achieved ≥1 point improvement in Hammersmith scores). A substantial proportion of patients in each cohort achieved a ≥3 point increase in Hammersmith scores (35% of total patients achieved ≥3 point increase in Hammersmith scores). A dose response was also observed in the double-blind, randomized portion of the study (Cohort 3), with efficacy increasing with dose. Numerically greater improvements in HFMSE scores were observed for the high-dose arm as compared to the low-dose arm across all evaluated timepoints. The high dose arm of Cohort 3 attained a 5.6 point mean improvement from baseline in the Hammersmith Functional Motor Scale Expanded (HFMSE) as compared to the low dose arm, which attained a 2.4 point mean improvement at the six-month interim analysis timepoint. This observed dose response was supported by PK and PD data.

**Table 12.**

| | **Ambulatory Patients (Revised Hammersmith Scale)** | | | **Non-Ambulatory Patients (Hammersmith Functional Motor Scale Expanded)** | | |
|---|---|---|---|---|---|---|
| | **Cohort 1** | | | **Cohort 2*** | **Cohort 3*** | |
| | Pooled (n=23) | 20 mg/kg monotherapy (n=11) | 20 mg/kg + nusinersen (n=12) | 20 mg/kg + nusinersen (n=14) | 2 mg/kg + nusinersen (n=9) | 20 mg/kg + nusinersen (n=9) |
| Mean change from baseline (95% CI) | 0.5 (-1.1, 2.2) | 0.7 (-2.5, 4.0) | 0.3 (-1.4, 2.0) | 1.4 (0.1, 2.7) | 2.4 (-0.9, 5.8) | 5.6 (2.5, 8.7) |
| # (%) patients achieving ≥1-pt increase | 12/23 (52%) | 7/11 (64%) | 5/12 (42%) | 10/14 (71%) | 6/9 (67%) | 9/9 (100%) |
| # (%) patients achieving ≥3-pt increase | 6/23 (26%) | 4/11 (36%) | 2/12 (17%) | 3/14 (21%) | 4/9 (44%) | 6/9 (67%) |

| | | | | | | |
|---|---|---|---|---|---|---|
| * 3 patients (1 in Cohort 2 and 2 in Cohort 3) each missed 3 doses of apitegromab and the six-month interim analysis timepoint due to site access restrictions; the six-month timepoint from these patients was not included in the interim analysis. | | | | | | |

Surprisingly, in a patient population where patients typically experience motor function stabilization on nusinersen treatment, such as Cohort 2, a mean improvement in the HFMSE from baseline was observed upon treatment with apitegromab. Furthermore, substantial proportions of patients meeting this high bar were observed in each of the 3 cohorts. Attaining a 3-point improvement in any given patient is a high bar outcome and is rare to observe. Most patients across the study experienced improvement as measured by at least a 1-point improvement from baseline. Gaining improvements in the ability to perform motor tasks is important for patients living with SMA, and improvements in motor function as measured by HFMSE or RHS could meaningfully impact each patient's life.

### 7.2 Baseline Demographics and Characteristics

Baseline demographics and characteristics are shown in Table 13.

**Table 13. Baseline Demographics and Characteristics**

| | **Ambulatory Patients** | | | **Non-Ambulatory Patients** | | | |
|---|---|---|---|---|---|---|---|
| | **Cohort 1** | | | **Cohort 2** | **Cohort 3** | | |
| | Pooled | 20 mg/kg monotherapy | 20 mg/kg + nusinersen | 20 mg/kg + nusinersen | Pooled | 2 mg/kg + nusinersen | 20 mg/kg + nusinersen |
| N | 23 | 11 | 12 | 15 | 20 | 10 | 10 |
| Mean age (min, max) | 12.6 (7, 21) | 12.1 (7, 19) | 13.1 (7, 21) | 11.7 (8, 19) | 4.0 (2, 6) | 4.1 (2, 6) | 3.8 (2, 6) |
| Female (%) | 65% | 73% | 58% | 53% | 40% | 30% | 50% |

| SMN2 Gene Copy* (#, %) | | | | | | | |
|---|---|---|---|---|---|---|---|
| 2 | 1 (4%) | 1 (9%) | 0 (0%) | | 2 (10%) | 1 (10%) | 1 (10%) |
| 3 | 13 (57%) | 4 (36%) | 9 (75%) | 11 (73%) | 16 (80%) | 8 (80%) | 8 (80%) |
| 4 | 5 (22%) | 4 (36%) | 1 (8%) | 2 (13%) | 1 (5%) | 1 (10%) | 0 (0%) |
| Mean # of nusinersen maintenance doses (min, max) | N/A | N/A | 5.6 (2, 8) | 5.1 (2, 9) | 5.5 (2, 9) | 5.5 (2, 9) | 5.4 (3, 8) |
| Discontinuation(s) | 1** | 0 | 1** | 0 | 0 | 0 | 0 |
| Mean RHS score (min, max) | 49.6 (26, 63) | 47.6 (26, 63) | 51.3 (43, 62) | | | | |
| Mean HFMSE score (min, max) | | | | 22.7 (13, 39) | 24.8 (12, 44) | 26.1 (12, 44) | 23.5 (14, 42) |

| | | | | | | | |
|---|---|---|---|---|---|---|---|
| *1 patient answered 3-4, 1 patient answered >4 , both patients are in Cohort 1 treated with 20 mg/kg + nusinersen; data not available for all patients **patient who discontinued study for reasons unrelated to study drug; | | | | | | | |

### 7.3 Cohort Analyses

### 7.3.1 Cohort 3

This randomized, double-blind, portion of the study enrolled patients with type 2 SMA who had initiated treatment with an approved SMN upregulator (nusinersen) before 5 years of age. These patients had been on nusinersen for about 2 years. Even with nusinersen, the baseline mean HFMSE score for these patients was in the low-20s.

Twenty patients were randomized in a 1:1 ratio to receive the low dose (2 mg /kg apitegromab Q4W) or high dose (20 mg/kg apitegromab Q4W); both treatment arms are in conjunction with an approved SMN upregulator therapy (nusinersen). Two patients (one in high dose arm and one in low dose arm) each missed three doses of apitegromab and the six-month interim analysis timepoint due to site access restrictions; the six-month timepoint from these patients was not included in the interim analysis. The primary objectives of the cohort are to assess safety and the mean change from baseline in HFMSE.

At baseline, patients in the high dose arm had a mean age of 3.8 years (range 2-6 years) and mean HFMSE score of 23.5 (range 14-42) out of a total possible score of 66 points, while patients in the low dose arm had a mean age of 4.1 years old (range 2-6 years) and a mean HFMSE score of 26.1 (range 12-44).

At the six-month interim analysis timepoint:
- Mean change from baseline in HFMSE score:
   ∘ 20 mg/kg dose (n = 9): +5.6 points (95% Cl of +2.5, +8.7)
   ∘ 2 mg/kg dose (n = 9): +2.4 points (95% Cl of -0.9, +5.8)
- Proportion of patients attaining ≥1 point increase in HFMSE score:
   ∘ 20 mg/kg dose: 100% (9/9)
   ∘ 2 mg/kg dose: 67% (6/9)
- Proportion of patients attaining ≥3 point increase in HFMSE score:
   ∘ 20 mg/kg dose: 67% (6/9)
   ∘ 2 mg/kg dose: 44% (4/9)
- Proportion of patients attaining ≥5 point increase in HFMSE score:
   ∘ 20 mg/kg dose: 56% (5/9)
   ∘ 2 mg/kg dose: 33% (3/9)

In the high-dose arm, 100% of patients attained at least a 1 point improvement as compared to 67% with low dose. Looking at the high bar of 3-point improvement, 67% of patients in the high-dose arm achieved this threshold compared to 44% in the low-dose arm. More than half of the patients in the high dose arm achieved at least a 5 point increase from baseline. *Greater improvement achieved with high dose:* Patients treated with high dose (20 mg/kg) achieved numerically greater improvements from baseline in HFMSE scores as compared to the low dose (2 mg/kg) at all assessed timepoints (week 8, week 16, and the six-month interim analysis timepoint) (Table 14; FIG. 3A-B). Numerically greater improvements with high dose were observed both in terms of mean change from baseline and in proportions of patients attaining ≥3 point increase in HFMSE score.

**Table 14.**

| **Type 2 SMA** | **Apitegromab low dose (2 mg/kg) + nusinersen (n=9)** | **Apitegromab high dose (20 mg/kg) + nusinersen (n=9)** |
|---|---|---|
| Mean change from baseline in HFMSE (95% CI) | 2.4 (-0.9, 5.8) | 5.6 (2.5, 8.7) |
| # (%) patients achieving ≥1-pt increase in HFMSE | 6/9 (67%) | 9/9 (100%) |
| # (%) patients achieving ≥3-pt increase in HFMSE | 4/9 (44%) | 6/9 (67%) |
| # (%) patients achieving ≥5-pt increase in HFMSE | 3/9 (33%) | 5/9 (56%) |

*Plateau in improvement appears to not have yet been reached:* Improvements in HFMSE scores progressively increased over the six-month treatment period. Twelve-month data may provide additional insights evaluating the potential for durability of effect and for further motor function gains.

*PK and PD results are supportive of the observed dose response in efficacy:*
- Treatment with the high dose led to higher levels of drug exposure than with the low dose. Apitegromab exhibited a PK profile consistent with that commonly observed with monoclonal antibodies, and drug exposure was dose proportional (FIG. 4).
- Treatment with high dose achieved higher levels of target engagement, and treatment with low dose did not attain full target saturation (FIG. 5).

### 7.3.2 Cohort 2

This open-label, single-arm cohort enrolled 15 patients with a mean age of 11.7 years old (range 8-19 years) with type 2 or non-ambulatory type 3 SMA and who are already receiving treatment with an approved SMN upregulator. This is a patient population for which SMN upregulator therapy alone historically appears to primarily offer motor function stabilization rather than improvement. This patient population had been treated with nusinersen for about 2 years before enrolling in the study and had a baseline HFMSE score in the mid-20s.

Patients are treated with 20 mg/kg of apitegromab Q4W in conjunction with an approved SMN upregulator therapy (nusinersen). At baseline, patients had a mean HFMSE score of 22.7 (range 13-39) out of a total possible score of 66. One patient missed three doses of apitegromab and the six-month interim analysis timepoint due to site access restrictions; the six-month timepoint from this patient was not included in the interim analysis. The primary objectives of the cohort are to assess safety and the mean change from baseline in HFMSE.

In this patient population in which background SMN therapy is generally expected to primarily offer motor function stabilization, apitegromab showed consistent improvements in HFMSE scores (Table 15; FIG. 6A-B). 71% of the patients in the cohort achieved at least a 1-point increase in HFMSE while 21% of the patients achieved at least a 3-point increase. At the six-month interim analysis timepoint:
- Mean change from baseline in HFMSE score (n = 14): +1.4 points (95% CI of +0.1, +2.7)
- Proportion of patients attaining ≥1 point increase in HFMSE score: 71% (10/14)
- Proportion of patients attaining ≥3 point increase in HFMSE score: 21% (3/14)
- Proportion of patients attaining ≥5 point increase in HFMSE score: 14% (2/14)
- Improvements in HFMSE scores progressively increased over the six-month treatment period, and a plateau in improvement appears to not have yet been reached. Twelve-month data may provide additional insights evaluating the potential for durability of effect and for further motor function gains.

**Table 15.**

| **Type 2 and Non-Ambulatory Type 3 SMA** | **Apitegromab (20 mg/kg) + nusinersen (n=14)** |
|---|---|
| Mean change from baseline in HFMSE (95% CI) | 1.4 (0.1, 2.7) |
| # (%) patients achieving ≥1-pt increase in HFMSE | 10/14 (71%) |
| # (%) patients achieving ≥3-pt increase in HFMSE | 3/14 (21%) |
| # (%) patients achieving ≥5-pt increase in HFMSE | 2/14 (14%) |

### 7.3.3 Cohort 1

This open-label, single-arm cohort enrolled 23 patients with ambulatory type 3 SMA. Patients are treated with 20 mg/kg of apitegromab Q4W either as a monotherapy or in conjunction with an approved SMN upregulator therapy (nusinersen). This is a patient population for which SMN upregulator therapy alone historically appears to primarily offer motor function stabilization rather than improvement. The primary objectives of the cohort are to assess safety and the mean change from baseline in Revised Hammersmith Scale (RHS).

At baseline, patients across both subgroups of patients had a mean age of 12.6 (range 7-21 years) and a RHS score of 49.6 (range 26-63) out of a total possible score of 69. Patients in the apitegromab monotherapy group had a mean age of 12.1 years old (range 7-19 years) and a mean RHS score of 47.6 (range 26-63). Patients in the group treated with apitegromab and receiving an SMN upregulator (nusinersen) had a mean age of 13.1 (range 7-21 years) and a mean RHS score of 51.3 (range 43-62). One patient discontinued from the trial for reasons assessed to be unrelated to apitegromab but was included in the intent-to-treat interim analysis.

At the six-month interim analysis timepoint:
- Mean change from baseline in RHS score:
   ∘ Apitegromab pooled (n = 23): +0.5 points (95% Cl of -1.2, +2.2)
   ∘ Apitegromab monotherapy (n = 11): +0.7 points (95% Cl of -2.5, +4.0)
   ∘ Apitegromab + nusinersen (n = 12): +0.3 points (95% Cl of -1.4, +2.0)
- Proportion of patients attaining ≥1 point increase in RHS score:
   ∘ Apitegromab pooled: 52% (12/23)
   ∘ Apitegromab monotherapy: 64% (7/11)
   ∘ Apitegromab + nusinersen: 42% (5/12)
- Proportion of patients attaining ≥3 point increase in RHS score:
   ∘ Apitegromab pooled: 26% (6/23)
   ∘ Apitegromab monotherapy: 36% (4/11)
   ∘ Apitegromab + nusinersen: 17% (2/12)
- Proportion of patients attaining ≥5 point increase in RHS score:
   ∘ Apitegromab pooled: 9% (2/23)
   ∘ Apitegromab monotherapy: 9% (1/11)
   ∘ Apitegromab + nusinersen: 8% (1/12)

Improvements in RHS were observed with both apitegromab monotherapy and as add-on to background nusinersen (Table 16; FIG. 7A-B). 52% of the patients saw an improvement, as measured by at least a 1-point increase in RHS at the interim analysis timepoint, and about 26% of the patients achieved at least a 3-point increase. Natural history indicates considerable heterogeneity in ambulatory population, with declines in motor function over time in many patients. Observed comparable effects between monotherapy and add-on arms suggest that clinical effects are due to apitegromab.

**Table 16.**

| **Ambulatory Type** 3 **SMA** | **Apitegromab pooled (n=23)** | **Apitegromab (20 mg/kg) monotherapy (n=11)** | **Apitegromab (20 mg/kg) + nusinersen (n=12)** |
|---|---|---|---|
| Mean change from baseline in RHS (95% CI) | 0.5 (-1.1, 2.2) | 0.7 (-2.5, 4.0) | 0.3 (-1.4, 2.0) |
| # (%) patients achieving ≥1-pt increase in RHS | 12/23 (52%) | 7/11 (64%) | 5/12 (42%) |
| # (%) patients achieving ≥3-pt increase in RHS | 6/23 (26%) | 4/11 (36%) | 2/12 (17%) |
| # (%) patients achieving ≥5-pt increase in RHS | 2/23 (9%) | 1/11 (9%) | 1/12 (8%) |

### 7.4 Additional Secondary Endpoints

Interim analysis results showed initial trends for improvements in motor function as measured by Revised Upper Limb Module (RULM) and World Health Organization (WHO) motor developmental milestones for non-ambulatory patients. No meaningful improvements were observed in timed motor tests of ambulatory type 3 patients.

### 7.5 Overall Safety and Tolerability

No safety signals were identified during the six-month interim analysis (Table 17). The incidence and severity of adverse events were consistent with underlying patient population and background therapy.

**Table 17.**

| **Treatment-emergent adverse events (TEAEs)** | **Apitegromab 2 mg/kg dose (n=10)** | **Apitegromab 20 mg/kg dose (n=48)** | **Total (n=58)** |
|---|---|---|---|
| Any TEAE | 9 (90%) | 40 (83.3%) | 49 (84.5%) |
| Any Serious TEAE | 0 (0.0%) | 1 (2.1%) | 1 (1.7%) |
| Any TEAE leading to study drug discontinuation | 0 (0.0%) | 1 (2.1%) | 1 (1.7%) |
| Any Grade 3 (severe) or higher TEAE | 0 (0.0%) | 0 (0.0%) | 0 (0.0%) |

The five most frequently reported TEAEs were headache, upper respiratory tract infection, pyrexia, nasopharyngitis, and cough.

One patient (Cohort 1) with a prior history of upper respiratory infections experienced a serious TEAE of Grade 2 viral upper respiratory tract infection leading to hospitalization. The event resolved without sequelae and was assessed as unrelated to apitegromab.

One patient (Cohort 1) discontinued from the study due to Grade 2 muscle fatigue that started prior to initiation of dosing with apitegromab. The event was assessed as unrelated to apitegromab.

Two patients (Cohort 1) had gait inability that was considered a significant disability (both Grade 3). Events remain ongoing. The event was assessed as unrelated to apitegromab.

One patient (Cohort 1) was hospitalized with post lumbar puncture syndrome (Grade 2). The event was resolved without sequelae and was assessed as unrelated to apitegromab.

One patient (Cohort 1) presented with post lumbar puncture syndrome (non-serious Grade 3). The event was resolved without sequelae and was assessed as unrelated to apitegromab.

One patient (Cohort 3) receiving 2 mg/kg apitegromab was hospitalized due to adenoidal and tonsillar hypertrophy and scheduled adenotonsillectomy (Grade 2). The event was resolved without sequelae and was assessed as unrelated to apitegromab.

One patient (Cohort 1) with Grade 2 leg muscle fatigue (developed prior to enrollment) withdrew consent after approximately two months in the trial.

Treatment-emergent adverse events (TEAEs) with incidence >5% at six-month interim analysis are listed in Table 18. No grade 3 (severe) or higher adverse events were reported.

**Table 18.**

| **TEAEs > 5% by Maximum Severity - Cohorts 1+2+3** | | | | | | | | | | | | |
|---|---|---|---|---|---|---|---|---|---|---|---|---|
| | **Apitegromab 2 mg/kg dose (N=10)** | | | | **Apitegromab 20 mg/kg dose (N=48)** | | | | **Total (N=58)** | | | |
| **Preferred Term (PT)** | **Grade 1 (Mild)** | **Grade 2 (Mod.)** | **Severe** | **Total** | **Grade 1 (Mild)** | **Grade 2 (Mod.)** | **Severe** | **Total** | **Grade 1 (Mild)** | **Grade 2 (Mod.)** | **Severe** | **Total** |
| Headache | 2 (20.0) | | | 2 (20.0) | 11 (22.9) | 1 (2.1) | | 12 (25.0) | 13 (22.4) | 1 (1.7) | | 14 (24.1) |
| Upper respiratory tract infection | 2 (20.0) | 1 (10.0) | | 3 (30.0) | 7 (14.6) | 3 (6.3) | | 10 (20.8) | 9 (15.5) | 4 (6.9) | | 13 (22.4) |
| Pyrexia | 1 (10.0) | 2 (20.0) | | 3 (30.0) | 4 (8.3) | 4 (8.3) | | 8 (16.7) | 5 (8.6) | 6 (10.3) | | 11 (19.0) |
| Nasopharyngitis | 1 (10.0) | 1 (10.0) | | 2 (20.0) | 6 (12.5) | 1 (2.1) | | 7 (14.6) | 7 (12.1) | 2 (3.4) | | 9 (15.5) |
| Cough | | 2 (20.0) | | 2 (20.0) | 5 (10.4) | 2 (4.2) | | 7 (14.6) | 5 (8.6) | 4 (6.9) | | 9 (15.5) |
| Vomiting | 2 (20.0) | 1 (10.0) | | 3 (30.0) | 4 (8.3) | 1 (2.1) | | 5 (10.4) | 6 (10.3) | 2 (3.4) | | 8 (13.8) |
| Nausea | 1 (10.0) | 1 (10.0) | | 2 (20.0) | 4 (8.3) | 1 (2.1) | | 5 (10.4) | 5 (8.6) | 2 (3.4) | | 7 (12.1) |
| Dizziness | | | | | 4 (8.3) | 2 (4.2) | | 6 (12.5) | 4 (6.9) | 2 (3.4) | | 6 (10.3) |
| Rash | 2 (20.0) | | | 2 (20.0) | 4 (8.3) | | | 4 (8.3) | 6 (10.3) | | | 6 (10.3) |
| Influenza | 1 (10.0) | 1 (10.0) | | 2 (20.0) | 1 (2.1) | 1 (2.1) | | 2 (4.2) | 2 (3.4) | 2 (3.4) | | 4 (6.9) |
| Diarrhoea | 2 (20.0) | | | 2 (20.0) | | 2 (4.2) | | 2 (4.2) | 2 (3.4) | 2 (3.4) | | 4 (6.9) |
| Nasal congestion | | | | | 4 (8.3) | | | 4 (8.3) | 4 (6.9) | | | 4 (6.9) |
| Fall | | | | | 3 (6.3) | 1 (2.1) | | 4 (8.3) | 3 (5.2) | 1 (1.7) | | 4 (6.9) |
| Ear infection | | 2 (20.0) | | 2 (20.0) | 1 (2.1) | | | 1 (2.1) | 1 (1.7) | 2 (3.4) | | 3 (5.2) |
| Rhinorrhoea | 1 (10.0) | | | 1 (10.0) | 2 (4.2) | | | 2 (4.2) | 3 (5.2) | | | 3 (5.2) |
| Tonsillar hypertrophy | 1 (10.0) | | | 1 (10.0) | 2 (4.2) | | | 2 (4.2) | 3 (5.2) | | | 3 (5.2) |
| Muscle spasms | 1 (10.0) | | | 1 (10.0) | 2 (4.2) | | | 2 (4.2) | 3 (5.2) | | | 3 (5.2) |
| Musculoskeletal pain | | | | | 2 (4.2) | 1 (2.1) | | 3 (6.3) | 2 (3.4) | 1 (1.7) | | 3 (5.2) |

### 8 12-MONTH EFFICACY AND SAFETY RESULTS

### 8.1 Summary

The efficacy and safety of intravenous apitegromab dosed every four weeks (Q4W) was evaluated over a 12-month treatment period. Consistent with the six-month interim analysis, the 12-month top-line efficacy and safety data further demonstrate proof-of-concept in patients with type 2 or type 3 SMA.

With the exception of the monotherapy patients, all patients were receiving background nusinersen (Table 13). Such patients were required to be past the loading phase of treatment, and these patients had received an average of about 5 maintenance doses of nusinersen, which translates to roughly two years of treatment, before they enrolled in the study. The baseline HFMSE scores for Cohorts 2 and 3 were in the low to mid 20s even after two years of treatment with nusinersen. These patients had been treated with nusinersen for an average of about 3 years at the end of the 12-month treatment period.

Cohort 1 (20 mg/kg apitegromab monotherapy or in conjunction with nusinersen, ambulatory type 3 patients 7-21 years of age) observed a mean change from baseline in Revised Hammersmith Scale (RHS) of 0.3-point decline (Table 23). The majority of patients (57% (13/23) of patients) maintained or improved their motor function as reflected by a >0-point change from baseline in RHS score. This efficacy signal may suggest a potential clinical effect of apitegromab in a patient population that is otherwise generally expected to decline (Coratti et al. (2020) American Neurology Association, 88:1109-1117).

Cohort 2 (20 mg/kg apitegromab in conjunction with nusinersen, type 2 and non-ambulatory type 3 patients 8-19 years of age) observed a mean change from baseline in Hammersmith Functional Motor Scale Expanded (HFMSE) of 0.6-point improvement (Table 22) at the end of the 12-month treatment period. 64% (9/14) of patients attained >1-point increase from baseline and 29% (4/14) of patients attained >3-point increase from baseline. These results demonstrate the potential durability of the improvements in motor function observed at the six-month interim analysis. Since this is a patient population for which motor function improvement over a 12-month timeframe would generally not be expected, these data also demonstrate the potential additional therapeutic potential of apitegromab (Mercuri et.al. (2018) N Engl J Med. 378:625-635; Mercuri et al. (2016) Neuromuscul Disord. 26(2):126-131).

Cohort 3 (2 or 20 mg/kg apitegromab in conjunction with nusinersen, type 2 patients 2-6 years of age who initiated nusinersen <5 years of age) observed a mean change from baseline in HFMSE of 7.1-point and 5.3-point improvements in the 20 mg/kg dose and the 2 mg/kg dose arms, respectively (Table 21). Across the full cohort, 59% (10/17) of patients attained >5-point increase and 35% (6/17) of patients attained >10-point increase from baseline. These results demonstrate further improvements in motor function relative to the six-month interim analysis.

WHO Motor Developmental Milestones, representing major functional stage achievements that are substantially more challenging than HFMSE, were gained by 7 of 35 non-ambulatory patients, including 3 patients who initiated background nusinersen therapy later in life (≥5 years of age). During the chronic maintenance phase of nusinersen, 12 months of apitegromab treatment led to 20% of the patients gaining ≥1 new WHO Motor Developmental Milestone in cohort 2 (3/15) and cohort 3 (2/10 in 2 mg/kg arm and 2/10 in 20 mg/kg arm). This was observed in patients 2-6 years of age and in patients 8-19 years of age. Five patients achieved 1 new WHO Motor Developmental Milestone, including one patient who gained the ability to walk independently and one patient who gained the ability to stand independently. One patient in cohort 2 gained 2 new WHO Motor Developmental Milestones (standing with assistance and hands and knees crawling) and one patient in cohort 3 (20 mg/kg arm) gained 3 new WHO Motor Developmental Milestones (standing with assistance, hands and knees crawling, and walking with assistance). Mean number of WHO Motor Developmental Milestones at baseline was 1.4 for cohort 2, and 1.6 for cohort 3 high-dose arm, and 1.8 for cohort 3 low-dose arm.

A dose response continued to be observed in relation to clinical efficacy (HFMSE improvements) and target engagement (latent myostatin concentrations). While both doses (2 and 20 mg/kg) yielded high levels of target engagement, the 20 mg/kg dose yielded relatively higher levels than the low dose.

Regarding PK, dose proportional increases in exposure were observed for Cₘₐₓ and AUC_{inf} across all doses evaluated at 12 months. There was little to no accumulation observed for Cₘₐₓ (<2-fold increase). Area under the serum concentration-time curve (AUC) was computed utilizing the linear trapezoidal approximation method. For AUC_{inf}, there was an observed accumulation between the first and 6^{th} dose across all cohorts, as well as between the first and 13^{th} dose. However, there was little to no accumulation in AUC_{inf} between Dose 6 and Dose 13, which was expected since steady state was reached by Dose 6. There was no observed target-mediated drug disposition (TMDD) at either of the two studied doses (2 or 20 mg/kg). However, there was larger variability in the concentrations in the 2 mg/kg group. Terminal elimination rate and half-life were calculated by log-linear regression of the terminal phase of the mean concentration versus time profiles (ideal r² adjusted > 0.75). Mean half-life ranged between 8.4-31.7 days for Cohort 1, 6.92-33.5 days for Cohort 2, 5.67-41 days for Cohort 3 (2 mg/kg), and 12.3-37.5 days for Cohort 3 (20 mg/kg). There was sparse sampling for this data. There was a significant correlation between age and clearance, with younger patients having significantly lower clearance than older patients. There was also a correlation between weight and clearance, with heavier patients having higher clearance. Clearance was found to increase with increasing age. Full PK parameters were only calculated from data for Visit 1, 7, and 13. Comparison of trough values were utilized for all other doses.

Regarding PD, baseline latent myostatin concentrations were lower in all SMA cohorts than in healthy adults. There was a significant correlation between weight and baseline latent myostatin concentrations, with heavier patients having higher baseline latent myostatin concentrations. Cohort 1 had the highest average baseline latent myostatin concentrations across all measured timepoints. Cohort 2 and Cohort 3 (20 mg/kg) had similar latent myostatin concentrations throughout the duration of the study. Cohort 3 (2 mg/kg) had the lowest overall latent myostatin concentrations and the lowest fold-change from baseline. The largest fold-change in latent myostatin from baseline was observed in Cohort 3 (20 mg/kg) followed by Cohort 2, and Cohort 1.

The incidence and severity of adverse events were consistent with the underlying patient population and/or background therapy (nusinersen), with the five most frequently reported treatment-emergent adverse events being headache, pyrexia, upper respiratory tract infection, cough, and nasopharyngitis. Anti-drug antibodies were present at low titers in three out of the 58 enrolled patients. These ADAs had no apparent impact on drug exposure and were not associated with any hypersensitivity reactions. No safety signals for apitegromab were identified as part of the 12-month top-line analysis.

All analyses were prespecified in the Statistical Analysis Plan (SAP). The primary efficacy intent-to-treat analysis (N=58 at baseline, n=54 at 12 months) excluded four patients after they missed three consecutive doses each due to site access restrictions (all at same study site). The per protocol analysis (N=48) was similar to the primary efficacy intent-to-treat analysis but excluded additional patients who had protocol deviations considered major, such as >20% missed doses or the use of prohibited concomitant medication. The sensitivity analysis (N=58) was conducted to assess the robustness of the primary efficacy intent-to-treat analysis by also including the four additional patients excluded from the primary analysis in the statistical assessment. The sensitivity analysis included all patients in the study (including patients with three missed doses).

Overall, the 12-month top-line results suggest that apitegromab may offer therapeutic potential for improving motor function in patients with later-onset SMA. The safety profile of apitegromab, as of the 12-month top-line readout, supports the potential for chronic dosing in a pediatric SMA population.

**Table 19.**

| | **Ambulatory Patients (Revised Hammersmith Scale)** | | | **Non-Ambulatory Patients (Hammersmith Functional Motor Scale Expanded)** | | | |
|---|---|---|---|---|---|---|---|
| | **Cohort 1** | | | **Cohort 2*** | **Cohort 3*** | | |
| | Pooled (n=23) | 20 mg/kg monotherapy (n=11) | 20 mg/kg + nusinersen (n=12) | 20 mg/kg + nusinersen (n=14) | Pooled (n=17) | 2 mg/kg + nusinersen (n=9) | 20 mg/kg + nusinersen (n=8) |
| Mean change from baseline (95% CI) | -0.3 (-2.1, 1.4) | -0.4 (-3.9, 3.1) | -0.3 (-2.0, 1.4) | +0.6 (-1.4, 2.7) | +6.2 (2.2, 10.1) | +5.3 (-1.5, 12.2) | +7.1 (1.8, 12.5) |
| # (%) patients achieving ≥1-pt increase | 9/23 (39%) | 4/11 (36%) | 5/12 (42%) | 9/14 (64%) | 14/17 (82%) | 7/9 (78%) | 7/8 (88%) |
| # (%) patients achieving ≥3-pt increase | 5/23 (22%) | 3/11 (27%) | 2/12 (17%) | 4/14 (29%) | 10/17 (59%) | 5/9 (56%) | 5/8 (63%) |

| | | | | | | | |
|---|---|---|---|---|---|---|---|
| * Four patients (one in Cohort 2 and three in Cohort 3) each missed three consecutive doses of apitegromab over the course of the 12-month treatment period due to site access restrictions and were excluded from the prespecified, primary intent-to-treat analysis. | | | | | | | |

**Table 20.**

| | **Ambulatory Patients** | **Non-Ambulatory Patients** | |
|---|---|---|---|
| | **Cohort 1** | **Cohort 2** | **Cohort 3** |
| | Ambulatory Type 3 | Type 2 and Non-Ambulatory Type 3 (initiated nusinersen ≥5 years old) | Type 2 (initiated nusinersen <5 years old) |
| 6-month interim results | • Mean RHS increase from baseline | • Mean HFMSE increase from baseline | • Mean HFMSE increases from baseline |
| | • Majority of patients maintained or improved (≥0-pt change from baseline) | • Majority of patients improved (≥1-pt increase from baseline) | • Majority of patients achieved ≥3-pt increase |
| | | | • Dose response observed |
| 12-month top-line results | • Mean RHS decline from baseline | • Mean HFMSE increase from baseline | • Further HFMSE increases observed vs. 6-month interim analysis |
| | • Majority of patients maintained or improved (≥0-pt change from baseline) | • Majority of patients improved (≥1-pt increase from baseline) | |
| | | | • Majority of patients achieved ≥5-pt increase |
| | | • High % of patients achieved ≥3-pt increase (29%) | |
| | | | • Dose response continues to be observed |
| Natural history of patient population | *Motor function decline occurs on average in ambulatory type* 3 *SMA and can be severe in subset of patients (see, e.g.,* Coratti et al. (2020) American Neurology Association, 88:1109-1117*).* | *Patients who initiate nusinersen at* ≥5 *years of age commonly experience HFMSE decline and rarely achieve ≥3-point increase (see, e.g.,* Mercuri et.al. (2018) N Engl J Med. 378:625-635*).* | *Patients who initiate nusinersen at <5 years of age primarily stabilize or experience modest and gradual improvement (see, e.g.,* Darras et.al. (2019) Neurology. 92(21) e2492-e2506*).* |

| | | | |
|---|---|---|---|
| Database for HFMSE and RHS scores for the 12-month topline analysis are locked. The 6-month interim analysis was a snapshot and subsequent adjustments by site investigators resulted in the following changes to the 6-month interim results: • Cohort 2: Mean change in HFMSE score from baseline was updated to +1.1-points (from +1.4-points). Proportion of patients with ≥3-point increases was updated to 2/14 (from 3/14) and updated to 1/14 (from 2/14) for patients with ≥5-point increases. • Cohort 3 high dose arm (20 mg/kg): Mean change in HFMSE score from baseline was revised to +5.3-points (from +5.6-points). | | | |

### 8.2 Cohort Analyses

### 8.2.1 Cohort 3

This randomized, double-blind, parallel arm portion of the study enrolled patients with type 2 SMA, age 2 years and older, who had initiated treatment with an approved SMN upregulator (nusinersen) before 5 years of age. Twenty patients were randomized in a 1:1 ratio to receive the low dose (2 mg /kg apitegromab Q4W) or high dose (20 mg/kg apitegromab Q4W). Both treatment arms were in conjunction with an approved SMN upregulator (nusinersen). Three patients (two in high dose arm and one in low dose arm) each missed three consecutive doses of apitegromab due to site access restrictions and were excluded from the pre-specified, primary intent-to-treat analysis. The primary objectives of Cohort 3 were to assess safety and the mean change from baseline in HFMSE following 12 months of treatment. Patients on average had received approximately 2 years of treatment with nusinersen at baseline and approximately 3 years at the end of the 12-month treatment period.

Clinical data from the CHERISH study and the SHINE extension study offer background insights into the clinical course of patients with non-ambulatory later-onset SMA treated with nusinersen, including patients who initiated treatment at a young age. Most nusinersen-treated patients in the CHERISH study were under 5 years of age at the time of therapy initiation. In the CHERISH study, patients who received nusinersen experienced a mean HFMSE improvement of almost 4 points during the first 15 months of treatment. Beyond this initial 15 months of nusinersen treatment, when patients rolled over into the SHINE study, these patients primarily had motor function stabilization or only a modest and gradual mean improvement over the subsequent long-term phase of nusinersen treatment (FIG. 8, which is reproduced from Williams et al. Minimal clinically important differences of the Expanded Hammersmith Functional Motor Scale in later-onset spinal muscular atrophy: results from the phase 3 CHERISH trial. Presented at: 31st Annual Meeting of the American Academy of Managed Care Pharmacy; 25-28 March 2019; San Diego, CA, USA. In contrast, patients in Cohort 3 of the current study (who had been receiving nusinersen prior to study initiation) exhibited further mean increases in HFMSE score when treated with adjunctive apitegromab therapy over a 12-month period rather than the expected stabilization of HFMSE scores based on data from the CHERISH and SHINE studies.

Cohort 3 efficacy data demonstrate sizable further improvements in motor function at 12 months, in patients who had initiated SMN-directed therapy before 5 years of age. The mean change from baseline in HFMSE scores was a 7.1-point and a 5.3-point improvement for the 20 mg/kg and 2 mg/kg arms, respectively (Table 21; FIG. 9A-D). A majority of type 2 non-ambulatory >age 2 patients (63%) experienced > 3-point improvements in HFMSE, which is often considered to be a clinically meaningful improvement. Notably, a 7.1-point increase was observed in type 2 non-ambulatory >age 2 patients during the previously reported plateau in response to chronic nusinersen treatment (Dagbay KB et al. J Biol Chem. 2020; 295(16):5404-5418). The majority (59%) of patients in the whole of Cohort 3 achieved at least a 5-point increase in HFMSE and 35% of patients achieved greater than a 10-point increase in HFMSE over baseline. One patient experienced a 20-point gain and another patient an 18-point gain. Of note, at the six-month interim analysis, only one patient had greater than a 10-point improvement from baseline in HFMSE score. These observed improvements in motor function for both the high and low dose apitegromab arms were greater than expected with nusinersen alone. These results provide evidence of the therapeutic potential of apitegromab in SMA, and demonstrate that further improvements in HFMSE scores occurred between the six-month interim analysis and the 12-month top-line analysis in both the 20 mg/kg and 2 mg/kg dose arms. The per protocol and sensitivity analyses showed results similar to the primary intent-to-treat analysis. Further, dose response was observed in patients from both the 2 mg/kg arm as well as the 20 mg/kg arm with increased benefit observed as early as around 2 months following administration of apitegromab (FIG. 25; type 2 non-ambulatory >age 2 cohort).

The difference in both the magnitude and latency to improvement provides evidence supporting a role of apitegromab in benefitting patients beyond the underlying SMN enhancing treatment, which subjects had been on for an average of 2 years.

Efficacy data from non-ambulatory patients also demonstrated improvements in motor function at 12 months as measured by the revised Upper Limb Motor (RULM) function scores. The mean change from baseline in RULM scores was +1.0, with a 95% confidence interval of -1.7 to 3.7, for the pooled 20 mg/kg and 2 mg/kg arms. Further, 33% (3/9) of the patients in the 2 mg/kg arm and 29% (2/7) of the patients in the 20 mg/kg arm achieved a ≥ 2-point improvement in RULM function relative to baseline, where a 2-point increase in RULM is often considered to be clinically meaningful.

**Table 21.**

| **Type 2 SMA** | **Apitegromab pooled (n=17)** | **Apitegromab low dose (2 mg/kg) + nusinersen (n=9)** | **Apitegromab high dose (20 mg/kg) + nusinersen (n=8)** |
|---|---|---|---|
| Mean change from baseline in HFMSE (95% CI) | +6.2 (+2.2, +10.1) | +5.3 (-1.5, +12.2) | +7.1 (+1.8, +12.5) |
| # (%) patients achieving ≥1-pt increase in HFMSE | 14/17 (82%) | 7/9 (78%) | 7/8 (88%) |
| # (%) patients achieving ≥3-pt increase in HFMSE | 10/17 (59%) | 5/9 (56%) | 5/8 (63%) |
| # (%) patients achieving ≥5-pt increase in HFMSE | 10/17 (59%) | 5/9 (56%) | 5/8 (63%) |
| # (%) patients achieving ≥10-pt increase in HFMSE | 6/17 (35%) | 3/9 (33%) | 3/8 (38%) |

### 8.2.2 Cohort 2

This open-label, single-arm cohort enrolled 15 patients with type 2 or non-ambulatory type 3 SMA, between the ages of 5 and 21, who had initiated treatment with an approved SMN upregulator (nusinersen) after 5 years of age. Patients were treated with 20 mg/kg of apitegromab Q4W in conjunction with an approved SMN upregulator (nusinersen). One patient missed three consecutive doses of apitegromab due to site access restrictions and was excluded from the pre-specified, primary intent-to-treat analysis. The primary objectives of Cohort 2 were to assess safety and the mean change from baseline in HFMSE following the 12-month treatment period.

This is a patient population for which SMN upregulator therapy alone historically appears to primarily offer motor function stabilization, rather than improvement. Clinical data from the nusinersen CHERISH study in non-ambulatory later-onset SMA offers background insights into this patient population. In the CHERISH study, in the subset of patients who started on nusinersen at the age of 5 years or older, there was a mean HFMSE decline of >0.5 points after 15 months of treatment, and less than 15% of patients attained a 3-point or greater increase in HFMSE score (Mercuri et.al. (2018) N Engl J Med. 378:625-635, e.g., at Figure 2A). A majority of patients in this study did not experience HFMSE improvements and rarely achieved a ≥3-point increase. In a separate study, natural history data showed that for patients who are 5 years of age or older, there is a mean decline in HFMSE scores over the course of 12 months. Furthermore, less than 5% of patients achieved a 3-point or greater increase in HFMSE score (Mercuri et al. (2016) Neuromuscul Disord. 26(2):126-131).

In contrast, in Cohort 2 of the current study, improvement in mean HFMSE at 12 months were observed, with a majority of patients achieving ≥1-point increase. Efficacy data demonstrated improvement of motor function from baseline. The mean change from baseline in HFMSE scores was 0.6 points (Table 22; FIG. 10A-C). Potential durability of effect from the six-month interim analysis was observed, as nine patients demonstrated an improvement in HFMSE score at the 12-month timepoint, as compared to 10 patients at the six-month interim analysis. The majority (64%) of patients achieved at least a 1-point increase in HFMSE. A sizeable portion (43%) of patients achieved at least a 2-point increase in HFMSE and (29%) of patients achieved at least a 3-point increase in HFMSE from baseline after 12 months of treatment, which is a rare outcome in this patient population. Further, 14% of the patients achieved at least a 4-point improvement, 14% of the patients achieved at least a 5-point improvement, and 7% of the patients achieved at least a 6-point improvement in HFMSE scores. 29% of the patients with experienced > 3-point improvements in HFMSE; an improvement of 3-point or higher in HFMSE scores is often considered clinically meaningful (FIG. 10C; type 2/3 non-ambulatory; age 5-21 cohort).

Efficacy data from non-ambulatory patients also demonstrated improvements in motor function at 12 months as measured by the revised Upper Limb Motor (RULM) function scores. The mean change from baseline in RULM scores was +1.2, with a 95% confidence interval of -0.5 to 2.9. Further, 36% (5/14) of the patients a ≥2-point in RULM function relative to baseline, where a 2-point increase in RULM is considered to be clinically meaningful.

Additionally, it is contemplated that among patients who started on SMA upregulator/corrector therapy at the age of 5 years or older, HFMSE increases following apitegromab treatment may be more pronounced in patients that are ≤ 12 years of age. Twelve-month efficacy results showed that, in patients aged 5-12 (cohort 2), 75% (6/8) of patients attained >1-point increase of HFMSE from baseline and 50% (4/8) attained >3-point increase of HFMSE from baseline. In non-ambulatory patients, a mean improvement in HFMSE score of +1.6 (-1.3, 4.6) was observed in patients aged 5-12 as compared to a mean improvement in HFMSE score of +0.6 (-1.4, 2.7) overall. An analysis of 12-month HFMSE changes and the duration of prior nusinersen therapy in non-ambulatory patients showed minimal correlation **(****FIGs.** 22A-B), suggesting that HFMSE increases observed are more attributable to apitegromab than nusinersen treatment. Together, these results suggest the therapeutic potential of apitegromab for improving motor function in a patient population that is otherwise not expected to improve on average, regardless of the number of maintenance doses received. Sensitivity analysis showed results similar to the primary intent-to-treat and per protocol analyses. The primary efficacy endpoint improvements in HFMSE score were additive and may be synergistic with the background therapy utilized in this study.

Post hoc analyses of cohort 2 and cohort 3 data showed that patients under 12 years old and/or patients with stable disease decline had higher improvements in HFMSE change from baseline following apitegromab treatment **(****FIGs.** 23A-D). **FIG** 23D shows HFMSE change from baseline in pooled cohorts of non-ambulatory patients treated with apitegromab 20 mg/kg or 2 mg/kg; data excludes 4 patients who each missed 3 doses of apitegromab due to COVID-19-related site access restrictions and were not included in the primary (intent-to-treat) analysis. HFMSE improvements observed across age range of pooled non-ambulatory patients showed greater clinical benefit associated with initiation of apitegromab treatment at an earlier age. It is contemplated that further stratification of patients based on the age of initiation of SMN upregulator/corrector therapy may be helpful in identifying a patient population that is particularly responsive to apitegromab treatment. For instance, in some embodiments, patients who are under the age of 12 and who initiated SMN upregulator/corrector therapy (e.g., nusinersen or risdiplam) before the age of 5 may be particularly responsive to apitegromab treatment.

Pooled data from non-ambulatory type 2 and type 2/3 patients demonstrated an inverse correlation between greater improvements in HFMSE scores and the presence of scoliosis and/or joint contracture in patients such that greater increases in HFMSE scores were observed in non-ambulatory patients who were not symptomatic for scoliosis or joint contractures **(****FIG.** 24A). Patients who skipped 3 consecutive doses due to site restrictions due to COVID-19 were excluded; SD Scoliosis (7.7) min-7, max 20, SD (5.4) min -7, max 13; SD Contracture (7.1) min-3, max 20, SD (2.3) min -7, max 13.

One patient in Cohort 2 (a different patient than the one who missed three consecutive doses of apitegromab due to site access restrictions, as noted above) was identified as having received concomitant treatment with an acetylcholinesterase inhibitor before and during the study, which was not permitted by the study protocol. This patient experienced a 7-point decline in HFMSE score at the 12-month timepoint. In the per protocol analysis, which excluded this patient in accordance with the prespecified approach, the mean change from baseline in HFMSE score for Cohort 2 was a 1.2-point improvement.

**Table 22.**

| **Type 2 and Non-Ambulatory Type 3 SMA** | **Apitegromab (20 mg/kg) + nusinersen** | |
|---|---|---|
| | **Intent-to-treat (n=14)** | **Per protocol* (n=13)** |
| Mean change from baseline in HFMSE (95% CI) | +0.6 (-1.4, +2.7) | +1.2 (-0.5, 2.9) |
| # (%) patients achieving ≥1-pt increase in HFMSE | 9/14 (64%) | 9/13 (69%) |
| # (%) patients achieving ≥3-pt increase in HFMSE | 4/14 (29%) | 4/13 (31%) |
| # (%) patients achieving ≥5-pt increase in HFMSE | 2/14 (14%) | 2/13 (15%) |

| | | |
|---|---|---|
| * One patient was treated with an acetylcholinesterase inhibitor, which was not permitted by the study protocol. | | |

### 8.2.3 Cohort 1

This open-label, single-arm cohort enrolled 23 patients with ambulatory type 3 SMA between the ages of 5 and 21. Patients were treated with 20 mg/kg of apitegromab Q4W either as a monotherapy or in conjunction with an approved SMN upregulator (nusinersen). The primary objectives of Cohort 1 were to assess safety and the mean change from baseline in Revised Hammersmith Scale (RHS) following the 12-month treatment period.

This is a patient population for which SMN upregulator therapy alone historically appears to primarily offer motor function stabilization rather than improvement. Ambulant SMA patients show improved motor function until about age 6 years, a slowly declining phase until adolescence, then a steeper decline around puberty and until age 20. Since the age around puberty appears to be the most vulnerable period, stabilization of motor function may therefore provide an important therapeutic benefit (C Vuillerot et al. Archives of Physical Medicine and Rehabilitation 2013;94:1555-61).

Although the clinical efficacy of SMN upregulator therapy alone in this patient population has not been well-established or characterized, natural history data from a longitudinal study offer background insights. The longitudinal study indicates that patients with ambulatory type 3 SMA commonly experience motor function declines, which in some cases are severe (e.g., loss of ambulation) (Coratti et al. (2020) American Neurology Association, 88:1109-1117, e.g., at Figure 1). In that study, 130 patients with ambulatory type 3 SMA (with some patients lost to follow-up over time) had a mean age of 10.05 and a mean HFMSE score of 52.81 at baseline. At the 12-month assessment, the mean change in HFMSE from baseline was -0.79 points and 11 patients had lost ambulation (mean age of ambulation loss was 10.21 years (SD±6.43)). Relative stability with modest functional improvement was observed in patients until 7 years of age, followed by a steeper decline in subsequent years.

In the current study, in Cohort 1 (pooled population), there was a mean decline in RHS at 12 months, but a majority of patients maintained or improved in RHS score from baseline. The mean change from baseline in RHS score was a 0.3-point decline (Table 23; FIG. 11A-C). The Cohort 1 efficacy data suggest a potential clinical effect of apitegromab in this patient population, as 57% of pooled patients observed a maintenance or improvement in RHS score (>0-point change from baseline), 22% of pooled patients achieved at least a 3-point increase in RHS score from baseline, and 17% of ambulatory patients with type 3 SMA experienced > 3-point improvements in RHS score from the baseline. A RHS score improvement of > 3-points is often considered clinically meaningful. The per protocol and sensitivity analyses showed results similar to the primary intent-to-treat analysis.

Pooled RHS scores from ambulatory type 3 patients also demonstrated an inverse correlation between greater improvements in RHS scores and characteristics of scoliosis or joint contractures such that greater increases in RHS scores were observed in ambulatory patients who were not limited by scoliosis or joint contractures **(****FIG.** 24B). Patients who skipped 3 consecutive doses due to site restrictions due to COVID-19 were excluded; SD Scoliosis (0.7) min-4, max 4, SD (-1.5) min -12, max 8; SD Contracture (1.1) min-2, max 4, SD (-1.5) min -12, max 8.

**Table 23.**

| **Ambulatory Type 3 SMA** | **Apitegromab pooled (n=23)** | **Apitegromab (20 mg/kg) monotherapy (n=11)** | **Apitegromab (20 mg/kg) + nusinersen (n=12)** |
|---|---|---|---|
| Mean change from baseline in RHS (95% CI) | -0.3 (-2.1, +1.4) | -0.4 (-3.9, +3.1) | -0.3 (-2.0, +1.4) |
| # (%) patients achieving ≥0-pt increase in RHS | 13/23 (57%) | 6/11 (55%) | 7/12 (58%) |
| # (%) patients achieving ≥1-pt increase in RHS | 9/23 (39%) | 4/11 (36%) | 5/12 (42%) |
| # (%) patients achieving ≥3-pt increase in RHS | 5/23 (22%) | 3/11 (27%) | 2/12 (17%) |
| # (%) patients achieving ≥5-pt increase in RHS | 1/23 (4%) | 1/11 (9%) | 0/12 (0%) |

### 8.3 Overall Safety and Tolerability

No safety signals were identified during the 12-month treatment period (Table 24). The incidence and severity of adverse events were consistent with the underlying patient population and/or background therapy.

**Table 24.**

| **Treatment-emergent adverse events (TEAEs)** | **Apitegromab 2 mg/kg dose (n=10)** | **Apitegromab 20 mg/kg dose (n=48)** | **Total (n=58)** |
|---|---|---|---|
| Any TEAE | 9 (90%) | 44 (91.7%) | 53 (91.4%) |
| Any Serious TEAE | 1 (10.0%) | 4 (8.3%) | 5 (8.6%) |
| Any TEAE leading to study drug discontinuation | 0 (0.0%) | 1 (2.1%) | 1 (1.7%) |
| Any Grade 3 (severe) or higher TEAE | 0 (0.0%) | 3 (6.2%) | 3 (5.2%) |

The five most frequently reported TEAEs were headache (24%), pyrexia (22%), upper respiratory tract infection (22%), cough (22%), and nasopharyngitis (21%) (Table 25).

Five patients experienced a serious TEAE, which were all assessed as unrelated to apitegromab. One additional patient (Cohort 1) presented with a non-serious Grade 3 post lumbar puncture syndrome. This event resolved without sequelae and was assessed as unrelated to apitegromab.

One patient (Cohort 1) discontinued from the study due to Grade 2 muscle fatigue that started prior to initiation of dosing with apitegromab. The event was assessed as unrelated to apitegromab.

Anti-drug antibodies (ADA) were present at low titers following apitegromab treatment in three out of 58 enrolled patients. No apparent impact on drug exposure was observed, nor were the ADA associated with any hypersensitivity reactions. In one patient, ADA was observed only once during last visit, in another patient it was observed 2 times and in the third patient low titer ADA persisted over 200 days. There was larger variability in the concentrations of apitegromab in the 2 mg/kg group possibly caused by ADA. There is no apparent effect on the clearance of apitegromab in these patients. There was no observed target-mediated drug disposition (TMDD) at either of the two studied doses (2 or 20 mg/kg).

**Table 25.**

| | **Apitegromab 2 mg/kg dose (N=10)** | | | | **Apitegromab 20 mg/kg dose (N=48)** | | | | **Total (N=58)** | | | |
|---|---|---|---|---|---|---|---|---|---|---|---|---|
| **Preferred Term (PT)** | **Grade 1** | **Grade 2** | **Grade 3** | **Total** | **Grade 1** | **Grade 2** | **Grade 3** | **Total** | **Grade 1** | **Grade 2** | **Grade 3** | **Total** |
| **Headache** | 2 (20.0) | | | 2 (20.0) | 11 (22.9) | 1 (2.1) | | 12 (25.0) | 13 (22.4) | 1 (1.7) | | 14 (24.1) |
| **Upper resp. tract infection** | 2 (20.0) | 1 (10.0) | | 3 (30.0) | 7 (14.6) | 3 (6.3) | | 10 (20.8) | 9 (15.5) | 4 (6.9) | | 13 (22.4) |
| **Pyrexia** | 1 (10.0) | 2 (20.0) | | 3 (30.0) | 6 (12.5) | 4 (8.3) | | 10 (20.8) | 7 (12.1) | 6 (10.3) | | 13 (22.4) |
| **Cough** | | 3 (30.0) | | 3 (30.0) | 8 (16.7) | 2 (4.2) | | 10 (20.8) | 8 (13.8) | 5 (8.6) | | 13 (22.4) |
| **Nasophary ngitis** | 1 (10.0) | 2 (20.0) | | 3 (30.0) | 8 (16.7) | 1 (2.1) | | 9 (18.8) | 9 (15.5) | 3 (5.2) | | 12 (20.7) |

| | | | | | | | | | | | | |
|---|---|---|---|---|---|---|---|---|---|---|---|---|
| Grade 1 = mild; Grade 2 = moderate; Grade 3 = severe. | | | | | | | | | | | | |

### 8.4 PK/PD

Pharmacokinetic (PK) and pharmacodynamic (PD) results are supportive of a continued dose response in efficacy. A dose-proportional and sustained drug exposure was observed following chronic administration of apitegromab (FIG. 12A-B). The 20 mg/kg dose achieved numerically greater mean improvements from baseline in HFMSE scores relative to the 2 mg/kg dose across all of the assessed timepoints. The clinically observed dose response was consistent with the pharmacodynamic (target engagement) results. Both the 20 mg/kg and 2 mg/kg doses yielded high levels of target engagement (>100-fold increase from baseline), with the 20 mg/kg dose exhibiting a relatively higher absolute level of target engagement (FIG. 13). Cohort 1 had the highest average baseline latent myostatin concentrations. Target engagement was plotted against change in HFMSE score for patients receiving the 2 mg/kg and 20 mg/kg doses in conjunction with nusinersen (FIG. 14).

FIG. 15A shows a comparison between baseline latent myostatin concentrations in healthy adult volunteers (SAD and MAD) vs. Cohorts 1, 2, and 3. Baseline latent myostatin concentrations were significantly higher in the healthy adult volunteers than in the cohorts. This correlation may be associated with both weight, as well as disease state. FIGs. 15B-C show fold change in latent myostatin concentrations in healthy adult volunteers (SAD) and Cohorts 1, 2, and 3. Increases in serum latent myostatin concentrations following treatment may indicate target engagement by apitegromab, e.g., in skeletal muscle. Such target engagement may correlate with disease severity, weight, age, SMA type and/or functional efficacy measurements.

FIG. 16 shows a correlation between baseline latent myostatin concentration and weight in healthy adult volunteers (SAD and MAD) vs. Cohorts 1, 2, and 3. Baseline latent myostatin concentrations were correlated with patient weight. Overall, the greater the patient weight, the higher the baseline latent myostatin concentration. FIG. 17 shows a linear regression for the correlation between clearance and weight in Cohorts, 1, 2, and 3.

FIG. 18 shows a linear regression for the correlation between clearance and age in Cohorts, 1, 2, and 3. Clearance increased with increasing age. Younger patients had significantly slower clearance than older patients.

### 8.5 Latent Myostatin as a Pharmacodynamic or Predictive Biomarker

FIG. 26 shows analysis of the relationship between improvement in motor function as measured by change of HFMSE scores with respect to age, fold-change in latent myostatin levels (comparing level at baseline and after apitegromab administration), and pharmacokinetic concentration of apitegromab, based on data collected on day 365 following apitegromab treatment from patients with type 2 SMA. FIG. 26A shows strong correlation between change in HFMSE score and age (n=23, p=0.0043). The data suggest that within the patient populations with later onset SMA, myostatin-selective inhition may particularly benefit younger patients. FIG. 26B shows a positive correlation between fold change of latent myostatin levels (LM fold) and the PK concentration of apitegromab (n=14, p=0.0086), indicating that serum latent myostatin levels serve as a target engagement biomarker for myostatin inhibitors that target pro/latent myostatin. FIG. 26C shows a strong positive correlation between age-normalized change in motor function and age-normalized fold change in latent myostatin (LM) levels. FIG. 26D shows a positive correlation between change in age-normalized change in motor function and age-normalized LM Fold/PK concentration (n=14, p=0.0084).

**FIG. 27A** shows the relationship between change in HFME score and baseline latent myostatin. FIG. 27B shows a significant correlation between age-normalized baseline latent myostatin levels and age-normalized change in motor function (p<0.0001). This observation suggests that baseline latent myostatin levels are indicative of efficacy.

### 9 LIST OF ABBREVIATIONS AND DEFINITIONS OF TERMS

The following abbreviations and specialist terms are used throughout this example:
- 6MWT: 6-minute Walk Test
- ADA: Anti-Drug Antibody
- AE: Adverse Event
- APTT: Activated Partial Thromboplastin Time
- ASO: Antisense Oligonucleotide
- CFR: Code of Federal Regulations
- CHO: Chinese Hamster Ovary
- ECG: Electrocardiogram
- eCRF: Electronic Case Report Form
- EDC: Electronic Data Capture
- EIU: Exposure in Utero
- ESNHPT: Endurance Shuttle Nine Hole Peg Test
- ESBBT: Endurance Shuttle Box and Block Test
- FDA: Food and Drug Administration
- GCP: Good Clinical Practice
- GDF11: Growth Differentiation Factor 11
- GLP: Good Laboratory Practice
- HFMSE: Hammersmith Functional Motor Scale Expanded
- ICF: Informed Consent Form
- ICH: International Council for Harmonisation
- IEC: Independent Ethics Committee
- IgG4: Immunoglobulin G4
- IRB: Institutional Review Board
- ITT: Intention to Treat
- IV: Intravenous
- IWRS: Interactive Web-based Randomization System
- LSMEANS: Least Square Means
- mAb: Monoclonal Antibody
- MAD: Multiple Ascending Dose
- MedDRA: Medical Dictionary for Regulatory Activities
- NOAEL: No-Observed-Adverse-Effect Level
- PD: Pharmacodynamics
- PEDICAT: Pediatric Evaluation of Disability Inventory Computer Adaptive Test
- PK: Pharmacokinetic(s)
- PT/INR: Prothrombin Time/International Normalized Ratio
- PROMIS: Patient-Reported Outcomes Measurement Information System
- RHS: Revised Hammersmith Scale
- RULM: Revised Upper Limb Module
- SAD: Single Ascending Dose
- SAE: Serious adverse event
- SAP: Statistical Analysis Plan
- SAS: Statistical Analysis System
- SD: Standard Deviation
- SMA: Spinal Muscular Atrophy
- SMN: Survival Motor Neuron
- SOP: Standard Operating Procedure
- SRK-015: Anti-proMyostatin antibody (apitegromab)
- SST: Safety Surveillance Team
- TEAE: Treatment-emergent Adverse Event
- WHO: World Health Organization

### Example 2. Preclinical Safety Assessment and Toxicokinetics of Apitegromab for the Treatment of Muscle-Atrophying Disease

A comprehensive set of preclinical studies using apitegromab, including pharmacology and pharmacodynamic assessments and *in vivo* toxicology and toxicokinetic (TK) studies in adult cynomolgus monkeys and adult and juvenile rats, was performed. *In vitro* studies confirmed the ability of apitegromab to inhibit the activation of promyostatin. Toxicology studies, in which apitegromab was dosed in monkeys for 4 weeks and in adult rats for up to 26 weeks, showed that weekly intravenous administration achieved sustained serum exposure and target engagement, and was well-tolerated, with no treatment-related adverse findings at the highest doses tested of up to 300 mg/kg. Additionally, results from an 8-week juvenile rat study showed no adverse effects on any endpoint, including neurodevelopmental, motor, and reproductive outcomes at 300 mg/kg. In summary, nonclinical pharmacology, pharmacokinetic, and toxicology data demonstrated that apitegromab is a selective inhibitor of myostatin activation that does not exhibit toxicities observed with other myostatin pathway inhibitors. These data support the conduct of ongoing clinical studies of apitegromab in both adult and pediatric (>2 years) patients with spinal muscular atrophy (SMA).

### MATERIALS AND METHODS

### Test Article and Vehicle

For the *in vitro* studies, apitegromab was produced by transiently transfecting Expi293^{™} cells with heavy and light chain expression vectors (Pirruccello-Straub et al. (2018) Sci Rep. 8(1):2292). For the rat and cynomolgus monkey studies, apitegromab was expressed in a Chinese Hamster Ovary (CHO) cell line that stably expresses the heavy and light chains. Apitegromab was provided as a stock solution at a nominal concentration of 50 mg/mL in buffer and stored at -60 to -80 °C. The vehicle control was matched but without the antibody.

### In Vitro Pharmacology and Tissue Cross-Reactivity

*Sequence homology for the myostatin prodomain and mature growth factor.* The sequence identity of human, cynomolgus monkey, and rat myostatin prodomains and mature growth factor peptide were obtained from the UniProt Database (References 014793, Q95J86, 035312) (UniProt Consortium et al. (2021) Nucleic Acids Res. 49(D1):D480-D489). A pairwise alignment was performed using ClustalOmega (Madeira et al. (2019) Nucleic Acids Res. 47(W1):W636-W641) using the mature protein sequence without its predicted signal peptide, followed by analysis of the percent sequence identity.

*Apitegromab binding affinities.* The binding affinity of apitegromab for human, cynomolgus monkey, and rat promyostatin isoforms was measured using biolayer interferometry (FortéBio Octet QKe) following methods previously described (Pirruccello-Straub et al. (2018) Sci Rep. 8(1):2292). Briefly, in the binding experiments, apitegromab was immobilized on human Fc capture tips (FortéBio), and the association and dissociation of each species' promyostatin was evaluated. All baseline steps, loading of biotinylated antigens, and antibody association and dissociation steps were performed in 1x kinetics buffer containing PBS pH 7.4 with 0.002% Tween 20 and 0.01% BSA. Association proceeded for 300 seconds and dissociations were then performed in 1x kinetics buffer for at least 600 seconds. Data analysis was performed on FortéBio Data Analysis software version 8.2. A 1:1 global fitting model considering both association and dissociation was then utilized for the single concentration K_{D} determination for each antigen antibody/construct pair.

*Promyostatin activation assay.* Ability of apitegromab to inhibit the activation of human, cynomolgus monkey, and rat promyostatin was measured in a previously described cell-based activation assay (Pirruccello-Straub et al. (2018) Sci Rep. 8(1):2292). A sample of human, cynomolgus monkey, or rat promyostatin at 500 nM was incubated with varying amounts of apitegromab to equilibrium. This mixture was then incubated at 37 °C for 16 hours with cell supernatants containing recombinantly overexpressed proprotein convertase (Furin/PACE3 or PSCK5) and a tolloid protease (BMP-1 or mTLL2). The extent of proteolytic release of the mature myostatin growth factor was assessed by adding samples to a culture of 293T cells, which contain a stably integrated pGL4 plasmid (Promega, Madison, WI) responsive to the SMAD activation. Cells were incubated with the myostatin reaction for 6 hours, followed by detection of luciferase expression using BRIGHT-GLOTM reagent (Promega, Madison, WI) according to manufacturer's instructions.

*Tissue cross-reactivity study (TCR).* A GLP tissue cross reactivity study in human tissues that evaluated the potential on- and off-target tissue binding of apitegromab was conducted per ICHS6 (R1) at Charles River Laboratories in Reno, NV. Apitegromab was applied two concentrations (optimal = 5.0 and high = 25.0 µg/mL) to cryosections of a panel of normal human tissues, at least 3 donors per tissue and 38 tissues (sourced from the Special Pathology Services (SPS) Human Tissue Bank). The tissue panels used included all tissues on the suggested list from the EMEA guidelines document for the development and characterization of monoclonal antibodies and all of the tissues recommended by the FDA/Center for Biologics Evaluation and Research (CBER) (EMEA, "Guideline on development, production, characterization and specification for monoclonal antibodies and related products" (Annex I), 2016; FDA/CBER, "Points to Consider in the Manufacture and Testing of Monoclonal Antibody Products for Human Use," 1997). A human IgG4 lambda antibody was used as the control article, to evaluate the specificity of apitegromab. Additionally, the Flp-In T-Rex 293 cell line (Invitrogen, Cat. No. R78007) was transfected with a DNA construct to express pro/latent myostatin as a positive control and used without transfection as a negative control. Localized binding of apitegromab in the cellular tissue samples was performed using a biotinylated antihuman IgG4 secondary antibody.

### In Vivo Toxicology and Toxicokinetic Studies in Cynomolqus Monkeys and Rats

The *in vivo* toxicology studies were conducted at Covance Laboratories in Madison, WI except for the juvenile rat toxicology study, which was conducted at Covance Laboratories in Greenfield, IN. All studies followed applicable International Conference on Harmonisation of Technical Requirements for Registration of Pharmaceuticals for Human Use (ICH) guidelines and were conducted in compliance with United States Food and Drug Administration (FDA) GLP Regulations (21 CFR Part 58). Protocols were reviewed and approved by an institutional animal care and use committee (IACUC). Histology, microscopic evaluation, and bone densitometry of the femur tissue was conducted by Charles River Laboratories Montreal ULC, Senneville, QC, Canada, in compliance with the Organisation for Economic Co-operation and Development (OECD) Principles of Good Laboratory Practice. For the juvenile rat study, developmental neurotoxicity histology was conducted at Tox Path Specialists (TPS) in Frederick, MD and developmental neurotoxicity pathology was conducted (non-GLP) by GEMpath, Inc. in Longmont, CO. For the 26-week rat study, radiograph evaluation (non-GLP) was conducted by a qualified consultant for Veterinary Diagnostic Imaging in Madison, WI. The study designs are summarized in Table 26.

*Cynomolgus Monkey Toxicology Study.* Male and female cynomolgus monkeys (24 to 50 months of age) were randomly assigned to treatment groups and group housed in stainless steel cages in a controlled environment (20 to 26 °C; 30 to 70% relative humidity; 12-hour light and dark cycles). Animals were offered cage enrichment devices and food, Certified Primate Diet No. 5048 (PMI Nutrition International Certified LabDiet^{®}) one to two times daily, and tap water ad libitum.

*Adult and Juvenile Rat Toxicology Studies.* Male and female Sprague Dawley (SD) rats (adult studies, 6 to 8 weeks of age; juvenile study, PND 21 for F1 developmental arm and 15-16 weeks of age for the mating arm) were randomly assigned to treatment groups. In adult studies, all animals were group housed and in the juvenile study F1 animals were individually housed starting on PND 21, naïve males for mating were individually housed (except when pairing), and naïve females were group housed (except when pairing and during gestation). Housing was in polycarbonate cages with Diamond Soft^{®} bedding in a controlled environment (20 to 26 °C; 30 to 70% relative humidity; 12-hour light and dark cycles) except during mating where animals were placed in stainless steel, wire bottom cages. Food, Certified Rodent Diet No. 2014C or 2016C (Envigo RMS, Inc.) and tap water were offered ad libitum, and animals were provided cage-enrichment devices.

*Safety and other Specialized Evaluations.* All toxicology studies evaluated the following general endpoints: mortality, clinical observations, body weights, food consumption. General clinical observations of animals were performed twice daily and cageside observations were conducted 2 to 3 hours post-dose to assess acute toxicity in monkeys and once daily in rats. Detailed observations were performed weekly on dosing days. Food consumption was measured once daily in monkeys, once weekly in adult rats, and every 3-4 days (corresponding to body weight intervals) for juvenile animals. Body weight was measured once weekly for monkeys and adult rats and every 3-4 days in the juvenile study. All studies also included clinical pathology (hematology, serum chemistry, coagulation, and urinalysis) and anatomic pathology (gross and microscopic) evaluations with additional collection of skeletal muscle weights for the biceps femoris and gastrocnemius. Blood samples were collected for toxicokinetic, anti-drug antibody (ADA) and target engagement (serum latent myostatin) analysis. Other safety evaluations performed for the monkey and adult rat studies included ophthalmic examinations (both species); and for the monkey only, vital-sign measurements; electrocardiograms; neurologic exams; and measurements of respiration rate and heart rate.

The following specialized endpoints were evaluated in the 26-week rat study: functional observational battery (FOB), locomotor activity assessments, *in vivo* radiographic image evaluation (femur head and femur neck), *ex vivo* bone mineral content and densitometry by peripheral quantitative computed tomography (pQCT), and histopathology of the femur.

The following specialized endpoints were evaluated in the juvenile rat study: development landmarks, anatomic and developmental neurotoxicity (DNT) and neuropathology, male and female fertility endpoints, neurobehavioral findings and *ex vivo* bone analyses of the femur (i.e., mineral content and densitometry by pQCT, radiography, length, histopathology). DNT pathology examination involved an assessment of numerous sections from the brain, spinal cord, dorsal root ganglia, and nerve, including key neural structures as listed in the "best practice" recommendations for nervous system sampling during nonclinical toxicity studies (Bolon et al. (2013) Toxicol Pathol. 41:1028-1048; Bolon et al. (2018) Toxicol Pathol. 46:372-402).

**Table 26. Apitegromab GLP Multi-Dose Toxicology and Toxicokinetic Studies in Cynomolgus Monkeys and Sprague Dawley Rats**

| | **Study Duration** | **N** | | **Groups** | **IV Bolus Dose** | | **TK and ADA Sampling** | **Clinical Pathology^{e} Sampling** | **Additional Safety, PD, and Specialized Endpoints** |
|---|---|---|---|---|---|---|---|---|---|
| **Species** | | **M** | **F** | | **Levels (mg/kg/ week)** | **Total Doses** | | | |
| Cynomolgus Monkey, adult | 4-week dosing phase with 4-week recovery phase | 24 | 24 | 6/sex/group^{a} | 0, 10, 30, 100 | 5 | Day 1: predose^{d} and 1, 48, 96, 120, 168 hours postdose | Day 30 (dosing) | Ophthalmic; ECG; Vital signs; Respiration; Neurologic Evaluation; Muscle weights; Latent myostatin |
| | | | | | | | Day 15: predose^{d} | | |
| | | | | | | | Day 22: predose^{d} and 1, 48, 96, 120 postdose | Day 29 (recovery) | |
| | | | | | | | Day 29: predose^{d} | | |
| | | | | | | | Once on Days 8, 15^{d}, 22, and 29^{d} (recovery phase) | | |
| SD Rat, adult | 4-week dosing phase with 4-week recovery phase | 93 | 93 | 15/sex/group^{b} | 0, 10, 30, 100 | 5 | Day 1: predose^{d} and 1, 24, 48, 72, 120, 168 hours postdose | Day 30 (dosing) | Ophthalmic; Muscle weights; Latent myostatin |
| | | | | | | | Day 15: predose^{d} | | |
| | | | | TK and ADA: 6/sex (control) and 9/sex/group (treated) | | | Day 22: predose | Day 29 (recovery) | |
| | | | | | | | Day 29: predose^{d} and 1, 24, 48, 72, 120, 168 hours postdose | | |
| | | | | | | | Once on Days 15^{d}, 22, and 29^{d} (recovery phase) | | |
| SD Rat, adult | 26-week dosing phase with 8-week recovery phase | 11 7 | 11 7 | 15/sex/group^{b} | 0, 30, 100, 300 | 27 | Day 1: predose^{d} and 1, 24, 48, 72, 120, 168 hours postdose | Day 91 and 184 (dosing) | Ophthalmic; FOB; Locomotor activity; *in vivo* radiography; Femur histopathology and densitometry; Muscle weights; Latent myostatin |
| | | | | TK and ADA: 6/sex (control) and 9/sex/group (treated) | | | Days 15, 22, 29, 43, 57, 71, 85, 99, 113, 127, 141, 155, 169: predose^{d} | | |
| | | | | | | | Day 183: predose^{d} and 1, 24, 48, 72, 120, 168^{d} hours postdose | Day 57 (recovery) | |
| | | | | | | | Once on Days 14, 21, 28, 35, 42, 49 and 56 (recovery phase) ^{d} | | |
| SD Rat, juvenile (PND 21 - PND 63) | 7-week dosing phase with 8-week | 20 2 | 24 2 | 40 M/group; 50 F/group | 0, 30, 100, 300 | 7 | PND 21: predose^{d} and 1, 24, 48, 72, 96, 120, 168 hours postdose | PND 64 and 132/133 | Fertility; Sperm Assessment; Femur length, Densitometry, *ex vivo* radiography, histopathology; Locomotor Activity; Neurobehavioral |
| | | | | | | | PND 35^{d}, 42, 49^{d}, 56: predose | | |
| | | | | TK and ADA: 6/sex | | | PND 63: predose^{d} and 1, 24, 48, 72, 96, 120, 168 hours postdose | | |
| | recovery phase | | | (control) and 12/sex/group treated | | | Once on PND 77^{d}, 84, 91^{d} (recovery phase) | | Assessment; DNT histopathology; Muscle weights; Latent myostatin |

| | | | | | | | | | |
|---|---|---|---|---|---|---|---|---|---|
| ^{a} 4/sex/group dosing phase; 2/sex/group recovery phase ^{b} 10/sex/group dosing phase; 5/sex/group recovery phase ^{c} 20/sex/group dosing phase; 20 M/group and 30 F/group recovery phase ^{d} ADA also collected ^{e} Clinical pathology included hematology, serum chemistry, coagulation, and urinalysis Abbreviations: DNT, developmental neurotoxicity; F, female; FOB, functional observational battery; GLP, good laboratory practices; IV, intravenous; M, male; PND, postnatal day; PD, pharmacodynamic; TK, toxicokinetic. | | | | | | | | | |

*Quantification of Apitegromab in Cynomolgus Monkey and Sprague Dawley (SD) Rat Serum.* Serum samples were analyzed for the presence of apitegromab using an ELISA validated in both cynomolgus monkey and SD rat serum. Assay plates were coated with recombinant promyostatin and blocked with a solution of bovine serum albumin (BSA). Serum samples were diluted to the minimum required dilution (MRD) (1:100) or further to ensure the analyte was within the quantitative range of the assay. The diluted samples were then incubated on the assay plate. Bound apitegromab was detected using a goat-antihuman horseradish peroxidase (HRP) reagent, and the assay signal was generated using tetramethylbenzidine (TMB). The standard curve was fitted to a 5-parameter logistic function with 1/Y2 weighting. The quantitative range is 200 to 10,000 ng/mL apitegromab in serum. For all studies, sample analysis was conducted (GLP) at Biologics Development Services in Tampa, FL.

*Detection of Antiapitegromab Antibodies in Cynomolgus Monkey and SD Rat Serum.* Serum samples were assessed for the presence of anti-drug antibodies (ADA) against apitegromab using an electrochemiluminescent (ECL) bridging-format plate-based assay. Samples were acidified with 100 mM acetic acid to dissociate any ADA complexes, followed by neutralization with a Tris buffer and incubation with biotinylated and ruthenium-conjugated apitegromab. The neutralized samples were then incubated on streptavidin-coated assay plates, and the assay signal was measured. Samples that resulted in assay signals above an established cut-point value were then assayed in a confirmatory assay, in which excess apitegromab was added to the neutralized sample to show immunodepletion of the assay signal. This assay was validated for use in both cynomolgus monkey and SD rat serum, using an MRD of 1:20. The sensitivity was measured at 4.23 ng/mL in the SD rat assay and 23.5 ng/mL in the cynomolgus monkey assay. For all studies, sample analysis was conducted (GLP) at Biologics Development Services in Tampa, FL.

*Quantification of Latent Myostatin in Cynomolgus Monkey and SD Rat Serum.* Latent myostatin concentrations were quantified using a qualified ECL assay that selectively detects total latent myostatin (i.e., bound and unbound). The qualification of this assay in cynomolgus monkey serum is described in Cote et al. (2020) SLAS Discov Adv Sci Drug Discov. 25(1):95-103, and that protocol was used here without modification. A version of this assay was also qualified in SD rat serum. The assay sensitivity was 10 ng/mL of latent myostatin in serum and achieved a drug tolerance of up to 1 mg/mL of apitegromab. For all studies, latent myostatin was measured (non-GLP) at Scholar Rock, Inc. in Cambridge, MA.

*Toxicokinetics.* Toxicokinetic parameters were calculated for each study using Phoenix WinNonLin (Certara, Princeton, NJ). Noncompartmental analyses were performed using an IV bolus dose administration route, nominal dose levels, and nominal sampling times. Concentrations that were below the limit of quantitation (<200 ng/mL) were treated as 0 for descriptive statistics. Area under the curve (AUC) assessments were calculated using the linear trapezoidal rule. Terminal half-life (t½) was estimated by log-linear regression of the terminal phase of the mean concentration versus time profiles. At least 3 points clearly visible in the terminal phase, an r2 adjusted value >0.7, and a time interval of at least 3 half-lives were used to characterize half-life. Accumulation ratio (AR) was calculated as [Cₘₐₓ or AUC₀₋₁₆₈ on the last day of dosing] / [Cₘₐₓ or AUC₀₋₁₆₈ on the first day of dosing] for all studies except the 4-week cynomolgus monkey study. In that study, AR was calculated using the day 22 values rather than the last dosing day.

### RESULTS

### Species Selection for Toxicology Studies

Prior to initiating the *in vivo* toxicology studies, rat and cynomolgus monkey were evaluated as the appropriate rodent and nonrodent species for assessing apitegromab toxicity, per ICH S6 and S9 guidelines (FDA, Guidance for Industry: S9 Nonclinical Evaluation for Anticancer Pharmaceuticals. Silver Spring, MD 20993: FDA, 2010; FDA, Guidance for Industry: S6(R1) Preclinical Safety Evaluation of Biotechnology-Derived Pharmaceuticals. Silver Spring, MD 20993: FDA, 2012). The ability of apitegromab to increase and strengthen muscle mass has previously been demonstrated in preclinical pharmacology studies in healthy animals and in multiple rodent models of muscle atrophy, including two models of SMA (Pirruccello-Straub et al. (2018) Sci Rep. 8(1):2292; Long et al. (2019) Hum Mol Genet. 28(7):1076-1089). For the studies described here, the sequence identity of myostatin prodomain was compared and the *in vitro* binding and activity of apitegromab was confirmed across human, rat and cynomolgus monkey.

*Myostatin prodomain has similar sequence identity and binding to apitegromab across species.* Apitegromab selectively binds to the human myostatin prodomain and not to other closely related TGFβ family growth factors, notably GDF-11 and activins (Pirruccello-Straub et al. (2018) Sci Rep. 8(1):2292). Human and rat myostatin prodomains share 94.5% sequence homology, while the sequence identity between the human and cynomolgus monkey myostatin prodomain is 99.7% (Table 27). Similarly, the sequence of the mature myostatin growth factor in human, rat and cynomolgus monkey share high sequence identity. Furthermore, apitegromab has similar binding affinity to promyostatin across human, rat and cynomolgus monkeys, ranging from 2.00x10⁻⁹ M to 4.86x10⁻⁹ M (Table 27), suggesting potentially similar activity of apitegromab across these species.

**Table 27. Myostatin Prodomain Sequence Identity / Promyostatin Binding Affinity Across Species**

| **Species** | **Prodomain (% Identity)** | | **Binding Affinity to promyostatin*** |
|---|---|---|---|
| | **Rat** | **Monkey** | **K_{D} (M)** |
| **Human** | 94.9% | 99.7% | 3.42x10⁻⁹ |
| **Rat** | - | 95.2% | 2.0x10⁻⁹ |
| **Monkey** | - | - | 4.86x10⁻⁹ |

| | | | |
|---|---|---|---|
| *promyostatin contained approximately 10-15% latent myostatin | | | |

*Apitegromab inhibits proteolytic activation of promyostatin. In vitro* activity of apitegromab was assessed using a previously described a cell-based activity assay (Pirruccello-Straub et al. (2018) Sci Rep. 8(1):2292). To confirm similar inhibitory activity of apitegromab across species, the assay measured the ability of apitegromab to block the proteolytic activation of human, cynomolgus monkey and rat promyostatin to the mature myostatin growth factor. The IC₅₀ values for each promyostatin form were calculated from the resulting curve fits. Apitegromab inhibits proteolytic activation of human promyostatin (IC₅₀ = 1017 nM), and also prevents activation of cynomolgus promyostatin (IC₅₀ = 584 nM) and of rat promyostatin (IC₅₀ = 153 nM) (FIG. 19).

### Human Tissue Cross-Reactivity of Apitegromab

*Apitegromab does not exhibit off-target binding in a human tissues.* To evaluate the potential on- and off-target tissue binding of apitegromab, a GLP tissue cross reactivity study was conducted using a full panel of human tissues from three different human donors (ICHS6 (R1)). Apitegromab bound to positive and negative control cell lines; however, it did not show specific positive staining in any human tissue.

### In vivo Safety Assessment of Apitegromab in Cynomolgus Monkeys and Rats

Four-week GLP toxicology studies were conducted in cynomolgus monkeys and rats to support the initiation of a Phase 1 clinical study (Barrett et al. (2021) Abstract 253. 24th Annual International Annual Congress of the World Muscle Society, Copenhagen, Denmark). Additional repeat-dose GLP studies were conducted in rats, which included a juvenile rat toxicity study to support dosing in pediatric populations and a long term, 26-week toxicity study in adult animals.

*Four-week Toxicology Study in Cynomolgus Monkeys.* Male and female cynomolgus monkeys were administered apitegromab once weekly by IV bolus at doses of 0 (vehicle), 10, 30, and 100 mg/kg, on Days 1, 8, 15, 22, and 29 (5 doses total) for 4 weeks of the dosing phase, followed by a 4 week (treatment-free) recovery phase. No mortality was observed on the study and all animals survived until the scheduled necropsy. There were no apitegromab-related adverse effects on clinical observations, food consumption, ophthalmic examinations, clinical chemistry, hematology, coagulation, or urinalysis. Muscle mass (gastrocnemius and biceps) was slightly higher in apitegromab-treated animals compared to control animals, although the effects lacked a dose response and showed individual variability. Safety pharmacology endpoints (i.e., neurological examinations, respiration rates, and electrocardiography) demonstrated no treatment-related effects. No apitegromab-related macroscopic or microscopic findings were observed in the dosing or recovery phase animals. Overall, there were no treatment-related adverse findings observed on any endpoint evaluated and the NOAEL was 100 mg/kg, which was the highest dose tested.

*Four-week Toxicology Study in Adult SD Rats.* Male and female rats were administered apitegromab once weekly by IV bolus at doses of 0 (vehicle), 10, 30, and 100 mg/kg, on Days 1, 8, 15, 22, and 29 (5 doses total) for 4 weeks of dosing phase, followed by a 4-week (treatment-free) recovery phase. No apitegromab-related mortality was observed. There were no apitegromab-related adverse effects on clinical observations, food consumption, ophthalmic examinations, hematology, coagulation, or urinalysis. Apitegromab-related body weight increases were observed in animals administered ≥10 mg/kg that did not correlate with higher food consumption but did correlate with increased skeletal muscle weights, at termination. Specifically, anatomic pathology findings were limited to weight increases for skeletal muscle tissues (left biceps, right biceps, left gastrocnemius, or right gastrocnemius) of all groups administered apitegromab, that occasionally reached significance, with a mean effect that ranged from approximately 3-32% over controls; however, there were no microscopic correlates. Increased muscle mass was an anticipated pharmacological effect of apitegromab, an antibody that targets promyostatin and latent myostatin, as previously reported in healthy and disease animal models (Pirruccello-Straub et al. (2018) Sci Rep. 8(1):2292; Long et al. (2019) Hum Mol Genet. 28(7):1076-1089). Several minor apitegromab-related clinical chemistry findings (e.g., mildly higher globulin concentration, mildly higher total protein concentrations and lower albumin:globulin (A:G) ratios) were observed at the end of the dosing phase, predominately in animals administered 100 mg/kg, and most exhibited evidence of reversibility. Increases in globulin concentrations may have been related to the high levels of apitegromab (an IgG4 immunoglobulin) in circulation. No treatment-related adverse findings were observed on any endpoint evaluated and the no observed adverse effect level (NOAEL) was 100 mg/kg, which was the highest dose tested.

*Twenty Six-week Toxicology Study in Adult SD Rats.* Rats were selected as the single toxicity species for further toxicology assessment of apitegromab because of similar results (i.e., no toxicity observed) in the 4-week rat and cynomolgus monkey toxicology studies, per ICH S6 (R1) (Cavagnaro et al. (2002) Nat Rev Drug Discov. 1(6):469-475; FDA, Guidance for Industry: S6(R1) Preclinical Safety Evaluation of Biotechnology-Derived Pharmaceuticals. Silver Spring, MD 20993: FDA, 2012).

Apitegromab was administered by IV-bolus at doses of 30, 100, and 300 mg/kg once weekly to male and female rats, for 26 weeks for a total of 27 doses. No apitegromab-related mortality was observed. There were no adverse apitegromab-related effects on clinical observations, food consumption, clinical chemistry, hematology, ophthalmic examinations, functional observational battery (FOB) testing, bone mineral density (femur), *in vivo* radiography (femur), macroscopic pathology and microscopic pathology evaluations. Similar to the 4-week rat study, apitegromab-related clinical chemistry effects were limited to minimally higher total protein and globulin concentrations and anatomic pathology findings were limited to slight increases in skeletal muscle weights (left and right biceps brachii). The skeletal muscle mass increases occurred in all apitegromab-treated groups, ranged from approximately 15-33% over controls and correlated with microscopic findings of minimal to slight hypertrophy of muscle fibers. These muscle changes persisted during the recovery phase but were not dose-responsive (Pirruccello-Straub et al. (2018) Sci Rep. 8(1):2292; Long et al. (2019) Hum Mol Genet. 28(7):1076-1089). Administration of apitegromab once weekly by IV bolus administration for up to 26-weeks was well-tolerated in adult SD rats and the NOAEL was the highest dose tested of 300 mg/kg.

*Juvenile Toxicology Study in SD Rats.* To support the dosing of pediatric patients ( ≥ 2 years of age) with SMA, a juvenile rat GLP toxicology study was conducted. Juvenile rats were dosed for 7 weeks from postnatal day (PND) 21, the equivalent of a 2-year old human, until PND 63, when animals are sexually mature, and the development of major organs and the immune system are generally complete (FDA, "S11 Nonclinical Safety Testing in Support of Development of Paediatric Medicines," 2020). The study design also incorporated neurotoxicology, fertility, and reproductive and developmental toxicology endpoints to investigate the effects of myostatin pathway inhibition on young animal development and reproductive biology (Table 26).

In this study, apitegromab was administered by IV bolus administration at weekly doses of 30, 100, and 300 mg/kg. There were no apitegromab-related effects observed on mortality, clinical observations, food consumption, clinical pathology, ophthalmic examinations, and anatomic pathology evaluations. No apitegromab-related effect was observed on mean body weight or weight gain in males; however, an apitegromab-related, non-adverse, significant increase in mean body weight gain was noted for females over the entire dosing period (PND 21 through 63) that persisted into the recovery phase for some animals. Several clinical pathology findings were observed in apitegromab-treated juvenile rats, though due to their overall small magnitude, none were considered adverse. These included minimally to mildly higher total protein and/or globulin concentrations, which resulted in minimally lower albumin:globulin ratio in both sexes administered ≥100 mg/kg/dose, mildly lower triglyceride concentration in males administered 300 mg/kg/dose and minimally lower sodium concentration in males administered ≥100 mg/kg/dose and females administered 300 mg/kg/dose. The changes in globulin may have been due to apitegromab in circulation. Similar to the studies performed in adult animals, in apitegromab-treated males and females there was an increase in absolute muscle weights (biceps and/or gastrocnemius), with a mean effect that ranged from approximately 13-28% over controls, and with no apparent microscopic correlates or dose response. Additionally, no adverse toxicity was observed for any of the specialized study endpoints including anatomic and developmental neurotoxicity (DNT) and neurohistopathology, neurobehavioral evaluations (acoustic startle, locomotor activity, or Morris water maze), developmental sexual landmarks, and bone analyses (densitometry, radiography, femur length, and femur histopathology). Lastly, following the breeding of treated males and females with naïve animals, apitegromab had no adverse effects on reproductive parameters (estrous cycles, reproductive performance, fecundity, fertility, sperm assessment); however, an apitegromab-related increase in mean body weight was observed during gestation for some females. Administration of apitegromab once weekly by IV bolus administration, from PND 21 to PND 63 (7 doses total), was well-tolerated in juvenile SD rats and the NOAEL was the highest dose tested of 300 mg/kg.

### Multi-dose Toxicokinetics of Apitegromab in Cynomolgus Monkeys and Rats

The apitegromab multi-dose TK profile, TK parameters, and anti-drug antibodies were evaluated in the 4-week studies in adult rats and cynomolgus monkeys, the 26-week study in adult rats, and the juvenile rat study. In all studies, systemic exposure to apitegromab was confirmed in treated animals (FIG. 20A-D). The Cₘₐₓ and AUC₀₋₁₆₈ parameters increased dose proportionally with increasing dose level and showed accumulation after multiple doses (Table 28). Additionally, there were no differences in apitegromab exposure between male and female animals. Across all studies and dose groups, apitegromab showed minimal-to-no sex-differences in kinetics, achieved dose-proportional exposure, and demonstrated accumulation following multiple doses.

*Multi-dose TK in Cynomolgus Monkeys (4-weeks).* Male and female cynomolgus monkeys were administered 5 IV bolus doses of apitegromab at dose levels of 10, 30, and 100 mg/kg, on Days 1, 8, 15, 22, and 29. The TK parameters were determined after day 1 and day 22. Cₘₐₓ was observed at the first sampling point of 1 hour following dosing in all dosed animals, with the exception of day 22 where one animal in 100 mg/kg group had a Cₘₐₓ observed at 48 hours after dosing (Table 28). The t½ for apitegromab was not measured in this study due to a lack of a distinct elimination phase. Exposure, as assessed by apitegromab Cₘₐₓ and AUC₀₋₁₆₈, demonstrated a dose-dependent increase on days 1 and 22 and was approximately dose proportional from 10 to 100 mg/kg, suggesting that the target was fully saturated. Animals maintained exposure throughout dosing and recovery periods (FIG. 20A) and accumulation was observed at all doses from day 1 on day 22 (Table 28). No animals developed ADA following apitegromab administration at any dose.

*Multi-dose TK in Adult Sprague Dawley Rats (4-weeks).* Male and female rats were administered 5 IV bolus doses of apitegromab at dose levels of 10, 30, and 100 mg/kg, on Days 1, 8, 15, 22, and 29. The TK parameters were determined after day 1 and day 29. Cₘₐₓ was observed at the first sampling point of 1 hour in all groups on both days and apitegromab attained a t½ ranging from 326 to 358 hours on day 29 (Table 28). Exposure, as assessed by apitegromab Cₘₐₓ and AUC₀₋₁₆₈, demonstrated a dose-dependent increase on days 1 and 29 and was generally dose proportional from 10 to 100 mg/kg, suggesting that the target was fully saturated. Animals maintained exposure throughout dosing and recovery periods and accumulation was observed at all doses, following weekly administration (FIG. 20B). Two females, one in the 10 mg/kg group and one in the 30 mg/kg group, were ADA-negative for all intervals in the dosing phase but were ADA-positive on Days 15 and 29 of the recovery phase, which correlated with lower concentrations of apitegromab measured for these animals following the Day 29 dose (compared with other animals in the group). All other animals were ADA-negative throughout the study.

*Multi-dose TK in Adult Sprague Dawley Rats (26-weeks).* Male and female rats were administered 27 IV bolus doses of apitegromab at dose levels of 30, 100, and 300 mg/kg for 26 weeks. The TK parameters were determined after day 1 and day 183. Cₘₐₓ was observed at the first sampling point of 1 hour in all groups on both days and apitegromab attained a t½ ranging from 256 to 346 hours on day 183 (Table 28). Exposure, as assessed by apitegromab Cₘₐₓ and AUC₀₋₁₆₈, demonstrated a dose-dependent increase and was generally dose proportional. Animals maintained exposure throughout dosing and recovery periods and accumulation was observed at all doses, following weekly administration (FIG. 20C). In the 30 mg/kg group, ADA were detected in one male (on day 43) and two females (one each on day 42 and day 99). Serum concentrations of apitegromab were not affected for the male but both females showed decreased exposure. ADA were not detected at any interval in any animal in the 100 and 300 mg/kg dose groups.

*Multi-dose TK in Juvenile Sprague Dawley Rats.* The PK profile and parameters of apitegromab were evaluated in the multi-dose TK rat juvenile study. Juvenile male and female rats were administered 7 IV bolus doses of apitegromab at dose levels of 30, 100, and 300 mg/kg from PND 21 to PND 63. The PK parameters were determined after PND 21 and PND 63. Cₘₐₓ was observed at the first sampling point of 1 hour in all groups on both days and apitegromab attained a t½ ranging from 265 to 330 hours on PND 63 (Table 28). Exposure, as assessed by apitegromab Cₘₐₓ and AUC₀₋₁₆₈, demonstrated a dose-dependent increase and was generally dose proportional. Animals maintained exposure throughout dosing and recovery periods and accumulation was observed at all doses, following weekly administration (FIG. 20D). All animals were determined to be ADA-negative.

### Target Engagement with Apitegromab in Cynomolgus Monkey and Rats

Target engagement with apitegromab was determined by measuring serum latent myostatin levels across the dosing and recovery phases of the study (FIG. 21A-D). The baseline levels of latent myostatin ranged from 20-100 ng/mL across all studies. In all studies, after an initial dose of apitegromab, serum latent myostatin concentrations increased over baseline and remained at this elevated concentration throughout the dosing and recovery phases. The absolute level of latent myostatin achieved after dosing increased with increasing dose but was not dose proportional. As with apitegromab exposure, there was a notable change in latent myostatin concentrations associated with confirmed ADA responses. ADA positive animals were excluded from the group averaged values.

**Table 28. Group Mean PK Parameters for Apitegromab in Cynomolgus Monkey and Rat Serum From GLP Multi-Dose TK Studies**

| **Study** | **Day** | **Dose Level (mg/kg/week)** | **Cₘₐₓ (µg/mL)** | **Tₘₐₓ (h)** | **t½ (h)** | **Dose Normalized Cₘₐₓ [(µg/mL) / (mg/kg/week)]** | **Cₘₐₓ AR** | **AUC₀₋₁₆₈ (h*µg/mL)** | **Dose Normalized AUC₀₋₁₆₈ [(h*µg/mL) / (mg/kg/week)]** | **AUC₀₋₁₆₈ AR** |
|---|---|---|---|---|---|---|---|---|---|---|
| **4-week Cynomolgus Monkey** | Day 1 | 10 | 369 | 1.0 | - | 36.9 | - | 30,400 | 3,040 | - |
| | | 30 | 876 | 1.0 | - | 29.2 | - | 77,200 | 2,570 | - |
| | | 100 | 2,560 | 1.0 | - | 25.6 | - | 240,000 | 2,400 | - |
| | Day 22 | 10 | 744 | 1.0 | NC | 74.4 | 1.94 | 73,900 | 7,390 | 2.41 |
| | | 30 | 1,610 | 1.0 | NC | 53.5 | 1.87 | 186,000 | 6,200 | 2.41 |
| | | 100 | 5,210 | 4.92^{a} | NC | 52.1 | 2.08 | 577,000 | 5,770 | 2.41 |
| **4-week Rat** | Day 1 | 10 | 213 | 1.0 | - | 21.3 | - | 16,200 | 1,620 | - |
| | | 30 | 695 | 1.0 | - | 23.2 | - | 51,100 | 1,700 | - |
| | | 100 | 2,360 | 1.0 | - | 23.6 | - | 170,000 | 1,700 | - |
| | Day 29 | 10 | 425 | 1.0 | 342 | 42.5 | 1.99 | 47,300 | 4,730 | 2.93 |
| | | 30 | 1,420 | 1.0 | 358 | 47.4 | 2.05 | 156,000 | 5,190 | 3.05 |
| | | 100 | 4,390 | 1.0 | 326 | 43.9 | 1.86 | 452,000 | 4,520 | 2.67 |
| **26-week Rat** | Day 1 | 30 | 440 | 1.0 | - | 14.7 | - | 33,100 | 1,100 | - |
| | | 100 | 1,600 | 1.0 | - | 16.0 | - | 117,000 | 1,170 | - |
| | | 300 | 4,220 | 1.0 | - | 14.1 | - | 312,000 | 1,040 | - |
| | Day 183 | 30 | 1,460 | 1.0 | 346 | 48.8 | 3.32 | 126,000 | 4,210 | 3.81 |
| | | 100 | 4,760 | 1.0 | 270 | 47.6 | 2.97 | 409,000 | 4,090 | 3.48 |
| | | 300 | 10,500 | 1.0 | 256 | 34.8 | 2.48 | 978,000 | 3,260 | 3.13 |
| **7-week Juvenile Rat** | PND 21 | 30 | 352 | 1.0 | - | 11.7 | - | 26,600 | 886 | - |
| | | 100 | 1,310 | 1.0 | - | 13.1 | - | 96,400 | 964 | - |
| | | 300 | 4,130 | 1.0 | - | 13.8 | - | 296,000 | 988 | - |
| | **PND** 63 | 30 | 1,050 | 1.0 | NC | 35.0 | 2.99 | 106,000 | 3,540 | 3.99 |
| | | 100 | 4,020 | 1.0 | 330 | 40.2 | 3.07 | 361,000 | 3,610 | 3.74 |
| | | 300 | 11,200 | 1.0 | 265 | 37.3 | 2.71 | 867,000 | 2,890 | 2.92 |

| | | | | | | | | | | |
|---|---|---|---|---|---|---|---|---|---|---|
| ^{a} One male animal had a Tₘₐₓ of 48 hours Pharmacokinetic parameters of apitegromab after weekly administration to adult rats, juvenile rats, and cynomolgus monkeys. The mean toxicokinetic parameters of apitegromab were calculated across nonclinical studies as arithmetic means except for Tₘₐₓ, which is reported as the median time. Select mean toxicokinetic parameters were calculated in each study following the first dose and after multiple doses and are shown as male and female combined. Abbreviations: Cₘₐₓ, maximum concentration; Tₘₐₓ, time at maximum observed concentration; AR, accumulation ratio; AUC₀₋₁₆₈, area under the curve from 0 to 168 hours; NC, not calculated due to a lack of a distinct elimination phase | | | | | | | | | | |

### SUMMARY

Preclinical toxicology and TK studies (Table 1) were conducted to characterize the safety and exposure profile of apitegromab (Pirruccello-Straub et al. (2018) Sci Rep. 8(1):2292).

Nonclinical safety data from the 4-week toxicology studies in both rats and cynomolgus monkeys showed that apitegromab was well-tolerated when administered by IV bolus injection once weekly for 4 weeks at doses up to 100 mg/kg. Apitegromab was also well-tolerated in both a 26-week study in adult rats and an 8-week study in juvenile rats, each of which had a NOAEL of 300 mg/kg, the highest dose tested.

The studies with apitegromab did not identify alterations in the weight or histopathology of the testes in adult and juvenile rats or in adult monkeys, and there were no apitegromab-related effects on caudal epididymal weight, sperm motility, concentration, or morphology in the juvenile rat study. Fertility endpoints were also investigated in the juvenile rat study as a functional outcome, to determine any potential reproductive effects caused by promyostatin inhibition. Apitegromab had no effect on fertility parameters such as estrous cyclicity, reproductive performance, and fecundity in juvenile rats. These data suggest that apitegromab can circumvent the potential off-target reproductive organ effects observed with nonspecific myostatin inhibitors.

The impact of apitegromab treatment on the FOB, locomotor activity, and developmental neurohistopathology was evaluated in juvenile rats. There were no apitegromab-related adverse observations on any of these endpoints, confirming that in juvenile rodents, the specific inhibition of latent myostatin by apitegromab does not result in any undesirable impact on the CNS.

The impact of apitegromab on cardiovascular histopathology and/or physiology was evaluated in rats and monkeys. There were no apitegromab-related changes in the weight and histopathology of the heart. Moreover, apitegromab did not have any adverse impact on heart function, as determined by quantitative and qualitative assessment of electrocardiograms. These data suggest that specific targeting of promyostatin and latent myostatin is not associated with any unwanted cardiac toxicities in rats and monkeys.

The effects of apitegromab on bone were evaluated in adult and juvenile animals. Apitegromab did not alter bone mineral density, bone length, bone histopathology and showed no effect on radiographic observations. No apitegromab-related adverse effects on bone parameters were observed in any of the safety assessments.

The multi-dose TK profile of apitegromab in rats and monkeys indicated systemic exposure in all animals with approximate dose proportionality and accumulation of drug after multiple doses. Apitegromab exposures achieved in toxicology studies were sufficient to produce pharmacodynamic effects, as evidenced by the increase in skeletal muscle mass in treated animals. Furthermore, increases in circulatory levels of latent myostatin indicated the ability of apitegromab to engage and saturate the target.

Overall, the *in vivo* toxicology assessment of apitegromab demonstrated a favorable safety profile with no toxicity identified in chronic studies of up to 26 weeks in adult rats and 8 weeks in juvenile rats, at up to 300 mg/kg. The nonclinical toxicology data complement the preclinical pharmacology studies in mouse models of SMA.

### Example 3. Trial to Evaluate the Efficacy and Safety of Apitegromab in Patients with Later-Onset Spinal Muscular Atrophy Who Are Being Treated with Nusinersen or Risdiplam

The purpose of this Phase 3 trial is to assess the safety and efficacy of apitegromab compared with placebo for improving motor function in patients with later-onset spinal muscular atrophy (SMA) who are receiving background therapy with nusinersen or risdiplam. Survivor motor neuron (SMN) upregulator therapies have been shown to significantly improve clinical outcomes by preventing or reducing the decline in motor function, however, patients may continue to suffer from substantial motor functional impairment because targeted SMN upregulator therapies focus on SMN-dependent pathways and do not directly impact skeletal muscle to reverse the atrophy that has already taken place. Consequently, there remains an unmet medical need for a complementary therapeutic muscle-directed therapy that may address muscle atrophy and thereby improve motor function in patients with SMA. Through its novel mechanism of action as a selective inhibitor of myostatin activation, apitegromab has the potential to produce a clinically meaningful effect on motor function in a broad population of patients with SMA who are being treated with background SMN upregulator therapies.

This trial will be conducted in patients 2 through 21 years of age at screening, who have been diagnosed with later-onset SMA (i.e., Type 2 and Type 3 SMA) and are receiving an approved SMN upregulator therapy to confirm the efficacy and safety of apitegromab as an adjunctive therapy to nusinersen and evaluate the efficacy and safety of apitegromab as an adjunctive therapy to risdiplam. Since both nusinersen and the SMN upregulator risdiplam treat the SMN protein deficiency in SMA by increasing the production of functional SMN protein, previously reported positive results observed with nusinersen support investigating apitegromab as an adjunctive therapy.

### Patient population

Patients will meet prespecified inclusion criteria that include that they are age 2 through 21 at screening and have a life expectancy of at least two years from screening. They must have a documented diagnosis of 5q SMA and have been diagnosed with later onset, i.e., Type 2 or Type 3 SMA before receiving either nusinersin or risdiplam. Patients must be nonambulatory at screening and able to sit independently according to WHO motor milestones at screening. Patients must have completed either at least ten months of dosing with nusinersin or at least six months of dosing with risdiplam prior to screening. Patients must have an HFMSE score ≥10 and ≤45 at screening and have no physical limitations that would prevent them from undergoing motor function outcome measures. Patients must be able to receive study drug infusions and provide blood samples intravenously and female patients of reproductive age must agree to adhere to contraception.

Exclusion criteria include having received onasemnogene abeparvovec-xioi at any time; previous treatment with apitegromab; a prior history of severe hypersensitivity reaction or intolerance to SMN upregulator therapies; and a prior history of a hypersensitivity reaction to a monoclonal antibody or recombinant protein bearing an Fc domain, apitegromab, or excipients of apitegromab. Exclusion criteria also include a requirement for invasive ventilation or tracheostomy; nutritional status that was not stable over the past six months and is not anticipated to be stable throughout the trial; medical necessity for a gastric/nasogastric feeding tube, where the majority of feeds are given by this route; and major orthopedic or other interventional procedure, including spine or hip surgery having the potential to substantially limit the ability of the patient to be evaluated on any motor function outcome measures within six months before screening or anticipated during the trial. Exclusion criteria further include treatment with other investigational drugs in a clinical trial within three months or five half- lives, whichever is longer, prior to screening; use of valproic acid or hydroxyurea within 90 days before screening; use of therapies with potentially significant muscle effects or potentially significant neuromuscular effects other than approved SMN upregulator therapy within 60 days before screening; use of systemic corticosteroids within 60 days before screening (inhaled or topical steroids are permitted); any acute or comorbid condition interfering with the well-being of the patient within seven days before screening, including active systemic infection, the need for acute treatment, or inpatient observation due to any reason; severe contractures or scoliosis at screening; use of chronic daytime noninvasive ventilatory support for more than 16 hours daily in the two weeks before dosing, or are anticipated to regularly receive such daytime ventilator support chronically throughout the trial; pregnant or breastfeeding; and any other condition or clinically significant laboratory result or ECG value that, in the opinion of the Investigator, may compromise safety or compliance, would preclude the patient from successful completion of the trial, or interfere with the interpretation of the results.

### Trial Design

The trial will include Screening, Treatment, and Safety Follow-up Periods. Patients will be randomized to receive 10 mg/kg or 20 mg/kg apitegromab or placebo by intravenous (IV) infusion. Approximately 204 male and female patients with later-onset SMA will be randomized into either the Main Efficacy Population (2 through 12 years old at screening) or the Exploratory Subpopulation (13 through 21 years old at screening). Patients in the Main Efficacy Population will be randomized separately from the patients in the Exploratory Subpopulation.

For the Main Efficacy Population, approximately 156 patients who are 2 through 12 years old at screening will be randomized 1:1:1 double-blind to receive apitegromab 10 mg/kg, apitegromab 20 mg/kg, or placebo every four weeks during a 52-week Treatment Period. Randomization for the Main Efficacy Population will be stratified by type of background therapy (i.e., nusinersen or risdiplam) and age at initiation of SMN upregulator therapy (≥5 and <5).

For the Exploratory Subpopulation, a maximum of 48 patients who are 13 through 21 years old at screening will be randomized 2:1 double-blind to receive apitegromab 20 mg/kg or placebo every four weeks during the 52-week Treatment Period. Randomization for the Exploratory Subpopulation will be stratified by the type of background therapy (i.e., nusinersen or risdiplam).

During the Screening Period (day -28 through day -1) screening and eligibility determinations will be conducted after informed consent has been provided. Screening motor function outcome measures will be conducted a minimum of seven days before the first dose.

The Treatment Period commences on day 1. After dosing, patients will be monitored for hypersensitivity reactions and after each dose patients will be contacted by the site by telephone within seven days for a safety check-in. Patient site visits will occur every four weeks through the end of the Treatment Period. Activity assessments in the Treatment Period will include the Pediatric Evaluation of Disability Inventory-Computer Adapted Test (PEDI-CAT), the Patient Reported Outcomes Measurement Information System Fatigue Short Form (PROMIS), the Assessment of Caregiver Experience with Neuromuscular Disease (ACEND) Questionnaire and the Columbia-Suicide Severity Rating Scale (C-SSRS). The PEDI-CAT is a questionnaire completed by the caregiver that assess the patient's ability to perform daily functions and is suitable to assess function in newborn to 21 year old SMA patients. The PROMIS is a person-centered measure of fatigue symptoms suitable for patients 5 years or older. It is self reported by patients eight years or older and by their proxies if the patient is under eight years old. The PROMIS measures symptoms ranging from mild subjective feelings of tiredness to overwhelming, debilitating and sustained sense of exhaustion. The C-SSRS measures suicidal ideation and behavior and treatment emergent suicidal ideation and behavior.

Two dose levels (10 mg/kg and 20 mg/kg) will be evaluated for the Main Efficacy Population as compared to placebo. The Exploratory Subpoplation will be given a dose of 20 mg/kg or placebo every four weeks or monthly. All administrations of apitegromab will be via intravenous infusion.

All patients, including those randomized to the placebo group, will continue to receive an approved SMA treatment (i.e., nusinersen or risdiplam). Patients are expected to have remained on the same background SMA therapy from at least ten months before screening for nusinersen or at least six months before screening for risdiplam and throughout the duration of the trial. Patients who receive nusinersen must receive their maintenance doses at least 24 hours after receiving study drug, or at least 14 days prior to any scheduled study drug administration visit. Any change in the timing of the patient's nusinersen treatment that would fall within 14 days prior to the scheduled administration of study drug should be discussed with the Medical Monitor in advance.

Patients who complete the Treatment Period (12-month assessments at Visit 14) may be offered the option to enter an extension trial at that time. Patients who choose not to enroll in the extension trial will be followed for a 20-week Safety Follow-up Period after Visit 14. Activity assessments in the Safety Follow-up Period will also include PEDI-CAT, PROMIS, ACEND Questionnaire and C-SSRS.

Total trial participation for an individual patient will consist of approximately four weeks for screening, 52 weeks of trial visits and twenty weeks of safety follow up.

An interim analysis will be conducted when at least 50% of the patients in the Main Efficacy Population complete the Visit 14 assessments. The trial may claim early efficacy at the interim analysis if the boundary based on the prespecified alpha spending function is crossed. If early efficacy is claimed, the trial will be stopped, treatment assignment will be unblinded for both the Main Efficacy Population and the Exploratory Subpopulation, and all patients will be offered the option to enter an extension trial. If early efficacy is not claimed, the trial will continue as double-blind randomized until all patients in the Main Efficacy Population complete the 12-month assessments to further characterize the efficacy and safety profile.

The primary analysis of this trial will be performed using data from a "Main Efficacy Population," i.e., patients who are 2 through 12 years old at screening. Focusing on the 2- through 12-year age range as the Main Efficacy Population facilitates a reliably powered trial, while investigation of apitegromab's effect on a broader age range can be achieved through an Exploratory Subpopulation analysis in a 13- through 21-year age range.

### Efficacy and Safety Assessments

The motor function outcome measures to be tested are the HFSME, the WHO Motor Development Milestones and the RULM. All patients will perform the HFSME, which consists of 33 items graded on a scale of 0, 1 and 2, where 0 denotes unable, 1 denotes performed with modification or adaption and 2 denotes performance without modification or adaption. All patients will also perform the WHO Motor Development Milestones assessment, which are a set of six gross motor milestones that are universal and fundamental to acquiring the ability to walk independently. Patients who are at least 30 months of age at screening will perform the RULM, a 19 item assessment of upper limb function in nonambulatory patients which tests functions that relate to everyday life.

Safety assessments will include demographics and medical history, vital signs, weight and height, physical examination, electrocardiogram, and concomitant medications will be collected and documented. Clinical laboratory testing will include serum chemistry, hematology, urinalysis, and coagulation, PK and PD sample draw, pregnancy and ADA testing; they will be performed using established methods. Because risdiplam is a central nervous system interventional suicidal ideation and behavior risk monitoring will be performed. The C-SSRS will be used for patients six years of age and older and the Very Young Child/Cognitively Impaired Version of the C-SSRS will be used for patients who are four to five years old.

The efficacy of apitegromab will be assessed by the change in the HFMSE total score from baseline to 12 months compared with placebo. The primary outcome is the difference in HFMSE mean change from baseline to 12 months in the intended to treat (ITT) population in the Main Efficacy Population treated with apitegromab vs. placebo. The ITT population is defined as all randomized patients in the Main Efficiency Population, even if the patient does not receive the correct treatment or otherwise does not follow the protocol. The primary hypothesis of interest is whether apitegromab 20 mg/kg has superior efficacy compared with placebo in patients from 2 through 12 years old treated with background SMN upregulator therapies. If superiority is demonstrated with the 20 mg/kg group, the hypothesis of whether apitegromab 10 mg/kg has superior efficacy compared with placebo in patients from 2 through 12 years old treated with background SMA upregulator therapies will be tested to determine the biologically optimal dose.

A secondary endpoint, the proportion of patients with a greater than or equal to three point change from baseline in the HFMSE total score at 12 months, will be compared between the apitegromab 20 mg/kg (and/or 10 mg/kg) group vs. placebo using a logistic model, adjusting for baseline HFMSE total score and the stratification factors at randomization.

### Pharmacokinetics, Pharmacodynamics and Immunogenicity

Whole blood samples will be collected to evaluate the pharmacokinetics (PK) of apitegromab, including measuring the serum concentration, on Treatment Period days 1, 29, 113, 169, 225, 281, 337 and 365 and at the end of the Follow-up Period. Once emerging PK data have been collected and analyzed, the PK sample schedule may be adjusted to reflect a decrease in the number of samples to be collected.

Whole blood will be collected for pharmacodynamic studies (PD) and immunogenicity assessments at the same time that PK samples are collected. Patient confidentiality will be maintained and apitegromab concentration information that would unblind the treatment assignment will not be reported to investigative sites or to blinded personnel until the treatment assignment has been unblinded. The PD of apitegromab will be evaluated by measuring serum circulating latent myostatin concentration. Once emerging PD data have been collected and analyzed, the PD sample schedule may be adjusted to reflect a decrease in the number of samples to be collected.

Serum samples will be screened for antibodies that bind to apitegromab and the titer of confirmed positive samples will be reported. Other analyses may be performed to verify the stability of any detected antibodies to apitegromab and/or to further characterize the immunogenicity of apitegromab. The detection and characterization of antibodies to apitegromab will be performed using a validated assay method. All samples collected for detection of antibodies to apitegromab will also be evaluated for apitegromab serum concentration to enable interpretation of the antibody data. Antibodies may be further characterized and/or evaluated for their ability to neutralize the activity of apitegromab. Samples may be stored for a maximum of fifteen years or according to local regulations following the last patient's last visit to enable further analysis of immune responses to apitegromab.

### Objectives and Endpoints

### Main efficacy population

An objective is to assess the efficacy of apitegromab compared with placebo using the HFMSE in patients 2 through 12 years old. The endpoint is a change in baseline in HFMSE total score at twelve months

An objective is to assess the efficacy of apitegromab compared with placebo based on the number of patients with clinical improvement in patients 2 through 12 years old. The endpoint is the proportion of patients with ≥3-point change from baseline in the HFMSE total score at 12 months.

An objective is to assess the efficacy of apitegromab compared with placebo by measuring changes in upper limb function using the Revised Upper Limb Module (RULM) in patients 2 through 12 years old. The endpoint is the change from baseline in RULM total score at 12 months.

An objective is to assess the efficacy of apitegromab compared with placebo by measuring changes in number of World Health Organization (WHO) motor development milestones in patients 2 through 12 years old. The endpoint is the change from baseline in the number of WHO motor development milestones attained at 12 months.

An objective is to further assess the efficacy of apitegromab compared with placebo by evaluating changes in additional motor function outcome measures and changes in HFMSE at other prespecified time points in patients 2 through 12 years old. The endpoints are (i) the proportion of patients achieving various magnitudes of change in HFMSE score from baseline at 12 months; (ii) the proportion of patients achieving various magnitudes of change in RULM score from baseline at 12 months; (iii) the proportion of patients who attain a new WHO motor development milestone relative to baseline at 12 months; (iv) the change from baseline in HFMSE total score at other prespecified time points; (v) the change from baseline in RULM total score at other prespecified time points; and (vi) the change from baseline in the number of WHO motor development milestones attained at other prespecified time points.

An objective is to assess the efficacy of apitegromab compared with placebo by measuring changes from baseline in motor function across the treatment period using the HFMSE in patients 2 through 12 years old. The endpoint is change from baseline in HFMSE total score across time during the 12-month treatment period.

An objective is to assess the time to therapeutic effect of apitegromab compared with placebo using the HFMSE in patients 2 through 12 years old. The endpoint is time to therapeutic effect (≥3-point change from baseline in HFMSE total score) compared between apitegromab and placebo.

An objective is to evaluate the effects of apitegromab on patient/caregiver-reported disability, fatigability, and suicidal ideation and behavior in patients 2 through 12 years old. The endpoints are (i) change from baseline in PEDI-CAT; (ii) change from baseline in the PROMIS fatigue questionnaire; (iii) change from baseline in ACEND; and (iv) change from baseline in C-SSRS.

### Main efficacy population/exploratory subpopulation

An objective is to assess the safety and tolerability of apitegromab in all randomized patients with later-onset SMA who receive at least one dose of apitegromab. The endpoint is the incidence of treatment-emergent adverse events (TEAEs) and serious adverse events (SAEs) by severity.

An objective is to characterize the pharmacokinetics (PK) of apitegromab in all randomized patients with later-onset SMA who receive at least one dose of apitegromab. The endpoint is the apitegromab concentration in serum from blood samples.

An objective is to evaluate the pharmacodynamic (PD) effects of apitegromab in all randomized patients with later-onset SMA who receive at least one dose of apitegromab. The endpoint is the level of circulating latent myostatin concentrations in blood samples.

An objective is to evaluate the immunogenicity of apitegromab in all randomized patients with later-onset SMA who receive at least one dose of apitegromab. The endpoint is the presence or absence of antidrug antibody (ADA) against apitegromab in serum from blood samples.

An objective is to assess the efficacy of apitegromab compared with placebo using the HFMSE in patients 13 through 21 years old and in patients 2 through 21 years old. The endpoint is the change from baseline in HFMSE total score at 12 months.

An objective is to assess the efficacy of apitegromab compared with placebo based on the number of patients with clinical improvement in patients 2 through 21 years old. The endpoint is the proportion of patients with ≥3-point change from baseline in the HFMSE total score at 12 months.

An objective is to assess the efficacy of apitegromab compared with placebo by measuring changes in upper limb function between baseline and the end of the treatment period using the RULM in patients 13 through 21 years old and in patients 2 through 21 years old. The endpoint is the change in baseline in RULM total score at 12 months.

An objective is to assess the efficacy of apitegromab compared with placebo by measuring changes in the number of WHO motor development milestones in patients 13 through 21 years old and in patients 2 through 21 years old. The endpoint is the change from baseline in the number of WHO motor development milestones attained at 12 months.

An objective is to assess the efficacy of apitegromab compared with placebo by evaluating changes in additional motor function outcome measures and changes in HFMSE at other prespecified time points in patients 13 through 21 years old and in patients 2 through 21 years old. The endpoints are (i) the proportion of patients achieving various magnitudes of change in HFMSE score from baseline at 12 months; (ii) the proportion of patients achieving various magnitudes of change in RULM score from baseline at 12 months; (iii) the proportion of patients who attain a new WHO motor development milestone relative to baseline at 12 months; (iv) the change from baseline in HFMSE total score at other prespecified time points; (v) the change from baseline in RULM total score at other prespecified time points; and (vi) the change from baseline in the number of WHO motor development milestones attained at other prespecified time points.

An objective is to assess the efficacy of apitegromab compared with placebo by measuring changes from baseline in motor function across the treatment period using the HFMSE in patients 13 through 21 years old and in patients 2 through 21 years old. The endpoint is the change from baseline in HFMSE total score across time during the 12-month treatment period.

An objective is to assess the time to stabilization of effect of apitegromab compared with placebo using the HFMSE in patients 13 through 21 years old. The endpoint is the time to decline (at least a -3-point change from baseline in HFMSE total score) compared between apitegromab and placebo.

An objective is to assess the time to therapeutic effect of apitegromab compared with placebo using the HFMSE in patients 2 through 21 years old. The endpoint is the time to therapeutic effect (≥1-point change from baseline in HFMSE total score) compared between apitegromab and placebo.

An objective is to evaluate the effects of apitegromab on fatigability, caregiver-reported disability, and suicidal ideation and behavior in patients 13 through 21 years old and in patients 2 through 21 years old. The endpoints are (i) the change from baseline in PEDI-CAT; (ii) the change from baseline in PROMIS fatigue questionnaire; (iii) the change from baseline in ACEND; and (iv) the change from baseline in C-SSRS.

## Claims

1. A composition comprising apitegromab for use in the treatment of spinal muscular atrophy (SMA) in a human subject, wherein the treatment comprises intravenous administration of apitegromab in an amount of 10 mg/kg at an interval of once every four weeks or once a month for at least 24 weeks or six months, wherein the treatment increases motor function as measured by a Hammersmith Functional Motor Scale Expanded score (HFMSE) at 6 months after the start of treatment relative to HFMSE at baseline.

2. The composition for use according to claim 1, wherein the treatment achieves an increase in HFMSE score of at least one point, at least two points or at least three points relative to baseline.

3. The composition for use according to claim 1 or claim 2, wherein the apitegromab is administered for at least 52 weeks or twelve months.

4. The composition for use according to any of the preceding claims, wherein the human subject is:
a) 2 years of age or older;
b) 2-21 years of age;
c) 2-10 years of age; and/or
d) 2-5 years of age.

5. The composition for use according to any of the preceding claims, wherein the subject has later onset SMA, optionally wherein the later-onset SMA is:
a) non-ambulatory SMA; or
b) ambulatory SMA.

6. The composition for use according to any of the preceding claims, wherein the subject is or has been identified as a carrier of an SMN mutation.

7. The composition for use according to any of the preceding claims, wherein the subject has at least two copies of the SMN2 gene.

8. The composition for use according to any of the preceding claims wherein the human subject is also administered an SMN upregulation therapy, wherein the SMN upregulation therapy is a splice modifier, SMN gene replacement or gene therapy, an SMN transcription enhancer, an SMN protein translation enhancer or an SMN protein stabilizer.

9. The composition for use according to claim 8, wherein the SMN upregulation therapy is selected from nusinersen and/or risdiplam.

10. The composition for use according to any of the preceding claims, wherein the baseline serum concentration of latent myostatin in the human subject selected for treatment is at least 1 ng/mL, and optionally wherein the human subject has later-onset SMA, preferably type 2 SMA.

11. The composition for use according to claim 10, wherein the baseline serum concentration of latent myostatin in the human subject is greater than 1.5 ng/ml, greater than 2 ng/ml, greater than 2.5 ng/ml, or greater than 3 ng/ml.

12. The composition for use according to any of the preceding claims, wherein the treatment:
a) achieves a serum concentration of latent myostatin in the human subject of at least 250 ng/ml at steady state after the administration of apitegromab; and/or
b) achieves a serum concentration of apitegromab in the human subject of at least 50 ug/ml at steady state after the administration of apitegromab.

13. The composition for use according to claim 12, wherein the treatment achieves a serum concentration of latent myostatin in the human subject of 550-2400 ng/ml at steady state after the administration of apitegromab.

14. The composition for use according to claim 12 or claim 13, wherein the steady state is at 14 days or later after administration of apitegromab.

## Patentansprüche

1. Zusammensetzung, umfassend Apitegromab, zur Verwendung bei der Behandlung von Spinaler Muskelatrophie (SMA) bei einem Menschen, wobei die Behandlung intravenöse Verabreichung von Apitegromab in einer Menge von 10 mg/kg in einem Intervall von einmal alle vier Wochen oder einmal pro Monat mindestens 24 Wochen oder sechs Monate lang umfasst, wobei die Behandlung motorische Funktion gemessen mittels eines HFMSE(Hammersmith Functional Motor Scale Expanded)-Scores 6 Monate nach dem Beginn der Behandlung bezogen auf den HFMSE-Ausgangswert erhöht.

2. Zusammensetzung zur Verwendung nach Anspruch 1, wobei die Behandlung eine Erhöhung des HFMSE-Scores von mindestens einem Punkt, mindestens zwei Punkten oder mindestens drei Punkten bezogen auf den Ausgangswert erreicht.

3. Zusammensetzung zur Verwendung nach Anspruch 1 oder Anspruch 2, wobei der Apitegromab mindestens 52 Wochen oder zwölf Monate lang verabreicht wird.

4. Zusammensetzung zur Verwendung nach einem der vorhergehenden Ansprüche, wobei der Mensch Folgendes ist:
a) 2 Jahre alt oder älter;
b) 2-21 Jahre alt;
c) 2-10 Jahre alt; und/oder
d) 2-5 Jahre alt.

5. Zusammensetzung zur Verwendung nach einem der vorhergehenden Ansprüche, wobei das Individuum später einsetzende SMA aufweist, gegebenenfalls wobei es sich bei der später einsetzenden SMA um Folgendes handelt:
a) SMA ohne Gehfähigkeit; oder
b) SMA mit Gehfähigkeit.

6. Zusammensetzung zur Verwendung nach einem der vorhergehenden Ansprüche, wobei das Individuum ein Träger einer SMN-Mutation ist oder als solcher identifiziert wurde.

7. Zusammensetzung zur Verwendung nach einem der vorhergehenden Ansprüche, wobei das Individuum mindestens zwei Kopien des SMN2-Gens aufweist.

8. Zusammensetzung zur Verwendung nach einem der vorhergehenden Ansprüche, wobei dem Menschen auch eine SMN-Hochregulierungstherapie verabreicht wird, wobei es sich bei der SMN-Hochregulierungstherapie um einen Spleißmodifikator, SMN-Genersatz- oder -Gentherapie, einen SMN-Transkriptionsenhancer, einen SMN-Protein-Translationsenhancer oder einen SMN-Protein-Stabilisator handelt.

9. Zusammensetzung zur Verwendung nach Anspruch 8, wobei die SMN-Hochregulierungstherapie aus Nusinersen und/oder Risdiplam ausgewählt ist.

10. Zusammensetzung zur Verwendung nach einem der vorhergehenden Ansprüche, wobei die Ausgangsserumkonzentration von Latent-Myostatin in dem zur Behandlung ausgewählten Menschen mindestens 1 ng/ml beträgt und gegebenenfalls wobei der Mensch später einsetzende SMA, vorzugsweise SMA des Typs 2 aufweist.

11. Zusammensetzung zur Verwendung nach Anspruch 10, wobei die Ausgangsserumkonzentration von Latent-Myostatin in dem Menschen mehr als 1,5 ng/ml, mehr als 2 ng/ml, mehr als 2,5 ng/ml oder mehr als 3 ng/ml beträgt.

12. Zusammensetzung zur Verwendung nach einem der vorhergehenden Ansprüche, wobei die Behandlung:
a) eine Steady-State-Serumkonzentration von Latent-Myostatin von mindestens 250 ng/ml in dem Menschen nach Verabreichung von Apitegromab erreicht; und/oder
b) eine Steady-State-Serumkonzentration von Apitegromab von mindestens 50 ug/ml in dem Menschen nach Verabreichung von Apitegromab erreicht.

13. Zusammensetzung zur Verwendung nach Anspruch 12, wobei die Behandlung eine Steady-State-Serumkonzentration von Latent-Myostatin von 550-2400 ng/ml in dem Menschen nach der Verabreichung von Apitegromab erreicht.

14. Zusammensetzung zur Verwendung nach Anspruch 12 oder Anspruch 13, wobei der Steady State 14 Tage nach Verabreichung von Apitegromab oder später vorliegt.

## Revendications

1. Composition comprenant de l'apitégromab pour utilisation dans le traitement de l'atrophie musculaire spinale (SMA) chez un sujet humain, dans laquelle le traitement comprend l'administration intraveineuse d'apitégromab en une quantité de 10 mg/kg à un intervalle d'une fois toutes les quatre semaines ou une fois par mois pendant au moins 24 semaines ou six mois, dans laquelle le traitement augmente la fonction motrice telle que mesurée par un score étendu sur l'échelle motrice fonctionnelle de Hammersmith (HFMSE) 6 mois après le début du traitement par rapport au HFMSE à la ligne de base.

2. Composition pour utilisation selon la revendication 1, dans laquelle le traitement atteint une augmentation du score HFMSE d'au moins un point, d'au moins deux points ou d'au moins trois points par rapport à la ligne de base.

3. Composition pour utilisation selon la revendication 1 ou la revendication 2, dans laquelle l'apitégromab est administré pendant au moins 52 semaines ou douze mois.

4. Composition pour utilisation selon l'une quelconque des revendications précédentes, dans laquelle le sujet humain est âgé de :
a) 2 ans ou plus ;
b) 2-21 ans;
c) 2-10 ans ; et/ou
d) 2-5 ans.

5. Composition pour utilisation selon l'une quelconque des revendications précédentes, dans laquelle le sujet a une SMA d'apparition ultérieure, éventuellement dans laquelle la SMA d'apparition ultérieure est :
a) une SMA non ambulatoire ; ou
b) une SMA ambulatoire.

6. Composition pour utilisation selon l'une quelconque des revendications précédentes, dans laquelle le sujet est ou a été identifié comme étant un porteur d'une mutation SMN.

7. Composition pour utilisation selon l'une quelconque des revendications précédentes, dans laquelle le sujet a au moins deux copies du gène SMN2.

8. Composition pour utilisation selon l'une quelconque des revendications précédentes, dans laquelle le sujet humain reçoit également une thérapie de régulation à la hausse de SMN, dans laquelle la thérapie de régulation à la hausse de SMN est un modificateur d'épissage, un remplacement de gène SMN ou une thérapie génique, un amplificateur de transcription de SMN, un amplificateur de traduction de protéine SMN ou un stabilisant de protéine SMN.

9. Composition pour utilisation selon la revendication 8, dans laquelle la thérapie de régulation à la hausse de SMN est choisie parmi nusinersen et/ou risdiplam.

10. Composition pour utilisation selon l'une quelconque des revendications précédentes, dans laquelle la concentration sérique de ligne de base de myostatine latente chez le sujet humain choisi pour le traitement est d'au moins 1 ng/mL, et éventuellement dans laquelle le sujet humain a une SMA d'apparition ultérieure, de préférence une SMA de type 2.

11. Composition pour utilisation selon la revendication 10, dans laquelle la concentration sérique de ligne de base de myostatine latente chez le sujet humain est supérieure à 1,5 ng/mL, supérieure à 2 ng/mL, supérieure à 2,5 ng/mL ou supérieure à 3 ng/mL.

12. Composition pour utilisation selon l'une quelconque des revendications =- précédentes, dans laquelle le traitement :
a) atteint une concentration sérique de myostatine latente chez le sujet humain d'au moins 250 ng/mL à l'état d'équilibre après l'administration d'apitégromab ; et/ou
b) atteint une concentration sérique d'apitégromab chez le sujet humain d'au moins 50 ug/mL à l'état d'équilibre après l'administration d'apitégromab.

13. Composition pour utilisation selon la revendication 12, dans laquelle le traitement atteint une concentration sérique de myostatine latente chez le sujet humain de 550-2400 ng/mL à l'état d'équilibre après l'administration d'apitégromab.

14. Composition pour utilisation selon la revendication 12 ou la revendication 13, dans laquelle l'état d'équilibre est 14 jours ou plus tard après l'administration d'apitégromab.
